# EUROPEAN PATENT SPECIFICATION

(11) **EP 2 326 731 B1**
(45) Date of publication and mention of the grant of the patent: **13.11.2013**
(21) Application number: 09820957.0
(22) Date of filing: 19.08.2009
(51) Int. Cl.: C12Q 1/68

(54) **BIOMARKERS FOR ANTI-TNF TREATMENT IN ULCERATIVE COLITIS AND RELATED DISORDERS**
BIOMARKER ZUR ANTI-TNF-BEHANDLUNG BEI COLITIS ULCEROSA UND DAMIT ZUSAMMENHÄNGENDEN ERKRANKUNGEN
BIOMARQUEURS POUR LE TRAITEMENT ANTI-TNF DANS LA RECTOCOLITE HÉMORRAGIQUE ET DE TROUBLES ASSOCIÉS

(30) Priority: 25.08.2008 US 91641 P
(43) Date of publication of application: 01.06.2011
(73) Proprietor: Janssen Biotech, Inc., Horsham, PA 19044 (US)
(72) Inventor: LI, Katherine, Radnor, PA 19087 (US); BARIBAUD, Frederic, Radnor, PA 19087 (US)
(74) Representative: Goodfellow, Hugh Robin
(86) International application number: PCT/US2009/054287
(87) International publication number: WO 2010/044952

(56) References cited:
- WO-A2-2008/028044
- WO-A2-2008/067195
- US-A1- 2001 036 650
- US-A1- 2006 275 834
- US-A1- 2007 083 334
- US-B1- 6 500 418

### SEQUENCE LISTING

<110> CENTOCOR ORTHO BIOTECH INC.
   LI, Katherine
   BARIBAUD, Frederic
<120> BIOMARKERS FOR ANTI-TNF TREATMENT IN ULCERATIVE COLITIS AND RELATED DISORDERS
<130> CEN5228WOPCT
<140> Unknown
   <141> Herewith
<150> 61/091,641
   <151> 2009-08-25
<160> 203
<170> FastSEQ for Windows Version 4.0
<210> 1
   <211> 560
   <212> DNA
   <213> Homo sapiens
<400> 1
   aaagctgcaa ggtcaagatg gttcattatg gctgtgttca ccatagcaaa ctggaaagaa 60
   tctagatatc caacagtgag ggttaagcaa catggtgcat ctgtggatag aacaccaccc 120
   agccgcccgg agcagggact gtcattcagg gaggctaagg agagaggctt gcttgggata 180
   tagaaagata tcctgacatt ggccaggcat ggtggctcac gcctgtaatc ctggcacttt 240
   gggaggacga agcgagtgga tcactgaagt ccaagagttt gagaccggcc tgcgagacat 300
   ggcaaaaccc tgtctcaaaa aagaaagaat gatgtcctga catgaaacag caggctacaa 360
   aaccactgca tgctgtgatc ccaattttgt gtttttcttt ctatatatgg attaaaacaa 420
   aaatcctaaa gggaaatacg ccaaaatgtt gacaatgact gtctccaggt caaaggagag 480
   aggtgggatt gtgggtgact tttaatgtgt atgattgtct gtattttaca gaatttctgc 540
   catgactgtg tattttgcat                                             560
<210> 2
   <211> 404
   <212> DNA
   <213> Homo sapiens
<400> 2
   cacatcttgt tgactggagg catctagtgg aaaaatgcag tatttcagcc tgattgtgtt 60
   tgaagtaaat gattaaaaga ggaggaagtt accacattct ggaagattta cttgagacag 120
   acgaaccttg aattacggga aaaggccccg tgatttagga aataacaaat ttgggaaaca 180
   tgtaatgggg agagactggg gaatacccca gttgtgaaag tacttcctgt aaggcaacat 240
   ctgacaccag gaacctttct cttcagtatt ttaaaaacaa cttaatttca gtcctttact 300
   tgtggaatca gagccttact tatgtaatac aacccactgg aaaaaagctt tttattgtat 360
   tgtactatat tgtttataag tgattgagta cctgcagagc tttc                   404
<210> 3
   <211> 392
   <212> DNA
   <213> Homo sapiens
<220>
   <221> misc_feature
   <222> 342
   <223> n = A,T,C or G
<400> 3
   gaaatattca gttcagtttg tacctgttag cagtctttca gtttggggga gaattaaata 60
   ctgtgctaag ctggtgcttg gatacatatt acagcatctt gtgttttatt tgacaaacag 120
   aattttggtg ccataatatt ttgagaatta gagaagattg tgatgcatat atataaacac 180
   tatttttaaa aaatatctaa atatgtctca catatttata taatcctcaa atatactgta 240
   ccattttaga tattttttaa acagattaat ttggagaagt tttattcatt acctaattct 300
   gtggcaaaaa tggtgcctct gatgttgtga tatagtattg tnagtgtgta catatataaa 360
   acctgtgtaa acctctgtcc ttatgaacca ta                               392
<210> 4
   <211> 462
   <212> DNA
   <213> Homo sapiens
<400> 4
   ccgtgcttgt ccctgtggaa gtatcttggt tgtgacagag tcaagggtgt gtgcagcatt 60
   gttggctgtt cctgcagtag aatgggggca gcacctccta agaaggcacc tctctgggtt 120
   gaagggcagt gttccctggg gctttaactc ctgctagaac agtctcttga ggcacagaaa 180
   ctcctgttca tgcccatacc cctggccaag gaagatccct ttgtccacaa gtaaaaggaa 240
   atcctcctcc agggagtctc agcttcaccc tgaggtgagc atcatcttct gggttaggcc 300
   ttgcctaggc atagcctgcc tcaagctatg tgagctcacc agtccctccc caaatgcttt 360
   ccatgagttg cagttttttc ctagtctgtt ttccctcctt ggagaacagg gccctgtcgg 420
   tttgttcact gtatgtcctt ggtgcctgga gcctactaaa tg                    462
<210> 5
   <211> 553
   <212> DNA
   <213> Homo sapiens
<400> 5
   gatctacttg tgacttgttg gccttcttcc cacatctgcc tcagactggg gggggctcag 60
   ctcctcgggt gatatctagc ctgcttgtga gctctagcag ggataaggag agctgagatt 120
   ggagggaatt gtgttgctcc tggaggaagc ccaggcatca ttaaacaagc cagtaggtca 180
   cctggcttcc gtggaccaat tcatctttca gacaagcttt agagaaatgg actcagggaa 240
   gagactcaca tgctttggtt agtatctgtg tttccggtgg gtgtaatagg ggattagccc 300
   cagaagggac tgagctaaac agtgttatta tgggaaagga aatggcattg ctgctttcaa 360
   ccagcgacta atgcaatcca ttcctctctt gtttatagta atctaagggt tgagcagtta 420
   aaacggcttc aggatagaaa gctgtttccc acctgtttcg ttttaccatt aaaagggaaa 480
   cgtgcctctg ccccacgggt agagggggtg cacgttcctc ctggttcctt cgcttgtgtt 540
   tctgtactta cca                                                    553
<210> 6
   <211> 542
   <212> DNA
   <213> Homo sapiens
<220>
   <221> misc_feature
   <222> 416, 417, 418
   <223> n = A,T,C or G
<400> 6
   acctgtcact caagaggact tcagctctgc tgttgggctg gtgtgtggac agaaggaatg 60
   gaaagccaaa ttaatttagt ccagatttct aggtttgggt ttttctaaaa ataaaagatt 120
   acatttactt cttttacttt ttataaagtt ttttttcctt agtctcctac ttagagatat 180
   tctagaaaat gtcacttgaa gaggaagtat ttattttaat ctggcacaac actaattacc 240
   atttttaaag cggtattaag ttgtaattta aaccttgttt gtaactgaaa ggtcgattgt 300
   aatggattgc cgtttgtacc tgtatcagta ttgctgtgta aaaattctgt atcagaataa 360
   taacagtact gtatatcatt tgatttattt taatattata tccttatttt tgtcannntt 420
   gttgtcagtt tttattataa gcaaatttct aaataggcta cgtcccaaca tggataaaga 480
   ccccaaggtt ggtaaaatag ccccaaagat atataataat cattttacat acagcagagt 540
   tg                                                                542
<210> 7
   <211> 216
   <212> DNA
   <213> Homo sapiens
<400> 7
   gtgtgaaggt gcagttttgc caaggagtgc taaagaactt agatgtcagt gcataaagac 60
   atactccaaa cctttccacc ccaaatttat caaagaactg agagtgattg agagtggacc 120
   acactgcgcc aacacagaaa ttattgtaaa gctttctgat ggaagagagc tctgtctgga 180
   ccccaaggaa aactgggtgc agagggttgt ggagaa                           216
<210> 8
   <211> 280
   <212> DNA
   <213> Homo sapiens
<400> 8
   ccatccaggt ttaaccctgc aagctgtttc cacatcagaa ctcccttcaa acacaaagat 60
   tgctgtgaaa acgaaaatgt gtagacgcac tctcaaaaac tgcacggaca tacaaaatca 120
   atcttttcct ttgatcttgt ggctaaacca gcatttctgt gtttgagaga tttcctgtta 180
   ggtgcttcgt ctgaaagtga actctcataa ttcaaattgt ataaaataaa gctacatttc 240
   taagagcttg gtgtagggca attggaataa tgtcctgtta                       280
<210> 9
   <211> 424
   <212> DNA
   <213> Homo sapiens
<400> 9
   cctggtactc agattttgct atgaggttaa tgaagtacca agctgtgctt gaataacgat 60
   atgttttctc agattttctg ttgtacagtt taatttagca gtccatatca cattgcaaaa 120
   gtagcaatga cctcataaaa tacctcttca aaatgcttaa attcatttca cacattaatt 180
   ttatctcagt cttgaagcca attcagtagg tgcattggaa tcaagcctgg ctacctgcat 240
   gctgttcctt ttcttttctt cttttagcca ttttgctaag agacacagtc ttctcaaaca 300
   cttcgtttct cctattttgt tttactagtt ttaagatcag agttcacttt ctttggactc 360
   tgcctatatt ttcttacctg aacttttgca agttttcagg taaacctcag ctcaggactg 420
   ctat                                                               424
<210> 10
   <211> 467
   <212> DNA
   <213> Homo sapiens
<400> 10
   ccatgatcat ggtttactgc gcgtccgaat ggtcaacctt caagtggaag attctggact 60
   gtatcagtgt gtgatctacc agcctcccaa ggagcctcac atgctgttcg atcgcatccg 120
   cttggtggtg accaagggtt tttcagggac ccctggctcc aatgagaatt ctacccagaa 180
   tgtgtataag attcctccta ccaccactaa ggccttgtgc ccactctata ccagccccag 240
   aactgtgacc caagctccac ccaagtcaac tgccgatgtc tccactcctg actctgaaat 300
   caaccttaca aatgtgacag atatcatcag ggttccggtg ttcaacattg tcattctcct 360
   ggctggtgga ttcctgagta agagcctggt cttctctgtc ctgtttgctg tcacgctgag 420
   gtcatttgta ccctaggccc acgaacccac gagaatgtcc tctgact                 467
<210> 11
   <211> 307
   <212> DNA
   <213> Homo sapiens
<220>
   <221> misc_feature
   <222> 60
   <223> n = A,T,C or G
<400> 11
   agttagaggc tgtacaaggc ccccactgcc tgtcggttgc ttggattccc tgacgtaagn 60
   tggatattaa aaatctgtaa atcaggacag gtggtgcaaa tggcgctggg aggtgtacac 120
   ggaggtctct gtaaaagcag acccacctcc cagcgccggg aagcccgtct tgggtcctcg 180
   ctgctggctg ctccccctgg tggtggatcc tggaattttc tcacgcagga gccattgctc 240
   tcctagaggg ggtctcagaa actgcgaggc cagttccttg gagggacatg actaatttat 300
   cgatttt                                                            307
<210> 12
   <211> 484
   <212> DNA
   <213> Homo sapiens
<400> 12
   ggttgaatgt ttgtccttag gataggccta tgtgctagcc cacaaagaat attgtctcat 60
   tagcctgaat gtgccataag actgaccttt taaaatgttt tgagggatct gtggatgctt 120
   cgttaatttg ttcagccaca atttattgag aaaatattct gtgtcaagca ctgtgggttt 180
   taatattttt aaatcaaacg ctgattacag ataatagtat ttatataaat aattgaaaaa 240
   aattttcttt tgggaagagg gagaaaatga aataaatatc attaaagata actcaggaga 300
   atcttcttta caattttacg tttagaatgt ttaaggttaa gaaagaaata gtcaatatgc 360
   ttgtataaaa cactgttcac tgtttttttt aaaaaaaaaa cttgatttgt tattaacatt 420
   gatctgctga caaaacctgg gaatttgggt tgtgtatgcg aatgtttcag tgcctcagac 480
   aaat 484
<210> 13
   <211> 503
   <212> DNA
   <213> Homo sapiens
<220>
   <221> misc_feature
   <222> 419
   <223> n = A,T,C or G
<400> 13
   gtgctcggcc tgatggagac tgtgtgcagc cccttcacgg ccgccagacg tctgcgggac 60
   caggaggcag ccgtggcgga gctgcaggcc gccctggagc gacaggctct ccagaagcaa 120
   gccctgcagg agaaaggcaa gcagcaggac acggtcctcg gcggccgggc cctgtccaac 180
   cggcagcacg cctcctagga actgtgggag accagcggag tgggagggag acgcagtaga 240
   cagagacaga ccgagaagga agggagagac agagggggcg cgcgcacagg agcctgactc 300
   cgctgggaga gtgcaggagc acgtgctgtt ttttatttgg acttaacttc agagaaaccg 360
   ctgacatcta gaactgacct accacaagca tccaccaaag gagtttggga ttgagtttnt 420
   gctgctgtgc agcactgcat tgtcatgaca tttccaacac tgtgtgaatt atctaaatgc 480
   gtctaccatt ttgcactagg gag 503
<210> 14
   <211> 542
   <212> DNA
   <213> Homo sapiens
<400> 14
   tttcctcttt ttgactacag taactattcc aaatggggac acatactgta ctttcaactt 60
   tgcatcctgg ggtggcaccc ctgaggagag gctgaaggtg gccattacca tgctgacagc 120
   cagagggatt atccggtttg tcattggctt tagcttgccg atgtccattg ttgccatctg 180
   ctatgggctc attgcagcca agatccacaa aaagggcatg attaaatcca gccgtccctt 240
   acgggtcctc actgctgtgg tggcttcttt cttcatctgt tggtttccct ttcaactggt 300
   tgcccttctg ggcaccgtct ggctcaaaga gatgttgttc tatggcaagt acaaaatcat 360
   tgacatcctg gttaacccaa cgagctccct ggccttcttc aacagctgcc tcaaccccat 420
   gctttacgtc tttgtgggcc aagacttccg agagagactg atccactccc tgcccaccag 480
   tctggagagg gccctgtctg aggactcagc cccaactaat gacacggctg ccaattctgc 540
   tt 542
<210> 15
   <211> 294
   <212> DNA
   <213> Homo sapiens
<400> 15
   ccccttgccg gtgaacggat gcggcaggtg ctctgtggat gagaggaacc atcgcaggat 60
   gacagctccc gggtccccaa agctgttccc ctcttgctac tagccactga gaagtgcact 120
   ttaagaggtg ggagctgggc agacccctct acctcctcca ggctgggaga cagagtcagg 180
   ctgttgcggc tcccaactca gccccaagtt ccccaggccc agtgagggtg gccgggcatg 240
   ggccacgcgg gaaccgactt tccattgatt cagggcgtct gatgacacag gctg       294
<210> 16
   <211> 367
   <212> DNA
   <213> Homo sapiens
<400> 16
   accctcattg tctgggtcta ttcccacact tactgagtac agatgaagga aagtggtagc 60
   aatttaatca taactttcat ttgctgaaaa acattatgag aaggcctccc ttcctaagcc 120
   acctctggtc ttgctaagtc ttgatcttgc ttcctgccag caccaaacat tacattcagg 180
   ggatttcctc tggctcagtc ttttcccctt gaagttctct aatagatgtt acttttgaca 240
   aaagatcgcc tatgagttac aagcaccagg ggatgctcta catcaaggga tgcaccttca 300
   gtcaaactgt caaaaagccc agaattccca aaggcattag gtttcccaac tgctttgtgc 360
   tgatatc                                                           367
<210> 17
   <211> 229
   <212> DNA
   <213> Homo sapiens
<400> 17
   cccacaaccc gccaggattt aatcttttat ggattttttt ttcagtttat agtttccagg 60
   aggaaagtct tgtaacgctt tactttttct cttctttttt taaagtaaag tgagtgtcag 120
   tagatgtgtt ttttacagtc tggattttaa aaaaagtggg gcaggagaga gggaaccagg 180
   gaaagggctg tttagaaata cagtttgtgg ctctgagcga tgccaccgg             229
<210> 18
   <211> 490
   <212> DNA
   <213> Homo sapiens
<400> 18
   ggatacccca ttgtgaagcc agggcccaac tgtggatttg gaaaaactgg cattattgat 60
   tatggaatcc gtctcaatag gagtgaaaga tgggatgcct attgctacaa cccacacgca 120
   aaggagtgtg gtggcgtctt tacagatcca aagcaaattt ttaaatctcc aggcttccca 180
   aatgagtacg aagataacca aatctgctac tggcacatta gactcaagta tggtcagcgt 240
   attcacctga gttttttaga ttttgacctt gaagatgacc caggttgctt ggctgattat 300
   gttgaaatat atgacagtta cgatgatgtc catggctttg tgggaagata ctgtggagat 360
   gagcttccag atgacatcat cagtacagga aatgtcatga ccttgaagtt tctaagtgat 420
   gcttcagtga cagctggagg tttccaaatc aaatatgttg caatggatcc tgtatccaaa 480
   tccagtcaag                                                        490
<210> 19
   <211> 294
   <212> DNA
   <213> Homo sapiens
<220>
   <221> misc_feature
   <222> 45, 53, 62, 77, 84
   <223> n = A,T,C or G
<400> 19
   gaatatgcag caatggaaag tatcagtatc catacaaaga ttttncaaga aancgaatgg 60
   gntgagccaa gtagctngag attnagaatg agttcataga ctgtccaaga tctttatccc 120
   aaaaacattt ggattgttta ttttcagtat ttatcctctt tccctactac ctggatactc 180
   caaatttgca tatacttgta atattaatat acagttcctt tgcaatattt attttgttgc 240
   ttcttagaga aacccaacta aatgagttta aaaaatctgt gaaagggctg ggca       294
<210> 20
   <211> 453
   <212> DNA
   <213> Homo sapiens
<400> 20
   acctaacgaa gtatccttca gcctgaaaga ggaatgaagt actcatacat gttacaacac 60
   ggacgaacct tgaaaacttt atgctaagtg aaataagcca gacatcaaca gataaatagt 120
   ttatgattcc acctacatga ggtactgaga gtgaacaaat ttacagagac agaaagcaga 180
   acagtgatta ccagggactg aggggagggg agcatgggaa gtgacggttt aatgggcaca 240
   gggtttatgt ttaggatgtt gaaaaagttc tgcagataaa cagtagtgat agttgtaccg 300
   caatgtgact taatgccact aaattgacac ttaaaaatgg tttaaatggt caattttgtt 360
   atgtatattt tatatcaatt taaaaaaaaa cctgagcccc aaaaggtatt ttaatcacca 420
   aggctgatta aaccaaggct agaaccacct gcc                              453
<210> 21
   <211> 541
   <212> DNA
   <213> Homo sapiens
<400> 21
   ctatgtgctc cagggggacc caagagcagt ttccacccag ccccaatccc agtctggcac 60
   cagcgatcag gtcctttatg ggcagctgct gggcagcccc acaagcccag ggccagggca 120
   ctatctccgc tgtgactcca ctcagcccct cttggcgggc ctcaccccca gccccaagtc 180
   ctatgagaac ctctggttcc aggccagccc cttggggacc ctggtaaccc cagccccaag 240
   ccaggaggac gactgtgtct ttgggccact gctcaacttc cccctcctgc aggggatccg 300
   ggtccatggg atggaggcgc tggggagctt ctagggcttc ctggggttcc cttcttgggc 360
   ctgcctctta aaggcctgag ctagctggag aagaggggag ggtccataag cccatgacta 420
   aaaactaccc cagcccaggc tctcaccatc tccagtcacc agcatctccc tctcctccca 480
   atctccatag gctgggcctc ccaggcgatc tgcatacttt aaggaccaga tcatgctcca 540
   t                                                                 541
<210> 22
   <211> 265
   <212> DNA
   <213> Homo sapiens
<400> 22
   catcgcccac caaggcctgg gtgggtgaga acagtgccca caaggagacc ctgagtaaca 60
   gagactcaca gcccatccag gtctctgggc aggaaattga aggaatcatc acattttaca 120
   gaggaggaga ctgcagctca gagtggggga agtgtgtgca ccaggccaca ggcaagtctg 180
   tccagagcac tggtaggaat gagggaaact aggaatgacc actttaaaaa gttagatgag 240
   aagaatttca aggccgggcg cggtg                                       265
<210> 23
   <211> 395
   <212> DNA
   <213> Homo sapiens
<400> 23
   gtcagtcaca gggagggatg cacactttcc ttttgaccaa ggagggggca gctgattccc 60
   cgctgcgtct aaaatcaaag cgccaatctc ataagtacca ggctgaattc cccatgagtc 120
   ctgtgacctc ggcccacgcg gggacctaca ggtgctacgg ctcactcagc tccaacccct 180
   acctgctgac tcaccccagt gaccccctgg agctcgtggt ctcaggagca gctgagaccc 240
   tcagcccacc acaaaacaag tccgactcca aggctggtga gtgaggagat gcttgccgtg 300
   atgacgctgg gcacagaggg tcaggtcctg tcaagaggag ctgggtgtcc tgggtggaca 360
   tttgaagaat tatattcatt ccaacttgaa gaatt                            395
<210> 24
   <211> 536
   <212> DNA
   <213> Homo sapiens
<220>
   <221> misc_feature
   <222> 55, 56, 57, 80, 109
   <223> n = A,T,C or G
<400> 24
   ccatcttgag gcttatcatc accatggaga tatccttctt cagcttcttc atctnnnctg 60
   tacagctttg ccattcatan atacataccc ttcatcctgt ggcccattnc tgacctcttc 120
   aacgacctga ttgcttgtgc gttccttgtg ggagccgtgg tctttgctgt gagaagtcgg 180
   cgatccatga atctccacta cttacttgct gtgatcctta ttggtgcggc tggagttttt 240
   gcttttatcg atgtgtgtct tcaaagaaac cacttcagag gcaagaaggc caaaaagcat 300
   atgctggttc ctcctccagg aaaggaaaaa ggaccccagc agggcaaggg accagaaccc 360
   gccaagccac cagaacctgg caagccacca gggccagcaa agggaaagaa atgacttgga 420
   ggaggctcct ggtgtctgaa acggcagtgt attttacagc aatatgtttc cactctcttc 480
   cttgtcttct ttctggaatg gttttctttt ccattttcat taccaccttt gcttgg     536
<210> 25
   <211> 511
   <212> DNA
   <213> Homo sapiens
<400> 25
   gaagtgtgtg tatgtccacg tttgtaatca tagaatggac cccattcttt tgttaaatac 60
   acaagagaaa gctttctgtg acagttccag gtcttgaagc taatcagcat ctcaagaaag 120
   tatccagaaa gaacatctgc tagttggtta taggcggtgg gaggaataat atacctaatt 180
   ggttataggt ggggggagca tgataagcaa agaaaaggca aacacaagga aagatcagat 240
   gaaacagaag atgatagtaa aagtgatcct aagtaagaac ataatgtaaa attgtcagca 300
   gcctcatggg gaggaaaaag gaagagtcaa ctcacttgaa gaagagggtc ttgagaaatc 360
   cttagcataa agggctactg gtgagattga gatctgagca ggcaaagctc aaaagagagt 420
   ttggaggtta aaaataattt atttttgcag tagtgtgctt tgaaatgtgt aaatcttatt 480
   tctaatgtat acaaccacat ttcacataaa a                                511
<210> 26
   <211> 365
   <212> DNA
   <213> Homo sapiens
<220>
   <221> misc_feature
   <222> 171, 334
   <223> n = A,T,C or G
<400> 26
   aagaatcata ttccttcact agcaggatca agaaagcttt tatagaaata tacaaaatct 60
   tcacttcttg aaggattggt aaaatttaat agccaacatt gggcacttat tcattctctg 120
   agtaaatatt tattgcatgc ttatcttgta tcaagcattg tgatgaaagc ncaagaatga 180
   aagaggaggg agaatgttta gagaataagg gctgaaacac agattttgta gggagcgtag 240
   gggagactga gaagacaggt tcaggttagt aagggcgctc atattttgac actgaatgtt 300
   aactatgtgc acatcatgct agctattcta aatnaggcat tttcaaatgg aagcaggcac 360
   tgaca                                                             365
<210> 27
   <211> 540
   <212> DNA
   <213> Homo sapiens
<400> 27
   tacaagtact acttctactg gaaataaaaa ctttttagct ggaagattta gccacttata 60
   aaaaaaaaaa aggatgatca aaacacacag tgtttatgtt ggaatctttt ggaactcctt 120
   tgatctcact gttattatta acatttattt attatttttc taaatgtgaa agaaatacat 180
   aatttaggga aaattggaaa atataggaaa ctttaaacga gaaaatgaaa cctctcataa 240
   tcccactgca tagaaataac aagcgttaac attttcatat ttttttcttt cagtcatttt 300
   tgtatttgtg gtatatgtat atatgtacct atatgtattt gcatttgaaa ttttggaatc 360
   ctgctctatg tacagttttg tattatactt tttaaatctt gaactttatg aacattttct 420
   gaaatcattg attattctac aaaaacatga ttttaaacag ctgtaaaata ttctatgata 480
   tgaatgtttt atgcattatt taagcctgtc tctattgttg gaatttcagg tcattttcat 540
<210> 28
   <211> 523
   <212> DNA
   <213> Homo sapiens
<400> 28
   atgtccatcg ttgctgtcag ttatgggctt attgccacca agatccacaa gcaaggcttg 60
   attaagtcca gtcgtccctt acgggtcctc tcctttgtcg cagcagcctt ttttctctgc 120
   tggtccccat atcaggtggt ggcccttata gccacagtca gaatccgtga gttattgcaa 180
   ggcatgtaca aagaaattgg tattgcagtg gatgtgacaa gtgccctggc cttcttcaac 240
   agctgcctca accccatgct ctatgtcttc atgggccagg acttccggga gaggctgatc 300
   cacgcccttc ccgccagtct ggagagggcc ctgaccgagg actcaaccca aaccagtgac 360
   acagctacca attctacttt accttctgca gaggtggagt tacaggcaaa gtgaggaggg 420
   agctggggga cactttcgag ctcccagctc cagcttcgtc tcaccttgag ttaggctgag 480
   cacaggcatt tcctgcttat tttaggatta cccactcatc aga 523
<210> 29
   <211> 537
   <212> DNA
   <213> Homo sapiens
<220>
   <221> misc_feature
   <222> 323
   <223> n = A,T,C or G
<400> 29
   gctcacagac gccagcaaag cagtatgctc ccgatcaagt agatttttaa aaaatcagag 60
   tgggccgggc gcggctgcac gcctgtaatc ccagcacttt ggaggccaag gcgggtggat 120
   cacgaggtca ggagatcgag accatcctgg ctaacacggt gaaaccctgt ctctactaaa 180
   aatacaaaaa attagccagg ggtggtgcgg gcgcctgtag tcccagctac tctggaggct 240
   gaggcaggag agtacgtgaa cccgggaggc agagcttgcg gtgagccgag attgcgctac 300
   tgcactccag cctgggcgac agnaccagac tccatctcaa aaaaaaaaaa aaccagactg 360
   aattaatttt aactgaaaat ttctcttatg ttccaaggta cacaataggt aagattatgc 420
   tcaatattct cagaataatt ttcaatgtat taatgaaatg aaatgataat ttggcttcat 480
   atctagacta acacaaaatt aagaatcttc cataattgct tttgctcagt aactgtg 537
<210> 30
   <211> 551
   <212> DNA
   <213> Homo sapiens
<220>
   <221> misc_feature
   <222> 231, 492, 493
   <223> n = A,T,C or G
<400> 30
   actactatga ccctgctggg gcagaagatc ccctgggagc aattcacttg agaggctgtg 60
   tggtgacttc agtggagagc aactcaaatg gcaggaagag tgaggaagag aacctttttg 120
   agatcatcac agcagatgaa gtgcactatt tcttgcaagc agccaccccc aaggagcgca 180
   cagagtggat caaagccatc cagatggcct cccgaactgg gaagtaaaga nactcctgca 240
   ttcctcctcc cctcctgagg gaagcccatg gacaagctca gtccaggacc tgtccacttc 300
   tgtgacaaat caacgggaaa cagcccaggg gtgggaagtt ttcatttgca ggggggtctg 360
   aatgtaactc accatgtggt gtgcaaggtt cccctgcatt gtattgctca ctgcagcccc 420
   tctgccccta tccatgaccc ccaagcagat ataacaagct gtgcagcctc agtaggctgc 480
   ttgccctctc cnngcctcag ggcctcttct ggaaaatgaa gaaattcaac tagtagattc 540
   ctgaggtccc c 551
<210> 31
   <211> 457
   <212> DNA
   <213> Homo sapiens
<400> 31
   gttcatgtaa aacctctcag attttaaaga gaaggccaaa taatttggta tttgtgctct 60
   tgctcagaga agcatcatat tcggaaatat cttcctaggt ttatctacca tttagtgttg 120
   tttagtcaga ctgaaacaac ttaaaacctg taatgactaa gacaatgaaa atgataggct 180
   tgtaagaaaa atacaatttg ttattctttg gcaaataagg aatcatgtct aaataagacg 240
   gaggtcatgg cttgatagag agatggctga acctatagta gaaaaacact aggttccgcc 300
   aaatggtaag ggaaatgttg agtcacaatg acacacatgt cctagatttg tttcgtcaaa 360
   gcgacttttg gttgtcatga tcttacttcc ggtggaggtt gcagtgagct gagatcatgc 420
   cattgcactc caggctaggc aacagagtgc aactccg                          457
<210> 32
   <211> 532
   <212> DNA
   <213> Homo sapiens
<400> 32
   gtcttccagg ggactctatc agaactggac catttccaag tatataacga tgagccctct 60
   aatgctagga gtagcaaatg gtcctaggaa ggggactgag gattggggtg ggggtggggt 120
   ggaaaagaaa gtacagaaca aaccctgtgt cactgtccca agttaagcta agtgaacaga 180
   actatctcag catcagaatg agaatgagaa agcctgagaa gaaagaacca accacaagca 240
   cacaggaagg aaagcgcagg aggtgaaaat gctttcttgg ccagggtagt aagaattaga 300
   ggttaatgca gggactgtaa aaccaccttt tctgcttcaa tgtctagttc ctgtatagct 360
   ttgttcattg catttattaa acaaatgttg tataaccaat actaaatgta ctactgagct 420
   tcactgagtt acgctgtgaa actttcaaat ccttcttcag tcagttccaa tgaggtgggg 480
   atggagaaga caattgttgc ttatgaaaaa aagctttagc tgtctcctgt tt         532
<210> 33
   <211> 432
   <212> DNA
   <213> Homo sapiens
<400> 33
   aagtgcttca agtattagtt gtcatattaa tccaagagaa aattagatct gagactacct 60
   cgaggagtaa acgaacaact tctttaaacc aatactagtc aaaatgcagt ccacggacca 120
   atgctggttc atgccctatt tgatccaagt ccacattaag tacagaaatg aagagtaagc 180
   atttagaaac ttggggcaat ttgacattgc ctcaacatcc aagtttcagt gtcctttata 240
   aactattgat ccatgacaga tcatgaaact ttctaaaaat tgatcctttt atcacatagt 300
   ctttcaaaat attatttgct tctttgaagc tttggataga aacttgataa tgtgaagttc 360
   tctgctatgt ggacctggtg tgcttatctt ctttgttgct gatttgagag ctgagctatg 420
   tactatatct gg                                                     432
<210> 34
   <211> 403
   <212> DNA
   <213> Homo sapiens
<400> 34
   aaaatgttgc gttctcagtc caaaaagaag tggaaaagaa tctgaagtca tgcttggaca 60
   atgttaatgt tgtgtccgta gacactgcca gaacactatt caaccaagtg atggaaaagg 120
   agtttgaaga cggcatcatt aactggggaa gaattgtaac catatttgca tttgaaggta 180
   ttctcatcaa gaaacttcta cgacagcaaa ttgccccgga tgtggatacc tataaggaga 240
   tttcatattt tgttgcggag ttcataatga ataacacagg agaatggata aggcaaaacg 300
   gaggctggga aaatggcttt gtaaagaagt ttgaacctaa atctggctgg atgacttttc 360
   tagaagttac aggaaagatc tgtgaaatgc tatctctcct gaa                   403
<210> 35
   <211> 491
   <212> DNA
   <213> Homo sapiens
<400> 35
   agggatattt tgagttctgt tcatcctacc ctaatgccag ttccagcttc atctaccctt 60
   gagtcatatt gaggcattca aggatgcaca gctcaagtat ttattcagga aaaatgtttt 120
   tgtgtccctg atttggggct aagaaataga cagtcaggct actaaaatat tagtgttatt 180
   ttttgttttt tgacttctgc ctataccctg gggtaagtgg agttgggaaa tacaagaaga 240
   gaaagaccag tggggatttg taagacttag atgagatagc gcataataag gggaagactt 300
   taaagtataa agtaaaatgt ttgctgtagg ttttttatag ctattaaaaa aaatcagatt 360
   atggaagttt tcttctattt ttagtttgct aagagttttc tgtttctttt ttcttacatc 420
   atgagtggac tttgcatttt atcaatgcat tttctacatg tattaagatg gtcatattat 480
   tcttcttctt t                                                      491
<210> 36
   <211> 569
   <212> DNA
   <213> Homo sapiens
<220>
   <221> misc_feature
   <222> 118, 120, 180, 395, 413, 416, 417
   <223> n = A,T,C or G
<400> 36
   tggggcgcct atgatggaga ccctcagccc ctagggactc catcttttga ccttgaagtc 60
   tcgctcaaac attcccctaa aagaacgggg gtgagggaga atggaaggca aaaagagngn 120
   aaaaagcaaa taaacttcgg aatcggacaa cttaaagtct cgatatgagc ctcggattgn 180
   attcgaactc ctgggttcta gacccagctc tgctactaac ttgtctgacc ttccccattt 240
   ataaactgtt gtgggtgaga ggtgggacta ggtgaactct gagagccctg cactcggcgc 300
   ggacgaggga tttaggggaa agtgtgctgt attccccgcg agccttcccc agcggccccg 360
   cctccccggg tgaatccccg gcggccagcg cagcncaggc agcgatcctg gcngcnnccg 420
   gggccggcag cttcctggaa agttacttct tgttggagcc ggcaataggc aaagtgtgtt 480
   ctgtgaaatc cgcagagccg agattgacta cgttccggga atgagagggc tgtgtcattc 540
   ccctcattgc ctgctgcatt gacggcgta                                   569
<210> 37
   <211> 462
   <212> DNA
   <213> Homo sapiens
<220>
   <221> misc_feature
   <222> 304
   <223> n = A,T,C or G
<400> 37
   ggtactatgt tagccccata attttttttt tccttttaaa acacttccat aatctggact 60
   cctctgtcca ggcactgctg cccagcctcc aagctccatc tccactccag attttttaca 120
   gctgcctgca gtactttacc tcctatcaga agtttctcag ctcccaaggc tctgagcaaa 180
   tgtggctcct gggggttctt tcttcctctg ctgaaggaat aaattgctcc ttgacattgt 240
   agagcttctg gcacttggag acttgtatga aagatggctg tgcctctgcc tgtctccccc 300
   accnggctgg gagctctgca gagcaggaaa catgactcgt atatgtctca ggtccctgca 360
   gggccaagca cctagcctcg ctcttggcag gtactcagcg aatgaatgct gtatatgttg 420
   ggtgcaaagt tccctacttc ctgtgacttc agctctgttt ta                   462
<210> 38
   <211> 379
   <212> DNA
   <213> Homo sapiens
<400> 38
   aataacttgc atctgtcatt accatgatat gtttcataac ctttatatgc acatggagct 60
   taaaaatgta atttaacaat aaataatgac atataccaga tatgctcact gtttattcca 120
   gtactcagcc aaaaacctaa atatcattta aattataaat acataatgca aatataatgg 180
   cacaaaaatg tctaaagtgc aaccaaatca caagagaaga atcatgcaaa ctggtctagg 240
   tcagcccccg aatcactctg cagcaaacac aagacgaagc tttggaagtt taagggggaa 300
   ttggagggag tagggtgggg gaaagagaag caaaaaacta gcaacattgt gagaactgct 360
   tctttctgta tgtgtagat                                              379
<210> 39
   <211> 421
   <212> DNA
   <213> Homo sapiens
<400> 39
   gagatctttt taaatccctg ctactgtccc ttctcacagg cattcacaga acccttctga 60
   ttcgaagggt tacgaaactc atgttcttct ccagtcccct gtggtttctg ttggagcata 120
   aggtttccag taagcgggag ggcagatcca actcagaacc atgcagataa ggagcctctg 180
   gcaaatgggt gctgcatcag aacgcgtgga ttctctttca tggcagatgc tcttggactc 240
   ggttctccag gcctgattcc ccgactccat cctttttcag ggttatttaa aaatctgcct 300
   tagattctat agtgaagaca agcatttcaa gaaagagtta cctggatcag ccatgctcag 360
   ctgtgacgcc tgataactgt ctactttatc ttcactgaac cactcactct gtgtaaaggc 420
   c                                                                 421
<210> 40
   <211> 491
   <212> DNA
   <213> Homo sapiens
<400> 40
   agctgtaccc agagagtcct gtgctgaatg tggactcaat ccctagggct ggcagaaagg 60
   gaacagaaag gtttttgagt acggctatag cctggacttt cctgttgtct acaccaatgc 120
   ccaactgcct gccttagggt agtgctaaga ggatctcctg tccatcagcc aggacagtca 180
   gctctctcct ttcagggcca atccccagcc cttttgttga gccaggcctc tctcacctct 240
   cctactcact taaagcccgc ctgacagaaa ccacggccac atttggttct aagaaaccct 300
   ctgtcattcg ctcccacatt ctgatgagca accgcttccc tatttattta tttatttgtt 360
   tgtttgtttt attcattggt ctaatttatt caaagggggc aagaagtagc agtgtctgta 420
   aaagagccta gtttttaata gctatggaat caattcaatt tggactggtg tgctctcttt 480
   aaatcaagtc c                                                      491
<210> 41
   <211> 456
   <212> DNA
   <213> Homo sapiens
<220>
   <221> misc_feature
   <222> 38, 52, 54, 56, 64, 69, 79, 82, 86, 88, 95, 99, 104, 116
   <223> n = A,T,C or G
<400> 41
   actgcagcaa actttctagc atctgatatt ggataagnat agcttgtgct anangntgga 60
   gatnaatcng gtctgctgnc tngcancntt agagnctgna tctnatggtt ggtgtnagga 120
   tgttgttgac agttctgaaa gttagccatc aattcctgtg cagggtggag tcagacccag 180
   tgacttcctt ttcaatgtca gcaagagttt tctcatgcct gctttggtca ctttctcttg 240
   gaaacttcac gcatttgact tgcagcttct tgacccgagg aatcaactga gctcccagtg 300
   ctggctacct tgggcaaata aaatctgtgg ttgagagagt tctctttgct gtgccacagt 360
   ccctgtgatg tgccaaatgg caccagcatt tgcagcaaag ctcgttaaat ctttttggaa 420
   tgggcaaata ttttttcaac ttgagccctg ctgagc                          456
<210> 42
   <211> 512
   <212> DNA
   <213> Homo sapiens
<400> 42
   gagtacaggt gccagacaaa cctctccacc ctcagtgacc cggtgcagct agaagtccat 60
   atcggctggc tgttgctcca ggcccctcgg tgggtgttca aggaggaaga ccctattcac 120
   ctgaggtgtc acagctggaa gaacactgct ctgcataagg tcacatattt acagaatggc 180
   aaagacagga agtattttca tcataattct gacttccaca ttccaaaagc cacactcaaa 240
   gatagcggct cctacttctg cagggggctt gttgggagta aaaatgtgtc ttcagagact 300
   gtgaacatca ccatcactca aggtttggca gtgtcaacca tctcatcatt ctctccacct 360
   gggtaccaag tctctttctg cttggtgatg gtactccttt ttgcagtgga cacaggacta 420
   tatttctctg tgaagacaaa catttgaagc tcaacaagag actggaagga ccataaactt 480
   aaatggagaa aggaccctca agacaaatga cc                               512
<210> 43
   <211> 448
   <212> DNA
   <213> Homo sapiens
<400> 43
   ccttcttgtc cacggttttg ttgagttttc actcttctaa tgcaagggtc tcacactgtg 60
   aaccacttag gatgtgatca ctttcaggtg gccaggaatg ttgaatgtct ttggctcagt 120
   tcatttaaaa aagatatcta tttgaaagtt ctcagagttg tacatatgtt tcacagtaca 180
   ggatctgtac ataaaagttt ctttcctaaa ccattcacca agagccaata tctaggcatt 240
   ttcttggtag cacaaatttt cttattgctt agaaaattgt cctccttgtt atttctgttt 300
   gtaagactta agtgagttag gtctttaagg aaagcaacgc tcctctgaaa tgcttgtctt 360
   ttttctgttg ccgaaatagc tggtcctttt tcgggagtta gatgtataga gtgtttgtat 420
   gtaaacattt cttgtaggca tcaccatg                                   448
<210> 44
   <211> 515
   <212> DNA
   <213> Homo sapiens
<400> 44
   gaggtcccaa acagaattat cgaaatagca aataaaactc ccaagatcag tcaactttac 60
   aagcaagcat ctggaacaat ggtgtatggg ttgtttatgt tacaaaagaa aagcgtacac 120
   aagaagaaca caatttatga aatacaggat aatacaggat ccatggatgt agtggggagt 180
   ggaaaatggc acaatatcaa gtgtgagaaa ggagataaac ttcgactctt ctgccttcaa 240
   ctgagaacag ttgaccgcaa gctgaaactg gtgtgtggaa gtcacagctt catcaaggtc 300
   atcaaggcca agaaaaacaa ggaaggacca atgaatgtta attgaaatat gaaagctgaa 360
   atgcaacaaa caacttccgc ttaaaacaat taagttgtta ataactgtga ttttgtaaat 420
   ttcagtaatt catttaaatg atgtttcagt agatatattc tagcatatta agagctttta 480
   taactgagtt atagattagt ttgctttctg gaata                             515
<210> 45
   <211> 478
   <212> DNA
   <213> Homo sapiens
<400> 45
   gtgagtggtc ttttctgtaa gctaatccac aacctgttac cacttcctga atcagttatt 60
   atttcttcat ttttttttct accagaggac agattaatag atttaaccct tcacaacagt 120
   tcttgttaga atcatgggat gtgtggccca gaggtaagaa tagaatttct ttccctaaag 180
   aacatacctt ttgtagatga actcttctca actctgtttt gctatgctat aattccgaaa 240
   catacaagac aaaaaaaatg aagacactca atttagaaca aactaagcca ggtatgcaaa 300
   tatcgctgaa tagaaacaga tggaattaga aatatatctt ctatttttag gcttctattt 360
   cctttccacc cactcttcac aggctatttt actttaaagg aagccttttt attttgctgc 420
   acacaatcta gcaggaatct tttttttttt ttaagagctg tgtcatcctt atgtaggc   478
<210> 46
   <211> 381
   <212> DNA
   <213> Homo sapiens
<400> 46
   tgccgaccct ctgggagaaa atccagcaag atgcaagcct tcagaatctg ggatgttaac 60
   cagaagacct tctatctgag gaacaaccaa ctagttgccg gatacttgca aggaccaaat 120
   gtcaatttag aagaaaagat agatgtggta cccattgagc ctcatgctct gttcttggga 180
   atccatggag ggaagatgtg cctgtcctgt gtcaagtctg gtgatgagac cagactccag 240
   ctggaggcag ttaacatcac tgacctgagc gagaacagaa agcaggacaa gcgcttcgcc 300
   ttcatccgct cagacagtgg ccccacacca gtttgtgagg tctgcgctgc ccggtttggt 360
   tcctctgcac agcgatggaa g                                           381
<210> 47
   <211> 550
   <212> DNA
   <213> Homo sapiens
<220>
   <221> misc_feature
   <222> 51, 123, 124, 125, 126, 127, 128, 129, 130, 131, 132, 133, 134, 135, 136, 137, 138, 139, 272, 273, 274, 301, 302, 303, 304, 305, 306, 307, 308, 309, 310, 311, 312, 313, 314, 315, 316, 317, 318, 319, 320, 321, 322, 323, 324, 325, 326
<223> n = A,T,C or G
<220>
   <221> misc_feature
   <222> 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369
   <223> n = A,T,C or G
<400> 47
   aatgtggact caatccctag ggctggcaga aagggaacag aaaggttttt nagtacggct 60
   atagcctgga ctttcctgtt gtctacacca atgcccaact gcctgcctta gggtagtgct 120
   aannnnnnnn nnnnnnnnnc agccaggaca gtcagctctc tcctttcagg gccaatcccc 180
   agcccttttg ttgagccagg cctctctcac ctctcctact cacttaaagc ccgcctgaca 240
   gaaaccacgg ccacatttgg ttctaagaaa cnnncctctg tcattcgctc ccacattctg 300
   nnnnnnnnnn nnnnnnnnnn nnnnnnnnnn nnnnnnnnnn nnnnnnnnnn nnnnnnnnnn 360
   nnnnnnnnna agggggcaag aagtagcagt gtctgtaaaa gagcctagtt tttaatagct 420
   atggaatcaa ttcaatttgg actggtgtgc tctctttaaa tcaagtcctt taattaagac 480
   tgaaaatata taagctcaga ttatttaaat gggaatattt ataaatgagc aaatatcata 540
   ctgttcaatg                                                        550
<210> 48
   <211> 519
   <212> DNA
   <213> Homo sapiens
<220>
   <221> misc_feature
   <222> 325, 326, 327, 328, 329, 330, 331
   <223> n = A,T,C or G
<400> 48
   aacagtacag tcctcaccct gatgaccttg accccagagg ggtcggagct acacatcatc 60
   ctgggcctgt tcggcctcct gctgttgctc acctgcctct gtggaactgc ctggctctgt 120
   tgcagcccca acaggaagaa tcccctctgg ccaagtgtcc cagacccagc tcacagcagc 180
   ctgggctcct gggtgcccac aatcatggag gaggatgcct tccagctgcc cggccttggc 240
   acgccaccca tcaccaagct cacagtgctg gaggaggatg aaaagaagcc ggtgccctgg 300
   gagtcccata acagctcaga gaccnnnnnn ntccccactc tggtccagac ctatgtgctc 360
   cagggggacc caagagcagt ttccacccag ccccaatccc agtctggcac cagcgatcag 420
   gctgggcctc ccaggcgatc tgcatacttt aaggaccaga tcatgctcca tccagcccca 480
   cccaatggcc ttttgtgctt gtttcctata acttcagta                        519
<210> 49
   <211> 399
   <212> DNA
   <213> Homo sapiens
<400> 49
   aaatgttctt ataatgctac ctgatttcta tgaaatgtgt ttgacaagcc aaaattctag 60
   gatgtagaaa tctggaaagt tcatttcctg ggattcactt ctccagggat tttttaaagt 120
   taatttggga aattaacagc agttcacttt attgtgagtc tttgccacat ttgactgaat 180
   tgagctgtca tttgtacatt taaagcagct gttttggggt ctgtgagagt acatgtatta 240
   tatacaagca caacagggct tgcactaaag aattgtcatt gtaataacac tacttggtag 300
   cctaacttca tatatgtatt cttaattgca caaaaagtca ataatttgtc accttggggt 360
   tttgaatgtt tgctttaagt gttggctatt tctatgttt                        399
<210> 50
   <211> 466
   <212> DNA
   <213> Homo sapiens
<220>
   <221> misc_feature
   <222> 227
   <223> n = A,T,C or G
<400> 50
   cgatggaagc tgaccagccc gtcagcctca ccaatatgcc tgacgaaggc gtcatggtca 60
   ccaaattcta cttccaggag gacgagtagt actgcccagg cctgcctgtt cccattcttg 120
   catggcaagg actgcaggga ctgccagtcc ccctgcccca gggctcccgg ctatgggggc 180
   actgaggacc agccattgag gggtggaccc tcagaaggcg tcacaanaac ctggtcacag 240
   gactctgcct cctcttcaac tgaccagcct ccatgctgcc tccagaatgg tctttctaat 300
   gtgtgaatca gagcacagca gcccctgcac aaagcccttc catgtcgcct ctgcattcag 360
   gatcaaaccc cgaccacctg cccaacctgc tctcctcttg ccactgcctc ttcctccctc 420
   attccacctt cccatgccct ggatccatca ggccacttga tgaccc                466
<210> 51
   <211> 462
   <212> DNA
   <213> Homo sapiens
<400> 51
   gtttcttaca gtgagcggga tgcctactgg gtggagaacc ttatggtcca ggagctggag 60
   aacttcaatc cccccttcaa gttgtgtctt cataagcggg acttcattcc tggcaagtgg 120
   atcattgaca atatcattga ctccattgaa aagagccaca aaactgtctt tgtgctttct 180
   gaaaactttg tgaagagtga gtggtgcaag tatgaactgg acttctccca tttccgtctt 240
   tttgaagaga acaatgatgc tgccattctc attcttctgg agcccattga gaaaaaagcc 300
   attccccagc gcttctgcaa gctgcggaag ataatgaaca ccaagaccta cctggagtgg 360
   cccatggacg aggctcagcg ggaaggattt tgggtaaatc tgagagctgc gataaagtcc 420
   taggttccca tatttaagac cagtctttgt ctagttggga tc                    462
<210> 52
   <211> 450
   <212> DNA
   <213> Homo sapiens
<400> 52
   tagtggggaa gtccaagtca gtaattgtac gtcctgggat gatatccagt gtgttgaaga 60
   atttggtgcc aatgccactg tggaaacccc agctgctgaa gagacaatga acaccagccc 120
   ggggactcct gccccagctg ctgaagagac aatgaacacc agcccaggga ctcctgcccc 180
   agctgctgaa gagacaatga ccaccagccc ggggactcct gccccagctg ctgaagagac 240
   aatgaccacc agcccgggga ctcctgcccc agctgctgaa gagacaatga ccaccagccc 300
   ggggactcct gcctcttctc attacctctc atgcaccatc gtagggatca tagttctaat 360
   tgtgcttctg attgtgtttg tttgaaagac ttcactgtgg aagaaattcc ttccttacct 420
   gaaaggttca ggtaggcgct ggctgagggc                                  450
<210> 53
   <211> 517
   <212> DNA
   <213> Homo sapiens
<400> 53
   tgtaaatcct tcttgtacag cgctgctaaa tttgacactc agggaaaagc attcgtcaaa 60
   gagagcttaa aatgcatcgc caacggggtc acctccaagg tcttcctcgc cattcggagg 120
   tgctccactt tccaaaggat gattgctgag gtgcaggaag agtgctacag caagctgaat 180
   gtgtgcagca tcgccaagcg gaaccctgaa gccatcactg aggtcgtcca gctgcccaat 240
   cacttctcca acagatacta taacagactt gtccgaagcc tgctggaatg tgatgaagac 300
   acagtcagca caatcagaga cagcctgatg gagaaaattg ggcctaacat ggccagcctc 360
   ttccacatcc tgcagacaga ccactgtgcc caaacacacc cacgagctga cttcaacagg 420
   agacgcacca atgagccgca gaagctgaaa gtcctcctca ggaacctccg aggtgaggag 480
   gactctccct cccacatcaa acgcacatcc catgaga                          517
<210> 54
   <211> 442
   <212> DNA
   <213> Homo sapiens
<400> 54
   caattttcac atcttccaca aactccattt agggagaaat gtttaaatct ctggtataag 60
   tttactccat accagagtaa actatatatt actctatata agcagtcttg caataactaa 120
   tcaccaccat agaagaaaga aacagactgc aaggaacaga gttgagtgtc tggagtcatc 180
   aaaggcatta aaaactccag taaaagctgg ggccgtagca aaaatcatga aaaacacttc 240
   aacgtgtcct ttcaatcatc caattaaatg tgggtagatt aatgaaaatg tattacatca 300
   atattaactc atctatagca ctttgagtat ctttgtagtt catgatatcc tatcctataa 360
   tgtggaggta aatgatttta tatgcattgg gggtcatata taaaacttca atgtaatttc 420
   actacaataa attgccttcc tt                                         442
<210> 55
   <211> 533
   <212> DNA
   <213> Homo sapiens
<220>
   <221> misc_feature
   <222> 294, 444, 449, 455, 474
   <223> n = A,T,C or G
<400> 55
   gtggtctgga agatcttcac cctgtcccgt gctacagcgg tcaaggagcg ggggaagaac 60
   cggaagaagg tgctcaccct gctgggcctc tcgagcctgg tgggtgtgac atgggggttg 120
   gccatcttca ccccgttggg cctctccacc gtctacatct ttgcactttt caactccttg 180
   caaggtgtct tcatctgctg ctggttcacc atcctttacc tcccaagtca gagcaccaca 240
   gtctcctcct ctactgcaag attggaccag gcccactccg catctcaaga atangaaggc 300
   acggccctgc aatatggact cagctctggc tctctgtgtg accttgggca gctccgtgcc 360
   tctctctgta ctccctcagt ttccttctct gtacaatgtg gctggggagg gagaggatgg 420
   gaccaggttg gaccacgtgg catncagang gtccnatcca gatccaacta tagntccaag 480
   agtccacgta agcaggtttg caaggctcta aagttcctat agtcctgaga ccc        533
<210> 56
   <211> 380
   <212> DNA
   <213> Homo sapiens
<400> 56
   tactgcttgc agtaattcaa ctggaaatta aaaaaaaaaa actagactcc attgtgcctt 60
   actaaatatg ggaatgtcta acttaaatag ctttgagatt tcagctatgc tagaggcttt 120
   tattagaaag ccatattttt ttctgtaaaa gttactaata tatctgtaac actattacag 180
   tattgctatt tatattcatt cagatataag atttgtacat attatcatcc tataaagaaa 240
   cggtatgact taattttaga aagaaaatta tattctgttt attatgacaa atgaaagaga 300
   aaatatatat ttttaatgga aagtttgtag catttttcta ataggtactg ccatattttt 360
   ctgtgtggag tatttttata                                            380
<210> 57
   <211> 340
   <212> DNA
   <213> Homo sapiens
<400> 57
   attatgcttt ctattttgag atcattgcaa actcaacaca attgtaagta atgatacaga 60
   gggatcttgt gtacccttca cccagcctcc cccaatggca acatcttgca aaactacaat 120
   gtagtctcat aaccaggata ttgacattga tacagtgaag atacaggaca ttctcatcac 180
   cacagggatc cccaggatgc ccacttccct ccacccccac accccagccg tgtccctaac 240
   ccctggcaac caggaatcca ctctccattt ctataatgtt gtcatttcaa gaatgttatt 300
   caatggaatc atatagtatg taacctgttt tgagcttaaa                       340
<210> 58
   <211> 418
   <212> DNA
   <213> Homo sapiens
<400> 58
   gagggcaacc accagtttgc taagtacaaa tcattcaagg tggctgacga ggcagagaag 60
   tacaagctgg tactgggagc ctttgtcggg ggcagtgcgg gtaattctct aacgggccac 120
   aacaacaact tcttctccac caaagaccaa gacaatgatg tgagttcttc gaattgtgct 180
   gagaagttcc agggagcctg gtggtacgcc gactgtcatg cttcaaacct caatggtctc 240
   tacctcatgg gaccccatga gagctatgcc aatggtatca actggagtgc ggcgaagggg 300
   tacaaatata gctacaaggt gtcagagatg aaggtgcggc ccgcctagac gggccaggac 360
   ccctccacat gcacctgcta gtggggaggc cacacccaca agcgctgcgt cgtggaag   418
<210> 59
   <211> 349
   <212> DNA
   <213> Homo sapiens
<400> 59
   ataccgctgt tactactaca gccctgcagg ctggtcagag cccagcgacc ccctggagct 60
   ggtggtgaca ggattctaca acaaacccac cctctcagcc ctgcccagtc ctgtggtgac 120
   ctcaggagag aacgtgaccc tccagtgtgg ctcacggctg agattcgaca ggttcattct 180
   gactgaggaa ggagaccaca agctctcctg gaccttggac tcacagctga cccccagtgg 240
   gcagttccag gccctgttcc ctgtgggccc tgtgaccccc agccacaggt ggatgctcag 300
   atgctatggc tctcgcaggc atatcctgca ggtatggtca gaacccagt             349
<210> 60
   <211> 474
   <212> DNA
   <213> Homo sapiens
<220>
   <221> misc_feature
   <222> 155, 224, 245
   <223> n = A,T,C or G
<400> 60
   aattcaatgt ttcattccgc tgcctccatc atgtaataga atcgctttcc agaaaggcag 60
   ttaactggaa gcagcagagg ctcccagccg tgagaggact gctcaacaat gccccccatc 120
   gccgcccccc cacccctcgc accccttgtg ttttnccctc tgagggcccc aagggttatg 180
   gctttcatgt ctaggtgtgg ggacagagga gggagaggca gatncctggg ccgggagagg 240
   atggncctgg tctgaatctg gagtaattaa tgccacccaa agaaaaggcc ctgccaggtc 300
   caatgttgtc ttagatctga tgatgctgct atttacaaaa cactgatcgt ccgaaagctt 360
   gaatctgttc ctcctcgaat gaccctgtag atgcctgacc tccaccgtac ctccacatca 420
   ctattcatgt ccttctagga aaatgtgcac atgcctcacg cactatgtgg gaag       474
<210> 61
   <211> 496
   <212> DNA
   <213> Homo sapiens
<220>
   <221> misc_feature
   <222> 126, 127, 128, 130, 131, 132, 137, 138, 139
   <223> n = A,T,C or G
<400> 61
   gcaaagcctt taagtttttc cactggaatt ctaacctcac cattctgaaa accaacataa 60
   gtcacaatgg cacctaccat tgctcaggca tgggaaagca tcgctacaca tcagcaggaa 120
   tatcannntn nnctgtnnna ggcctccagt taccaactcc tgtctggttt catgtccttt 180
   tctatctggc agtgggaata atgtttttag tgaacactgt tctctgggtg acaatacgta 240
   aagaactgaa aagaaagaaa aagtggaatt tagaaatctc tttggattct ggtcatgaga 300
   agaaggtaat ttccagcctt caagaagaca gacatttaga agaagagctg aaatgtcagg 360
   aacaaaaaga agaacagctg caggaagggg tgcaccggaa ggagccccag ggggccacgt 420
   agcagcggct cagttggtgg ccatcgatct ggaccgtccc ctgcccactt gctccccatg 480
   agcactgcgt acaaac                                                  496
<210> 62
   <211> 399
   <212> DNA
   <213> Homo sapiens
<400> 62
   ttctttcccc aaatatcatg tagcacatca atatgtaggg aaacattctt atgcatcatt 60
   tggtttgttt tataaccaat tcattaaatg taattcataa aatgtactat gaaaaaaatt 120
   atacgctatg ggatactggc aacagtgcac atatttcata accaaattag cagcaccggt 180
   cttaatttga tgtttttcaa cttttattca ttgagatgtt ttgaagcaat taggatatgt 240
   gtgtttactg tactttttgt tttgatccgt ttgtataaat gatagcaata tcttggacac 300
   atttgaaata caaaatgttt ttgtctacca aagaaaaatg ttgaaaaata agcaaatgta 360
   tacctagcaa tcacttttac tttttgtaat tctgtctct                         399
<210> 63
   <211> 144
   <212> DNA
   <213> Homo sapiens
<400> 63
   tctcccagcc aagtccaaga gggcagggcc agttcctccc atcttcaggc ccagccaggc 60
   agggggcagt cggctcctca actgggtgac aagggtgagg atgagaagtg gtcacgggat 120
   ttattcagcc ttggtcagag caga                                         144
<210> 64
   <211> 474
   <212> DNA
   <213> Homo sapiens
<400> 64
   gcaaataggc acgaaggcac aggtctaaaa tactacattg tcactgtaag ctatactttt 60
   aaaatattta ttttttttaa agtattttct agtcttttct ctctctgtgg aatggtgaaa 120
   gagagatgcc gtgttttgaa agtaagatga tgaaatgaat ttttaattca agaaacattc 180
   agaaacatag gaattaaaac ttagagaaat gatctaattt ccctgttcac acaaacttta 240
   cactttaatc tgatgattgg atattttatt ttagtgaaac atcatcttgt tagctaactt 300
   taaaaaatgg atgtagaatg attaaaggtt ggtatgattt ttttttaatg tatcagtttg 360
   aacctagaat attgaattaa aatgctgtct cagtatttta aaagcaaaaa aggaatggag 420
   gaaaattgca tcttagacca tttttatatg cagtgtacaa tttgctgggc taga 474
<210> 65
   <211> 474
   <212> DNA
   <213> Homo sapiens
<400> 65
   gaaacaaagt tgctcttgca gaggcctggt ttgcagcttt acttctcctt ctacatgggc 60
   agcaagaccc tgcgaggcag gaacacatcc tctgaatacc aaatactaac tgctagaaga 120
   gaagactctg ggttatactg gtgcgaggct gccacagagg atggaaatgt ccttaagcgc 180
   agccctgagt tggagcttca agtgcttggc ctccagttac caactcctgt ctggtttcat 240
   gtccttttct atctggcagt gggaataatg tttttagtga acactgttct ctgggtgaca 300
   atacgtaaag aactgaaaag aaagaaaaag tgggatttag aaatctcttt ggattctgga 360
   ggccaagcac ttgaagctcc aactcagggc tgcgcttaag gacatttaca tcctctgaat 420
   accaaatact aactgctaga agagaagact ctgggttata ctggtgcgag gctg 474
<210> 66
   <211> 259
   <212> DNA
   <213> Homo sapiens
<400> 66
   gtctacttaa gacttctggt catttccaac ttatagagga agggagtctc taaaatctct 60
   tcttcagaag gcacctcact tctcagactt aaaattccac atcaagtgtt ccattaaaag 120
   aagataaggc attctgagtg caaacaaatg ggggcttctt aaactacaca ccagcagtca 180
   gtgaggaaaa ctttgaacaa ttattgagtt gctttcttgg gtctctataa tcaataacct 240
   gtctgcagat atctatcta 259
<210> 67
   <211> 423
   <212> DNA
   <213> Homo sapiens
<400> 67
   ggtgggagtt gatcctccca cccagtctgc ccctggtctc tgcccatcca atcagagccc 60
   accctcctgg aagagacccc cgtgttcaga gtgctggcag ccctgcacgt gtccagggac 120
   actgcatttc aaagaaccac tgagtgggtg agctaccttg ggcaaacccc ccactcctga 180
   ctctgactgc cacgtgggtg gcccgacctc tgacctgctg tcatcataga ggtagaaagc 240
   aaacaatctg gggctcagca cacctggggg tgctcccact cattcagtgt gtggggcccc 300
   tgagcagagg ctgggcattg ccactagaac ctgagctcct agagagcaag gacctgggtg 360
   gcctcgctta ctgttccagc ccaggccgag cacagggcct ggctcgtggc aaaccttgaa 420
   taa 423
<210> 68
   <211> 504
   <212> DNA
   <213> Homo sapiens
<400> 68
   gaaagagcta gattctctct ctcaaaaaaa aaaaaaaaaa ggaaagaaag aaagaaaaga 60
   aaagaaaatc cctttttgct ttaacttgcc cttgcaggtt tgtagaaact caattgttga 120
   aatttgggtg gataaatttc tggattttct atctattcca tgttggacca ataccacact 180
   gccctagtca ctgttgcatt atagtatatc tttaaaggag taatgggaat ccttcaacta 240
   catttttttc cccaataatt tttggctatt ctgcttcttt tgtgtttcta tgtaaatttt 300
   atcatcagtg tgtctatttc tacaaatagt cctgataggg tttgaattgg gatttctgtg 360
   aatctataga tcaatctgag gagacttaat aatgatattg attctcccaa ttcatgaata 420
   tagtataccc ctgtatttat ttgttttctt gaatttcttt tatcattgtt ttgtagtttt 480
   caccatgaca gtcttgcaca tatt                                        504
<210> 69
   <211> 406
   <212> DNA
   <213> Homo sapiens
<400> 69
   gataaaacgg caacacagct cacaagaaca gactttccag ctgctgaagt tatggaaaca 60
   tcaaaacaaa gcccaagata tagtcaagaa gatcatccaa gatattgacc tctgtgaaaa 120
   cagcgtgcag cggcacattg gacatgctaa cctcaccttc gagcagcttc gtagcttgat 180
   ggaaagctta ccgggaaaga aagtgggagc agaagacatt gaaaaaacaa taaaggcatg 240
   caaacccagt gaccagatcc tgaagctgct cagtttgtgg cgaataaaaa atggcgacca 300
   agacaccttg aagggcctaa tgcacgcact aaagcactca aagacgtacc actttcccaa 360
   aactgtcact cagagtctaa agaagaccat caggttcctt cacagc                406
<210> 70
   <211> 269
   <212> DNA
   <213> Homo sapiens
<400> 70
   ggaagaagct gtcatctgca tcctagtttg cctgaaatga acccaaataa tacccattat 60
   tattagtcct gaattatgag tagtgaatga tacccatcat tctggcatca tgatgagtag 120
   tgtccacttc cattctgaaa agtgccctgc tgtgaaaaat aaattatata gtcatcctag 180
   gtaaatgaag gaggagggag aagtgtgaaa gagtatggct taaatcagac aagatataca 240
   agaagatact ttatataggg caggagcgg                                   269
<210> 71
   <211> 479
   <212> DNA
   <213> Homo sapiens
<400> 71
   gtgcagtcat caccaagcag aatgggaagc tgtgcttggt gattcaggta gccaatatga 60
   ggaagagcct cttgattcag tgccagctct ctggcaagct cctgcagacc cacgtcacca 120
   aggaggggga gcggattctc ctcaaccaag ccactgtcaa attccacgtg gactcctcct 180
   ctgagggccc cttcctcatt ctgcccatga cattctacca tgtgctggat gagacgagcc 240
   ccctgagaga cctcacaccc caaaacctaa aggagaagga gtttgagctt gtggtcctcc 300
   tcaatgccac tgtggaatcc accagcgctg tctgccagag ccgaacatct tatatcccag 360
   aggaaatcta ctggggtttt gagtttgtgc ctgtggtatc tctctccaaa aatggaaaat 420
   atgtggctga tttcagtcag tttgaacaga ttcggaaaag cccagattgc acattttac  479
<210> 72
   <211> 416
   <212> DNA
   <213> Homo sapiens
<400> 72
   gagacttttc tatggttttg tgactttcaa cttttgtaca gttatgtgaa ggatgaaagg 60
   tgggtgaaag gaccaaaaac agaaatacag tcttcctgaa tgaatgacaa tcagaattcc 120
   actgcccaaa ggagtccaac aattaaatgg atttctagga aaagctacct taagaaaggc 180
   tggttaccat cggagtttac aaagtgcttt cacgttctta cttgttgtat tatacattca 240
   tgcatttcta ggctagagaa ccttctagat ttgatgctta caactattct gttgtgacta 300
   tgagaacatt tctgtctcta gaagttatct gtctgtattg atctttatgc tatattacta 360
   tctgtggtta cagtggagac attgacatta ttactggagt caagccctta taagtc     416
<210> 73
   <211> 515
   <212> DNA
   <213> Homo sapiens
<220>
   <221> misc_feature
   <222> 33, 34, 41, 431, 444
   <223> n = A,T,C or G
<400> 73
   gcctggcaag aatgcagtca ccctgcggaa ccnnaagggc ntttgtgaaa ctggccctgc 60
   gtcatggagc tgacctggtt cccatctact cctttggaga gaatgaagtg tacaagcagg 120
   tgatcttcga ggagggctcc tggggccgat gggtccagaa gaagttccag aaatacattg 180
   gtttcgcccc atgcatcttc catggtcgag gcctcttctc ctccgacacc tgggggctgg 240
   tgccctactc caagcccatc accactgttg tgggagagcc catcaccatc cccaagctgg 300
   agcacccaac ccagcaagac atcgacctgt accacaccat gtacatggag gccctggtga 360
   agctcttcga caagcacaag accaagttcg gcctcccgga gactgaggtc ctggaggtga 420
   actgagccag ncttcggggc caantccctg gaggaaccag ctgcaaatca cttttttgct 480
   ctgtaaattt ggaagtgtca tgggtgtctg tgggt                            515
<210> 74
   <211> 537
   <212> DNA
   <213> Homo sapiens
<400> 74
   tttttttttg gctgtgacct cttcaaaccg tggtaccccc ccttttctcc ccacgatgat 60
   atctatatat gtatctacaa tacatatatc tacacataca gaaagaagca gttctcacat 120
   gttgctagtt ttttgcttct ctttccccca ccctactccc tccaattccc cccttaaact 180
   tccaaagctt cgtcttgtgt ttgctgcaga gtgattcggg ggctgaccta gaccagtttg 240
   catgattctt ctcttgtgat ttggttgcac tttagacatt tttgtgccat tatatttgca 300
   ttatgtattt ataatttaaa tgatatttag gtttttggct gagtactgga ataaacagtg 360
   agcatatctg gtatatgtca ttatttattg ttaaattaca ttttttaagc tccatgtgca 420
   tataaaggtt atgaaacata tcatggtaat gacagatgca agttatttta tttgcttatt 480
   ttttataatt aaagatgcca tagcataata tgaagccttt ggtgaattcc ttctaag 537
<210> 75
   <211> 484
   <212> DNA
   <213> Homo sapiens
<400> 75
   aaactgcgtt aaaggctcga ttttgtatct gcaggcagac acggatctga gaatctttat 60
   tgagaaagag cacttaagag aatattttaa gtattgcatc tgtataagta agaaaatatt 120
   ttgtctaaaa tgcctcagtg tatttgtatt tttttgcaag tgaaggttta caatttacaa 180
   agtgtgtatt aaaaaaaacc caaagaaccc aaaaatctgc agaaggaaaa atgtgtaatt 240
   ttgttctagt tttcagtttg tatatacccg tacaacgtgt cctcacggtg ccttttttca 300
   cggaagtttt caatgatggg cgagcgtgca ccatcccttt ttgaagtgta ggcagacaca 360
   gggacttgaa gttgttacta actaaactct ctttgggaat gtttgtctca tcccattctg 420
   cgtcatgctt gtgtgataac tactccggag acagggtttg gctgtgtcta aactgcatta 480
   ccgc 484
<210> 76
   <211> 422
   <212> DNA
   <213> Homo sapiens
<400> 76
   aggtaagatg ttaatctccc agcccacctc aacccagagg ctactcttga cttagaccta 60
   tactgaaaga tctctgtcac atccaactgg aaattccagg aaccaaaaag agcatcccta 120
   tgggcttgga ccacttacag tgtgataagg cctactatac attaggaagt ggcagttctt 180
   tactcgtccc ctttcatcgg tgcctggtac tctggcaaat gatgatgggg tgggagactt 240
   tccattaaat caatcaggaa tgagtcaatc agcctttagg tctttagtcc gggggacttg 300
   gggctgagag agtataaata accctgggct gtccagcctt aatagacttc tcttacattt 360
   tcgtcctgta gcacgctgcc tgccaaagta gtcctggcag ctggaccatc tctgtaggat 420
   cg 422
<210> 77
   <211> 521
   <212> DNA
   <213> Homo sapiens
<400> 77
   atatcttctt caggctctga caggcctcct ggaaacttcc acatattttt caactgcagt 60
   ataaagtcag aaaataaagt taacataact ttcactaaca cacacatatg tagatttcac 120
   aaaatccacc tataattggt caaagtggtt gagaatatat tttttagtaa ttgcatgcaa 180
   aatttttcta gcttccatcc tttctccctc gtttcttctt tttttggggg agctggtaac 240
   tgatgaaatc ttttcccacc ttttctcttc aggaaatata agtggttttg tttggttaac 300
   gtgatacatt ctgtatgaat gaaacattgg agggaaacat ctactgaatt tctgtaattt 360
   aaaatatttt gctgctagtt aactatgaac agatagaaga atcttacaga tgctgctata 420
   aataagtaga aaatataaat ttcatcacta aaatatgcta ttttaaaatc tatttcctat 480
   attgtatttc taatcagatg tattactctt attatttcta t                      521
<210> 78
   <211> 533
   <212> DNA
   <213> Homo sapiens
<220>
   <221> misc_feature
   <222> 154, 305
   <223> n = A,T,C or G
<400> 78
   gctcatagtc cgtcaccgaa aatagaaaat gccatccata ggtaaaatgc tgacctatag 60
   aaaaaaatga actctacttt tatagcctag taaaaatgct ctacctgagt agttaaaagc 120
   aattcatgaa gcctgaagct aaagagcact ctgntggttt tggcataata gctgcatttc 180
   cagacctgac ctttggcccc aaccacaagt gctccaagcc ccaccagctg accaaagaaa 240
   gcccaagttc tccttctgtc cttcccacaa cctccctgct cccaaaacta tgaaattaat 300
   ttganccata ttaacacagc tgactcctcc agtttactta aggtagaaag aatgagttta 360
   caacagatga aaataagtgc tttgggcgaa ctgtattcct tttaacagat ccaaactatt 420
   ttacatttaa aaaaaaagtt aaactaaact tctttactgc tgatatgttt cctgtattct 480
   agaaaaattt ttacactttc acattatttt tgtacacttt ccccatgtta agg        533
<210> 79
   <211> 410
   <212> DNA
   <213> Homo sapiens
<400> 79
   atcacctcag ccaactgtgg ctcttaattt attgcataat gatatccaca tcagccaact 60
   gtggctcttt aatttattgc ataatgatat tcacatcccc tcagttgcag tgaattgtga 120
   gcaaaagatc ttgaaagcaa aaagcactaa ttagtttaaa atgtcacttt tttggttttt 180
   attatacaaa aaccatgaag tacttttttt atttgctaaa tcagattgtt cctttttagt 240
   gactcatgtt tatgaagaga gttgagttta acaatcctag cttttaaaag aaactattta 300
   atgtaaaata ttctacatgt cattcagata ttatgtatat cttctagcct ttattctgta 360
   cttttaatgt acatatttct gtcttgcgtg atttgtatat ttcactggtt             410
<210> 80
   <211> 285
   <212> DNA
   <213> Homo sapiens
<400> 80
   aagtgctcag accttgtcta gttatttata aactgtatgt acctccctca cttctctcct 60
   atcactgctt tcctactctc cttttatctc actctagtcc aggtgccaag aatttccctt 120
   ctaccctcta ttctcttgtg tctgtaagtt acaaagtcag gaaaagtctt ggctggaccc 180
   ctttcctgct gggtggatgc agtggtccag gactggggtc tgggcccagg tttgagggag 240
   aagtttgcag agcacttccc acctctctga atagtgtgta tgtgt                285
<210> 81
   <211> 376
   <212> DNA
   <213> Homo sapiens
<400> 81
   cctggttctg gaatcctcag ttcaaggtta tcctacgcag agagccaggt accaatgggt 60
   gcgctgcaat ccagacagta attctgcaaa ctgccttgaa gaaaaaggac caatgttcga 120
   actacttcca ggtgaatcca acaagatccc ccgtctgagg actgaccttt ttccaaagac 180
   gagaatccag gacttgaatc gtatcttccc actttctgag gactactctg gatcaggctt 240
   cggctccggc tccggctctg gatcaggatc tgggagtggc ttcctaacgg aaatggaaca 300
   ggattaccaa ctagtagacg aaagtgatgc tttccatgac aaccttaggt ctcttgacag 360
   gaatctgccc tcagac                                                376
<210> 82
   <211> 177
   <212> DNA
   <213> Homo sapiens
<400> 82
   gggagtttga ccagagatgc aaggggtgaa ggagcgcttc ctaccgttag ggaactctgg 60
   ggacagagcg ccccggccgc ctgatggccg aggcagggtg cgacccagga cccaggacgg 120
   cgtcgggaac cataccatgg cccggatccc caagacccta aagttcgtcg tcgtcat    177
<210> 83
   <211> 511
   <212> DNA
   <213> Homo sapiens
<400> 83
   gagaatccac cctgcaggat gttcagaggg acccactcct tcatttttca gagtcaaagg 60
   aatcagaggc tcacccatgg caggcagtga aaagagccag gagtcctggg ttctagtccc 120
   tgctctgccc ccaactggct gtataacctt tgaaaaatca ttttctttgt ctgagtctct 180
   ggttctccgt cagcaacagg ctggcataag gtcccctgca ggttccttct agctggagca 240
   ctcagagctt ccctgactgc tagcagcctc tctggccctc acagggctga ttgttctcct 300
   tctccctgga gctctctctc ctgaaaatct ccatcagagc aaggcagcca gagaagcccc 360
   tgagagggaa tgattgggaa gtgtccactt ctccaaccgg ctcatcaaac acactccttt 420
   gtctatgaat ggcacatgta aatgatgtta tattttgtat cttttatatc atatgcttca 480
   ccattctgta aagggcctct gcattgttgc t                               511
<210> 84
   <211> 422
   <212> DNA
   <213> Homo sapiens
<400> 84
   gtatgtggct ttctgtagcc gtggtaacgg ccaaactgtt catcctagct acccatgctc 60
   tgtgtccagg cttgctcctg gcaggtggca ttcatctcag atgtgagcac aaggcattgg 120
   ccctctggac tcctttctcc ttttctttcc tctctaggct gctcctgaat cctgttctct 180
   gacatccgtg gagcccctcc tgcatccacc tatgcctcct ataagtccag ttgaaatctc 240
   agcctccttc aacattttct tctcgtgtgt ggcccacatc cctccacttc tccaacttct 300
   gtttaatctg atcacggctc tttttaagcc ctggcagcat tttggtccct gctccttgcc 360
   catagtaaaa cagcttgaaa tatcccatgc aagagagtag tttcaagtgg gcgactctgc 420
   tc 422
<210> 85
   <211> 466
   <212> DNA
   <213> Homo sapiens
<400> 85
   ggtgctatgg tgcacacaac ctctcctccg agtggtcggc ccccagcgac cccctgaaca 60
   tcctgatggc aggacagatc tatgacaccg tctccctgtc agcacagccg ggccccacag 120
   tggcctcagg agagaacgtg accctgctgt gtcagtcatg gtggcagttt gacactttcc 180
   ttctgaccaa agaaggggca gcccatcccc cgctgcgtct gagatcaatg tacggagctc 240
   ataagtacca ggctgaattc cccatgagtc ctgtgacctc agcccacgcg gggacctaca 300
   ggtgctacgg ctcatacagc tccaaccccc acctgctgtc tttccccagt gagcccctgg 360
   aactcatggt ctcagcctca cacgccaagg attacacagt ggagaatctc atccgcatgg 420
   gcatggcagg cttggtcctg gtgttcctcg ggattctgtt atttga 466
<210> 86
   <211> 362
   <212> DNA
   <213> Homo sapiens
<220>
   <221> misc_feature
   <222> 90, 159, 160, 276
   <223> n = A,T,C or G
<400> 86
   cccacaccct tcctgggaaa cagatacgtc ccctgcccgg tgatgaccca ccatcaactg 60
   catcctccaa gctggattcc tagacatctn ttggtctcct ctcttcctca ctcccttccc 120
   ccagtcagtc ctgagtcctg ccgtcagact ctggcaggnn tcccagagga gcatcaactc 180
   caggagaaca gggatttcta tcaatgtcat tcactgctgc atccccagtg cctagaacag 240
   ggctggcagg tggtaggcgc ctgacagatg cttgtntgat gcctgaatgt ctccttaccc 300
   atgcccacgg cacaggatag atgtgctata gggcaaagaa ctttgagggt cgagcagcag 360
   gg 362
<210> 87
   <211> 450
   <212> DNA
   <213> Homo sapiens
<400> 87
   gctggtatca gaacagcttc cctcactgtg tacagaacgc aagaagggaa taggtggtct 60
   gaacgtggtg tctcactctg aaaagcagga atgtaagatg atgaaagaga caatgtaata 120
   ctgttggtcc aaaagcattt aaaatcaata gatctgggat tatgtggcct taggtagctg 180
   gttgtacatc tttccctaaa tcgatccatg ttaccacata gtagttttag tttaggattc 240
   agtaacagtg aagtgtttac tatgtgcaag ggtattgaag ttcttatgac cacagatcat 300
   cagtactgtt gtctcatgta atgctaaaac tgaaatggtc cgtgtttgca ttgttaaaaa 360
   tgatgtgtga aatagaatga gtgctatggt gttgaaaact gcagtgtccg ttatgagtgc 420
   caaaaatctg tcttgaaggc agctacactt 450
<210> 88
   <211> 533
   <212> DNA
   <213> Homo sapiens
<400> 88
   gcctcagggt cacacagggc aaacgacgct cagactcttg gcacataagt ctggagactg 60
   ctgtgggggt ggcagtggct gtcactgtgc tcggaatcat gattttggga ctgatctgcc 120
   tcctcaggtg gaggagaagg aaaggtcagc agcggactaa agccacaacc ccagccaggg 180
   aacccttcca aaacacagag gagccatatg agaatatcag gaatgaagga caaaatacag 240
   atcccaagct aaatcccaag gatgacggca tcgtctatgc ttcccttgcc ctctccagct 300
   ccacctcacc caaagcacct cccagccacc gtcccctcaa gagcccccag aacgagaccc 360
   tgtactctgt cttaaaggcc taaccaatgg acagccctct caaaactgaa tggtgaggcc 420
   aggtacagtg gcgcacacct gtaatcccag ctactctgaa gcctgaggca aaatcaagtg 480
   agcccaggag ttcagggcca gctttggata atgggagcga ggatgccatc tct 533
<210> 89
   <211> 478
   <212> DNA
   <213> Homo sapiens
<400> 89
   ccccctggac atcctgatca caggacagtt ctatgacaga ccctctctct cggtgcagcc 60
   ggtccccaca gtagccccag gaaagaacgt gaccctgctg tgtcagtcac gggggcagtt 120
   ccacactttc cttctgacca aggagggggc aggccatccc ccactgcatc tgagatcaga 180
   gcaccaagct cagcagaacc aggctgaatt ccgcatgggt cctgtgacct cagcccacgt 240
   ggggacctac agatgctaca gctcactcag ctccaacccc tacctgctgt ctctccccag 300
   tgaccccctg gagctcgtgg tctcagaagc agctgagacc ctcagcccat cacaaaacaa 360
   gacagactcc acgactacat ccctaggcca acacccccag gattacacag tggagaatct 420
   catccgcatg ggtgtggctg gcttggtcct ggtggtcctc gggattctgc tatttgag 478
<210> 90
   <211> 495
   <212> DNA
   <213> Homo sapiens
<400> 90
   ggaaatgtgc atataacgta caatggggat attattcagc ctaaaaaaag gggggatcct 60
   gttatttatg acaacatgaa taaacccgga ggccattatg ctatgtaaaa tgagcaagta 120
   acagaaagac aaatactgcc tgatttcatt tatatgaggt tctaaaatag tcaaactcat 180
   agaagcagag aatagaacag tggttcctag ggaaaaggag gaagggagaa atgaggaaat 240
   agggagttgt ctaattggta taaaattata gtatgcaaga tgaattagct ctaaagatca 300
   gctgtatagc agagttcgta taatgaacaa tactgtatta tgcacttaac attttgttaa 360
   gagggtacct ctcatgttaa gtgttcttac catatacata tacacaagga agcttttgga 420
   ggtgatggat atatttatta ccttgattgt ggtgatggtt tgacaggtat gtgactatgt 480
   ctaaactcat caaat 495
<210> 91
   <211> 437
   <212> DNA
   <213> Homo sapiens
<220>
   <221> misc_feature
   <222> 186, 199, 205, 217, 338, 353, 375, 376, 399
   <223> n = A,T,C or G
<400> 91
   ggatgccatg aatcgagact gtggaattcc actcagtcat ttgcaggttg atggaggaat 60
   gaccagcaac aaaattctta tgcagctaca agcagacatt ctgtatattc cagtagtgaa 120
   gcccttgatg cccgaaacca ctgcactggg tgctgccatg gcggcagggg ctgcagaagg 180
   agtcgncgta tggagtctng aaccngagga tttgtcngcc gtcacgatgg agcggtttga 240
   acctcagatt aatgctgagg aaagtgaaat tcgttattct acatggaaga aagctgtgat 300
   gaagtcaatg ggttgggtta caactcaatc tccagaangt ggtgacccta gtntcttctg 360
   tagtctgccc ttggnntttt ttatagtgag tagcatggna atgttaatcg gagcaaggta 420
   catctcaggt attccat                                                437
<210> 92
   <211> 360
   <212> DNA
   <213> Homo sapiens
<400> 92
   gcaataactc tgggaggggc tcgagagggc tggtccttat ttatttaact tcacccgagt 60
   tcctctgggt ttctaagcag ttatggtgat gacttagcgt caagacattt gctgaactca 120
   gcacattcgg gaccaatata tagtgggtac atcaagtcca tctgacaaaa tggggcagaa 180
   gagaaaggac tcagtgtgtg atccggtttc tttttgctcg cccctgtttt ttgtagaatc 240
   tcttcatgct tgacatacct accagtatta ttcccgacga cacatataca tatgagaata 300
   taccttattt atttttgtgt aggtgtctgc cttcacaaat gtcattgtct actcctagaa 360
<210> 93
   <211> 488
   <212> DNA
   <213> Homo sapiens
<400> 93
   tagctggtgc tgttattcgc tggctaagag acaatcttgg aattataaag acctcagaag 60
   aaattgaaaa acttgctaaa gaagtaggta cttcttatgg ctgctacttc gtcccagcat 120
   tttcggggtt atatgcacct tattgggagc ccagcgcaag agggataatc tgtggactca 180
   ctcagttcac caataaatgc catattgctt ttgctgcatt agaagctgtt tgtttccaaa 240
   ctcgagagat tttggatgcc atgaatcgag actgtggaat tccactcagt catttgcagg 300
   tagatggagg aatgaccagc aacaaaattc ttatgcagct acaagcagac attctgtata 360
   taccagtagt gaagccctca atgcccgaaa ccactgcact gggtcggcta tggcggcagg 420
   ggctgcagaa ggagtcggcg tatggagtct cgaacccgag gatttgtctg ccgtcacgat 480
   ggagcggt                                                          488
<210> 94
   <211> 437
   <212> DNA
   <213> Homo sapiens
<400> 94
   tactttgttt acctcggtgc ttacaacaga atgctgccct acatcgtcat gggtagtctg 60
   actgtcctga ttggaatctt cacccttttt ttccctgaaa gtttgggaat gactcttcca 120
   gaaaccttag agcagatgca gaaagtgaaa tggttcagat ctgggaaaaa aacaagagac 180
   tcaatggaga cagaagaaaa tcccaaggtt ctaataactg cattctgaaa aaatatctac 240
   cccatttggt gaagtgaaaa acagaaaaat aagaccctgt ggagaaattc gttgttccca 300
   ctgaaatgga ctgactgtaa cgattgacac caaaatgaac cttgctatca agaaatgctc 360
   gtcatacagt aaactctgga tgattcttcc agataatgtc cttgctttac aaaccaacca 420
   tttctagaga gtctcct                                                437
<210> 95
   <211> 416
   <212> DNA
   <213> Homo sapiens
<400> 95
   actacgtatt cattgtctac ctgctaagtc aagggttcac tgcatttctc cttcctcaga 60
   atacactctg gacctgtgct gtctaatatg gtagccacta acaaaatgtg gccattttga 120
   tggaaatcca ttaaaatcaa agaaaattta aaactcaatt cctcaattgg gctggccaca 180
   tttcaaatgc tcactaccca cctgtggcta gggtctactt ctactgtatt ggcgagcatg 240
   gatatagaac atttcatcac agttctgttg gaccaatcta gatagattca ttatcccatc 300
   tagagaagag actttagtca ctgtcttctt gcttcagacc catctatatt taaaacaaat 360
   ttccctaaat cctgagactg agacggagct acaaattctc agatggcgac cgtgta     416
<210> 96
   <211> 538
   <212> DNA
   <213> Homo sapiens
<400> 96
   agaaagactg atttccagcg tcctgcaggg gctgcggaga cagagcccaa ggacaggggc 60
   ctgctgagga ggtccagccc agctgctgac gtccaggaag aaaacctcta gcccacacga 120
   tgaagacccc caggcagtga cgtatgcccc ggtgaaacac tccagtccta ggagagaaat 180
   ggcctctcct ccctcctcac tgtctgggga attcctggac acaaaggaca gacaggtgga 240
   agaggacagg cagatggaca ctgaggctgc tgcatctgaa gcctcccagg atgtgaccta 300
   cgcccagctg cacagcttga cccttagacg gaaggcaact gagcctcctc catcccagga 360
   aggggaacct ccagctgagc ccagcatcta cgccactctg gccatccact agcccggggg 420
   gtacgcagac cccacactca gcagaaggag actcaggact gctgaaggca cgggagctgc 480
   ccccagtgga caccagtgaa ccccagtcag cctggacccc taacacagac catgagga   538
<210> 97
   <211> 538
   <212> DNA
   <213> Homo sapiens
<220>
   <221> misc_feature
   <222> 493, 494, 495, 498, 499, 500, 501
   <223> n = A,T,C or G
<400> 97
   tacttcgtcc cagcattttc ggggttatat gcaccttatt gggagcccag cgcaagaggg 60
   ataatctgtg gactcactca gttcaccaat aaatgccata ttgcttttgc tgcattagaa 120
   gctgtttgtt tccaaactcg agagattttg gatgccatga atcgagactg tggaattcca 180
   ctcagtcatt tgcaggtaga tggaggaatg accagcaaca aaattcttat gcagctacaa 240
   gcagacattc tgtatatacc agtagtgaag ccctcaatgc ccgaaaccac tgcactgggt 300
   gcggctatgg cggcaggggc tgcagaagga gtcggcgtat ggagtctcga acccgaggat 360
   ttgtctgccg tcacgatgga gcggtttgaa cctcagatta atgcggagga aagtgaaatt 420
   cgttattcta catggaagaa agctgtgatg aagtcaatgg gttgggttac aactcaatct 480
   ccagaaagtg gtnnnccnnn natcttctgt agtctgccct tgggcttttt tatagtga 538
<210> 98
   <211> 311
   <212> DNA
   <213> Homo sapiens
<400> 98
   gggagttgtg gctgctgctg agaaaaccaa acagggtgtg gcagaagcag caggaaagac 60
   aaaagagggt gttctctatg tagtggctga gaagaccaaa gagcaagtga caaatgttgg 120
   aggagcagtg gtgacgggtg tgacagcagt agcccagaag acagtggagg gagcagggag 180
   cattgcagca gccactggct ttgtcaaaaa ggaccagttg ggcaagaatg aagaaggagc 240
   cccacaggaa ggaattctgg aagatatgcc tgtggatcct gacaatgagg cttatgaaat 300
   gccttctgag g 311
<210> 99
   <211> 253
   <212> DNA
   <213> Homo sapiens
<400> 99
   gccagatttc tgctttttgg aagaagatcc tggaatatgt cgaggttata ttaccaggta 60
   tttttataac aatcagacaa aacagtgtga acgtttcaag tatggtggat gcctgggcaa 120
   tatgaacaat tttgagacac tggaagaatg caagaacatt tgtgaagatg gtccgaatgg 180
   tttccaggtg gataattatg gaacccagct caatgctgtg aataactccc tgactccgca 240
   atcaaccaag gtt 253
<210> 100
   <211> 528
   <212> DNA
   <213> Homo sapiens
<220>
   <221> misc_feature
   <222> 105, 120, 123, 439
   <223> n = A,T,C or G
<400> 100
   actggttttg caatatagag atcatgtggt aaagaatttt aataagatct tatactaaaa 60
   agccttaaat caatttattg agattcaaaa aatactatta taatnaatta catcccatan 120
   atntaggcaa actcatttaa aaaataaaac taattttggt aaaagtacat ggcctttgtt 180
   tttaaaatac ataattttaa aataaatcac ttgtcatgat aaagtccaaa aagaagttat 240
   cattcaacat tcaactaagg ttggagctaa gaatttacta atacaaaaaa agttaaaatt 300
   ttttggacca tatatatctt gacagtgtaa cttttaagta ggttcatttc catttgcaca 360
   gaaagtttct gtctttagga aactgaaaat gaaatactgt ggatgctatg actgtttgtc 420
   ttgtatgtaa ataggaaant aataagctgc ctattgagtg gtatagctgt atgcttaccc 480
   aaaaaaggga acactgtggt tatgacttgt attataaact ttctgtag 528
<210> 101
   <211> 443
   <212> DNA
   <213> Homo sapiens
<400> 101
   tctcaagccc atccattatt tcttaacttt tattttagct ttcgggggta catgttaaag 60
   gctttttata taggtaaact catgtcgtgg aggtttgttg tacagattat ttcatcaccc 120
   aggtattaag cccagtgcct aatattgttt ttttcggctc ctctccctcc tcctaccttc 180
   cgccctcaag tagactccag tgtctgttat tcccttcttt gtgtttatga attctcatca 240
   tttagctccc acttataagt gaggacatgc agtatttggt tttctgttcc catgtttgct 300
   aaggataatg gtttccagtt ctaccgatgt tcccacaaaa gacataattt tcttttttaa 360
   ggctgcttag tattccatgg tatctatgta tcacattttc tctatccaat ctattgttga 420
   ctcacattta gattgattcc atg                                         443
<210> 102
   <211> 389
   <212> DNA
   <213> Homo sapiens
<400> 102
   cctcccaact acgagatgct caaggaggag caggaagtgg ctatgctggg ggggccccac 60
   aaccctgctc ccccgacgtc caccgtgatc cacatccgca gcgagacctc cgtgcctgac 120
   catgtcgtct ggtccctgtt caacaccctc ttcatgaaca cctgctgcct gggcttcata 180
   gcattcgcct actccgtgaa gtctagggac aggaagatgg ttggcgacgt gaccggggcc 240
   caggcctatg cctccaccgc caagtgcctg aacatctggg ccctgatttt gggcatcttc 300
   atgaccattc tgctcgtcat catcccagtg ttggtcgtcc aggcccagcg atagatcagg 360
   aggcatcatt gaggccagga gctctgccc                                   389
<210> 103
   <211> 116
   <212> DNA
   <213> Homo sapiens
<400> 103
   tacagccagg cataacatat ccactgtgtg catagagggt ctcttcacgt tgatgcttgg 60
   cattccatca gctttctcta agtctttgct caagttcaac cttaaaatga tgttag     116
<210> 104
   <211> 527
   <212> DNA
   <213> Homo sapiens
<400> 104
   tcatgtagcc cctcaacaga atgcattgca tcatcaccat ggtaacagtt cccatcatca 60
   ccaccaccac caccaccatc accaccacca tggacaacaa gccttgggta accggaccag 120
   gccaagggtc tacaattctc caacgaatag ctcctctacc caagattcta tggaggttgg 180
   ccacagtcac cactccatga catccctgtc ttcctcaacg acttcttcct cgacatcttc 240
   ctcctctact ggtaaccaag gcaatcaggc ctaccacaat cgcccagtgg ctgctaatac 300
   cttggacttt ggacagaatg gagctatgga cgttaatttg accgtctact ccaatccccg 360
   ccaagagact ggcatagctg gacatccaac ataccaattt tctgctaata caggtcctgc 420
   acattacatg actgaaggac atctgacgat gaggcaaggg gctgatagag aagagtcccc 480
   catgacagga gtttgtgtgc aacagagtcc tgtagctagc tcgtgac               527
<210> 105
   <211> 422
   <212> DNA
   <213> Homo sapiens
<220>
   <221> misc_feature
   <222> 264
   <223> n = A,T,C or G
<400> 105
   gtggcacttt gattttaggc tgggcaggca gaagtctcac agtaaaatat atttcactag 60
   acgtccaaac caacactgca ttgtagtaac ctcttctgaa gtaactggtt aaattaataa 120
   ctggtcccat aagtcagggt caagccacat ttataaccca ttcgggccgg ccatgaaggg 180
   ttctgactcg acattggtat ttaaatgctg ttactttccc aatgcttagt agcacttgag 240
   actttatttc catcgacaaa aacnaagaag gccacagaag tagcctaaca gtcctccctg 300
   gtcatactcg tttcaaacag gtctgcctgt ctagacatat tcaggcagga ggttgcttgc 360
   tgctatcttg aaaaccactg gaagattcag cttcttgggg acctcagagc ccatattcat 420
   at                                                                422
<210> 106
   <211> 524
   <212> DNA
   <213> Homo sapiens
<400> 106
   aaaaccatgc tttccttgat ttctcctcat gtcaagacat ttcttacttc tctagtctcc 60
   tagcattttg tgcctcacaa ccctcaggac aggccagcta gtgtatggct gtgggttttt 120
   atctcacctc ccctgcctga ccctgagccc ttgtggggag tatactcacc ctactcctac 180
   agtgccttgc attccgtagc tgctcagtac attaacccat tcaatgtctt taagattttt 240
   acaagttagt tttcctgtaa ttactaatca tttatcttta attctgagta aaattcacaa 300
   caacaaataa aaggaaatag tagtaatttt ttaagctgtt tagtcaataa agatttaatg 360
   cgtcagttat tggactgttt ggtttgaagc caatcaacca ttctggtatg taaatctact 420
   tttttattat acaagtaaca caaaatgtat tgcctcagtc aaggaagaag aacagaaaaa 480
   aacaacaccc caaaatcctg tcacctctgt aaaactgcta ttaa                  524
<210> 107
   <211> 384
   <212> DNA
   <213> Homo sapiens
<400> 107
   gagtcatttt cataagatca attcattgat ttgttttttg taaagctatc attcaatata 60
   tattataaat taatataaat ttaagggaag ctctatgtaa ggagacttag agccaaactg 120
   tttaagctgt atcatcccaa caaagtatcc tttcatgaac ggggcatgca atagcttaag 180
   aattgctagg attaaattaa ggaaagtaaa gctactcaga gcaacaggtt ccacaagcac 240
   aaactttaca catttgtaca attttgaaat gcactacaat aaacaaatta gagcaacaca 300
   tttgaaatac aggcttcttt acataaactg agaggttata caaaactcag tttcacaagg 360
   gaacaatcta tacctttcta aaag                                        384
<210> 108
   <211> 492
   <212> DNA
   <213> Homo sapiens
<220>
   <221> misc_feature
   <222> 242
   <223> n = A,T,C or G
<400> 108
   aattcttcta gaggcttgtg tacactactg gttgttttag ttgggccttt tatatgaaag 60
   ttatactgta cttatctttt tgttgttgtt taggtttgtt gactaccatt tctggcttcc 120
   tttctttggt tttggattaa atccaacagt ttatttgtaa gccctgcagc agatttcttt 180
   gataatttag cttttactga atctcttgca atgaagaaag ctatttcatc agtgatttat 240
   cnctttcaat tcattgtgtg agctggaaat attattttat atgagagcta tagcaaaata 300
   atctgtataa acaaggaatg tgttagctta aactggatca ttttactttt tggcatcatg 360
   catctgtact gtaccaaaag tgtttatatg tctgcaaatt aaaggtatat attttcataa 420
   tttctttctc acttttagca ttttatattt gaacatgggc ttgttatttc actgaagtgc 480
   ctgtcatttg tt                                                     492
<210> 109
   <211> 502
   <212> DNA
   <213> Homo sapiens
<220>
   <221> misc_feature
   <222> 371
   <223> n = A,T,C or G
<400> 109
   tttcctacca atttcacatt ttgcagaaac ttgttcacat ttccaacaat atcagaatta 60
   gaaaacagtt cagataacaa gaaagattaa aaattaggga aattctgata tcaccataaa 120
   gcactatttt acatttagag attacattta agataaagtc atcatacaca aaaacaataa 180
   atatttataa ctttctctat aaggtccgca tatactgtat atattgaaac aatctgaatg 240
   actagtagat ttcatatgac cattgttatt tccactttct ccaatacttg tattttatgc 300
   tacatgtaat gaagttggac ctttttatta tttagtaatt cctatatgtt cctatacttt 360
   tcattttcaa natgattgct ctattgtttc atgttgtttc tagcaatata tctccatgag 420
   atatgcactt tgtttcatat tgaaaagtat aaaatttatc tttcaattcc tgtgtgtgta 480
   tcctatggtt atctgtatgt at                                          502
<210> 110
   <211> 258
   <212> PRT
   <213> Homo sapiens
<400> 110
   Met Ile Ala Ile Ser Ala Val Ser Ser Ala Leu Leu Phe Ser Leu Leu
    1               5                  10                  15
   Cys Glu Ala Ser Thr Val Val Leu Leu Asn Ser Thr Asp Ser Ser Pro
               20                  25                  30
   Pro Thr Asn Asn Phe Thr Asp Ile Glu Ala Ala Leu Lys Ala Gln Leu
           35                  40                  45
   Asp Ser Ala Asp Ile Pro Lys Ala Arg Arg Lys Arg Tyr Ile Ser Gln
       50                  55                  60
   Asn Asp Met Ile Ala Ile Leu Asp Tyr His Asn Gln Val Arg Gly Lys
   65                  70                  75                  80
   Val Phe Pro Pro Ala Ala Asn Met Glu Tyr Met Val Trp Asp Glu Asn
                   85                  90                  95
   Leu Ala Lys Ser Ala Glu Ala Trp Ala Ala Thr Cys Ile Trp Asp His
               100                 105                 110
   Gly Pro Ser Tyr Leu Leu Arg Phe Leu Gly Gln Asn Leu Ser Val Arg
           115                 120                 125
   Thr Gly Arg Tyr Arg Ser Ile Leu Gln Leu Val Lys Pro Trp Tyr Asp
       130                 135                 140
   Glu Val Lys Asp Tyr Ala Phe Pro Tyr Pro Gln Asp Cys Asn Pro Arg
   145                 150                 155                 160
   Cys Pro Met Arg Cys Phe Gly Pro Met Cys Thr His Tyr Thr Gln Met
                   165                 170                 175
   Val Trp Ala Thr Ser Asn Arg Ile Gly Cys Ala Ile His Thr Cys Gln
               180                 185                 190
   Asn Met Asn Val Trp Gly Ser Val Trp Arg Arg Ala Val Tyr Leu Val
           195                 200                 205
   Cys Asn Tyr Ala Pro Lys Gly Asn Trp Ile Gly Glu Ala Pro Tyr Lys
       210                 215                 220
   Val Gly Val Pro Cys Ser Ser Cys Pro Pro Ser Tyr Gly Gly Ser Cys
   225                 230                 235                 240
   Thr Asp Asn Leu Cys Phe Pro Gly Val Thr Ser Asn Tyr Leu Tyr Trp
                   245                 250                 255
   Phe Lys
<210> 111
   <211> 491
   <212> PRT
   <213> Homo sapiens
<400> 111
<210> 112
   <211> 265
   <212> PRT
   <213> Homo sapiens
<400> 112
   Met Asn Met Ser Val Leu Thr Leu Gln Glu Tyr Glu Phe Glu Lys Gln
    1              5                   10                  15
   Phe Asn Glu Asn Glu Ala Ile Gln Trp Met Gln Glu Asn Trp Lys Lys
               20                  25                  30
   Ser Phe Leu Phe Ser Ala Leu Tyr Ala Ala Phe Ile Phe Gly Gly Arg
           35                  40                  45
   His Leu Met Asn Lys Arg Ala Lys Phe Glu Leu Arg Lys Pro Leu Val
       50                  55                  60
   Leu Trp Ser Leu Thr Leu Ala Val Phe Ser Ile Phe Gly Ala Leu Arg
   65                  70                  75                  80
   Thr Gly Ala Tyr Met Val Tyr Ile Leu Met Thr Lys Gly Leu Lys Gln
                   85                  90                  95
   Ser Val Cys Asp Gln Gly Phe Tyr Asn Gly Pro Val Ser Lys Phe Trp
               100                 105                 110
   Ala Tyr Ala Phe Val Leu Ser Lys Ala Pro Glu Leu Gly Asp Thr Ile
           115                 120                 125
   Phe Ile Ile Leu Arg Lys Gln Lys Leu Ile Phe Leu His Trp Tyr His
       130                 135                 140
   His Ile Thr Val Leu Leu Tyr Ser Trp Tyr Ser Tyr Lys Asp Met Val
   145                 150                 155                 160
   Ala Gly Gly Gly Trp Phe Met Thr Met Asn Tyr Gly Val His Ala Val
                   165                 170                 175
   Met Tyr Ser Tyr Tyr Ala Leu Arg Ala Ala Gly Phe Arg Val Ser Arg
               180                 185                 190
   Lys Phe Ala Met Phe Ile Thr Leu Ser Gln Ile Thr Gln Met Leu Met
           195                 200                 205
   Gly Cys Val Val Asn Tyr Leu Val Phe Cys Trp Met Gln His Asp Gln
       210                 215                 220
   Cys His Ser His Phe Gln Asn Ile Phe Trp Ser Ser Leu Met Tyr Leu
   225                 230                 235                 240
   Ser Tyr Leu Val Leu Phe Cys His Phe Phe Phe Glu Ala Tyr Ile Gly
                   245                 250                 255
   Lys Met Arg Lys Thr Thr Lys Ala Glu
               260                 265
<210> 113
   <211> 698
   <212> PRT
   <213> Homo sapiens
<400> 113
   Met Gln Ala His Glu Leu Phe Arg Tyr Phe Arg Met Pro Glu Leu Val
    1               5                  10                  15
   Asp Phe Arg Gln Tyr Val Arg Thr Leu Pro Thr Asn Thr Leu Met Gly
               20                  25                  30
   Phe Gly Ala Phe Ala Ala Leu Thr Thr Phe Trp Tyr Ala Thr Arg Pro
           35                  40                  45
   Lys Pro Leu Lys Pro Pro Cys Asp Leu Ser Met Gln Ser Val Glu Val
       50                  55                  60
   Ala Gly Ser Gly Gly Ala Arg Arg Ser Ala Leu Leu Asp Ser Asp Glu
   65                  70                  75                  80
   Pro Leu Val Tyr Phe Tyr Asp Asp Val Thr Thr Leu Tyr Glu Gly Phe
                   85                  90                  95
   Gln Arg Gly Ile Gln Val Ser Asn Asn Gly Pro Cys Leu Gly Ser Arg
               100                 105                 110
   Lys Pro Asp Gln Pro Tyr Glu Trp Leu Ser Tyr Lys Gln Val Ala Glu
           115                 120                 125
   Leu Ser Glu Cys Ile Gly Ser Ala Leu Ile Gln Lys Gly Phe Lys Thr
       130                 135                 140
   Ala Pro Asp Gln Phe Ile Gly Ile Phe Ala Gln Asn Arg Pro Glu Trp
   145                 150                 155                 160
   Val Ile Ile Glu Gln Gly Cys Phe Ala Tyr Ser Met Val Ile Val Pro
                   165                 170                 175
   Leu Tyr Asp Thr Leu Gly Asn Glu Ala Ile Thr Tyr Ile Val Asn Lys
               180                 185                 190
   Ala Glu Leu Ser Leu Val Phe Val Asp Lys Pro Glu Lys Ala Lys Leu
           195                 200                 205
   Leu Leu Glu Gly Val Glu Asn Lys Leu Ile Pro Gly Leu Lys Ile Ile
       210                 215                 220
   Val Val Met Asp Ala Tyr Gly Ser Glu Leu Val Glu Arg Gly Gln Arg
   225                 230                 235                 240
   Cys Gly Val Glu Val Thr Ser Met Lys Ala Met Glu Asp Leu Gly Arg
                   245                 250                 255
   Ala Asn Arg Arg Lys Pro Lys Pro Pro Ala Pro Glu Asp Leu Ala Val
               260                 265                 270
   Ile Cys Phe Thr Ser Gly Thr Thr Gly Asn Pro Lys Gly Ala Met Val
           275                 280                 285
   Thr His Arg Asn Ile Val Ser Asp Cys Ser Ala Phe Val Lys Ala Thr
       290                 295                 300
   Glu Asn Thr Val Asn Pro Cys Pro Asp Asp Thr Leu Ile Ser Phe Leu
   305                 310                 315                 320
   Pro Leu Ala His Met Phe Glu Arg Val Val Glu Cys Val Met Leu Cys
                   325                 330                 335
   His Gly Ala Lys Ile Gly Phe Phe Gln Gly Asp Ile Arg Leu Leu Met
               340                 345                 350
   Asp Asp Leu Lys Val Leu Gln Pro Thr Val Phe Pro Val Val Pro Arg
           355                 360                 365
   Leu Leu Asn Arg Met Phe Asp Arg Ile Phe Gly Gln Ala Asn Thr Thr
       370                 375                 380
   Leu Lys Arg Trp Leu Leu Asp Phe Ala Ser Lys Arg Lys Glu Ala Glu
   385                 390                 395                 400
   Leu Arg Ser Gly Ile Ile Arg Asn Asn Ser Leu Trp Asp Arg Leu Ile
                   405                 410                 415
   Phe His Lys Val Gln Ser Ser Leu Gly Gly Arg Val Arg Leu Met Val
               420                 425                 430
   Thr Gly Ala Ala Pro Val Ser Ala Thr Val Leu Thr Phe Leu Arg Ala
           435                 440                 445
   Ala Leu Gly Cys Gln Phe Tyr Glu Gly Tyr Gly Gln Thr Glu Cys Thr
       450                 455                 460
   Ala Gly Cys Cys Leu Thr Met Pro Gly Asp Trp Thr Ala Gly His Val
   465                 470                 475                 480
   Gly Ala Pro Met Pro Cys Asn Leu Ile Lys Leu Val Asp Val Glu Glu
                   485                 490                 495
   Met Asn Tyr Met Ala Ala Glu Gly Glu Gly Glu Val Cys Val Lys Gly
               500                 505                 510
   Pro Asn Val Phe Gln Gly Tyr Leu Lys Asp Pro Ala Lys Thr Ala Glu
           515                 520                 525
   Ala Leu Asp Lys Asp Gly Trp Leu His Thr Gly Asp Ile Gly Lys Trp
       530                 535                 540
   Leu Pro Asn Gly Thr Leu Lys Ile Ile Asp Arg Lys Lys His Ile Phe
   545                 550                 555                 560
   Lys Leu Ala Gln Gly Glu Tyr Ile Ala Pro Glu Lys Ile Glu Asn Ile
                   565                 570                 575
   Tyr Met Arg Ser Glu Pro Val Ala Gln Val Phe Val His Gly Glu Ser
               580                 585                 590
   Leu Gln Ala Phe Leu Ile Ala Ile Val Val Pro Asp Val Glu Thr Leu
           595                 600                 605
   Cys Ser Trp Ala Gln Lys Arg Gly Phe Glu Gly Ser Phe Glu Glu Leu
       610                 615                 620
   Cys Arg Asn Lys Asp Val Lys Lys Ala Ile Leu Glu Asp Met Val Arg
   625                 630                 635                 640
   Leu Gly Lys Asp Ser Gly Leu Lys Pro Phe Glu Gln Val Lys Gly Ile
                   645                 650                 655
   Thr Leu His Pro Glu Leu Phe Ser Ile Asp Asn Gly Leu Leu Thr Pro
               660                 665                 670
   Thr Met Lys Ala Lys Arg Pro Glu Leu Arg Asn Tyr Phe Arg Ser Gln
           675                 680                 685
   Ile Asp Asp Leu Tyr Ser Thr Ile Lys Val
       690                 695
<210> 114
   <211> 698
   <212> PRT
   <213> Homo sapiens
<400> 114
   Met Gln Ala His Glu Leu Phe Arg Tyr Phe Arg Met Pro Glu Leu Val
    1               5                   10                  15
   Asp Phe Arg Gln Tyr Val Arg Thr Leu Pro Thr Asn Thr Leu Met Gly
               20                  25                  30
   Phe Gly Ala Phe Ala Ala Leu Thr Thr Phe Trp Tyr Ala Thr Arg Pro
           35                  40                  45
   Lys Pro Leu Lys Pro Pro Cys Asp Leu Ser Met Gln Ser Val Glu Val
       50                  55                  60
   Ala Gly Ser Gly Gly Ala Arg Arg Ser Ala Leu Leu Asp Ser Asp Glu
   65                  70                  75                  80
   Pro Leu Val Tyr Phe Tyr Asp Asp Val Thr Thr Leu Tyr Glu Gly Phe
                   85                  90                  95
   Gln Arg Gly Ile Gln Val Ser Asn Asn Gly Pro Cys Leu Gly Ser Arg
               100                 105                 110
   Lys Pro Asp Gln Pro Tyr Glu Trp Leu Ser Tyr Lys Gln Val Ala Glu
           115                 120                 125
   Leu Ser Glu Cys Ile Gly Ser Ala Leu Ile Gln Lys Gly Phe Lys Thr
       130                 135                 140
   Ala Pro Asp Gln Phe Ile Gly Ile Phe Ala Gln Asn Arg Pro Glu Trp
   145                 150                 155                 160
   Val Ile Ile Glu Gln Gly Cys Phe Ala Tyr Ser Met Val Ile Val Pro
                   165                 170                 175
   Leu Tyr Asp Thr Leu Gly Asn Glu Ala Ile Thr Tyr Ile Val Asn Lys
               180                 185                 190
   Ala Glu Leu Ser Leu Val Phe Val Asp Lys Pro Glu Lys Ala Lys Leu
           195                 200                 205
   Leu Leu Glu Gly Val Glu Asn Lys Leu Ile Pro Gly Leu Lys Ile Ile
       210                 215                 220
   Val Val Met Asp Ala Tyr Gly Ser Glu Leu Val Glu Arg Gly Gln Arg
   225                 230                 235                 240
   Cys Gly Val Glu Val Thr Ser Met Lys Ala Met Glu Asp Leu Gly Arg
                   245                 250                 255
   Ala Asn Arg Arg Lys Pro Lys Pro Pro Ala Pro Glu Asp Leu Ala Val
               260                 265                 270
   Ile Cys Phe Thr Ser Gly Thr Thr Gly Asn Pro Lys Gly Ala Met Val
           275                 280                 285
   Thr His Arg Asn Ile Val Ser Asp Cys Ser Ala Phe Val Lys Ala Thr
       290                 295                 300
   Glu Asn Thr Val Asn Pro Cys Pro Asp Asp Thr Leu Ile Ser Phe Leu
   305                 310                 315                 320
   Pro Leu Ala His Met Phe Glu Arg Val Val Glu Cys Val Met Leu Cys
                   325                 330                 335
   His Gly Ala Lys Ile Gly Phe Phe Gln Gly Asp Ile Arg Leu Leu Met
               340                 345                 350
   Asp Asp Leu Lys Val Leu Gln Pro Thr Val Phe Pro Val Val Pro Arg
           355                 360                 365
   Leu Leu Asn Arg Met Phe Asp Arg Ile Phe Gly Gln Ala Asn Thr Thr
       370                 375                 380
   Leu Lys Arg Trp Leu Leu Asp Phe Ala Ser Lys Arg Lys Glu Ala Glu
   385                 390                 395                 400
   Leu Arg Ser Gly Ile Ile Arg Asn Asn Ser Leu Trp Asp Arg Leu Ile
                   405                 410                 415
   Phe His Lys Val Gln Ser Ser Leu Gly Gly Arg Val Arg Leu Met Val
               420                 425                 430
   Thr Gly Ala Ala Pro Val Ser Ala Thr Val Leu Thr Phe Leu Arg Ala
           435                 440                 445
   Ala Leu Gly Cys Gln Phe Tyr Glu Gly Tyr Gly Gln Thr Glu Cys Thr
       450                 455                 460
   Ala Gly Cys Cys Leu Thr Met Pro Gly Asp Trp Thr Ala Gly His Val
   465                 470                 475                 480
   Gly Ala Pro Met Pro Cys Asn Leu Ile Lys Leu Val Asp Val Glu Glu
                   485                 490                 495
   Met Asn Tyr Met Ala Ala Glu Gly Glu Gly Glu Val Cys Val Lys Gly
               500                 505                 510
   Pro Asn Val Phe Gln Gly Tyr Leu Lys Asp Pro Ala Lys Thr Ala Glu
           515                 520                 525
   Ala Leu Asp Lys Asp Gly Trp Leu His Thr Gly Asp Ile Gly Lys Trp
               530                 535                 540
   Leu Pro Asn Gly Thr Leu Lys Ile Ile Asp Arg Lys Lys His Ile Phe
   545                 550                 555                 560
   Lys Leu Ala Gln Gly Glu Tyr Ile Ala Pro Glu Lys Ile Glu Asn Ile
                   565                 570                 575
   Tyr Met Arg Ser Glu Pro Val Ala Gln Val Phe Val His Gly Glu Ser
               580                 585                 590
   Leu Gln Ala Phe Leu Ile Ala Ile Val Val Pro Asp Val Glu Thr Leu
           595                 600                 605
   Cys Ser Trp Ala Gln Lys Arg Gly Phe Glu Gly Ser Phe Glu Glu Leu
       610                 615                 620
   Cys Arg Asn Lys Asp Val Lys Lys Ala Ile Leu Glu Asp Met Val Arg
   625                 630                 635                 640
   Leu Gly Lys Asp Ser Gly Leu Lys Pro Phe Glu Gln Val Lys Gly Ile
                   645                 650                 655
   Thr Leu His Pro Glu Leu Phe Ser Ile Asp Asn Gly Leu Leu Thr Pro
               660                 665                 670
   Thr Met Lys Ala Lys Arg Pro Glu Leu Arg Asn Tyr Phe Arg Ser Gln
           675                 680                 685
   Ile Asp Asp Leu Tyr Ser Thr Ile Lys Val
       690                 695
<210> 115
   <211> 199
   <212> PRT
   <213> Homo sapiens
<400> 115
   Met Glu Ala Ser Pro Ala Ser Gly Pro Arg His Leu Met Asp Pro His
    1               5                  10                  15
   Ile Phe Thr Ser Asn Phe Asn Asn Gly Ile Gly Arg His Lys Thr Tyr
               20                  25                  30
   Leu Cys Tyr Glu Val Glu Arg Leu Asp Asn Gly Thr Ser Val Lys Met
           35                  40                  45
   Asp Gln His Arg Gly Phe Leu His Asn Gln Ala Lys Asn Leu Leu Cys
       50                  55                  60
   Gly Phe Tyr Gly Arg His Ala Glu Leu Arg Phe Leu Asp Leu Val Pro
   65                  70                  75                  80
   Ser Leu Gln Leu Asp Pro Ala Gln Ile Tyr Arg Val Thr Trp Phe Ile
                   85                  90                  95
   Ser Trp Ser Pro Cys Phe Ser Trp Gly Cys Ala Gly Glu Val Arg Ala
               100                 105                 110
   Phe Leu Gln Glu Asn Thr His Val Arg Leu Arg Ile Phe Ala Ala Arg
           115                 120                 125
   Ile Tyr Asp Tyr Asp Pro Leu Tyr Lys Glu Ala Leu Gln Met Leu Arg
       130                 135                 140
   Asp Ala Gly Ala Gln Val Ser Ile Met Thr Tyr Asp Glu Phe Lys His
   145                 150                 155                 160
   Cys Trp Asp Thr Phe Val Asp His Gln Gly Cys Pro Phe Gln Pro Trp
                   165                 170                 175
   Asp Gly Leu Asp Glu His Ser Gln Ala Leu Ser Gly Arg Leu Arg Ala
               180                 185                 190
   Ile Leu Gln Asn Gln Gly Asn
           195
<210> 116
   <211> 295
   <212> PRT
   <213> Homo sapiens
<400> 116
   Met Gln Pro Glu Gly Ala Glu Lys Gly Lys Ser Phe Lys Gln Arg Leu
    1               5                  10                  15
   Val Leu Lys Ser Ser Leu Ala Lys Glu Thr Leu Ser Glu Phe Leu Gly
               20                  25                  30
   Thr Phe Ile Leu Ile Val Leu Gly Cys Gly Cys Val Ala Gln Ala Ile
           35                  40                  45
   Leu Ser Arg Gly Arg Phe Gly Gly Val Ile Thr Ile Asn Val Gly Phe
       50                  55                  60
   Ser Met Ala Val Ala Met Ala Ile Tyr Val Ala Gly Gly Val Ser Gly
   65                  70                  75                  80
   Gly His Ile Asn Pro Ala Val Ser Leu Ala Met Cys Leu Phe Gly Arg
                   85                  90                  95
   Met Lys Trp Phe Lys Leu Pro Phe Tyr Val Gly Ala Gln Phe Leu Gly
               100                 105                 110
   Ala Phe Val Gly Ala Ala Thr Val Phe Gly Ile Tyr Tyr Asp Gly Leu
           115                 120                 125
   Met Ser Phe Ala Gly Gly Lys Leu Leu Ile Val Gly Glu Asn Ala Thr
       130                 135                 140
   Ala His Ile Phe Ala Thr Tyr Pro Ala Pro Tyr Leu Ser Leu Ala Asn
   145                 150                 155                 160
   Ala Phe Ala Asp Gln Val Val Ala Thr Met Ile Leu Leu Ile Ile Val
                   165                 170                 175
   Phe Ala Ile Phe Asp Ser Arg Asn Leu Gly Ala Pro Arg Gly Leu Glu
               180                 185                 190
   Pro Ile Ala Ile Gly Leu Leu Ile Ile Val Ile Ala Ser Ser Leu Gly
           195                 200                 205
   Leu Asn Ser Gly Cys Ala Met Asn Pro Ala Arg Asp Leu Ser Pro Arg
       210                 215                 220
   Leu Phe Thr Ala Leu Ala Gly Trp Gly Phe Glu Val Phe Arg Ala Gly
   225                 230                 235                 240
   Asn Asn Phe Trp Trp Ile Pro Val Val Gly Pro Leu Val Gly Ala Val
                   245                 250                 255
   Ile Gly Gly Leu Ile Tyr Val Leu Val Ile Glu Ile His His Pro Glu
               260                 265                 270
   Pro Asp Ser Val Phe Lys Thr Glu Gln Ser Glu Asp Lys Pro Glu Lys
           275                 280                 285
   Tyr Glu Leu Ser Val Ile Met
       290                 295
<210> 117
   <211> 163
   <212> PRT
   <213> Homo sapiens
<400> 117
   Met Thr Asp Cys Glu Phe Gly Tyr Ile Tyr Arg Leu Ala Gln Asp Tyr
    1               5                  10                  15
   Leu Gln Cys Val Leu Gln Ile Pro Gln Pro Gly Ser Gly Pro Ser Lys
               20                  25                  30
   Thr Ser Arg Val Leu Gln Asn Val Ala Phe Ser Val Gln Lys Glu Val
           35                  40                  45
   Glu Lys Asn Leu Lys Ser Cys Leu Asp Asn Val Asn Val Val Ser Val
       50                  55                  60
   Asp Thr Ala Arg Thr Leu Phe Asn Gln Val Met Glu Lys Glu Phe Glu
   65                  70                  75                  80
   Asp Gly Ile Ile Asn Trp Gly Arg Ile Val Thr Ile Phe Ala Phe Glu
                   85                  90                  95
   Gly Ile Leu Ile Lys Lys Leu Leu Arg Gln Gln Ile Ala Pro Asp Val
               100                 105                 110
   Asp Thr Tyr Lys Glu Ile Ser Tyr Phe Val Ala Glu Phe Ile Met Asn
           115                 120                 125
   Asn Thr Gly Glu Trp Ile Arg Gln Asn Gly Gly Trp Gly Lys Trp His
       130                 135                 140
   Asn His Thr Pro Met Leu Val Glu Ser Val Ala His Lys Lys Arg Lys
   145                 150                 155                 160
   Met Ala Leu
<210> 118
   <211> 650
   <212> PRT
   <213> Homo sapiens
<400> 118
   Met Ala Ser Pro Ala Asp Ser Cys Ile Gln Phe Thr Arg His Ala Ser
    1               5                  10                  15
   Asp Val Leu Leu Asn Leu Asn Arg Leu Arg Ser Arg Asp Ile Leu Thr
               20                  25                  30
   Asp Val Val Ile Val Val Ser Arg Glu Gln Phe Arg Ala His Lys Thr
           35                  40                  45
   Val Leu Met Ala Cys Ser Gly Leu Phe Tyr Ser Ile Phe Thr Asp Gln
       50                  55                  60
   Leu Lys Cys Asn Leu Ser Val Ile Asn Leu Asp Pro Glu Ile Asn Pro
   65                  70                  75                  80
   Glu Gly Phe Cys Ile Leu Leu Asp Phe Met Tyr Thr Ser Arg Leu Asn
                   85                  90                  95
   Leu Arg Glu Gly Asn Ile Met Ala Val Met Ala Thr Ala Met Tyr Leu
               100                 105                 110
   Gln Met Glu His Val Val Asp Thr Cys Arg Lys Phe Ile Lys Ala Ser
           115                 120                 125
   Glu Ala Glu Met Val Ser Ala Ile Lys Pro Pro Arg Glu Glu Phe Leu
       130                 135                 140
   Asn Ser Arg Met Leu Met Pro Gln Asp Ile Met Ala Tyr Arg Gly Arg
   145                 150                 155                 160
   Glu Val Val Glu Asn Asn Leu Pro Leu Arg Ser Ala Pro Gly Cys Glu
                   165                 170                 175
   Ser Arg Ala Phe Ala Pro Ser Leu Tyr Ser Gly Leu Ser Thr Pro Pro
               180                 185                 190
   Ala Ser Tyr Ser Met Tyr Ser His Leu Pro Val Ser Ser Leu Leu Phe
           195                 200                 205
   Ser Asp Glu Glu Phe Arg Asp Val Arg Met Pro Val Ala Asn Pro Phe
       210                 215                 220
   Pro Lys Glu Arg Ala Leu Pro Cys Asp Ser Ala Arg Pro Val Pro Gly
   225                 230                 235                 240
   Glu Tyr Ser Arg Pro Thr Leu Glu Val Ser Pro Asn Val Cys His Ser
                   245                 250                 255
   Asn Ile Tyr Ser Pro Lys Glu Thr Ile Pro Glu Glu Ala Arg Ser Asp
               260                 265                 270
   Met His Tyr Ser Val Ala Glu Gly Leu Lys Pro Ala Ala Pro Ser Ala
           275                 280                 285
   Arg Asn Ala Pro Tyr Phe Pro Cys Asp Lys Ala Ser Lys Glu Glu Glu
       290                 295                 300
   Arg Pro Ser Ser Glu Asp Glu Ile Ala Leu His Phe Glu Pro Pro Asn
   305                 310                 315                 320
   Ala Pro Leu Asn Arg Lys Gly Leu Val Ser Pro Gln Ser Pro Gln Lys
                       325                 330                 335
   Ser Asp Cys Gln Pro Asn Ser Pro Thr Glu Ser Cys Ser Ser Lys Asn
                   340                 345                 350
   Ala Cys Ile Leu Gln Ala Ser Gly Ser Pro Pro Ala Lys Ser Pro Thr
               355                 360                 365
   Asp Pro Lys Ala Cys Asn Trp Lys Lys Tyr Lys Phe Ile Val Leu Asn
       370                 375                 380
   Ser Leu Asn Gln Asn Ala Lys Pro Glu Gly Pro Glu Gln Ala Glu Leu
   385                 390                 395                 400
   Gly Arg Leu Ser Pro Arg Ala Tyr Thr Ala Pro Pro Ala Cys Gln Pro
                   405                 410                 415
   Pro Met Glu Pro Glu Asn Leu Asp Leu Gln Ser Pro Thr Lys Leu Ser
                   420                 425                 430
   Ala Ser Gly Glu Asp Ser Thr Ile Pro Gln Ala Ser Arg Leu Asn Asn
               435                 440                 445
   Ile Val Asn Arg Ser Met Thr Gly Ser Pro Arg Ser Ser Ser Glu Ser
       450                 455                 460
   His Ser Pro Leu Tyr Met His Pro Pro Lys Cys Thr Ser Cys Gly Ser
   465                 470                 475                 480
   Gln Ser Pro Gln His Ala Glu Met Cys Leu His Thr Ala Gly Pro Thr
                   485                 490                 495
   Phe Pro Glu Glu Met Gly Glu Thr Gln Ser Glu Tyr Ser Asp Ser Ser
                   500                 505                 510
   Cys Gly Glu Lys Pro Tyr Arg Cys Asn Ile Cys Gly Ala Gln Phe Asn
               515                 520                 525
   Arg Pro Ala Asn Leu Lys Thr His Thr Arg Ile His Ser Gly Glu Lys
       530                 535                 540
   Pro Tyr Lys Cys Glu Thr Cys Gly Ala Arg Phe Val Gln Val Ala His
   545                 550                 555                 560
   Leu Arg Ala His Val Leu Ile His Thr Gly Glu Lys Pro Tyr Pro Cys
                   565                 570                 575
   Glu Ile Cys Gly Thr Arg Phe Arg His Leu Gln Thr Leu Lys Ser His
                   580                 585                 590
   Leu Arg Ile His Thr Gly Glu Lys Pro Tyr His Cys Glu Lys Cys Asn
               595                 600                 605
   Leu His Phe Arg His Lys Ser Gln Leu Arg Leu His Leu Arg Gln Lys
       610                 615                 620
   His Gly Ala Ile Thr Asn Thr Lys Val Gln Tyr Arg Val Ser Ala Thr
   625                 630                 635                 640
   Asp Leu Pro Pro Glu Leu Pro Lys Ala Cys
                   645                 650
<210> 119
   <211> 350
   <212> PRT
   <213> Homo sapiens
<400> 119
   Met Asn Ser Phe Asn Tyr Thr Thr Pro Asp Tyr Gly His Tyr Asp Asp
    1               5                  10                  15
   Lys Asp Thr Leu Asp Leu Asn Thr Pro Val Asp Lys Thr Ser Asn Thr
               20                  25                  30
   Leu Arg Val Pro Asp Ile Leu Ala Leu Val Ile Phe Ala Val Val Phe
           35                  40                  45
   Leu Val Gly Val Leu Gly Asn Ala Leu Val Val Trp Val Thr Ala Phe
       50                  55                  60
   Glu Ala Lys Arg Thr Ile Asn Ala Ile Trp Phe Leu Asn Leu Ala Val
   65                  70                  75                  80
   Ala Asp Phe Leu Ser Cys Leu Ala Leu Pro Ile Leu Phe Thr Ser Ile
                   85                  90                  95
   Val Gln His His His Trp Pro Phe Gly Gly Ala Ala Cys Ser Ile Leu
               100                 105                 110
   Pro Ser Leu Ile Leu Leu Asn Met Tyr Ala Ser Ile Leu Leu Leu Ala
           115                 120                 125
   Thr Ile Ser Ala Asp Arg Phe Leu Leu Val Phe Lys Pro Ile Trp Cys
       130                 135                 140
   Gln Asn Phe Arg Gly Ala Gly Leu Ala Trp Ile Ala Cys Ala Val Ala
   145                 150                 155                 160
   Trp Gly Leu Ala Leu Leu Leu Thr Ile Pro Ser Phe Leu Tyr Arg Val
                   165                 170                 175
   Val Arg Glu Glu Tyr Phe Pro Pro Lys Val Leu Cys Gly Val Asp Tyr
               180                 185                 190
   Ser His Asp Lys Arg Arg Glu Arg Ala Val Ala Ile Val Arg Leu Val
           195                 200                 205
   Leu Gly Phe Leu Trp Pro Leu Leu Thr Leu Thr Ile Cys Tyr Thr Phe
       210                 215                 220
   Ile Leu Leu Arg Thr Trp Ser Arg Arg Ala Thr Arg Ser Thr Lys Thr
   225                 230                 235                 240
   Leu Lys Val Val Val Ala Val Val Ala Ser Phe Phe Ile Phe Trp Leu
                   245                 250                 255
   Pro Tyr Gln Val Thr Gly Ile Met Met Ser Phe Leu Glu Pro Ser Ser
               260                 265                 270
   Pro Thr Phe Leu Leu Leu Asn Lys Leu Asp Ser Leu Cys Val Ser Phe
           275                 280                 285
   Ala Tyr Ile Asn Cys Cys Ile Asn Pro Ile Ile Tyr Val Val Ala Gly
       290                 295                 300
   Gln Gly Phe Gln Gly Arg Leu Arg Lys Ser Leu Pro Ser Leu Leu Arg
   305                 310                 315                 320
   Asn Val Leu Thr Glu Glu Ser Val Val Arg Glu Ser Lys Ser Phe Thr
                   325                 330                 335
   Arg Ser Thr Val Asp Thr Met Ala Gln Lys Thr Gln Ala Val
               340                 345                 350
<210> 120
   <211> 248
   <212> PRT
   <213> Homo sapiens
<400> 120
   Met Ala Pro Lys Ala Ala Lys Gly Ala Lys Pro Glu Pro Ala Pro Ala
   1               5                   10                  15
   Pro Pro Pro Pro Gly Ala Lys Pro Glu Glu Asp Lys Lys Asp Gly Lys
               20                  25                  30
   Glu Pro Ser Asp Lys Pro Gln Lys Ala Val Gln Asp His Lys Glu Pro
           35                  40                  45
   Ser Asp Lys Pro Gln Lys Ala Val Gln Pro Lys His Glu Val Gly Thr
       50                  55                  60
   Arg Arg Gly Cys Arg Arg Tyr Arg Trp Glu Leu Lys Asp Ser Asn Lys
   65                  70                  75                  80
   Glu Phe Trp Leu Leu Gly His Ala Glu Ile Lys Ile Arg Ser Leu Gly
                   85                  90                  95
   Cys Leu Ile Ala Ala Met Ile Leu Leu Ser Ser Leu Thr Val His Pro
               100                 105                 110
   Ile Leu Arg Leu Ile Ile Thr Met Glu Ile Ser Phe Phe Ser Phe Phe
           115                 120                 125
   Ile Leu Leu Tyr Ser Phe Ala Ile His Arg Tyr Ile Pro Phe Ile Leu
       130                 135                 140
   Trp Pro Ile Ser Asp Leu Phe Asn Asp Leu Ile Ala Cys Ala Phe Leu
   145                 150                 155                 160
   Val Gly Ala Val Val Phe Ala Val Arg Ser Arg Arg Ser Met Asn Leu
                   165                 170                 175
   His Tyr Leu Leu Ala Val Ile Leu Ile Gly Ala Ala Gly Val Phe Ala
               180                 185                 190
   Phe Ile Asp Val Cys Leu Gln Arg Asn His Phe Arg Gly Lys Lys Ala
           195                 200                 205
   Lys Lys His Met Leu Val Pro Pro Pro Gly Lys Glu Lys Gly Pro Gln
       210                 215                 220
   Gln Gly Lys Gly Pro Glu Pro Ala Lys Pro Pro Glu Pro Gly Lys Pro
   225                 230                 235                 240
   Pro Gly Pro Ala Lys Gly Lys Lys
                   245
<210> 121
   <211> 219
   <212> PRT
   <213> Homo sapiens
<400> 121
   Met Asn Ser Ser Lys Ser Ser Glu Thr Gln Cys Thr Glu Arg Gly Cys
    1               5                  10                  15
   Phe Ser Ser Gln Met Phe Leu Trp Thr Val Ala Gly Ile Pro Ile Leu
               20                  25                  30
   Phe Leu Ser Ala Cys Phe Ile Thr Arg Cys Val Val Thr Phe Arg Ile
           35                  40                  45
   Phe Gln Thr Cys Asp Glu Lys Lys Phe Gln Leu Pro Glu Asn Phe Thr
       50                  55                  60
   Glu Leu Ser Cys Tyr Asn Tyr Gly Ser Gly Ser Val Lys Asn Cys Cys
   65                  70                  75                  80
   Pro Leu Asn Trp Glu Tyr Phe Gln Ser Ser Cys Tyr Phe Phe Ser Thr
                   85                  90                  95
   Asp Thr Ile Ser Trp Ala Leu Ser Leu Lys Asn Cys Ser Ala Met Gly
               100                 105                 110
   Ala His Leu Val Val Ile Asn Ser Gln Glu Glu Gln Glu Phe Leu Ser
           115                 120                 125
   Tyr Lys Lys Pro Lys Met Arg Glu Phe Phe Ile Gly Leu Ser Asp Gln
       130                 135                 140
   Val Val Glu Gly Gln Trp Gln Trp Val Asp Gly Thr Pro Leu Thr Lys
   145                 150                 155                 160
   Ser Leu Ser Phe Trp Asp Val Gly Glu Pro Asn Asn Ile Ala Thr Leu
                   165                 170                 175
   Glu Asp Cys Ala Thr Met Arg Asp Ser Ser Asn Pro Arg Gln Asn Trp
               180                 185                 190
   Asn Asp Val Thr Cys Phe Leu Asn Tyr Phe Arg Ile Cys Glu Met Val
           195                 200                 205
   Gly Ile Asn Pro Leu Asn Lys Gly Lys Ser Leu
       210                 215
<210> 122
   <211> 1028
   <212> PRT
   <213> Homo sapiens
<400> 122
   Met Met Phe Pro Trp Lys Gln Leu Ile Leu Leu Ser Phe Ile Gly Cys
    1               5                  10                  15
   Leu Gly Gly Glu Leu Leu Leu Gln Gly Pro Val Phe Ile Lys Glu Pro
                   20                  25                  30
   Ser Asn Ser Ile Phe Pro Val Gly Ser Glu Asp Lys Lys Ile Thr Leu
               35                  40                  45
   His Cys Glu Ala Arg Gly Asn Pro Ser Pro His Tyr Arg Trp Gln Leu
       50                  55                  60
   Asn Gly Ser Asp Ile Asp Met Ser Met Glu His Arg Tyr Lys Leu Asn
   65                  70                  75                  80
   Gly Gly Asn Leu Val Val Ile Asn Pro Asn Arg Asn Trp Asp Thr Gly
                   85                  90                  95
   Thr Tyr Gln Cys Phe Ala Thr Asn Ser Leu Gly Thr Ile Val Ser Arg
               100                 105                 110
   Glu Ala Lys Leu Gln Phe Ala Tyr Leu Glu Asn Phe Lys Thr Lys Met
           115                 120                 125
   Arg Ser Thr Val Ser Val Arg Glu Gly Gln Gly Val Val Leu Leu Cys
       130                 135                 140
   Gly Pro Pro Pro His Ser Gly Glu Leu Ser Tyr Ala Trp Ile Phe Asn
   145                 150                 155                 160
   Glu Tyr Pro Ser Phe Val Glu Glu Asp Ser Arg Arg Phe Val Ser Gln
                   165                 170                 175
   Glu Thr Gly His Leu Tyr Ile Ser Lys Val Glu Pro Ser Asp Val Gly
               180                 185                 190
   Asn Tyr Thr Cys Val Val Thr Ser Met Val Thr Asn Ala Arg Val Leu
           195                 200                 205
   Gly Ser Pro Thr Pro Leu Val Leu Arg Ser Asp Gly Val Met Gly Glu
       210                 215                 220
   Tyr Glu Pro Lys Ile Glu Val Gln Phe Pro Glu Thr Leu Pro Ala Ala
   225                 230                 235                 240
   Lys Gly Ser Thr Val Lys Leu Glu Cys Phe Ala Leu Gly Asn Pro Ile
                   245                 250                 255
   Pro Gln Ile Asn Trp Arg Arg Ser Asp Gly Leu Pro Phe Ser Ser Lys
               260                 265                 270
   Ile Lys Leu Arg Lys Phe Ser Gly Val Leu Glu Ile Pro Asn Phe Gln
           275                 280                 285
   Gln Glu Asp Ala Gly Ser Tyr Glu Cys Ile Ala Glu Asn Ser Arg Gly
       290                 295                 300
   Lys Asn Val Ala Arg Gly Arg Leu Thr Tyr Tyr Ala Lys Pro His Trp
   305                 310                 315                 320
   Val Gln Leu Ile Lys Asp Val Glu Ile Ala Val Glu Asp Ser Leu Tyr
                   325                 330                 335
   Trp Glu Cys Arg Ala Ser Gly Lys Pro Lys Pro Ser Tyr Arg Trp Leu
               340                 345                 350
   Lys Asn Gly Ala Ala Leu Val Leu Glu Glu Arg Thr Gln Ile Glu Asn
           355                 360                 365
   Gly Ala Leu Thr Ile Ser Asn Leu Ser Val Thr Asp Ser Gly Met Phe
       370                 375                 380
   Gln Cys Ile Ala Glu Asn Lys His Gly Leu Val Tyr Ser Ser Ala Glu
   385                 390                 395                 400
   Leu Lys Val Val Ala Ser Ala Pro Asp Phe Ser Lys Asn Pro Met Lys
                   405                 410                 415
   Lys Leu Val Gln Val Gln Val Gly Ser Leu Val Ser Leu Asp Cys Lys
               420                 425                 430
   Pro Arg Ala Ser Pro Arg Ala Leu Ser Ser Trp Lys Lys Gly Asp Val
           435                 440                 445
   Ser Val Gln Glu His Glu Arg Ile Ser Leu Leu Asn Asp Gly Gly Leu
       450                 455                 460
   Lys Ile Ala Asn Val Thr Lys Ala Asp Ala Gly Thr Tyr Thr Cys Met
   465                 470                 475                 480
   Ala Glu Asn Gln Phe Gly Lys Ala Asn Gly Thr Thr His Leu Val Val
                   485                 490                 495
   Thr Glu Pro Thr Arg Ile Thr Leu Ala Pro Ser Asn Met Asp Val Ser
                   500                 505                 510
   Val Gly Glu Ser Val Ile Leu Pro Cys Gln Val Gln His Asp Pro Leu
               515                 520                 525
   Leu Asp Ile Ile Phe Thr Trp Tyr Phe Asn Gly Ala Leu Ala Asp Phe
       530                 535                 540
   Lys Lys Asp Gly Ser His Phe Glu Lys Val Gly Gly Ser Ser Ser Gly
   545                 550                 555                 560
   Asp Leu Met Ile Arg Asn Ile Gln Leu Lys His Ser Gly Lys Tyr Val
                   565                 570                 575
   Cys Met Val Gln Thr Gly Val Asp Ser Val Ser Ser Ala Ala Asp Leu
               580                 585                 590
   Ile Val Arg Gly Ser Pro Gly Pro Pro Glu Asn Val Lys Val Asp Glu
           595                 600                 605
   Ile Thr Asp Thr Thr Ala Gln Leu Ser Trp Lys Glu Gly Lys Asp Asn
       610                 615                 620
   His Ser Pro Val Ile Ser Tyr Ser Ile Gln Ala Arg Thr Pro Phe Ser
   625                 630                 635                 640
   Val Gly Trp Gln Thr Val Thr Thr Val Pro Glu Val Ile Asp Gly Lys
                   645                 650                 655
   Thr His Thr Ala Thr Val Val Glu Leu Asn Pro Trp Val Glu Tyr Glu
               660                 665                 670
   Phe Arg Val Val Ala Ser Asn Lys Ile Gly Gly Gly Glu Pro Ser Leu
           675                 680                 685
   Pro Ser Glu Lys Val Arg Thr Glu Glu Ala Val Pro Glu Val Pro Pro
       690                 695                 700
   Ser Glu Val Asn Gly Gly Gly Gly Ser Arg Ser Glu Leu Val Ile Thr
   705                 710                 715                 720
   Trp Asp Pro Val Pro Glu Glu Leu Gln Asn Gly Glu Gly Phe Gly Tyr
                   725                 730                 735
   Val Val Ala Phe Arg Pro Leu Gly Val Thr Thr Trp Ile Gln Thr Val
               740                 745                 750
   Val Thr Ser Pro Asp Thr Pro Arg Tyr Val Phe Arg Asn Glu Ser Ile
           755                 760                 765
   Val Pro Tyr Ser Pro Tyr Glu Val Lys Val Gly Val Tyr Asn Asn Lys
       770                 775                 780
   Gly Glu Gly Pro Phe Ser Pro Val Thr Thr Val Phe Ser Ala Glu Glu
   785                 790                 795                 800
   Glu Pro Thr Val Ala Pro Ser Gln Val Ser Ala Asn Ser Leu Ser Ser
                   805                 810                 815
   Ser Glu Ile Glu Val Ser Trp Asn Thr Ile Pro Trp Lys Leu Ser Asn
               820                 825                 830
   Gly His Leu Leu Gly Tyr Glu Val Arg Tyr Trp Asn Gly Gly Gly Lys
           835                 840                 845
   Glu Glu Ser Ser Ser Lys Met Lys Val Ala Gly Asn Glu Thr Ser Ala
       850                 855                 860
   Arg Leu Arg Gly Leu Lys Ser Asn Leu Ala Tyr Tyr Thr Ala Val Arg
   865                 870                 875                 880
   Ala Tyr Asn Ser Ala Gly Ala Gly Pro Phe Ser Ala Thr Val Asn Val
                   885                 890                 895
   Thr Thr Lys Lys Thr Pro Pro Ser Gln Pro Pro Gly Asn Val Val Trp
               900                 905                 910
   Asn Ala Thr Asp Thr Lys Val Leu Leu Asn Trp Glu Gln Val Lys Ala
           915                 920                 925
   Met Glu Asn Glu Ser Glu Val Thr Gly Tyr Lys Val Phe Tyr Arg Thr
       930                 935                 940
   Ser Ser Gln Asn Asn Val Gln Val Leu Asn Thr Asn Lys Thr Ser Ala
   945                 950                 955                 960
   Glu Leu Val Leu Pro Ile Lys Glu Asp Tyr Ile Ile Glu Val Lys Ala
                   965                 970                 975
   Thr Thr Asp Gly Gly Asp Gly Thr Ser Ser Glu Gln Ile Arg Ile Pro
                       980                 985                 990
   Arg Ile Thr Ser Met Asp Ala Arg Gly Ser Thr Ser Ala Ile Ser Asn
                   995                 1000                1005
   Val His Pro Met Ser Ser Tyr Met Pro Ile Val Leu Phe Leu Ile Val
       1010                1015                1020
   Tyr Val Leu Trp
   1025
<210> 123
   <211> 501
   <212> PRT
   <213> Homo sapiens
<400> 123
   Met Asn Leu Glu Gln Glu Arg Pro Phe Val Cys Ser Ala Pro Gly Cys
    1               5                  10                  15
   Ser Gln Arg Phe Pro Thr Glu Asp His Leu Met Ile His Arg His Lys
               20                  25                  30
   His Glu Met Thr Leu Lys Phe Pro Ser Ile Lys Thr Asp Asn Met Leu
           35                  40                  45
   Ser Asp Gln Thr Pro Thr Pro Thr Arg Phe Leu Lys Asn Cys Glu Glu
       50                  55                  60
   Val Gly Leu Phe Ser Glu Leu Asp Cys Ser Leu Glu His Glu Phe Arg
   65                  70                  75                  80
   Lys Ala Gln Glu Glu Glu Ser Ser Lys Arg Asn Ile Ser Met His Asn
                   85                  90                  95
   Ala Val Gly Gly Ala Met Thr Gly Pro Gly Thr His Gln Leu Ser Ser
               100                 105                 110
   Ala Arg Leu Pro Asn His Asp Thr Asn Val Val Ile Gln Gln Ala Met
           115                 120                 125
   Pro Ser Pro Gln Ser Ser Ser Val Ile Thr Gln Ala Pro Ser Thr Asn
       130                 135                 140
   Arg Gln Ile Gly Pro Val Pro Gly Ser Leu Ser Ser Leu Leu His Leu
   145                 150                 155                 160
   His Asn Arg Gln Arg Gln Pro Met Pro Ala Ser Met Pro Gly Thr Leu
                   165                 170                 175
   Pro Asn Pro Thr Met Pro Gly Ser Ser Ala Val Leu Met Pro Met Glu
               180                 185                 190
   Arg Gln Met Ser Val Asn Ser Ser Ile Met Gly Met Gln Gly Pro Asn
           195                 200                 205
   Leu Ser Asn Pro Cys Ala Ser Pro Gln Val Gln Pro Met His Ser Glu
       210                 215                 220
   Ala Lys Met Arg Leu Lys Ala Ala Leu Thr His His Pro Ala Ala Met
   225                 230                 235                 240
   Ser Asn Gly Asn Met Asn Thr Met Gly His Met Met Glu Met Met Gly
                   245                 250                 255
   Ser Arg Gln Asp Gln Thr Pro His His His Met His Ser His Pro His
               260                 265                 270
   Gln His Gln Thr Leu Pro Pro His His Pro Tyr Pro His Gln His Gln
           275                 280                 285
   His Pro Ala His His Pro His Pro Gln Pro His His Gln Gln Asn His
       290                 295                 300
   Pro His His His Ser His Ser His Leu His Ala His Pro Ala His His
   305                 310                 315                 320
   Gln Thr Ser Pro His Pro Pro Leu His Thr Gly Asn Gln Ala Gln Val
                   325                 330                 335
   Ser Pro Ala Thr Gln Gln Met Gln Pro Thr Gln Thr Ile Gln Pro Pro
               340                 345                 350
   Gln Pro Thr Gly Gly Arg Arg Arg Arg Val Val Asp Glu Asp Pro Asp
               355                 360                 365
   Glu Arg Arg Arg Lys Phe Leu Glu Arg Asn Arg Ala Ala Ala Thr Arg
           370                 375                 380
   Cys Arg Gln Lys Arg Lys Val Trp Val Met Ser Leu Glu Lys Lys Ala
   385                 390                 395                 400
   Glu Glu Leu Thr Gln Thr Asn Met Gln Leu Gln Asn Glu Val Ser Met
                   405                 410                 415
   Leu Lys Asn Glu Val Ala Gln Leu Lys Gln Leu Leu Leu Thr His Lys
               420                 425                 430
   Asp Cys Pro Ile Thr Ala Met Gln Lys Glu Ser Gln Gly Tyr Leu Ser
           435                 440                 445
   Pro Glu Ser Ser Pro Pro Ala Ser Pro Val Pro Ala Cys Ser Gln Gln
       450                 455                 460
   Gln Val Ile Gln His Asn Thr Ile Thr Thr Ser Ser Ser Val Ser Glu
   465                 470                 475                 480
   Val Val Gly Ser Ser Thr Leu Ser Gln Leu Thr Thr His Arg Thr Asp
                   485                 490                 495
   Leu Asn Pro Ile Leu
               500
<210> 124
   <211> 836
   <212> PRT
   <213> Homo sapiens
<400> 124
<210> 125
   <211> 836
   <212> PRT
   <213> Homo sapiens
<400> 125
<210> 126
   <211> 94
   <212> PRT
   <213> Homo sapiens
<400> 126
   Met Ser Val Lys Gly Met Ala Ile Ala Leu Ala Val Ile Leu Cys Ala
    1               5                  10                  15
   Thr Val Val Gln Gly Phe Pro Met Phe Lys Arg Gly Arg Cys Leu Cys
               20                  25                  30
   Ile Gly Pro Gly Val Lys Ala Val Lys Val Ala Asp Ile Glu Lys Ala
           35                  40                  45
   Ser Ile Met Tyr Pro Ser Asn Asn Cys Asp Lys Ile Glu Val Ile Ile
       50                  55                  60
   Thr Leu Lys Glu Asn Lys Gly Gln Arg Cys Leu Asn Pro Lys Ser Lys
   65                  70                  75                  80
   Gln Ala Arg Leu Ile Ile Lys Lys Val Glu Arg Lys Asn Phe
                   85                  90
<210> 127
   <211> 94
   <212> PRT
   <213> Homo sapiens
<400> 127
   Met Ser Val Lys Gly Met Ala Ile Ala Leu Ala Val Ile Leu Cys Ala
    1               5                  10                  15
   Thr Val Val Gln Gly Phe Pro Met Phe Lys Arg Gly Arg Cys Leu Cys
               20                  25                  30
   Ile Gly Pro Gly Val Lys Ala Val Lys Val Ala Asp Ile Glu Lys Ala
           35                  40                  45
   Ser Ile Met Tyr Pro Ser Asn Asn Cys Asp Lys Ile Glu Val Ile Ile
       50                  55                  60
   Thr Leu Lys Glu Asn Lys Gly Gln Arg Cys Leu Asn Pro Lys Ser Lys
   65                  70                  75                  80
   Gln Ala Arg Leu Ile Ile Lys Lys Val Glu Arg Lys Asn Phe
                   85                  90
<210> 128
   <211> 520
   <212> PRT
   <213> Homo sapiens
<400> 128
   Met Pro Gln Leu Ser Leu Ser Ser Leu Gly Leu Trp Pro Met Ala Ala
    1               5                  10                  15
   Ser Pro Trp Leu Leu Leu Leu Leu Val Gly Ala Ser Trp Leu Leu Ala
               20                  25                  30
   Arg Ile Leu Ala Trp Thr Tyr Thr Phe Tyr Asp Asn Cys Cys Arg Leu
               35                      40                      45
   Arg Cys Phe Pro Gln Pro Pro Lys Arg Asn Trp Phe Leu Gly His Leu
           50                      55                      60
   Gly Leu Ile His Ser Ser Glu Glu Gly Leu Leu Tyr Thr Gln Ser Leu
   65                      70                      75                      80
   Ala Cys Thr Phe Gly Asp Met Cys Cys Trp Trp Val Gly Pro Trp His
                       85                      90                      95
   Ala Ile Val Arg Ile Phe His Pro Thr Tyr Ile Lys Pro Val Leu Phe
               100                     105                     110
   Ala Pro Ala Ala Ile Val Pro Lys Asp Lys Val Phe Tyr Ser Phe Leu
           115                     120                     125
   Lys Pro Trp Leu Gly Asp Gly Leu Leu Leu Ser Ala Gly Glu Lys Trp
       130                     135                     140
   Ser Arg His Arg Arg Met Leu Thr Pro Ala Phe His Phe Asn Ile Leu
   145                     150                     155                     160
   Lys Pro Tyr Met Lys Ile Phe Asn Glu Ser Val Asn Ile Met His Ala
                   165                     170                     175
   Lys Trp Gln Leu Leu Ala Ser Glu Gly Ser Ala Arg Leu Asp Met Phe
               180                     185                     190
   Glu His Ile Ser Leu Met Thr Leu Asp Ser Leu Gln Lys Cys Val Phe
           195                     200                     205
   Ser Phe Asp Ser His Cys Gln Glu Lys Pro Ser Glu Tyr Ile Ala Ala
       210                     215                     220
   Ile Leu Glu Leu Ser Ala Leu Val Thr Lys Arg His Gln Gln Ile Leu
   225                     230                     235                     240
   Leu Tyr Ile Asp Phe Leu Tyr Tyr Leu Thr Pro Asp Gly Gln Arg Phe
                   245                     250                     255
   Arg Arg Ala Cys Arg Leu Val His Asp Phe Thr Asp Ala Val Ile Gln
               260                     265                     270
   Glu Arg Arg Arg Thr Leu Pro Ser Gln Gly Val Asp Asp Phe Leu Gln
           275                     280                     285
   Ala Lys Ala Lys Ser Lys Thr Leu Asp Phe Ile Asp Val Leu Leu Leu
       290                     295                     300
   Ser Lys Asp Glu Asp Gly Lys Lys Leu Ser Asp Glu Asp Ile Arg Ala
   305                     310                     315                     320
   Glu Ala Asp Thr Phe Met Phe Glu Gly His Asp Thr Thr Ala Ser Gly
                   325                     330                     335
   Leu Ser Trp Val Leu Tyr His Leu Ala Lys His Pro Glu Tyr Gln Glu
               340                     345                     350
   Arg Cys Arg Gln Glu Val Gln Glu Leu Leu Lys Asp Arg Glu Pro Lys
           355                     360                     365
   Glu Ile Glu Trp Asp Asp Leu Ala Gln Leu Pro Phe Leu Thr Met Cys
       370                     375                     380
   Ile Lys Glu Ser Leu Arg Leu His Pro Pro Val Pro Ala Val Ser Arg
   385                     390                     395                     400
   Cys Cys Thr Gln Asp Ile Val Leu Pro Asp Gly Arg Val Ile Pro Lys
                   405                     410                     415
   Gly Ile Ile Cys Leu Ile Ser Val Phe Gly Thr His His Asn Pro Ala
               420                     425                     430
   Val Trp Pro Asp Pro Glu Val Tyr Asp Pro Phe Arg Phe Asp Pro Lys
           435                     440                     445
   Asn Ile Lys Glu Arg Ser Pro Leu Ala Phe Ile Pro Phe Ser Ala Gly
       450                     455                     460
   Pro Arg Asn Cys Ile Gly Gln Ala Phe Ala Met Ala Glu Met Lys Val
   465                     470                     475                     480
   Val Leu Gly Leu Thr Leu Leu Arg Phe Arg Val Leu Pro Asp His Thr
                   485                     490                     495
   Glu Pro Arg Arg Lys Pro Glu Leu Val Leu Arg Ala Glu Gly Gly Leu
               500                     505                     510
   Trp Leu Arg Val Glu Pro Leu Ser
   515 520
<210> 129
   <211> 388
   <212> PRT
   <213> Homo sapiens
<400> 129
   Met Lys Thr Leu Ile Ala Ala Tyr Ser Gly Val Leu Arg Gly Glu Arg
    1               5                  10                  15
   Gln Ala Glu Ala Asp Arg Ser Gln Arg Ser His Gly Gly Pro Ala Leu
               20                  25                  30
   Ser Arg Glu Gly Ser Gly Arg Trp Gly Thr Gly Ser Ser Ile Leu Ser
           35                  40                  45
   Ala Leu Gln Asp Leu Phe Ser Val Thr Trp Leu Asn Arg Ser Lys Val
       50                  55                  60
   Glu Lys Gln Leu Gln Val Ile Ser Val Leu Gln Trp Val Leu Ser Phe
   65                  70                  75                  80
   Leu Val Leu Gly Val Ala Cys Ser Ala Ile Leu Met Tyr Ile Phe Cys
                   85                  90                  95
   Thr Asp Cys Trp Leu Ile Ala Val Leu Tyr Phe Thr Trp Leu Val Phe
               100                 105                 110
   Asp Trp Asn Thr Pro Lys Lys Gly Gly Arg Arg Ser Gln Trp Val Arg
           115                 120                 125
   Asn Trp Ala Val Trp Arg Tyr Phe Arg Asp Tyr Phe Pro Ile Gln Leu
       130                 135                 140
   Val Lys Thr His Asn Leu Leu Thr Thr Arg Asn Tyr Ile Phe Gly Tyr
   145                 150                 155                 160
   His Pro His Gly Ile Met Gly Leu Gly Ala Phe Cys Asn Phe Ser Thr
                   165                 170                 175
   Glu Ala Thr Glu Val Ser Lys Lys Phe Pro Gly Ile Arg Pro Tyr Leu
               180                 185                 190
   Ala Thr Leu Ala Gly Asn Phe Arg Met Pro Val Leu Arg Glu Tyr Leu
           195                 200                 205
   Met Ser Gly Gly Ile Cys Pro Val Ser Arg Asp Thr Ile Asp Tyr Leu
       210                 215                 220
   Leu Ser Lys Asn Gly Ser Gly Asn Ala Ile Ile Ile Val Val Gly Gly
   225                 230                 235                 240
   Ala Ala Glu Ser Leu Ser Ser Met Pro Gly Lys Asn Ala Val Thr Leu
                   245                 250                 255
   Arg Asn Arg Lys Gly Phe Val Lys Leu Ala Leu Arg His Gly Ala Asp
               260                 265                 270
   Leu Val Pro Ile Tyr Ser Phe Gly Glu Asn Glu Val Tyr Lys Gln Val
           275                 280                 285
   Ile Phe Glu Glu Gly Ser Trp Gly Arg Trp Val Gln Lys Lys Phe Gln
       290                 295                 300
   Lys Tyr Ile Gly Phe Ala Pro Cys Ile Phe His Gly Arg Gly Leu Phe
   305                 310                 315                 320
   Ser Ser Asp Thr Trp Gly Leu Val Pro Tyr Ser Lys Pro Ile Thr Thr
                   325                 330                 335
   Val Val Gly Glu Pro Ile Thr Ile Pro Lys Leu Glu His Pro Thr Gln
               340                 345                 350
   Gln Asp Ile Asp Leu Tyr His Thr Met Tyr Met Glu Ala Leu Val Lys
           355                 360                 365
   Leu Phe Asp Lys His Lys Thr Lys Phe Gly Leu Pro Glu Thr Glu Val
       370                 375                 380
   Leu Glu Val Asn
   385
<210> 130
   <211> 367
   <212> PRT
   <213> Homo sapiens
<400> 130
   Met Val Met Glu Val Gly Thr Leu Asp Ala Gly Gly Leu Arg Ala Leu
   1               5                   10                  15
   Leu Gly Glu Arg Ala Ala Gln Cys Leu Leu Leu Asp Cys Arg Ser Phe
               20                  25                  30
   Phe Ala Phe Asn Ala Gly His Ile Ala Gly Ser Val Asn Val Arg Phe
           35                  40                  45
   Ser Thr Ile Val Arg Arg Arg Ala Lys Gly Ala Met Gly Leu Glu His
       50                  55                  60
   Ile Val Pro Asn Ala Glu Leu Arg Gly Arg Leu Leu Ala Gly Ala Tyr
   65                  70                  75                  80
   His Ala Val Val Leu Leu Asp Glu Arg Ser Ala Ala Leu Asp Gly Ala
                   85                  90                  95
   Lys Arg Asp Gly Thr Leu Ala Leu Ala Ala Gly Ala Leu Cys Arg Glu
               100                 105                 110
   Ala Arg Ala Ala Gln Val Phe Phe Leu Lys Gly Gly Tyr Glu Ala Phe
           115                 120                 125
   Ser Ala Ser Cys Pro Glu Leu Cys Ser Lys Gln Ser Thr Pro Met Gly
       130                 135                 140
   Leu Ser Leu Pro Leu Ser Thr Ser Val Pro Asp Ser Ala Glu Ser Gly
   145                 150                 155                 160
   Cys Ser Ser Cys Ser Thr Pro Leu Tyr Asp Gln Gly Gly Pro Val Glu
                   165                 170                 175
   Ile Leu Pro Phe Leu Tyr Leu Gly Ser Ala Tyr His Ala Ser Arg Lys
               180                 185                 190
   Asp Met Leu Asp Ala Leu Gly Ile Thr Ala Leu Ile Asn Val Ser Ala
           195                 200                 205
   Asn Cys Pro Asn His Phe Glu Gly His Tyr Gln Tyr Lys Ser Ile Pro
       210                 215                 220
   Val Glu Asp Asn His Lys Ala Asp Ile Ser Ser Trp Phe Asn Glu Ala
   225                 230                 235                 240
   Ile Asp Phe Ile Asp Ser Ile Lys Asn Ala Gly Gly Arg Val Phe Val
                   245                 250                 255
   His Cys Gln Ala Gly Ile Ser Arg Ser Ala Thr Ile Cys Leu Ala Tyr
               260                 265                 270
   Leu Met Arg Thr Asn Arg Val Lys Leu Asp Glu Ala Phe Glu Phe Val
           275                 280                 285
   Lys Gln Arg Arg Ser Ile Ile Ser Pro Asn Phe Ser Phe Met Gly Gln
       290                 295                 300
   Leu Leu Gln Phe Glu Ser Gln Val Leu Ala Pro His Cys Ser Ala Glu
   305                 310                 315                 320
   Ala Gly Ser Pro Ala Met Ala Val Leu Asp Arg Gly Thr Ser Thr Thr
                   325                 330                 335
   Thr Val Phe Asn Phe Pro Val Ser Ile Pro Val His Ser Thr Asn Ser
               340                 345                 350
   Ala Leu Ser Tyr Leu Gln Ser Pro Ile Thr Thr Ser Pro Ser Cys
           355                 360                 365
<210> 131
   <211> 763
   <212> PRT
   <213> Homo sapiens
<400> 131
<210> 132
   <211> 188
   <212> PRT
   <213> Homo sapiens
<400> 132
   Met Trp Phe Leu Thr Thr Leu Leu Leu Trp Gly Trp Leu Leu Leu Gln
    1               5                  10                  15
   Val Ser Ser Arg Val Phe Met Glu Gly Glu Pro Leu Ala Leu Arg Cys
               20                  25                  30
   His Ala Trp Lys Asp Lys Leu Val Tyr Asn Val Leu Tyr Tyr Arg Asn
           35                  40                  45
   Gly Lys Ala Phe Lys Phe Phe His Trp Asn Ser Asn Leu Thr Ile Leu
       50                  55                  60
   Lys Thr Asn Ile Ser His Asn Gly Thr Tyr His Cys Ser Gly Met Gly
   65                  70                  75                  80
   Lys His Arg Tyr Thr Ser Ala Gly Ile Ser Gln Tyr Thr Val Lys Gly
                   85                  90                  95
   Leu Gln Leu Pro Thr Pro Val Trp Phe His Val Leu Phe Tyr Leu Ala
               100                 105                 110
   Val Gly Ile Met Phe Leu Val Asn Thr Val Leu Trp Val Thr Ile Arg
           115                 120                 125
   Lys Glu Leu Lys Arg Lys Lys Lys Trp Asn Leu Glu Ile Ser Leu Asp
       130                 135                 140
   Ser Gly His Glu Lys Lys Val Ile Ser Ser Leu Gln Glu Asp Arg His
   145                 150                 155                 160
   Leu Glu Glu Glu Leu Lys Cys Gln Glu Gln Lys Glu Glu Gln Leu Gln
                   165                 170                 175
   Glu Gly Val His Arg Lys Glu Pro Gln Gly Ala Thr
               180                 185
<210> 133
   <211> 374
   <212> PRT
   <213> Homo sapiens
<400> 133
   Met Trp Phe Leu Thr Thr Leu Leu Leu Trp Val Pro Val Asp Gly Gln
    1               5                  10                  15
   Val Asp Thr Thr Lys Ala Val Ile Thr Leu Gln Pro Pro Trp Val Ser
               20                  25                  30
   Val Phe Gln Glu Glu Thr Val Thr Leu His Cys Glu Val Leu His Leu
           35                  40                  45
   Pro Gly Ser Ser Ser Thr Gln Trp Phe Leu Asn Gly Thr Ala Thr Gln
       50                  55                  60
   Thr Ser Thr Pro Ser Tyr Arg Ile Thr Ser Ala Ser Val Asn Asp Ser
   65                  70                  75                  80
   Gly Glu Tyr Arg Cys Gln Arg Gly Leu Ser Gly Arg Ser Asp Pro Ile
                   85                  90                  95
   Gln Leu Glu Ile His Arg Gly Trp Leu Leu Leu Gln Val Ser Ser Arg
               100                 105                 110
   Val Phe Thr Glu Gly Glu Pro Leu Ala Leu Arg Cys His Ala Trp Lys
           115                 120                 125
   Asp Lys Leu Val Tyr Asn Val Leu Tyr Tyr Arg Asn Gly Lys Ala Phe
       130                 135                 140
   Lys Phe Phe His Trp Asn Ser Asn Leu Thr Ile Leu Lys Thr Asn Ile
   145                 150                 155                 160
   Ser His Asn Gly Thr Tyr His Cys Ser Gly Met Gly Lys His Arg Tyr
                   165                 170                 175
   Thr Ser Ala Gly Ile Ser Val Thr Val Lys Glu Leu Phe Pro Ala Pro
               180                 185                 190
   Val Leu Asn Ala Ser Val Thr Ser Pro Leu Leu Glu Gly Asn Leu Val
           195                 200                 205
   Thr Leu Ser Cys Glu Thr Lys Leu Leu Leu Gln Arg Pro Gly Leu Gln
       210                 215                 220
   Leu Tyr Phe Ser Phe Tyr Met Gly Ser Lys Thr Leu Arg Gly Arg Asn
   225                 230                 235                 240
   Thr Ser Ser Glu Tyr Gln Ile Leu Thr Ala Arg Arg Glu Asp Ser Gly
                   245                 250                 255
   Leu Tyr Trp Cys Glu Ala Ala Thr Glu Asp Gly Asn Val Leu Lys Arg
               260                 265                 270
   Ser Pro Glu Leu Glu Leu Gln Val Leu Gly Leu Gln Leu Pro Thr Pro
           275                 280                 285
   Val Trp Phe His Val Leu Phe Tyr Leu Ala Val Gly Ile Met Phe Leu
       290                 295                 300
   Val Asn Thr Val Leu Trp Val Thr Ile Arg Lys Glu Leu Lys Arg Lys
   305                 310                 315                 320
   Lys Lys Trp Asp Leu Glu Ile Ser Leu Asp Ser Gly His Glu Lys Lys
                   325                 330                 335
   Val Ile Ser Ser Leu Gln Glu Asp Arg His Leu Glu Glu Glu Leu Lys
               340                 345                 350
   Cys Gln Glu Gln Lys Glu Glu Gln Leu Gln Glu Gly Val His Arg Lys
           355                 360                 365
   Glu Pro Gln Gly Ala Thr
       370
<210> 134
   <211> 233
   <212> PRT
   <213> Homo sapiens
<400> 134
   Met Trp Gln Leu Leu Leu Pro Thr Ala Leu Leu Leu Leu Val Ser Ala
    1               5                  10                  15
   Gly Met Arg Thr Glu Asp Leu Pro Lys Ala Val Val Phe Leu Glu Pro
               20                  25                  30
   Gln Trp Tyr Ser Val Leu Glu Lys Asp Ser Val Thr Leu Lys Cys Gln
           35                  40                  45
   Gly Ala Tyr Ser Pro Glu Asp Asn Ser Thr Gln Trp Phe His Asn Glu
       50                  55                  60
   Asn Leu Ile Ser Ser Gln Ala Ser Ser Tyr Phe Ile Asp Ala Ala Thr
   65                  70                  75                  80
   Val Asn Asp Ser Gly Glu Tyr Arg Cys Gln Thr Asn Leu Ser Thr Leu
                   85                  90                  95
   Ser Asp Pro Val Gln Leu Glu Val His Ile Gly Trp Leu Leu Leu Gln
               100                 105                 110
   Ala Pro Arg Trp Val Phe Lys Glu Glu Asp Pro Ile His Leu Arg Cys
           115                 120                 125
   His Ser Trp Lys Asn Thr Ala Leu His Lys Val Thr Tyr Leu Gln Asn
       130                 135                 140
   Gly Lys Asp Arg Lys Tyr Phe His His Asn Ser Asp Phe His Ile Pro
   145                 150                 155                 160
   Lys Ala Thr Leu Lys Asp Ser Gly Ser Tyr Phe Cys Arg Gly Leu Val
                   165                 170                 175
   Gly Ser Lys Asn Val Ser Ser Glu Thr Val Asn Ile Thr Ile Thr Gln
               180                 185                 190
   Gly Leu Ala Val Ser Thr Ile Ser Ser Phe Ser Pro Pro Gly Tyr Gln
           195                 200                 205
   Val Ser Phe Cys Leu Val Met Val Leu Leu Phe Ala Val Asp Thr Gly
       210                 215                 220
   Leu Tyr Phe Ser Val Lys Thr Asn Ile
   225                 230
<210> 135
   <211> 326
   <212> PRT
   <213> Homo sapiens
<400> 135
   Met Glu Leu Ser Gly Ala Thr Met Ala Arg Gly Leu Ala Val Leu Leu
    1               5                  10                  15
   Val Leu Phe Leu His Ile Lys Asn Leu Pro Ala Gln Ala Ala Asp Thr
               20                  25                  30
   Cys Pro Glu Val Lys Val Val Gly Leu Glu Gly Ser Asp Lys Leu Thr
           35                  40                  45
   Ile Leu Arg Gly Cys Pro Gly Leu Pro Gly Ala Pro Gly Pro Lys Gly
       50                  55                  60
   Glu Ala Gly Val Ile Gly Glu Arg Gly Glu Arg Gly Leu Pro Gly Ala
   65                  70                  75                  80
   Pro Gly Lys Ala Gly Pro Val Gly Pro Lys Gly Asp Arg Gly Glu Lys
                   85                  90                  95
   Gly Met Arg Gly Glu Lys Gly Asp Ala Gly Gln Ser Gln Ser Cys Ala
               100                 105                 110
   Thr Gly Pro Arg Asn Cys Lys Asp Leu Leu Asp Arg Gly Tyr Phe Leu
           115                 120                 125
   Ser Gly Trp His Thr Ile Tyr Leu Pro Asp Cys Arg Pro Leu Thr Val
       130                 135                 140
   Leu Cys Asp Met Asp Thr Asp Gly Gly Gly Trp Thr Val Phe Gln Arg
   145                 150                 155                 160
   Arg Met Asp Gly Ser Val Asp Phe Tyr Arg Asp Trp Ala Ala Tyr Lys
                   165                 170                 175
   Gln Gly Phe Gly Ser Gln Leu Gly Glu Phe Trp Leu Gly Asn Asp Asn
               180                 185                 190
   Ile His Ala Leu Thr Ala Gln Gly Ser Ser Glu Leu Arg Val Asp Leu
           195                 200                 205
   Val Asp Phe Glu Gly Asn His Gln Phe Ala Lys Tyr Lys Ser Phe Lys
       210                 215                 220
   Val Ala Asp Glu Ala Glu Lys Tyr Lys Leu Val Leu Gly Ala Phe Val
   225                 230                 235                 240
   Gly Gly Ser Ala Gly Asn Ser Leu Thr Gly His Asn Asn Asn Phe Phe
                   245                 250                 255
   Ser Thr Lys Asp Gln Asp Asn Asp Val Ser Ser Ser Asn Cys Ala Glu
               260                 265                 270
   Lys Phe Gln Gly Ala Trp Trp Tyr Ala Asp Cys His Ala Ser Asn Leu
           275                 280                 285
   Asn Gly Leu Tyr Leu Met Gly Pro His Glu Ser Tyr Ala Asn Gly Ile
       290                 295                 300
   Asn Trp Ser Ala Ala Lys Gly Tyr Lys Tyr Ser Tyr Lys Val Ser Glu
   305                 310                 315                 320
   Met Lys Val Arg Pro Ala
                   325
<210> 136
   <211> 288
   <212> PRT
   <213> Homo sapiens
<400> 136
   Met Val Gly Val Gly Gly Gly Asp Val Glu Asp Val Thr Pro Arg Pro
   1 5 10 15
   Gly Gly Cys Gln Ile Ser Gly Arg Gly Ala Arg Gly Cys Asn Gly Ile
   20 25 30
   Pro Gly Ala Ala Ala Trp Glu Ala Ala Leu Pro Arg Arg Arg Pro Arg
   35 40 45
   Arg His Pro Ser Val Asn Pro Arg Ser Arg Ala Ala Gly Ser Pro Arg
   50 55 60
   Thr Arg Gly Arg Arg Thr Glu Glu Arg Pro Ser Gly Ser Arg Leu Gly
   65 70 75 80
   Asp Arg Gly Arg Gly Arg Ala Leu Pro Gly Gly Arg Leu Gly Gly Arg
   85 90 95
   Gly Arg Gly Arg Ala Pro Glu Arg Val Gly Gly Arg Gly Arg Gly Arg
   100 105 110
   Gly Thr Ala Ala Pro Arg Ala Ala Pro Ala Ala Arg Gly Ser Arg Pro
   115 120 125
   Gly Pro Ala Gly Thr Met Ala Ala Gly Ser Ile Thr Thr Leu Pro Ala
   130 135 140
   Leu Pro Glu Asp Gly Gly Ser Gly Ala Phe Pro Pro Gly His Phe Lys
   145 150 155 160
   Asp Pro Lys Arg Leu Tyr Cys Lys Asn Gly Gly Phe Phe Leu Arg Ile
   165 170 175
   His Pro Asp Gly Arg Val Asp Gly Val Arg Glu Lys Ser Asp Pro His
   180 185 190
   Ile Lys Leu Gln Leu Gln Ala Glu Glu Arg Gly Val Val Ser Ile Lys
   195 200 205
   Gly Val Cys Ala Asn Arg Tyr Leu Ala Met Lys Glu Asp Gly Arg Leu
   210 215 220
   Leu Ala Ser Lys Cys Val Thr Asp Glu Cys Phe Phe Phe Glu Arg Leu
   225 230 235 240
   Glu Ser Asn Asn Tyr Asn Thr Tyr Arg Ser Arg Lys Tyr Thr Ser Trp
   245 250 255
   Tyr Val Ala Leu Lys Arg Thr Gly Gln Tyr Lys Leu Gly Ser Lys Thr
   260 265 270
   Gly Pro Gly Gln Lys Ala Ile Leu Phe Leu Pro Met Ser Ala Lys Ser
   275 280 285
<210> 137
   <211> 529
   <212> PRT
   <213> Homo sapiens
<400> 137
   Met Gly Cys Val Phe Cys Lys Lys Leu Glu Pro Val Ala Thr Ala Lys
   1               5                   10                  15
   Glu Asp Ala Gly Leu Glu Gly Asp Phe Arg Ser Tyr Gly Ala Ala Asp
               20                  25                  30
   His Tyr Gly Pro Asp Pro Thr Lys Ala Arg Pro Ala Ser Ser Phe Ala
           35                  40                  45
   His Ile Pro Asn Tyr Ser Asn Phe Ser Ser Gln Ala Ile Asn Pro Gly
       50                  55                  60
   Phe Leu Asp Ser Gly Thr Ile Arg Gly Val Ser Gly Ile Gly Val Thr
   65                  70                  75                  80
   Leu Phe Ile Ala Leu Tyr Asp Tyr Glu Ala Arg Thr Glu Asp Asp Leu
                   85                  90                  95
   Thr Phe Thr Lys Gly Glu Lys Phe His Ile Leu Asn Asn Thr Glu Gly
               100                 105                 110
   Asp Trp Trp Glu Ala Arg Ser Leu Ser Ser Gly Lys Thr Gly Cys Ile
           115                 120                 125
   Pro Ser Asn Tyr Val Ala Pro Val Asp Ser Ile Gln Ala Glu Glu Trp
       130                 135                 140
   Tyr Phe Gly Lys Ile Gly Arg Lys Asp Ala Glu Arg Gln Leu Leu Ser
   145                 150                 155                 160
   Pro Gly Asn Pro Gln Gly Ala Phe Leu Ile Arg Glu Ser Glu Thr Thr
                   165                 170                 175
   Lys Gly Ala Tyr Ser Leu Ser Ile Arg Asp Trp Asp Gln Thr Arg Gly
               180                 185                 190
   Asp His Val Lys His Tyr Lys Ile Arg Lys Leu Asp Met Gly Gly Tyr
           195                 200                 205
   Tyr Ile Thr Thr Arg Val Gln Phe Asn Ser Val Gln Glu Leu Val Gln
       210                 215                 220
   His Tyr Met Glu Val Asn Asp Gly Leu Cys Asn Leu Leu Ile Ala Pro
   225                 230                 235                 240
   Cys Thr Ile Met Lys Pro Gln Thr Leu Gly Leu Ala Lys Asp Ala Trp
                   245                 250                 255
   Glu Ile Ser Arg Ser Ser Ile Thr Leu Glu Arg Arg Leu Gly Thr Gly
               260                 265                 270
   Cys Phe Gly Asp Val Trp Leu Gly Thr Trp Asn Gly Ser Thr Lys Val
           275                 280                 285
   Ala Val Lys Thr Leu Lys Pro Gly Thr Met Ser Pro Lys Ala Phe Leu
       290                 295                 300
   Glu Glu Ala Gln Val Met Lys Leu Leu Arg His Asp Lys Leu Val Gln
   305                 310                 315                 320
   Leu Tyr Ala Val Val Ser Glu Glu Pro Ile Tyr Ile Val Thr Glu Phe
                   325                 330                 335
   Met Cys His Gly Ser Leu Leu Asp Phe Leu Lys Asn Pro Glu Gly Gln
               340                 345                 350
   Asp Leu Arg Leu Pro Gln Leu Val Asp Met Ala Ala Gln Val Ala Glu
           355                 360                 365
   Gly Met Ala Tyr Met Glu Arg Met Asn Tyr Ile His Arg Asp Leu Arg
       370                 375                 380
   Ala Ala Asn Ile Leu Val Gly Glu Arg Leu Ala Cys Lys Ile Ala Asp
   385                 390                 395                 400
   Phe Gly Leu Ala Arg Leu Ile Lys Asp Asp Glu Tyr Asn Pro Cys Gln
                   405                 410                 415
   Gly Ser Lys Phe Pro Ile Lys Trp Thr Ala Pro Glu Ala Ala Leu Phe
               420                 425                 430
   Gly Arg Phe Thr Ile Lys Ser Asp Val Trp Ser Phe Gly Ile Leu Leu
           435                 440                 445
   Thr Glu Leu Ile Thr Lys Gly Arg Ile Pro Tyr Pro Gly Met Asn Lys
       450                 455                 460
   Arg Glu Val Leu Glu Gln Val Glu Gln Gly Tyr His Met Pro Cys Pro
   465                 470                 475                 480
   Pro Gly Cys Pro Ala Ser Leu Tyr Glu Ala Met Glu Gln Thr Trp Arg
                   485                 490                 495
   Leu Asp Pro Glu Glu Arg Pro Thr Phe Glu Tyr Leu Gln Ser Phe Leu
               500                 505                 510
   Glu Asp Tyr Phe Thr Ser Ala Glu Pro Gln Tyr Gln Pro Gly Asp Gln
           515                 520                 525
   Thr
<210> 138
   <211> 459
   <212> PRT
   <213> Homo sapiens
<400> 138
   Met Glu Lys Thr Cys Ile Asp Ala Leu Pro Leu Thr Met Asn Ser Ser
    1               5                  10                  15
   Glu Lys Gln Glu Thr Val Cys Ile Phe Gly Thr Gly Asp Phe Gly Arg
               20                  25                  30
   Ser Leu Gly Leu Lys Met Leu Gln Cys Gly Tyr Ser Val Val Phe Gly
           35                  40                  45
   Ser Arg Asn Pro Gln Lys Thr Thr Leu Leu Pro Ser Gly Ala Glu Val
       50                  55                  60
   Leu Ser Tyr Ser Glu Ala Ala Lys Lys Ser Gly Ile Ile Ile Ile Ala
   65                  70                  75                  80
   Ile His Arg Glu His Tyr Asp Phe Leu Thr Glu Leu Thr Glu Val Leu
                   85                  90                  95
   Asn Gly Lys Ile Leu Val Asp Ile Ser Asn Asn Leu Lys Ile Asn Gln
               100                 105                 110
   Tyr Pro Glu Ser Asn Ala Glu Tyr Leu Ala His Leu Val Pro Gly Ala
           115                 120                 125
   His Val Val Lys Ala Phe Asn Thr Ile Ser Ala Trp Ala Leu Gln Ser
       130                 135                 140
   Gly Ala Leu Asp Ala Ser Arg Gln Val Phe Val Cys Gly Asn Asp Ser
   145                 150                 155                 160
   Lys Ala Lys Gln Arg Val Met Asp Ile Val Arg Asn Leu Gly Leu Thr
                   165                 170                 175
   Pro Met Asp Gln Gly Ser Leu Met Ala Ala Lys Glu Ile Glu Lys Tyr
               180                 185                 190
   Pro Leu Gln Leu Phe Pro Met Trp Arg Phe Pro Phe Tyr Leu Ser Ala
           195                 200                 205
   Val Leu Cys Val Phe Leu Phe Phe Tyr Cys Val Ile Arg Asp Val Ile
       210                 215                 220
   Tyr Pro Tyr Val Tyr Glu Lys Lys Asp Asn Thr Phe Arg Met Ala Ile
   225                 230                 235                 240
   Ser Ile Pro Asn Arg Ile Phe Pro Ile Thr Ala Leu Thr Leu Leu Ala
                   245                 250                 255
   Leu Val Tyr Leu Pro Gly Val Ile Ala Ala Ile Leu Gln Leu Tyr Arg
               260                 265                 270
   Gly Thr Lys Tyr Arg Arg Phe Pro Asp Trp Leu Asp His Trp Met Leu
           275                 280                 285
   Cys Arg Lys Gln Leu Gly Leu Val Ala Leu Gly Phe Ala Phe Leu His
       290                 295                 300
   Val Leu Tyr Thr Leu Val Ile Pro Ile Arg Tyr Tyr Val Arg Trp Arg
   305                 310                 315                 320
   Leu Gly Asn Leu Thr Val Thr Gln Ala Ile Leu Lys Lys Glu Asn Pro
                   325                 330                 335
   Phe Ser Thr Ser Ser Ala Trp Leu Ser Asp Ser Tyr Val Ala Leu Gly
               340                 345                 350
   Ile Leu Gly Phe Phe Leu Phe Val Leu Leu Gly Ile Thr Ser Leu Pro
           355                 360                 365
   Ser Val Ser Asn Ala Val Asn Trp Arg Glu Phe Arg Phe Val Gln Ser
       370                 375                 380
   Lys Leu Gly Tyr Leu Thr Leu Ile Leu Cys Thr Ala His Thr Leu Val
   385                 390                 395                 400
   Tyr Gly Gly Lys Arg Phe Leu Ser Pro Ser Asn Leu Arg Trp Tyr Leu
                   405                 410                 415
   Pro Ala Ala Tyr Val Leu Gly Leu Ile Ile Pro Cys Thr Val Leu Val
               420                 425                 430
   Ile Lys Phe Val Leu Ile Met Pro Cys Val Asp Asn Thr Leu Thr Arg
           435                 440                 445
   Ile Arg Gln Gly Trp Glu Arg Asn Ser Lys His
       450                 455
<210> 139
   <211> 266
   <212> PRT
   <213> Homo sapiens
<400> 139
   Met Arg Leu Leu Phe Leu Ala Val Leu Arg Pro His Thr Gly Asn Ala
    1               5                  10                  15
   Val Thr Ala Gln Arg Val Arg Ala His Leu Glu Ala Ala Gly His Val
               20                  25                  30
   Cys Val Leu Lys Asp Ala Phe Asp Phe Glu Ser Arg Ser Glu Ile Ala
           35                  40                  45
   Asn Leu Ile Leu Ala Glu Asn Cys Glu Ala Ala Leu Ala Leu His Leu
       50                  55                  60
   Tyr Arg Gly Gly Arg Leu Leu Gln Gly His Arg Ile Pro Phe Gly Val
   65                  70                  75                  80
   Ile Phe Gly Gly Thr Asp Val Asn Glu Asp Ala Asn Gln Ala Glu Lys
                   85                  90                  95
   Asn Thr Val Met Gly Arg Val Leu Glu Glu Ala Arg Phe Ala Val Ala
               100                 105                 110
   Phe Thr Glu Ser Met Lys Glu Met Ala Gln Ala Gln Trp Val Asp Pro
           115                 120                 125
   Val Phe Thr Arg Glu Val Lys Ala Lys Val Lys Arg Ala Ala Gly Val
       130                 135                 140
   Arg Leu Ile Gly Glu Met Pro Gln Glu Asp Leu His Ala Val Val Lys
   145                 150                 155                 160
   Asn Cys Phe Ala Val Val Asn Ser Ser Val Ser Glu Gly Met Ser Ala
                   165                 170                 175
   Ala Ile Leu Glu Ala Met Asp Leu Glu Val Pro Val Leu Ala Arg Asn
               180                 185                 190
   Ile Pro Gly Asn Ala Ala Val Val Lys His Glu Val Thr Gly Leu Leu
           195                 200                 205
   Phe Ser Asn Pro Gln Glu Phe Val His Leu Ala Lys Arg Leu Val Ser
       210                 215                 220
   Asp Pro Ala Leu Glu Lys Glu Ile Val Val Asn Gly Arg Glu Tyr Val
   225                 230                 235                 240
   Arg Met Tyr His Ser Trp Gln Val Glu Arg Asp Thr Tyr Gln Gln Leu
                   245                 250                 255
   Ile Arg Lys Leu Glu Gly Ser Thr Glu Asp
               260                 265
<210> 140
   <211> 350
   <212> PRT
   <213> Homo sapiens
<400> 140
   Met Glu Thr Asn Ser Ser Leu Pro Thr Asn Ile Ser Gly Gly Thr Pro
    1               5                  10                  15
   Ala Val Ser Ala Gly Tyr Leu Phe Leu Asp Ile Ile Thr Tyr Leu Val
               20                  25                  30
   Phe Ala Val Thr Phe Val Leu Gly Val Leu Gly Asn Gly Leu Val Ile
           35                  40                  45
   Trp Val Ala Gly Phe Arg Met Thr His Thr Val Thr Thr Ile Ser Tyr
       50                  55                  60
   Leu Asn Leu Ala Val Ala Asp Phe Cys Phe Thr Ser Thr Leu Pro Phe
   65                  70                  75                  80
   Phe Met Val Arg Lys Ala Met Gly Gly His Trp Pro Phe Gly Trp Phe
                   85                  90                  95
   Leu Cys Lys Phe Val Phe Thr Ile Val Asp Ile Asn Leu Phe Gly Ser
               100                 105                 110
   Val Phe Leu Ile Ala Leu Ile Ala Leu Asp Arg Cys Val Cys Val Leu
           115                 120                 125
   His Pro Val Trp Thr Gln Asn His Arg Thr Val Ser Leu Ala Lys Lys
       130                 135                 140
   Val Ile Ile Gly Pro Trp Val Met Ala Leu Leu Leu Thr Leu Pro Val
   145                 150                 155                 160
   Ile Ile Arg Val Thr Thr Val Pro Gly Lys Thr Gly Thr Val Ala Cys
                   165                 170                 175
   Thr Phe Asn Phe Ser Pro Trp Thr Asn Asp Pro Lys Glu Arg Ile Asn
               180                 185                 190
   Val Ala Val Ala Met Leu Thr Val Arg Gly Ile Ile Arg Phe Ile Ile
           195                 200                 205
   Gly Phe Ser Ala Pro Met Ser Ile Val Ala Val Ser Tyr Gly Leu Ile
       210                 215                 220
   Ala Thr Lys Ile His Lys Gln Gly Leu Ile Lys Ser Ser Arg Pro Leu
   225                 230                 235                 240
   Arg Val Leu Ser Phe Val Ala Ala Ala Phe Phe Leu Cys Trp Ser Pro
                   245                 250                 255
   Tyr Gln Val Val Ala Leu Ile Ala Thr Val Arg Ile Arg Glu Leu Leu
               260                 265                 270
   Gln Gly Met Tyr Lys Glu Ile Gly Ile Ala Val Asp Val Thr Ser Ala
           275                 280                 285
   Leu Ala Phe Phe Asn Ser Cys Leu Asn Pro Met Leu Tyr Val Phe Met
       290                 295                 300
   Gly Gln Asp Phe Arg Glu Arg Leu Ile His Ala Leu Pro Ala Ser Leu
   305                 310                 315                 320
   Glu Arg Ala Leu Thr Glu Asp Ser Thr Gln Thr Ser Asp Thr Ala Thr
                   325                 330                 335
   Asn Ser Thr Leu Pro Ser Ala Glu Val Glu Leu Gln Ala Lys
               340                 345                 350
<210> 141
   <211> 350
   <212> PRT
   <213> Homo sapiens
<400> 141
   Met Glu Thr Asn Ser Ser Leu Pro Thr Asn Ile Ser Gly Gly Thr Pro
    1               5                  10                  15
   Ala Val Ser Ala Gly Tyr Leu Phe Leu Asp Ile Ile Thr Tyr Leu Val
               20                  25                  30
   Phe Ala Val Thr Phe Val Leu Gly Val Leu Gly Asn Gly Leu Val Ile
           35                  40                  45
   Trp Val Ala Gly Phe Arg Met Thr His Thr Val Thr Thr Ile Ser Tyr
       50                  55                  60
   Leu Asn Leu Ala Val Ala Asp Phe Cys Phe Thr Ser Thr Leu Pro Phe
   65                  70                  75                  80
   Phe Met Val Arg Lys Ala Met Gly Gly His Trp Pro Phe Gly Trp Phe
                   85                  90                  95
   Leu Cys Lys Phe Val Phe Thr Ile Val Asp Ile Asn Leu Phe Gly Ser
               100                 105                 110
   Val Phe Leu Ile Ala Leu Ile Ala Leu Asp Arg Cys Val Cys Val Leu
           115                 120                 125
   His Pro Val Trp Thr Gln Asn His Arg Thr Val Ser Leu Ala Lys Lys
       130                 135                 140
   Val Ile Ile Gly Pro Trp Val Met Ala Leu Leu Leu Thr Leu Pro Val
   145                 150                 155                 160
   Ile Ile Arg Val Thr Thr Val Pro Gly Lys Thr Gly Thr Val Ala Cys
                   165                 170                 175
   Thr Phe Asn Phe Ser Pro Trp Thr Asn Asp Pro Lys Glu Arg Ile Asn
               180                 185                 190
   Val Ala Val Ala Met Leu Thr Val Arg Gly Ile Ile Arg Phe Ile Ile
           195                 200                 205
   Gly Phe Ser Ala Pro Met Ser Ile Val Ala Val Ser Tyr Gly Leu Ile
       210                 215                 220
   Ala Thr Lys Ile His Lys Gln Gly Leu Ile Lys Ser Ser Arg Pro Leu
   225                 230                 235                 240
   Arg Val Leu Ser Phe Val Ala Ala Ala Phe Phe Leu Cys Trp Ser Pro
                   245                 250                 255
   Tyr Gln Val Val Ala Leu Ile Ala Thr Val Arg Ile Arg Glu Leu Leu
               260                 265                 270
   Gln Gly Met Tyr Lys Glu Ile Gly Ile Ala Val Asp Val Thr Ser Ala
           275                 280                 285
   Leu Ala Phe Phe Asn Ser Cys Leu Asn Pro Met Leu Tyr Val Phe Met
       290                 295                 300
   Gly Gln Asp Phe Arg Glu Arg Leu Ile His Ala Leu Pro Ala Ser Leu
   305                 310                 315                 320
   Glu Arg Ala Leu Thr Glu Asp Ser Thr Gln Thr Ser Asp Thr Ala Thr
                   325                 330                 335
   Asn Ser Thr Leu Pro Ser Ala Glu Val Glu Leu Gln Ala Lys
               340                 345                 350
<210> 142.
   <211> 351
   <212> PRT
   <213> Homo sapiens
<400> 142
   Met Glu Thr Asn Phe Ser Thr Pro Leu Asn Glu Tyr Glu Glu Val Ser
   1               5                   10                  15
   Tyr Glu Ser Ala Gly Tyr Thr Val Leu Arg Ile Leu Pro Leu Val Val
               20                  25                  30
   Leu Gly Val Thr Phe Val Leu Gly Val Leu Gly Asn Gly Leu Val Ile
           35                  40                  45
   Trp Val Ala Gly Phe Arg Met Thr Arg Thr Val Thr Thr Ile Cys Tyr
       50                  55                  60
   Leu Asn Leu Ala Leu Ala Asp Phe Ser Phe Thr Ala Thr Leu Pro Phe
   65                  70                  75                  80
   Leu Ile Val Ser Met Ala Met Gly Glu Lys Trp Pro Phe Gly Trp Phe
                   85                  90                  95
   Leu Cys Lys Leu Ile His Ile Val Val Asp Ile Asn Leu Phe Gly Ser
               100                 105                 110
   Val Phe Leu Ile Gly Phe Ile Ala Leu Asp Arg Cys Ile Cys Val Leu
           115                 120                 125
   His Pro Val Trp Ala Gln Asn His Arg Thr Val Ser Leu Ala Met Lys
       130                 135                 140
   Val Ile Val Gly Pro Trp Ile Leu Ala Leu Val Leu Thr Leu Pro Val
   145                 150                 155                 160
   Phe Leu Phe Leu Thr Thr Val Thr Ile Pro Asn Gly Asp Thr Tyr Cys
                   165                 170                 175
   Thr Phe Asn Phe Ala Ser Trp Gly Gly Thr Pro Glu Glu Arg Leu Lys
               180                 185                 190
   Val Ala Ile Thr Met Leu Thr Ala Arg Gly Ile Ile Arg Phe Val Ile
           195                 200                 205
   Gly Phe Ser Leu Pro Met Ser Ile Val Ala Ile Cys Tyr Gly Leu Ile
       210                 215                 220
   Ala Ala Lys Ile His Lys Lys Gly Met Ile Lys Ser Ser Arg Pro Leu
   225                 230                 235                 240
   Arg Val Leu Thr Ala Val Val Ala Ser Phe Phe Ile Cys Trp Phe Pro
                   245                 250                 255
   Phe Gln Leu Val Ala Leu Leu Gly Thr Val Trp Leu Lys Glu Met Leu
               260                 265                 270
   Phe Tyr Gly Lys Tyr Lys Ile Ile Asp Ile Leu Val Asn Pro Thr Ser
           275                 280                 285
   Ser Leu Ala Phe Phe Asn Ser Cys Leu Asn Pro Met Leu Tyr Val Phe
       290                 295                 300
   Val Gly Gln Asp Phe Arg Glu Arg Leu Ile His Ser Leu Pro Thr Ser
   305                 310                 315                 320
   Leu Glu Arg Ala Leu Ser Glu Asp Ser Ala Pro Thr Asn Asp Thr Ala
                   325                 330                 335
   Ala Asn Ser Ala Ser Pro Pro Ala Glu Thr Glu Leu Gln Ala Met
               340                 345                 350
<211> 351
   <212> PRT
   <213> Homo sapiens
<400> 143
<210> 144
   <211> 103
   <212> PRT
   <213> Homo sapiens
<400> 144
   Met Glu Thr Val Gln Glu Leu Ile Pro Leu Ala Lys Glu Met Met Ala
    1               5                  10                  15
   Gln Lys Arg Lys Gly Lys Met Val Lys Leu Tyr Val Leu Gly Ser Val
                       20                            25                            30
   Leu Ala Leu Phe Gly Val Val Leu Gly Leu Met Glu Thr Val Cys Ser
                   35                            40                            45
   Pro Phe Thr Ala Ala Arg Arg Leu Arg Asp Gln Glu Ala Ala Val Ala
           50                            55                            60
   Glu Leu Gln Ala Ala Leu Glu Arg Gln Ala Leu Gln Lys Gln Ala Leu
   65                            70                            75                            80
   Gln Glu Lys Gly Lys Gln Gln Asp Thr Val Leu Gly Gly Arg Ala Leu
                           85                            90                            95
   Ser Asn Arg Gln His Ala Ser
                       100
<210> 145
   <211> 639
   <212> PRT
   <213> Homo sapiens
<400> 145
   Met Leu Leu Arg Lys Arg Tyr Arg His Arg Pro Cys Arg Leu Gln Phe
    1               5                  10                  15
   Leu Leu Leu Leu Leu Met Leu Gly Cys Val Leu Met Met Val Ala Met
               20                  25                  30
   Leu His Pro Pro His His Thr Leu His Gln Thr Val Thr Ala Gln Ala
           35                  40                  45
   Ser Lys His Ser Pro Glu Ala Arg Tyr Arg Leu Asp Phe Gly Glu Ser
       50                  55                  60
   Gln Asp Trp Val Leu Glu Ala Glu Asp Glu Gly Glu Glu Tyr Ser Pro
   65                  70                  75                  80
   Leu Glu Gly Leu Pro Pro Phe Ile Ser Leu Arg Glu Asp Gln Leu Leu
                   85                  90                  95
   Val Ala Val Ala Leu Pro Gln Ala Arg Arg Asn Gln Ser Gln Gly Arg
               100                 105                 110
   Arg Gly Gly Ser Tyr Arg Leu Ile Lys Gln Pro Arg Arg Gln Asp Lys
           115                 120                 125
   Glu Ala Pro Lys Arg Asp Trp Gly Ala Asp Glu Asp Gly Glu Val Ser
       130                 135                 140
   Glu Glu Glu Glu Leu Thr Pro Phe Ser Leu Asp Pro Arg Gly Leu Gln
   145                 150                 155                 160
   Glu Ala Leu Ser Ala Arg Ile Pro Leu Gln Arg Ala Leu Pro Glu Val
                   165                 170                 175
   Arg His Pro Leu Cys Leu Gln Gln His Pro Gln Asp Ser Leu Pro Thr
               180                 185                 190
   Ala Ser Val Ile Leu Cys Phe His Asp Glu Ala Trp Ser Thr Leu Leu
           195                 200                 205
   Arg Thr Val His Ser Ile Leu Asp Thr Val Pro Arg Ala Phe Leu Lys
       210                 215                 220
   Glu Ile Ile Leu Val Asp Asp Leu Ser Gln Gln Gly Gln Leu Lys Ser
   225                 230                 235                 240
   Ala Leu Ser Glu Tyr Val Ala Arg Leu Glu Gly Val Lys Leu Leu Arg
                   245                 250                 255
   Ser Asn Lys Arg Leu Gly Ala Ile Arg Ala Arg Met Leu Gly Ala Thr
               260                 265                 270
   Arg Ala Thr Gly Asp Val Leu Val Phe Met Asp Ala His Cys Glu Cys
           275                 280                 285
   His Pro Gly Trp Leu Glu Pro Leu Leu Ser Arg Ile Ala Gly Asp Arg
       290                 295                 300
   Ser Arg Val Val Ser Pro Val Ile Asp Val Ile Asp Trp Lys Thr Phe
   305                 310                 315                 320
   Gln Tyr Tyr Pro Ser Lys Asp Leu Gln Arg Gly Val Leu Asp Trp Lys
                       325                 330                 335
   Leu Asp Phe His Trp Glu Pro Leu Pro Glu His Val Arg Lys Ala Leu
                   340                 345                 350
   Gln Ser Pro Ile Ser Pro Ile Arg Ser Pro Val Val Pro Gly Glu Val
               355                 360                 365
   Val Ala Met Asp Arg His Tyr Phe Gln Asn Thr Gly Ala Tyr Asp Ser
       370                 375                 380
   Leu Met Ser Leu Arg Gly Gly Glu Asn Leu Glu Leu Ser Phe Lys Ala
   385                 390                 395                 400
   Trp Leu Cys Gly Gly Ser Val Glu Ile Leu Pro Cys Ser Arg Val Gly
                   405                 410                 415
   His Ile Tyr Gln Asn Gln Asp Ser His Ser Pro Leu Asp Gln Glu Ala
               420                 425                 430
   Thr Leu Arg Asn Arg Val Arg Ile Ala Glu Thr Trp Leu Gly Ser Phe
           435                 440                 445
   Lys Glu Thr Phe Tyr Lys His Ser Pro Glu Ala Phe Ser Leu Ser Lys
       450                 455                 460
   Ala Glu Lys Pro Asp Cys Met Glu Arg Leu Gln Leu Gln Arg Arg Leu
   465                 470                 475                 480
   Gly Cys Arg Thr Phe His Trp Phe Leu Ala Asn Val Tyr Pro Glu Leu
                   485                 490                 495
   Tyr Pro Ser Glu Pro Arg Pro Ser Phe Ser Gly Lys Leu His Asn Thr
               500                 505                 510
   Gly Leu Gly Leu Cys Ala Asp Cys Gln Ala Glu Gly Asp Ile Leu Gly
           515                 520                 525
   Cys Pro Met Val Leu Ala Pro Cys Ser Asp Ser Arg Gln Gln Gln Tyr
       530                 535                 540
   Leu Gln His Thr Ser Arg Lys Glu Ile His Phe Gly Ser Pro Gln His
   545                 550                 555                 560
   Leu Cys Phe Ala Val Arg Gln Glu Gln Val Ile Leu Gln Asn Cys Thr
                   565                 570                 575
   Glu Glu Gly Leu Ala Ile His Gln Gln His Trp Asp Phe Gln Glu Asn
               580                 585                 590
   Gly Met Ile Val His Ile Leu Ser Gly Lys Cys Met Glu Ala Val Val
           595                 600                 605
   Gln Glu Asn Asn Lys Asp Leu Tyr Leu Arg Pro Cys Asp Gly Lys Ala
       610                 615                 620
   Arg Gln Gln Trp Arg Phe Asp Gln Ile Asn Ala Val Asp Glu Arg
   625                 630                 635
<210> 146
   <211> 682
   <212> PRT
   <213> Homo sapiens
<400> 146
   Met Cys Gly Ile Phe Ala Tyr Met Asn Tyr Arg Val Pro Arg Thr Arg
    1              5                  10                  15
   Lys Glu Ile Phe Glu Thr Leu Ile Lys Gly Leu Gln Arg Leu Glu Tyr
               20                  25                  30
   Arg Gly Tyr Asp Ser Ala Gly Val Ala Ile Asp Gly Asn Asn His Glu
           35                  40                  45
   Val Lys Glu Arg His Ile Gln Leu Val Lys Lys Arg Gly Lys Val Lys
       50                  55                  60
   Ala Leu Asp Glu Glu Leu Tyr Lys Gln Asp Ser Met Asp Leu Lys Val
   65                  70                  75                  80
   Glu Phe Glu Thr His Phe Gly Ile Ala His Thr Arg Trp Ala Thr His
                   85                  90                  95
   Gly Val Pro Ser Ala Val Asn Ser His Pro Gln Arg Ser Asp Lys Gly
                   100                 105                 110
   Asn Glu Phe Val Val Ile His Asn Gly Ile Ile Thr Asn Tyr Lys Asp
               115                 120                 125
   Leu Arg Lys Phe Leu Glu Ser Lys Gly Tyr Glu Phe Glu Ser Glu Thr
       130                 135                 140
   Asp Thr Glu Thr Ile Ala Lys Leu Ile Lys Tyr Val Phe Asp Asn Arg
   145                 150                 155                 160
   Glu Thr Glu Asp Ile Thr Phe Ser Thr Leu Val Glu Arg Val Ile Gln
                   165                 170                 175
   Gln Leu Glu Gly Ala Phe Ala Leu Val Phe Lys Ser Val His Tyr Pro
               180                 185                 190
   Gly Glu Ala Val Ala Thr Arg Arg Gly Ser Pro Leu Leu Ile Gly Val
           195                 200                 205
   Arg Ser Lys Tyr Lys Leu Ser Thr Glu Gln Ile Pro Ile Leu Tyr Arg
       210                 215                 220
   Thr Cys Thr Leu Glu Asn Val Lys Asn Ile Cys Lys Thr Arg Met Lys
   225                 230                 235                 240
   Arg Leu Asp Ser Ser Ala Cys Leu His Ala Val Gly Asp Lys Ala Val
                   245                 250                 255
   Glu Phe Phe Phe Ala Ser Asp Ala Ser Ala Ile Ile Glu His Thr Asn
               260                 265                 270
   Arg Val Ile Phe Leu Glu Asp Asp Asp Ile Ala Ala Val Ala Asp Gly
           275                 280                 285
   Lys Leu Ser Ile His Arg Val Lys Arg Ser Ala Ser Asp Asp Pro Ser
       290                 295                 300
   Arg Ala Ile Gln Thr Leu Gln Met Glu Leu Gln Gln Ile Met Lys Gly
   305                 310                 315                 320
   Asn Phe Ser Ala Phe Met Gln Lys Glu Ile Phe Glu Gln Pro Glu Ser
                   325                 330                 335
   Val Phe Asn Thr Met Arg Gly Arg Val Asn Phe Glu Thr Asn Thr Val
               340                 345                 350
   Leu Leu Gly Gly Leu Lys Asp His Leu Lys Glu Ile Arg Arg Cys Arg
           355                 360                 365
   Arg Leu Ile Val Ile Gly Cys Gly Thr Ser Tyr His Ala Ala Val Ala
       370                 375                 380
   Thr Arg Gln Val Leu Glu Glu Leu Thr Glu Leu Pro Val Met Val Glu
   385                 390                 395                 400
   Leu Ala Ser Asp Phe Leu Asp Arg Asn Thr Pro Val Phe Arg Asp Asp
                   405                 410                 415
   Val Cys Phe Phe Ile Ser Gln Ser Gly Glu Thr Ala Asp Thr Leu Leu
               420                 425                 430
   Ala Leu Arg Tyr Cys Lys Asp Arg Gly Ala Leu Thr Val Gly Val Thr
           435                 440                 445
   Asn Thr Val Gly Ser Ser Ile Ser Arg Glu Thr Asp Cys Gly Val His
       450                 455                 460
   Ile Asn Ala Gly Pro Glu Ile Gly Val Ala Ser Thr Lys Ala Tyr Thr
   465                 470                 475                 480
   Ser Gln Phe Ile Ser Leu Val Met Phe Gly Leu Met Met Ser Glu Asp
                   485                 490                 495
   Arg Ile Ser Leu Gln Asn Arg Arg Gln Glu Ile Ile Arg Gly Leu Arg
               500                 505                 510
   Ser Leu Pro Glu Leu Ile Lys Glu Val Leu Ser Leu Glu Glu Lys Ile
           515                 520                 525
   His Asp Leu Ala Leu Glu Leu Tyr Thr Gln Arg Ser Leu Leu Val Met
       530                 535                 540
   Gly Arg Gly Tyr Asn Tyr Ala Thr Cys Leu Glu Gly Ala Leu Lys Ile
   545                 550                 555                 560
   Lys Glu Ile Thr Tyr Met His Ser Glu Gly Ile Leu Ala Gly Glu Leu
                   565                 570                 575
   Lys His Gly Pro Leu Ala Leu Ile Asp Lys Gln Met Pro Val Ile Met
                       580                 585                 590
   Val Ile Met Lys Asp Pro Cys Phe Ala Lys Cys Gln Asn Ala Leu Gln
               595                 600                 605
   Gln Val Thr Ala Arg Gln Gly Arg Pro Ile Ile Leu Cys Ser Lys Asp
       610                 615                 620
   Asp Thr Glu Ser Ser Lys Phe Ala Tyr Lys Thr Ile Glu Leu Pro His
   625                 630                 635                 640
   Thr Val Asp Cys Leu Gln Gly Ile Leu Ser Val Ile Pro Leu Gln Leu
                   645                 650                 655
   Leu Ser Phe His Leu Ala Val Leu Arg Gly Tyr Asp Val Asp Phe Pro
               660                 665                 670
   Arg Asn Leu Ala Lys Ser Val Thr Val Glu
           675                 680
<210> 147
   <211> 553
   <212> PRT
   <213> Homo sapiens
<400> 147
   Met Ala Ala Ser Lys Lys Ala Val Leu Gly Pro Leu Val Gly Ala Val
    1               5                  10                  15
   Asp Gln Gly Thr Ser Ser Thr Arg Phe Leu Val Phe Asn Ser Lys Thr
               20                  25                  30
   Ala Glu Leu Leu Ser His His Gln Val Glu Ile Lys Gln Glu Phe Pro
           35                  40                  45
   Arg Glu Gly Trp Val Glu Gln Asp Pro Lys Glu Ile Leu His Ser Val
       50                  55                  60
   Tyr Glu Cys Ile Glu Lys Thr Cys Glu Lys Leu Gly Gln Leu Asn Ile
   65                  70                  75                  80
   Asp Ile Ser Asn Ile Lys Ala Ile Gly Val Ser Asn Gln Arg Glu Thr
                   85                  90                  95
   Thr Val Val Trp Asp Lys Ile Thr Gly Glu Pro Leu Tyr Asn Ala Val
               100                 105                 110
   Val Trp Leu Asp Leu Arg Thr Gln Ser Thr Val Glu Ser Leu Ser Lys
           115                 120                 125
   Arg Ile Pro Gly Asn Asn Asn Phe Val Lys Ser Lys Thr Gly Leu Pro
       130                 135                 140
   Leu Ser Thr Tyr Phe Ser Ala Val Lys Leu Arg Trp Leu Leu Asp Asn
   145                 150                 155                 160
   Val Arg Lys Val Gln Lys Ala Val Glu Glu Lys Arg Ala Leu Phe Gly
                   165                 170                 175
   Thr Ile Asp Ser Trp Leu Ile Trp Ser Leu Thr Gly Gly Val Asn Gly
               180                 185                 190
   Gly Val His Cys Thr Asp Val Thr Asn Ala Ser Arg Thr Met Leu Phe
           195                 200                 205
   Asn Ile His Ser Leu Glu Trp Asp Lys Gln Leu Cys Glu Phe Phe Gly
       210                 215                 220
   Ile Pro Met Glu Ile Leu Pro Asn Val Arg Ser Ser Ser Glu Ile Tyr
   225                 230                 235                 240
   Gly Leu Met Lys Ala Gly Ala Leu Glu Gly Val Pro Ile Ser Gly Cys
                   245                 250                 255
   Leu Gly Asp Gln Ser Ala Ala Leu Val Gly Gln Met Cys Phe Gln Ile
               260                 265                 270
   Gly Gln Ala Lys Asn Thr Tyr Gly Thr Gly Cys Phe Leu Leu Cys Asn
           275                 280                 285
   Thr Gly His Lys Cys Val Phe Ser Asp His Gly Leu Leu Thr Thr Val
       290                 295                 300
   Ala Tyr Lys Leu Gly Arg Asp Lys Pro Val Tyr Tyr Ala Leu Glu Gly
   305                 310                 315                 320
   Ser Val Ala Ile Ala Gly Ala Val Ile Arg Trp Leu Arg Asp Asn Leu
                   325                 330                 335
   Gly Ile Ile Lys Thr Ser Glu Glu Ile Glu Lys Leu Ala Lys Glu Val
               340                 345                 350
   Gly Thr Ser Tyr Gly Cys Tyr Phe Val Pro Ala Phe Ser Gly Leu Tyr
           355                 360                 365
   Ala Pro Tyr Trp Glu Pro Ser Ala Arg Gly Ile Ile Cys Gly Leu Thr
       370                 375                 380
   Gln Phe Thr Asn Lys Cys His Ile Ala Phe Ala Ala Leu Glu Ala Val
   385                 390                 395                 400
   Cys Phe Gln Thr Arg Glu Ile Leu Asp Ala Met Asn Arg Asp Cys Gly
                   405                 410                 415
   Ile Pro Leu Ser His Leu Gln Val Asp Gly Gly Met Thr Ser Asn Lys
               420                 425                 430
   Ile Leu Met Gln Leu Gln Ala Asp Ile Leu Tyr Ile Pro Val Val Lys
           435                 440                 445
   Pro Ser Met Pro Glu Thr Thr Ala Leu Gly Ala Ala Met Ala Ala Gly
       450                 455                 460
   Ala Ala Glu Gly Val Gly Val Trp Ser Leu Glu Pro Glu Asp Leu Ser
   465                 470                 475                 480
   Ala Val Thr Met Glu Arg Phe Glu Pro Gln Ile Asn Ala Glu Glu Ser
                   485                 490                 495
   Glu Ile Arg Tyr Ser Thr Trp Lys Lys Ala Val Met Lys Ser Met Gly
               500                 505                 510
   Trp Val Thr Thr Gln Ser Pro Glu Ser Gly Asp Pro Ser Ile Phe Cys
           515                 520                 525
   Ser Leu Pro Leu Gly Phe Phe Ile Val Ser Ser Met Val Met Leu Ile
       530                 535                 540
   Gly Ala Arg Tyr Ile Ser Gly Ile Pro
   545                 550
<210> 148
   <211> 387
   <212> PRT
   <213> Homo sapiens
<400> 148
   Met Asn Arg His His Leu Gln Asp His Phe Leu Glu Ile Asp Lys Lys
    1               5                  10                  15
   Asn Cys Cys Val Phe Arg Asp Asp Phe Ile Ala Lys Val Leu Pro Pro
               20                  25                  30
   Val Leu Gly Leu Glu Phe Ile Phe Gly Leu Leu Gly Asn Gly Leu Ala
           35                  40                  45
   Leu Trp Ile Phe Cys Phe His Leu Lys Ser Trp Lys Ser Ser Arg Ile
       50                  55                  60
   Phe Leu Phe Asn Leu Ala Val Ala Asp Phe Leu Leu Ile Ile Cys Leu
   65                  70                  75                  80
   Pro Phe Val Met Asp Tyr Tyr Val Arg Arg Ser Asp Trp Lys Phe Gly
                   85                  90                  95
   Asp Ile Pro Cys Arg Leu Val Leu Phe Met Phe Ala Met Asn Arg Gln
               100                 105                 110
   Gly Ser Ile Ile Phe Leu Thr Val Val Ala Val Asp Arg Tyr Phe Arg
           115                 120                 125
   Val Val His Pro His His Ala Leu Asn Lys Ile Ser Asn Trp Thr Ala
       130                 135                 140
   Ala Ile Ile Ser Cys Leu Leu Trp Gly Ile Thr Val Gly Leu Thr Val
   145                 150                 155                 160
   His Leu Leu Lys Lys Lys Leu Leu Ile Gln Asn Gly Thr Ala Asn Val
                   165                 170                 175
   Cys Ile Ser Phe Ser Ile Cys His Thr Phe Arg Trp His Glu Ala Met
               180                 185                 190
   Phe Leu Leu Glu Phe Phe Leu Pro Leu Gly Ile Ile Leu Phe Cys Ser
           195                 200                 205
   Ala Arg Ile Ile Trp Ser Leu Arg Gln Arg Gln Met Asp Arg His Ala
       210                 215                 220
   Lys Ile Lys Arg Ala Ile Thr Phe Ile Met Val Val Ala Ile Val Phe
   225                 230                 235                 240
   Val Ile Cys Phe Leu Pro Ser Val Val Val Arg Ile His Ile Phe Trp
                   245                 250                 255
   Leu Leu His Thr Ser Gly Thr Gln Asn Cys Glu Val Tyr Arg Ser Val
               260                 265                 270
   Asp Leu Ala Phe Phe Ile Thr Leu Ser Phe Thr Tyr Met Asn Ser Met
           275                 280                 285
   Leu Asp Pro Val Val Tyr Tyr Phe Ser Ser Pro Ser Phe Pro Asn Phe
       290                 295                 300
   Phe Ser Thr Leu Ile Asn Arg Cys Leu Gln Arg Lys Ile Thr Gly Glu
   305                 310                 315                 320
   Pro Asp Asn Asn Arg Ser Thr Ser Val Glu Leu Thr Gly Asp Pro Asn
                   325                 330                 335
   Lys Thr Arg Gly Ala Pro Glu Ala Leu Ile Ala Asn Ser Gly Glu Pro
               340                 345                 350
   Trp Ser Pro Ser Tyr Leu Gly Pro Thr Ser Asn Asn His Ser Lys Lys
           355                 360                 365
   Gly His Cys His Gln Glu Pro Ala Ser Leu Glu Lys Gln Leu Gly Cys
       370                 375                 380
   Cys Ile Glu
   385
<210> 149
   <211> 549
   <212> PRT
   <213> Homo sapiens
<400> 149
   Met Ala Thr Pro Arg Gly Leu Gly Ala Leu Leu Leu Leu Leu Leu Leu
    1               5                  10                  15
   Pro Thr Ser Gly Gln Glu Lys Pro Thr Glu Gly Pro Arg Asn Thr Cys
               20                  25                  30
   Leu Gly Ser Asn Asn Met Tyr Asp Ile Phe Asn Leu Asn Asp Lys Ala
           35                  40                  45
   Leu Cys Phe Thr Lys Cys Arg Gln Ser Gly Ser Asp Ser Cys Asn Val
       50                  55                  60
   Glu Asn Leu Gln Arg Tyr Trp Leu Asn Tyr Glu Ala His Leu Met Lys
   65                  70                  75                  80
   Glu Gly Leu Thr Gln Lys Val Asn Thr Pro Phe Leu Lys Ala Leu Val
                   85                  90                  95
   Gln Asn Leu Ser Thr Asn Thr Ala Glu Asp Phe Tyr Phe Ser Leu Glu
               100                 105                 110
   Pro Ser Gln Val Pro Arg Gln Val Met Lys Asp Glu Asp Lys Pro Pro
           115                 120                 125
   Asp Arg Val Arg Leu Pro Lys Ser Leu Phe Arg Ser Leu Pro Gly Asn
       130                 135                 140
   Arg Ser Val Val Arg Leu Ala Val Thr Ile Leu Asp Ile Gly Pro Gly
   145                 150                 155                 160
   Thr Leu Phe Lys Gly Pro Arg Leu Gly Leu Gly Asp Gly Ser Gly Val
                   165                 170                 175
   Leu Asn Asn Arg Leu Val Gly Leu Ser Val Gly Gln Met His Val Thr
   180 185 190
   Lys Leu Ala Glu Pro Leu Glu Ile Val Phe Ser His Gln Arg Pro Pro
   195 200 205
   Pro Asn Met Thr Leu Thr Cys Val Phe Trp Asp Val Thr Lys Gly Thr
   210 215 220
   Thr Gly Asp Trp Ser Ser Glu Gly Cys Ser Thr Glu Val Arg Pro Glu
   225 230 235 240
   Gly Thr Val Cys Cys Cys Asp His Leu Thr Phe Phe Ala Leu Leu Leu
   245 250 255
   Arg Pro Thr Leu Asp Gln Ser Thr Val His Ile Leu Thr Arg Ile Ser
   260 265 270
   Gln Ala Gly Cys Gly Val Ser Met Ile Phe Leu Ala Phe Thr Ile Ile
   275 280 285
   Leu Tyr Ala Phe Leu Arg Leu Ser Arg Glu Arg Phe Lys Ser Glu Asp
   290 295 300
   Ala Pro Lys Ile His Val Ala Leu Gly Gly Ser Leu Phe Leu Leu Asn
   305 310 315 320
   Leu Ala Phe Leu Val Asn Val Gly Ser Gly Ser Lys Gly Ser Asp Ala
   325 330 335
   Ala Cys Trp Ala Arg Gly Ala Val Phe His Tyr Phe Leu Leu Cys Ala
   340 345 350
   Phe Thr Trp Met Gly Leu Glu Ala Phe His Leu Tyr Leu Leu Ala Val
   355 360 365
   Arg Val Phe Asn Thr Tyr Phe Gly His Tyr Phe Leu Lys Leu Ser Leu
   370 375 380
   Val Gly Trp Gly Leu Pro Ala Leu Met Val Ile Gly Thr Gly Ser Ala
   385 390 395 400
   Asn Ser Tyr Gly Leu Tyr Thr Ile Arg Asp Arg Glu Asn Arg Thr Ser
   405 410 415
   Leu Glu Leu Cys Trp Phe Arg Glu Gly Thr Thr Met Tyr Ala Leu Tyr
   420 425 430
   Ile Thr Val His Gly Tyr Phe Leu Ile Thr Phe Leu Phe Gly Met Val
   435 440 445
   Val Leu Ala Leu Val Val Trp Lys Ile Phe Thr Leu Ser Arg Ala Thr
   450 455 460
   Ala Val Lys Glu Arg Gly Lys Asn Arg Lys Lys Val Leu Thr Leu Leu
   465 470 475 480
   Gly Leu Ser Ser Leu Val Gly Val Thr Trp Gly Leu Ala Ile Phe Thr
   485 490 495
   Pro Leu Gly Leu Ser Thr Val Tyr Ile Phe Ala Leu Phe Asn Ser Leu
   500 505 510
   Gln Gly Val Phe Ile Cys Cys Trp Phe Thr Ile Leu Tyr Leu Pro Ser
   515 520 525
   Gln Ser Thr Thr Val Ser Ser Ser Thr Ala Arg Leu Asp Gln Ala His
   530 535 540
   Ser Ala Ser Gln Glu
   545
<210> 150
   <211> 549
   <212> PRT
   <213> Homo sapiens
<400> 150
   Met Ala Thr Pro Arg Gly Leu Gly Ala Leu Leu Leu Leu Leu Leu Leu
    1               5                  10                  15
   Pro Thr Ser Gly Gln Glu Lys Pro Thr Glu Gly Pro Arg Asn Thr Cys
               20                  25                  30
   Leu Gly Ser Asn Asn Met Tyr Asp Ile Phe Asn Leu Asn Asp Lys Ala
           35                  40                  45
   Leu Cys Phe Thr Lys Cys Arg Gln Ser Gly Ser Asp Ser Cys Asn Val
       50                  55                  60
   Glu Asn Leu Gln Arg Tyr Trp Leu Asn Tyr Glu Ala His Leu Met Lys
   65                  70                  75                  80
   Glu Gly Leu Thr Gln Lys Val Asn Thr Pro Phe Leu Lys Ala Leu Val
                   85                  90                  95
   Gln Asn Leu Ser Thr Asn Thr Ala Glu Asp Phe Tyr Phe Ser Leu Glu
               100                 105                 110
   Pro Ser Gln Val Pro Arg Gln Val Met Lys Asp Glu Asp Lys Pro Pro
           115                 120                 125
   Asp Arg Val Arg Leu Pro Lys Ser Leu Phe Arg Ser Leu Pro Gly Asn
       130                 135                 140
   Arg Ser Val Val Arg Leu Ala Val Thr Ile Leu Asp Ile Gly Pro Gly
   145                 150                 155                 160
   Thr Leu Phe Lys Gly Pro Arg Leu Gly Leu Gly Asp Gly Ser Gly Val
                   165                 170                 175
   Leu Asn Asn Arg Leu Val Gly Leu Ser Val Gly Gln Met His Val Thr
               180                 185                 190
   Lys Leu Ala Glu Pro Leu Glu Ile Val Phe Ser His Gln Arg Pro Pro
           195                 200                 205
   Pro Asn Met Thr Leu Thr Cys Val Phe Trp Asp Val Thr Lys Gly Thr
       210                 215                 220
   Thr Gly Asp Trp Ser Ser Glu Gly Cys Ser Thr Glu Val Arg Pro Glu
   225                 230                 235                 240
   Gly Thr Val Cys Cys Cys Asp His Leu Thr Phe Phe Ala Leu Leu Leu
                   245                 250                 255
   Arg Pro Thr Leu Asp Gln Ser Thr Val His Ile Leu Thr Arg Ile Ser
               260                 265                 270
   Gln Ala Gly Cys Gly Val Ser Met Ile Phe Leu Ala Phe Thr Ile Ile
           275                 280                 285
   Leu Tyr Ala Phe Leu Arg Leu Ser Arg Glu Arg Phe Lys Ser Glu Asp
       290                 295                 300
   Ala Pro Lys Ile His Val Ala Leu Gly Gly Ser Leu Phe Leu Leu Asn
   305                 310                 315                 320
   Leu Ala Phe Leu Val Asn Val Gly Ser Gly Ser Lys Gly Ser Asp Ala
                   325                 330                 335
   Ala Cys Trp Ala Arg Gly Ala Val Phe His Tyr Phe Leu Leu Cys Ala
               340                 345                 350
   Phe Thr Trp Met Gly Leu Glu Ala Phe His Leu Tyr Leu Leu Ala Val
           355                 360                 365
   Arg Val Phe Asn Thr Tyr Phe Gly His Tyr Phe Leu Lys Leu Ser Leu
       370                 375                 380
   Val Gly Trp Gly Leu Pro Ala Leu Met Val Ile Gly Thr Gly Ser Ala
   385                 390                 395                 400
   Asn Ser Tyr Gly Leu Tyr Thr Ile Arg Asp Arg Glu Asn Arg Thr Ser
                   405                 410                 415
   Leu Glu Leu Cys Trp Phe Arg Glu Gly Thr Thr Met Tyr Ala Leu Tyr
               420                 425                 430
   Ile Thr Val His Gly Tyr Phe Leu Ile Thr Phe Leu Phe Gly Met Val
           435                 440                 445
   Val Leu Ala Leu Val Val Trp Lys Ile Phe Thr Leu Ser Arg Ala Thr
       450                 455                 460
   Ala Val Lys Glu Arg Gly Lys Asn Arg Lys Lys Val Leu Thr Leu Leu
   465                 470                 475                 480
   Gly Leu Ser Ser Leu Val Gly Val Thr Trp Gly Leu Ala Ile Phe Thr
                   485                 490                 495
   Pro Leu Gly Leu Ser Thr Val Tyr Ile Phe Ala Leu Phe Asn Ser Leu
               500                 505                 510
   Gln Gly Val Phe Ile Cys Cys Trp Phe Thr Ile Leu Tyr Leu Pro Ser
           515                 520                 525
   Gln Ser Thr Thr Val Ser Ser Ser Thr Ala Arg Leu Asp Gln Ala His
       530                 535                 540
   Ser Ala Ser Gln Glu
   545
<210> 151
   <211> 354
   <212> PRT
   <213> Homo sapiens
<400> 151
   Met Lys Ser Leu Leu Leu Leu Val Leu Ile Ser Ile Cys Trp Ala Asp
    1               5                  10                  15
   His Leu Ser Asp Asn Tyr Thr Leu Asp His Asp Arg Ala Ile His Ile
               20                  25                  30
   Gln Ala Glu Asn Gly Pro His Leu Leu Val Glu Ala Glu Gln Ala Lys
           35                  40                  45
   Val Phe Ser His Arg Gly Gly Asn Val Thr Leu Pro Cys Lys Phe Tyr
       50                  55                  60
   Arg Asp Pro Thr Ala Phe Gly Ser Gly Ile His Lys Ile Arg Ile Lys
   65                  70                  75                  80
   Trp Thr Lys Leu Thr Ser Asp Tyr Leu Lys Glu Val Asp Val Phe Val
                   85                  90                  95
   Ser Met Gly Tyr His Lys Lys Thr Tyr Gly Gly Tyr Gln Gly Arg Val
               100                 105                 110
   Phe Leu Lys Gly Gly Ser Asp Ser Asp Ala Ser Leu Val Ile Thr Asp
           115                 120                 125
   Leu Thr Leu Glu Asp Tyr Gly Arg Tyr Lys Cys Glu Val Ile Glu Gly
       130                 135                 140
   Leu Glu Asp Asp Thr Val Val Val Ala Leu Asp Leu Gln Gly Val Val
   145                 150                 155                 160
   Phe Pro Tyr Phe Pro Arg Leu Gly Arg Tyr Asn Leu Asn Phe His Glu
                   165                 170                 175
   Ala Gln Gln Ala Cys Leu Asp Gln Asp Ala Val Ile Ala Ser Phe Asp
               180                 185                 190
   Gln Leu Tyr Asp Ala Trp Arg Gly Gly Leu Asp Trp Cys Asn Ala Gly
           195                 200                 205
   Trp Leu Ser Asp Gly Ser Val Gln Tyr Pro Ile Thr Lys Pro Arg Glu
       210                 215                 220
   Pro Cys Gly Gly Gln Asn Thr Val Pro Gly Val Arg Asn Tyr Gly Phe
   225                 230                 235                 240
   Trp Asp Lys Asp Lys Ser Arg Tyr Asp Val Phe Cys Phe Thr Ser Asn
                   245                 250                 255
   Phe Asn Gly Arg Phe Tyr Tyr Leu Ile His Pro Thr Lys Leu Thr Tyr
               260                 265                 270
   Asp Glu Ala Val Gln Ala Cys Leu Asn Asp Gly Ala Gln Ile Ala Lys
           275                 280                 285
   Val Gly Gln Ile Phe Ala Ala Trp Lys Ile Leu Gly Tyr Asp Arg Cys
       290                 295                 300
   Asp Ala Gly Trp Leu Ala Asp Gly Ser Val Arg Tyr Pro Ile Ser Arg
   305                 310                 315                 320
   Pro Arg Arg Arg Cys Ser Pro Thr Glu Ala Ala Val Arg Phe Val Gly
                   325                 330                 335
   Phe Pro Asp Lys Lys His Lys Leu Tyr Gly Val Tyr Cys Phe Arg Ala
               340                 345                 350
   Tyr Asn
<210> 152
   <211> 210
   <212> PRT
   <213> Homo sapiens
<400> 152
   Met Trp Val Thr Lys Leu Leu Pro Ala Leu Leu Leu Gln His Val Leu
    1               5                  10                  15
   Leu His Leu Leu Leu Leu Pro Ile Ala Ile Pro Tyr Ala Glu Gly Gln
               20                  25                  30
   Arg Lys Arg Arg Asn Thr Ile His Glu Phe Lys Lys Ser Ala Lys Thr
           35                  40                  45
   Thr Leu Ile Lys Ile Asp Pro Ala Leu Lys Ile Lys Thr Lys Lys Val
       50                  55                  60
   Asn Thr Ala Asp Gln Cys Ala Asn Arg Cys Thr Arg Asn Lys Gly Leu
   65                  70                  75                  80
   Pro Phe Thr Cys Lys Ala Phe Val Phe Asp Lys Ala Arg Lys Gln Cys
                   85                  90                  95
   Leu Trp Phe Pro Phe Asn Ser Met Ser Ser Gly Val Lys Lys Glu Phe
               100                 105                 110
   Gly His Glu Phe Asp Leu Tyr Glu Asn Lys Asp Tyr Ile Arg Asn Cys
           115                 120                 125
   Ile Ile Gly Lys Gly Arg Ser Tyr Lys Gly Thr Val Ser Ile Thr Lys
       130                 135                 140
   Ser Gly Ile Lys Cys Gln Pro Trp Ser Ser Met Ile Pro His Glu His
   145                 150                 155                 160
   Ser Phe Leu Pro Ser Ser Tyr Arg Gly Lys Asp Leu Gln Glu Asn Tyr
                   165                 170                 175
   Cys Arg Asn Pro Arg Gly Glu Glu Gly Gly Pro Trp Cys Phe Thr Ser
               180                 185                 190
   Asn Pro Glu Val Arg Tyr Glu Val Cys Asp Ile Pro Gln Cys Ser Glu
           195                 200                 205
   Gly Lys
       210
<210> 153
   <211> 132
   <212> PRT
   <213> Homo sapiens
<400> 153
   Met Asn His Ile Val Gln Thr Phe Ser Pro Val Asn Ser Gly Gln Pro
   1               5                   10                  15
   Pro Asn Tyr Glu Met Leu Lys Glu Glu Gln Glu Val Ala Met Leu Gly
               20                  25                  30
   Val Pro His Asn Pro Ala Pro Pro Met Ser Thr Val Ile His Ile Arg
           35                  40                  45
   Ser Glu Thr Ser Val Pro Asp His Val Val Trp Ser Leu Phe Asn Thr
       50                  55                  60
   Leu Phe Met Asn Thr Cys Cys Leu Gly Phe Ile Ala Phe Ala Tyr Ser
   65                  70                  75                  80
   Val Lys Ser Arg Asp Arg Lys Met Val Gly Asp Val Thr Gly Ala Gln
                   85                  90                  95
   Ala Tyr Ala Ser Thr Ala Lys Cys Leu Asn Ile Trp Ala Leu Ile Leu
               100                 105                 110
   Gly Ile Phe Met Thr Ile Leu Leu Ile Ile Ile Pro Val Leu Val Val
           115                 120                 125
   Gln Ala Gln Arg
       130
<210> 154
   <211> 199
   <212> PRT
   <213> Homo sapiens
<400> 154
   Met Asn Cys Val Cys Arg Leu Val Leu Val Val Leu Ser Leu Trp Pro
   1               5                   10                  15
   Asp Thr Ala Val Ala Pro Gly Pro Pro Pro Gly Pro Pro Arg Val Ser
               20                  25                  30
   Pro Asp Pro Arg Ala Glu Leu Asp Ser Thr Val Leu Leu Thr Arg Ser
           35                  40                  45
   Leu Leu Ala Asp Thr Arg Gln Leu Ala Ala Gln Leu Arg Asp Lys Phe
       50                  55                  60
   Pro Ala Asp Gly Asp His Asn Leu Asp Ser Leu Pro Thr Leu Ala Met
   65                  70                  75                  80
   Ser Ala Gly Ala Leu Gly Ala Leu Gln Leu Pro Gly Val Leu Thr Arg
                   85                  90                  95
   Leu Arg Ala Asp Leu Leu Ser Tyr Leu Arg His Val Gln Trp Leu Arg
               100                 105                 110
   Arg Ala Gly Gly Ser Ser Leu Lys Thr Leu Glu Pro Glu Leu Gly Thr
           115                 120                 125
   Leu Gln Ala Arg Leu Asp Arg Leu Leu Arg Arg Leu Gln Leu Leu Met
       130                 135                 140
   Ser Arg Leu Ala Leu Pro Gln Pro Pro Pro Asp Pro Pro Ala Pro Pro
   145                 150                 155                 160
   Leu Ala Pro Pro Ser Ser Ala Trp Gly Gly Ile Arg Ala Ala His Ala
                   165                 170                 175
   Ile Leu Gly Gly Leu His Leu Thr Leu Asp Trp Ala Val Arg Gly Leu
               180                 185                 190
   Leu Leu Leu Lys Thr Arg Leu
           195
<210> 155
   <211> 269
   <212> PRT
   <213> Homo sapiens
<400> 155
   Met Ala Glu Val Pro Glu Leu Ala Ser Glu Met Met Ala Tyr Tyr Ser
    1               5                  10                  15
   Gly Asn Glu Asp Asp Leu Phe Phe Glu Ala Asp Gly Pro Lys Gln Met
               20                  25                  30
   Lys Cys Ser Phe Gln Asp Leu Asp Leu Cys Pro Leu Asp Gly Gly Ile
           35                  40                  45
   Gln Leu Arg Ile Ser Asp His His Tyr Ser Lys Gly Phe Arg Gln Ala
       50                  55                  60
   Ala Ser Val Val Val Ala Met Asp Lys Leu Arg Lys Met Leu Val Pro
   65                  70                  75                  80
   Cys Pro Gln Thr Phe Gln Glu Asn Asp Leu Ser Thr Phe Phe Pro Phe
                   85                  90                  95
   Ile Phe Glu Glu Glu Pro Ile Phe Phe Asp Thr Trp Asp Asn Glu Ala
               100                 105                 110
   Tyr Val His Asp Ala Pro Val Arg Ser Leu Asn Cys Thr Leu Arg Asp
           115                 120                 125
   Ser Gln Gln Lys Ser Leu Val Met Ser Gly Pro Tyr Glu Leu Lys Ala
       130                 135                 140
   Leu His Leu Gln Gly Gln Asp Met Glu Gln Gln Val Val Phe Ser Met
   145                 150                 155                 160
   Ser Phe Val Gln Gly Glu Glu Ser Asn Asp Lys Ile Pro Val Ala Leu
                   165                 170                 175
   Gly Leu Lys Glu Lys Asn Leu Tyr Leu Ser Cys Val Leu Lys Asp Asp
               180                 185                 190
   Lys Pro Thr Leu Gln Leu Glu Ser Val Asp Pro Lys Asn Tyr Pro Lys
           195                 200                 205
   Lys Lys Met Glu Lys Arg Phe Val Phe Asn Lys Ile Glu Ile Asn Asn
       210                 215                 220
   Lys Leu Glu Phe Glu Ser Ala Gln Phe Pro Asn Trp Tyr Ile Ser Thr
   225                 230                 235                 240
   Ser Gln Ala Glu Asn Met Pro Val Phe Leu Gly Gly Thr Lys Gly Gly
                   245                 250                 255
   Gln Asp Ile Thr Asp Phe Thr Met Gln Phe Val Ser Ser
               260                 265
<210> 156
   <211> 180
   <212> PRT
   <213> Homo sapiens
<400> 156
   Met Ala Leu Ala Asp Leu Tyr Glu Glu Gly Gly Gly Gly Gly Gly Glu
    1               5                  10                  15
   Gly Glu Asp Asn Ala Asp Ser Lys Glu Thr Ile Cys Arg Pro Ser Gly
               20                  25                  30
   Arg Lys Ser Ser Lys Met Gln Ala Phe Arg Ile Trp Asp Val Asn Gln
           35                  40                  45
   Lys Thr Phe Tyr Leu Arg Asn Asn Gln Leu Val Ala Gly Tyr Leu Gln
       50                  55                  60
   Gly Pro Asn Val Asn Leu Glu Glu Lys Ile Asp Val Val Pro Ile Glu
   65                  70                  75                  80
   Pro His Ala Leu Phe Leu Gly Ile His Gly Gly Lys Met Cys Leu Ser
                   85                  90                  95
   Cys Val Lys Ser Gly Asp Glu Thr Arg Leu Gln Leu Glu Ala Val Asn
               100                 105                 110
   Ile Thr Asp Leu Ser Glu Asn Arg Lys Gln Asp Lys Arg Phe Ala Phe
           115                 120                 125
   Ile Arg Ser Asp Ser Gly Pro Thr Thr Ser Phe Glu Ser Ala Ala Cys
       130                 135                 140
   Pro Gly Trp Phe Leu Cys Thr Ala Met Glu Ala Asp Gln Pro Val Ser
   145                 150                 155                 160
   Leu Thr Asn Met Pro Asp Glu Gly Val Met Val Thr Lys Phe Tyr Phe
                   165                 170                 175
   Gln Glu Asp Glu
               180
<210> 157
   <211> 99
   <212> PRT
   <213> Homo sapiens
<400> 157
   Met Thr Ser Lys Leu Ala Val Ala Leu Leu Ala Ala Phe Leu Ile Ser
    1               5                  10                  15
   Ala Ala Leu Cys Glu Gly Ala Val Leu Pro Arg Ser Ala Lys Glu Leu
               20                  25                  30
   Arg Cys Gln Cys Ile Lys Thr Tyr Ser Lys Pro Phe His Pro Lys Phe
           35                  40                  45
   Ile Lys Glu Leu Arg Val Ile Glu Ser Gly Pro His Cys Ala Asn Thr
       50                  55                  60
   Glu Ile Ile Val Lys Leu Ser Asp Gly Arg Glu Leu Cys Leu Asp Pro
   65                  70                  75                  80
   Lys Glu Asn Trp Val Gln Arg Val Val Glu Lys Phe Leu Lys Arg Ala
                   85                  90                  95
   Glu Asn Ser
<210> 158
   <211> 350
   <212> PRT
   <213> Homo sapiens
<400> 158
   Met Ser Asn Ile Thr Asp Pro Gln Met Trp Asp Phe Asp Asp Leu Asn
    1               5                  10                  15
   Phe Thr Gly Met Pro Pro Ala Asp Glu Asp Tyr Ser Pro Cys Met Leu
               20                  25                  30
   Glu Thr Glu Thr Leu Asn Lys Tyr Val Val Ile Ile Ala Tyr Ala Leu
           35                  40                  45
   Val Phe Leu Leu Ser Leu Leu Gly Asn Ser Leu Val Met Leu Val Ile
       50                  55                  60
   Leu Tyr Ser Arg Val Gly Arg Ser Val Thr Asp Val Tyr Leu Leu Asn
   65                  70                  75                  80
   Leu Ala Leu Ala Asp Leu Leu Phe Ala Leu Thr Leu Pro Ile Trp Ala
                   85                  90                  95
   Ala Ser Lys Val Asn Gly Trp Ile Phe Gly Thr Phe Leu Cys Lys Val
               100                 105                 110
   Val Ser Leu Leu Lys Glu Val Asn Phe Tyr Ser Gly Ile Leu Leu Leu
           115                 120                 125
   Ala Cys Ile Ser Val Asp Arg Tyr Leu Ala Ile Val His Ala Thr Arg
       130                 135                 140
   Thr Leu Thr Gln Lys Arg His Leu Val Lys Phe Val Cys Leu Gly Cys
   145                 150                 155                 160
   Trp Gly Leu Ser Met Asn Leu Ser Leu Pro Phe Phe Leu Phe Arg Gln
                   165                 170                 175
   Ala Tyr His Pro Asn Asn Ser Ser Pro Val Cys Tyr Glu Val Leu Gly
               180                 185                 190
   Asn Asp Thr Ala Lys Trp Arg Met Val Leu Arg Ile Leu Pro His Thr
           195                 200                 205
   Phe Gly Phe Ile Val Pro Leu Phe Val Met Leu Phe Cys Tyr Gly Phe
       210                 215                 220
   Thr Leu Arg Thr Leu Phe Lys Ala His Met Gly Gln Lys His Arg Ala
   225                 230                 235                 240
   Met Arg Val Ile Phe Ala Val Val Leu Ile Phe Leu Leu Cys Trp Leu
                   245                 250                 255
   Pro Tyr Asn Leu Val Leu Leu Ala Asp Thr Leu Met Arg Thr Gln Val
               260                 265                 270
   Ile Gln Glu Ser Cys Glu Arg Arg Asn Asn Ile Gly Arg Ala Leu Asp
           275                 280                 285
   Ala Thr Glu Ile Leu Gly Phe Leu His Ser Cys Leu Asn Pro Ile Ile
       290                 295                 300
   Tyr Ala Phe Ile Gly Gln Asn Phe Arg His Gly Phe Leu Lys Ile Leu
   305                 310                 315                 320
   Ala Met His Gly Leu Val Ser Lys Glu Phe Leu Ala Arg His Arg Val
                   325                 330                 335
   Thr Ser Tyr Thr Ser Ser Ser Val Asn Val Ser Ser Asn Leu
               340                 345                 350
<210> 159
   <211> 360
   <212> PRT
   <213> Homo sapiens
<40b> 159
<210> 160
   <211> 481
   <212> PRT
   <213> Homo sapiens
<400> 160
   Met Thr Pro Ala Leu Thr Ala Leu Leu Cys Leu Gly Leu Ser Leu Gly
   1               5                   10                  15
   Pro Arg Thr Arg Val Gln Ala Gly Pro Phe Pro Lys Pro Thr Leu Trp
               20                  25                  30
   Ala Glu Pro Gly Ser Val Ile Ser Trp Gly Ser Pro Val Thr Ile Trp
           35                  40                  45
   Cys Gln Gly Ser Leu Glu Ala Gln Glu Tyr Gln Leu Asp Lys Glu Gly
       50                  55                  60
   Ser Pro Glu Pro Leu Asp Arg Asn Asn Pro Leu Glu Pro Lys Asn Lys
   65                  70                  75                  80
   Ala Arg Phe Ser Ile Pro Ser Met Thr Gln His His Ala Gly Arg Tyr
                   85                  90                  95
   Arg Cys His Tyr Tyr Ser Ser Ala Gly Trp Ser Glu Pro Ser Asp Pro
               100                 105                 110
   Leu Glu Leu Val Met Thr Gly Phe Tyr Asn Lys Pro Thr Leu Ser Ala
           115                 120                 125
   Leu Pro Ser Pro Val Val Ala Ser Gly Gly Asn Met Thr Leu Arg Cys
       130                 135                 140
   Gly Ser Gln Lys Gly Tyr His His Phe Val Leu Met Lys Glu Gly Glu
   145                 150                 155                 160
   His Gln Leu Pro Arg Thr Leu Asp Ser Gln Gln Leu His Ser Gly Gly
                   165                 170                 175
   Phe Gln Ala Leu Phe Pro Val Gly Pro Val Thr Pro Ser His Arg Trp
               180                 185                 190
   Arg Phe Thr Cys Tyr Tyr Tyr Tyr Thr Asn Thr Pro Arg Val Trp Ser
           195                 200                 205
   His Pro Ser Asp Pro Leu Glu Ile Leu Pro Ser Gly Val Ser Arg Lys
       210                 215                 220
   Pro Ser Leu Leu Thr Leu Gln Gly Pro Val Leu Ala Pro Gly Gln Ser
   225                 230                 235                 240
   Leu Thr Leu Gln Cys Gly Ser Asp Val Gly Tyr Asp Arg Phe Val Leu
                   245                 250                 255
   Tyr Lys Glu Gly Glu Arg Asp Phe Leu Gln Arg Pro Gly Gln Gln Pro
               260                 265                 270
   Gln Ala Gly Leu Ser Gln Ala Asn Phe Thr Leu Gly Pro Val Ser Pro
           275                 280                 285
   Ser His Gly Gly Gln Tyr Arg Cys Tyr Gly Ala His Asn Leu Ser Ser
       290                 295                 300
   Glu Trp Ser Ala Pro Ser Asp Pro Leu Asn Ile Leu Met Ala Gly Gln
   305                 310                 315                 320
   Ile Tyr Asp Thr Val Ser Leu Ser Ala Gln Pro Gly Pro Thr Val Ala
                   325                 330                 335
   Ser Gly Glu Asn Val Thr Leu Leu Cys Gln Ser Arg Gly Tyr Phe Asp
               340                 345                 350
   Thr Phe Leu Leu Thr Lys Glu Gly Ala Ala His Pro Pro Leu Arg Leu
           355                 360                 365
   Arg Ser Met Tyr Gly Ala His Lys Tyr Gln Ala Glu Phe Pro Met Ser
       370                 375                 380
   Pro Val Thr Ser Ala His Ala Gly Thr Tyr Arg Cys Tyr Gly Ser Tyr
   385                 390                 395                 400
   Ser Ser Asn Pro His Leu Leu Ser Phe Pro Ser Glu Pro Leu Glu Leu
                   405                 410                 415
   Met Val Ser Gly His Ser Gly Gly Ser Ser Leu Pro Pro Thr Gly Pro
               420                 425                 430
   Pro Ser Thr Pro Ala Ser His Ala Lys Asp Tyr Thr Val Glu Asn Leu
           435                 440                 445
   Ile Arg Met Gly Met Ala Gly Leu Val Leu Val Phe Leu Gly Ile Leu
       450                 455                 460
   Leu Phe Glu Ala Gln His Ser Gln Arg Asn Pro Gln Asp Ala Ala Gly
   465                 470                 475                 480
   Arg
<210> 161
   <211> 375
   <212> PRT
   <213> Homo sapiens
<400> 161
   Met Asp Ala Ile His Ile Gly Met Ser Ser Thr Pro Leu Val Lys His
    1               5                  10                  15
   Thr Ala Gly Ala Gly Leu Lys Ala Asn Arg Pro Arg Val Met Ser Lys
               20                  25                  30
   Ser Gly His Ser Asn Val Arg Ile Asp Lys Val Asp Gly Ile Tyr Leu
           35                  40                  45
   Leu Tyr Leu Gln Asp Leu Trp Thr Thr Val Ile Asp Met Lys Trp Arg
       50                  55                  60
   Tyr Lys Leu Thr Leu Phe Ala Ala Thr Phe Val Met Thr Trp Phe Leu
   65                  70                  75                  80
   Phe Gly Val Ile Tyr Tyr Ala Ile Ala Phe Ile His Gly Asp Leu Glu
                   85                  90                  95
   Pro Gly Glu Pro Ile Ser Asn His Thr Pro Cys Ile Met Lys Val Asp
               100                 105                 110
   Ser Leu Thr Gly Ala Phe Leu Phe Ser Leu Glu Ser Gln Thr Thr Ile
           115                 120                 125
   Gly Tyr Gly Val Arg Ser Ile Thr Glu Glu Cys Pro His Ala Ile Phe
       130                 135                 140
   Leu Leu Val Ala Gln Leu Val Ile Thr Thr Leu Ile Glu Ile Phe Ile
   145                 150                 155                 160
   Thr Gly Thr Phe Leu Ala Lys Ile Ala Arg Pro Lys Lys Arg Ala Glu
                   165                 170                 175
   Thr Ile Lys Phe Ser His Cys Ala Val Ile Thr Lys Gln Asn Gly Lys
               180                 185                 190
   Leu Cys Leu Val Ile Gln Val Ala Asn Met Arg Lys Ser Leu Leu Ile
           195                 200                 205
   Gln Cys Gln Leu Ser Gly Lys Leu Leu Gln Thr His Val Thr Lys Glu
       210                 215                 220
   Gly Glu Arg Ile Leu Leu Asn Gln Ala Thr Val Lys Phe His Val Asp
   225                 230                 235                 240
   Ser Ser Ser Glu Ser Pro Phe Leu Ile Leu Pro Met Thr Phe Tyr His
                   245                 250                 255
   Val Leu Asp Glu Thr Ser Pro Leu Arg Asp Leu Thr Pro Gln Asn Leu
               260                 265                 270
   Lys Glu Lys Glu Phe Glu Leu Val Val Leu Leu Asn Ala Thr Val Glu
           275                 280                 285
   Ser Thr Ser Ala Val Cys Gln Ser Arg Thr Ser Tyr Ile Pro Glu Glu
       290                 295                 300
   Ile Tyr Trp Gly Phe Glu Phe Val Pro Val Val Ser Leu Ser Lys Asn
   305                 310                 315                 320
   Gly Lys Tyr Val Ala Asp Phe Ser Gln Phe Glu Gln Ile Arg Lys Ser
                   325                 330                 335
   Pro Asp Cys Thr Phe Tyr Cys Ala Asp Ser Glu Lys Gln Gln Leu Glu
               340                 345                 350
   Glu Lys Tyr Arg Gln Glu Asp Gln Arg Glu Arg Glu Leu Arg Thr Leu
               355                 360                 365
   Leu Leu Gln Gln Ser Asn Val
           370                 375
<210> 162
   <211> 375
   <212> PRT
   <213> Homo sapiens
<400> 162
   Met Asp Ala Ile His Ile Gly Met Ser Ser Thr Pro Leu Val Lys His
   1               5                   10                  15
   Thr Ala Gly Ala Gly Leu Lys Ala Asn Arg Pro Arg Val Met Ser Lys
               20                  25                  30
   Ser Gly His Ser Asn Val Arg Ile Asp Lys Val Asp Gly Ile Tyr Leu
           35                  40                  45
   Leu Tyr Leu Gln Asp Leu Trp Thr Thr Val Ile Asp Met Lys Trp Arg
       50                  55                  60
   Tyr Lys Leu Thr Leu Phe Ala Ala Thr Phe Val Met Thr Trp Phe Leu
   65                  70                  75                  80
   Phe Gly Val Ile Tyr Tyr Ala Ile Ala Phe Ile His Gly Asp Leu Glu
                   85                  90                  95
   Pro Gly Glu Pro Ile Ser Asn His Thr Pro Cys Ile Met Lys Val Asp
               100                 105                 110
   Ser Leu Thr Gly Ala Phe Leu Phe Ser Leu Glu Ser Gln Thr Thr Ile
           115                 120                 125
   Gly Tyr Gly Val Arg Ser Ile Thr Glu Glu Cys Pro His Ala Ile Phe
       130                 135                 140
   Leu Leu Val Ala Gln Leu Val Ile Thr Thr Leu Ile Glu Ile Phe Ile
   145                 150                 155                 160
   Thr Gly Thr Phe Leu Ala Lys Ile Ala Arg Pro Lys Lys Arg Ala Glu
                   165                 170                 175
   Thr Ile Lys Phe Ser His Cys Ala Val Ile Thr Lys Gln Asn Gly Lys
               180                 185                 190
   Leu Cys Leu Val Ile Gln Val Ala Asn Met Arg Lys Ser Leu Leu Ile
           195                 200                 205
   Gln Cys Gln Leu Ser Gly Lys Leu Leu Gln Thr His Val Thr Lys Glu
       210                 215                 220
   Gly Glu Arg Ile Leu Leu Asn Gln Ala Thr Val Lys Phe His Val Asp
   225                 230                 235                 240
   Ser Ser Ser Glu Ser Pro Phe Leu Ile Leu Pro Met Thr Phe Tyr His
                   245                 250                 255
   Val Leu Asp Glu Thr Ser Pro Leu Arg Asp Leu Thr Pro Gln Asn Leu
               260                 265                 270
   Lys Glu Lys Glu Phe Glu Leu Val Val Leu Leu Asn Ala Thr Val Glu
           275                 280                 285
   Ser Thr Ser Ala Val Cys Gln Ser Arg Thr Ser Tyr Ile Pro Glu Glu
       290                 295                 300
   Ile Tyr Trp Gly Phe Glu Phe Val Pro Val Val Ser Leu Ser Lys Asn
   305                 310                 315                 320
   Gly Lys Tyr Val Ala Asp Phe Ser Gln Phe Glu Gln Ile Arg Lys Ser
                   325                 330                 335
   Pro Asp Cys Thr Phe Tyr Cys Ala Asp Ser Glu Lys Gln Gln Leu Glu
               340                 345                 350
   Glu Lys Tyr Arg Gln Glu Asp Gln Arg Glu Arg Glu Leu Arg Thr Leu
           355                 360                 365
   Leu Leu Gln Gln Ser Asn Val
       370                 375
<210> 163
   <211> 1361
   <212> PRT
   <213> Homo sapiens
<400> 163
   Met Gly Ala Ala Gly Arg Gln Asp Phe Leu Phe Lys Ala Met Leu Thr
   1               5                   10                  15
   Ile Ser Trp Leu Thr Leu Thr Cys Phe Pro Gly Ala Thr Ser Thr Val
               20                  25                  30
   Ala Ala Gly Cys Pro Asp Gln Ser Pro Glu Leu Gln Pro Trp Asn Pro
           35                  40                  45
   Gly His Asp Gln Asp His His Val His Ile Gly Gln Gly Lys Thr Leu
       50                  55                  60
   Leu Leu Thr Ser Ser Ala Thr Val Tyr Ser Ile His Ile Ser Glu Gly
   65                  70                  75                  80
   Gly Lys Leu Val Ile Lys Asp His Asp Glu Pro Ile Val Leu Arg Thr
                   85                  90                  95
   Arg His Ile Leu Ile Asp Asn Gly Gly Glu Leu His Ala Gly Ser Ala
               100                 105                 110
   Leu Cys Pro Phe Gln Gly Asn Phe Thr Ile Ile Leu Tyr Gly Arg Ala
           115                 120                 125
   Asp Glu Gly Ile Gln Pro Asp Pro Tyr Tyr Gly Leu Lys Tyr Ile Gly
       130                 135                 140
   Val Gly Lys Gly Gly Ala Leu Glu Leu His Gly Gln Lys Lys Leu Ser
   145                 150                 155                 160
   Trp Thr Phe Leu Asn Lys Thr Leu His Pro Gly Gly Met Ala Glu Gly
                   165                 170                 175
   Gly Tyr Phe Phe Glu Arg Ser Trp Gly His Arg Gly Val Ile Val His
               180                 185                 190
   Val Ile Asp Pro Lys Ser Gly Thr Val Ile His Ser Asp Arg Phe Asp
           195                 200                 205
   Thr Tyr Arg Ser Lys Lys Glu Ser Glu Arg Leu Val Gln Tyr Leu Asn
       210                 215                 220
   Ala Val Pro Asp Gly Arg Ile Leu Ser Val Ala Val Asn Asp Glu Gly
   225                 230                 235                 240
   Ser Arg Asn Leu Asp Asp Met Ala Arg Lys Ala Met Thr Lys Leu Gly
                   245                 250                 255
   Ser Lys His Phe Leu His Leu Gly Phe Arg His Pro Trp Ser Phe Leu
               260                 265                 270
   Thr Val Lys Gly Asn Pro Ser Ser Ser Val Glu Asp His Ile Glu Tyr
           275                 280                 285
   His Gly His Arg Gly Ser Ala Ala Ala Arg Val Phe Lys Leu Phe Gln
       290                 295                 300
   Thr Glu His Gly Glu Tyr Phe Asn Val Ser Leu Ser Ser Glu Trp Val
   305                 310                 315                 320
   Gln Asp Val Glu Trp Thr Glu Trp Phe Asp His Asp Lys Val Ser Gln
                   325                 330                 335
   Thr Lys Gly Gly Glu Lys Ile Ser Asp Leu Trp Lys Ala His Pro Gly
               340                 345                 350
   Lys Ile Cys Asn Arg Pro Ile Asp Ile Gln Ala Thr Thr Met Asp Gly
           355                 360                 365
   Val Asn Leu Ser Thr Glu Val Val Tyr Lys Lys Gly Gln Asp Tyr Arg
       370                 375                 380
   Phe Ala Cys Tyr Asp Arg Gly Arg Ala Cys Arg Ser Tyr Arg Val Arg
   385                 390                 395                 400
   Phe Leu Cys Gly Lys Pro Val Arg Pro Lys Leu Thr Val Thr Ile Asp
                   405                 410                 415
   Thr Asn Val Asn Ser Thr Ile Leu Asn Leu Glu Asp Asn Val Gln Ser
                   420                     425                     430
   Trp Lys Pro Gly Asp Thr Leu Val Ile Ala Ser Thr Asp Tyr Ser Met
               435                     440                     445
   Tyr Gln Ala Glu Glu Phe Gln Val Leu Pro Cys Arg Ser Cys Ala Pro
           450                     455                     460
   Asn Gln Val Lys Val Ala Gly Lys Pro Met Tyr Leu His Ile Gly Glu
   465                     470                     475                     480
   Glu Ile Asp Gly Val Asp Met Arg Ala Glu Val Gly Leu Leu Ser Arg
                       485                     490                     495
   Asn Ile Ile Val Met Gly Glu Met Glu Asp Lys Cys Tyr Pro Tyr Arg
                   500                     505                     510
   Asn His Ile Cys Asn Phe Phe Asp Phe Asp Thr Phe Gly Gly His Ile
               515                     520                     525
   Lys Phe Ala Leu Gly Phe Lys Ala Ala His Leu Glu Gly Thr Glu Leu
           530                     535                     540
   Lys His Met Gly Gln Gln Leu Val Gly Gln Tyr Pro Ile His Phe His
   545                     550                     555                     560
   Leu Ala Gly Asp Val Asp Glu Arg Gly Gly Tyr Asp Pro Pro Thr Tyr
                       565                     570                     575
   Ile Arg Asp Leu Ser Ile His His Thr Phe Ser Arg Cys Val Thr Val
                   580                     585                     590
   His Gly Ser Asn Gly Leu Leu Ile Lys Asp Val Val Gly Tyr Asn Ser
               595                     600                     605
   Leu Gly His Cys Phe Phe Thr Glu Asp Gly Pro Glu Glu Arg Asn Thr
           610                     615                     620
   Phe Asp His Cys Leu Gly Leu Leu Val Lys Ser Gly Thr Leu Leu Pro
   625                     630                     635                     640
   Ser Asp Arg Asp Ser Lys Met Cys Lys Met Ile Thr Glu Asp Ser Tyr
                       645                     650                     655
   Pro Gly Tyr Ile Pro Lys Pro Arg Gln Asp Cys Asn Ala Val Ser Thr
                   660                     665                     670
   Phe Trp Met Ala Asn Pro Asn Asn Asn Leu Ile Asn Cys Ala Ala Ala
               675                     680                     685
   Gly Ser Glu Glu Thr Gly Phe Trp Phe Ile Phe His His Val Pro Thr
           690                     695                     700
   Gly Pro Ser Val Gly Met Tyr Ser Pro Gly Tyr Ser Glu His Ile Pro
   705                     710                     715                     720
   Leu Gly Lys Phe Tyr Asn Asn Arg Ala His Ser Asn Tyr Arg Ala Gly
                       725                     730                     735
   Met Ile Ile Asp Asn Gly Val Lys Thr Thr Glu Ala Ser Ala Lys Asp
                   740                     745                     750
   Lys Arg Pro Phe Leu Ser Ile Ile Ser Ala Arg Tyr Ser Pro His Gln
               755                     760                     765
   Asp Ala Asp Pro Leu Lys Pro Arg Glu Pro Ala Ile Ile Arg His Phe
           770                     775                     780
   Ile Ala Tyr Lys Asn Gln Asp His Gly Ala Trp Leu Arg Gly Gly Asp
   785                     790                     795                     800
   Val Trp Leu Asp Ser Cys Arg Phe Ala Asp Asn Gly Ile Gly Leu Thr
                       805                     810                     815
   Leu Ala Ser Gly Gly Thr Phe Pro Tyr Asp Asp Gly Ser Lys Gln Glu
                   820                     825                     830
   Ile Lys Asn Ser Leu Phe Val Gly Glu Ser Gly Asn Val Gly Thr Glu
               835                     840                     845
   Met Met Asp Asn Arg Ile Trp Gly Pro Gly Gly Leu Asp His Ser Gly
           850                     855                     860
   Arg Thr Leu Pro Ile Gly Gln Asn Phe Pro Ile Arg Gly Ile Gln Leu
   865                     870                     875                     880
   Tyr Asp Gly Pro Ile Asn Ile Gln Asn Cys Thr Phe Arg Lys Phe Val
                       885                     890                     895
   Ala Leu Glu Gly Arg His Thr Ser Ala Leu Ala Phe Arg Leu Asn Asn
                   900                 905                 910
   Ala Trp Gln Ser Cys Pro His Asn Asn Val Thr Gly Ile Ala Phe Glu
               915                 920                 925
   Asp Val Pro Ile Thr Ser Arg Val Phe Phe Gly Glu Pro Gly Pro Trp
       930                 935                 940
   Phe Asn Gln Leu Asp Met Asp Gly Asp Lys Thr Ser Val Phe His Asp
   945                 950                 955                 960
   Val Asp Gly Ser Val Ser Glu Tyr Pro Gly Ser Tyr Leu Thr Lys Asn
                   965                 970                 975
   Asp Asn Trp Leu Val Arg His Pro Asp Cys Ile Asn Val Pro Asp Trp
               980                 985                 990
   Arg Gly Ala Ile Cys Ser Gly Cys Tyr Ala Gln Met Tyr Ile Gln Ala
           995                 1000                1005
   Tyr Lys Thr Ser Asn Leu Arg Met Lys Ile Ile Lys Asn Asp Phe Pro
       1010                1015                1020
   Ser His Pro Leu Tyr Leu Glu Gly Ala Leu Thr Arg Ser Thr His Tyr
   1025                1030                1035                1040
   Gln Gln Tyr Gln Pro Val Val Thr Leu Gln Lys Gly Tyr Thr Ile His
                   1045                1050                1055
   Trp Asp Gln Thr Ala Pro Ala Glu Leu Ala Ile Trp Leu Ile Asn Phe
               1060                1065                1070
   Asn Lys Gly Asp Trp Ile Arg Val Gly Leu Cys Tyr Pro Arg Gly Thr
           1075                1080                1085
   Thr Phe Ser Ile Leu Ser Asp Val His Asn Arg Leu Leu Lys Gln Thr
       1090                1095                1100
   Ser Lys Thr Gly Val Phe Val Arg Thr Leu Gln Met Asp Lys Val Glu
   1105                1110                1115                1120
   Gln Ser Tyr Pro Gly Arg Ser His Tyr Tyr Trp Asp Glu Asp Ser Gly
                   1125                1130                1135
   Leu Leu Phe Leu Lys Leu Lys Ala Gln Asn Glu Arg Glu Lys Phe Ala
               1140                1145                1150
   Phe Cys Ser Met Lys Gly Cys Glu Arg Ile Lys Ile Lys Ala Leu Ile
           1155                1160                1165
   Pro Lys Asn Ala Gly Val Ser Asp Cys Thr Ala Thr Ala Tyr Pro Lys
       1170                1175                1180
   Phe Thr Glu Arg Ala Val Val Asp Val Pro Met Pro Lys Lys Leu Phe
   1185                1190                1195                1200
   Gly Ser Gln Leu Lys Thr Lys Asp His Phe Leu Glu Val Lys Met Glu
                   1205                1210                1215
   Ser Ser Lys Gln His Phe Phe His Leu Trp Asn Asp Phe Ala Tyr Ile
               1220                1225                1230
   Glu Val Asp Gly Lys Lys Tyr Pro Ser Ser Glu Asp Gly Ile Gln Val
           1235                1240                1245
   Val Val Ile Asp Gly Asn Gln Gly Arg Val Val Ser His Thr Ser Phe
       1250                1255                1260
   Arg Asn Ser Ile Leu Gln Gly Ile Pro Trp Gln Leu Phe Asn Tyr Val
   1265                1270                1275                1280
   Ala Thr Ile Pro Asp Asn Ser Ile Val Leu Met Ala Ser Lys Gly Arg
                   1285                1290                1295
   Tyr Val Ser Arg Gly Pro Trp Thr Arg Val Leu Glu Lys Leu Gly Ala
               1300                1305                1310
   Asp Arg Gly Leu Lys Leu Lys Glu Gln Met Ala Phe Val Gly Phe Lys
           1315                1320                1325
   Gly Ser Phe Arg Pro Ile Trp Val Thr Leu Asp Thr Glu Asp His Lys
       1330                1335                1340
   Ala Lys Ile Phe Gln Val Val Pro Ile Pro Val Val Lys Lys Lys Lys
   1345                1350                1355                1360
   Leu
<210> 164
   <211> 483
   <212> PRT
   <213> Homo sapiens
<400> 164
   Met Thr Pro Ile Leu Thr Val Leu Ile Cys Leu Gly Leu Ser Leu Gly
    1               5                  10                  15
   Pro Arg Thr His Val Gln Ala Gly His Leu Pro Lys Pro Thr Leu Trp
               20                  25                  30
   Ala Glu Pro Gly Ser Val Ile Ile Gln Gly Ser Pro Val Thr Leu Arg
           35                  40                  45
   Cys Gln Gly Ser Leu Gln Ala Glu Glu Tyr His Leu Tyr Arg Glu Asn
       50                  55                  60
   Lys Ser Ala Ser Trp Val Arg Arg Ile Gln Glu Pro Gly Lys Asn Gly
   65                  70                  75                  80
   Gln Phe Pro Ile Pro Ser Ile Thr Trp Glu His Ala Gly Arg Tyr His
                   85                  90                  95
   Cys Gln Tyr Tyr Ser His Asn His Ser Ser Glu Tyr Ser Asp Pro Leu
               100                 105                 110
   Glu Leu Val Val Thr Gly Ala Tyr Ser Lys Pro Thr Leu Ser Ala Leu
           115                 120                 125
   Pro Ser Pro Val Val Thr Leu Gly Gly Asn Val Thr Leu Gln Cys Val
       130                 135                 140
   Ser Gln Val Ala Phe Asp Gly Phe Ile Leu Cys Lys Glu Gly Glu Asp
   145                 150                 155                 160
   Glu His Pro Gln Arg Leu Asn Ser His Ser His Ala Arg Gly Trp Ser
                   165                 170                 175
   Trp Ala Ile Phe Ser Val Gly Pro Val Ser Pro Ser Arg Arg Trp Ser
               180                 185                 190
   Tyr Arg Cys Tyr Ala Tyr Asp Ser Asn Ser Pro Tyr Val Trp Ser Leu
           195                 200                 205
   Pro Ser Asp Leu Leu Glu Leu Leu Val Pro Gly Val Ser Lys Lys Pro
       210                 215                 220
   Ser Leu Ser Val Gln Pro Gly Pro Met Val Ala Pro Gly Glu Ser Leu
   225                 230                 235                 240
   Thr Leu Gln Cys Val Ser Asp Val Gly Tyr Asp Arg Phe Val Leu Tyr
                   245                 250                 255
   Lys Glu Gly Glu Arg Asp Phe Leu Gln Arg Pro Gly Trp Gln Pro Gln
               260                 265                 270
   Ala Gly Leu Ser Gln Ala Asn Phe Thr Leu Gly Pro Val Ser Pro Ser
           275                 280                 285
   His Gly Gly Gln Tyr Arg Cys Tyr Ser Ala His Asn Leu Ser Ser Glu
       290                 295                 300
   Trp Ser Ala Pro Ser Asp Pro Leu Asp Ile Leu Ile Thr Gly Gln Phe
   305                 310                 315                 320
   Tyr Asp Arg Pro Ser Leu Ser Val Gln Pro Val Pro Thr Val Ala Pro
                   325                 330                 335
   Gly Lys Asn Val Thr Leu Leu Cys Gln Ser Arg Gly Gln Phe His Thr
               340                 345                 350
   Phe Leu Leu Thr Lys Glu Gly Ala Gly His Pro Pro Leu His Leu Arg
           355                 360                 365
   Ser Glu His Gln Ala Gln Gln Asn Gln Ala Glu Phe Arg Met Gly Pro
       370                 375                 380
   Val Thr Ser Ala His Val Gly Thr Tyr Arg Cys Tyr Ser Ser Leu Ser
   385                 390                 395                 400
   Ser Asn Pro Tyr Leu Leu Ser Leu Pro Ser Asp Pro Leu Glu Leu Val
                   405                 410                 415
   Val Ser Glu Ala Ala Glu Thr Leu Ser Pro Ser Gln Asn Lys Thr Asp
                   420                 425                 430
   Ser Thr Thr Thr Ser Leu Gly Gln His Pro Gln Asp Tyr Thr Val Glu
               435                 440                 445
   Asn Leu Ile Arg Met Gly Val Ala Gly Leu Val Leu Val Val Leu Gly
       450                 455                 460
   Ile Leu Leu Phe Glu Ala Gln His Ser Gln Arg Ser Leu Gln Asp Ala
   465                 470                 475                 480
   Ala Gly Arg
<210> 165
   <211> 439
   <212> PRT
   <213> Homo sapiens
<400> 165
   Met Thr Ser Ile Leu Thr Val Leu Ile Cys Leu Gly Leu Ser Leu Asp
   1               5                   10                  15
   Pro Arg Thr His Val Gln Ala Gly Pro Leu Pro Lys Pro Thr Leu Trp
               20                  25                  30
   Ala Glu Pro Gly Ser Val Ile Thr Gln Gly Ser Pro Val Thr Leu Arg
           35                  40                  45
   Cys Gln Gly Ser Leu Glu Thr Gln Glu Tyr His Leu Tyr Arg Glu Lys
       50                  55                  60
   Lys Thr Ala Leu Trp Ile Thr Arg Ile Pro Gln Glu Leu Val Lys Lys
   65                  70                  75                  80
   Gly Gln Phe Pro Ile Leu Ser Ile Thr Trp Glu His Ala Gly Arg Tyr
                   85                  90                  95
   Cys Cys Ile Tyr Gly Ser His Thr Val Gly Leu Ser Glu Ser Ser Asp
               100                 105                 110
   Pro Leu Glu Leu Val Val Thr Gly Ala Tyr Ser Lys Pro Thr Leu Ser
           115                 120                 125
   Ala Leu Pro Ser Pro Val Val Thr Ser Gly Gly Asn Val Thr Ile Gln
       130                 135                 140
   Cys Asp Ser Gln Val Ala Phe Asp Gly Phe Ile Leu Cys Lys Glu Gly
   145                 150                 155                 160
   Glu Asp Glu His Pro Gln Cys Leu Asn Ser His Ser His Ala Arg Gly
                   165                 170                 175
   Ser Ser Arg Ala Ile Phe Ser Val Gly Pro Val Ser Pro Ser Arg Arg
               180                 185                 190
   Trp Ser Tyr Arg Cys Tyr Gly Tyr Asp Ser Arg Ala Pro Tyr Val Trp
           195                 200                 205
   Ser Leu Pro Ser Asp Leu Leu Gly Leu Leu Val Pro Gly Val Ser Lys
       210                 215                 220
   Lys Pro Ser Leu Ser Val Gln Pro Gly Pro Val Val Ala Pro Gly Glu
   225                 230                 235                 240
   Lys Leu Thr Phe Gln Cys Gly Ser Asp Ala Gly Tyr Asp Arg Phe Val
                   245                 250                 255
   Leu Tyr Lys Glu Trp Gly Arg Asp Phe Leu Gln Arg Pro Gly Arg Gln
               260                 265                 270
   Pro Gln Ala Gly Leu Ser Gln Ala Asn Phe Thr Leu Gly Pro Val Ser
           275                 280                 285
   Arg Ser Tyr Gly Gly Gln Tyr Thr Cys Ser Gly Ala Tyr Asn Leu Ser
       290                 295                 300
   Ser Glu Trp Ser Ala Pro Ser Asp Pro Leu Asp Ile Leu Ile Thr Gly
   305                 310                 315                 320
   Gln Ile Arg Ala Arg Pro Phe Leu Ser Val Arg Pro Gly Pro Thr Val
                   325                 330                 335
   Ala Ser Gly Glu Asn Val Thr Leu Leu Cys Gln Ser Gln Gly Gly Met
                     340                           345                           350
   His Thr Phe Leu Leu Thr Lys Glu Gly Ala Ala Asp Ser Pro Leu Arg
               355                           360                           365
   Leu Lys Ser Lys Arg Gln Ser His Lys Tyr Gln Ala Glu Phe Pro Met
       370                           375                           380
   Ser Pro Val Thr Ser Ala His Ala Gly Thr Tyr Arg Cys Tyr Gly Ser
   385                           390                           395                           400
   Leu Ser Ser Asn Pro Tyr Leu Leu Thr His Pro Ser Asp Pro Leu Glu
                           405                           410                           415
   Leu Val Val Ser Gly Ala Ala Glu Thr Leu Ser Pro Pro Gln Asn Lys
                     420                           425                           430
   Ser Asp Ser Lys Ala Gly Glu
               435
<210> 166
   <211> 631
   <212> PRT
   <213> Homo sapiens
<400> 166
   Met Thr Pro Ala Leu Thr Ala Leu Leu Cys Leu Gly Leu Ser Leu Gly
    1               5                  10                  15
   Pro Arg Thr Arg Val Gln Ala Gly Pro Phe Pro Lys Pro Thr Leu Trp
               20                  25                  30
   Ala Glu Pro Gly Ser Val Ile Ser Trp Gly Ser Pro Val Thr Ile Trp
           35                  40                  45
   Cys Gln Gly Ser Gln Glu Ala Gln Glu Tyr Arg Leu His Lys Glu Gly
       50                  55                  60
   Ser Pro Glu Pro Leu Asp Arg Asn Asn Pro Leu Glu Pro Lys Asn Lys
   65                  70                  75                  80
   Ala Arg Phe Ser Ile Pro Ser Met Thr Glu His His Ala Gly Arg Tyr
                   85                  90                  95
   Arg Cys His Tyr Tyr Ser Ser Ala Gly Trp Ser Glu Pro Ser Asp Pro
               100                 105                 110
   Leu Glu Met Val Met Thr Gly Ala Tyr Ser Lys Pro Thr Leu Ser Ala
           115                 120                 125
   Leu Pro Ser Pro Val Val Ala Ser Gly Gly Asn Met Thr Leu Arg Cys
       130                 135                 140
   Gly Ser Gln Lys Gly Tyr His His Phe Val Leu Met Lys Glu Gly Glu
   145                 150                 155                 160
   His Gln Leu Pro Arg Thr Leu Asp Ser Gln Gln Leu His Ser Arg Gly
                   165                 170                 175
   Phe Gln Ala Leu Phe Pro Val Gly Pro Val Thr Pro Ser His Arg Trp
               180                 185                 190
   Arg Phe Thr Cys Tyr Tyr Tyr Tyr Thr Asn Thr Pro Trp Val Trp Ser
           195                 200                 205
   His Pro Ser Asp Pro Leu Glu Ile Leu Pro Ser Gly Val Ser Arg Lys
       210                 215                 220
   Pro Ser Leu Leu Thr Leu Gln Gly Pro Val Leu Ala Pro Gly Gln Ser
   225                 230                 235                 240
   Leu Thr Leu Gln Cys Gly Ser Asp Val Gly Tyr Asn Arg Phe Val Leu
                   245                 250                 255
   Tyr Lys Glu Gly Glu Arg Asp Phe Leu Gln Arg Pro Gly Gln Gln Pro
               260                 265                 270
   Gln Ala Gly Leu Ser Gln Ala Asn Phe Thr Leu Gly Pro Val Ser Pro
           275                 280                 285
   Ser Asn Gly Gly Gln Tyr Arg Cys Tyr Gly Ala His Asn Leu Ser Ser
       290                 295                 300
   Glu Trp Ser Ala Pro Ser Asp Pro Leu Asn Ile Leu Met Ala Gly Gln
   305                 310                 315                 320
   Ile Tyr Asp Thr Val Ser Leu Ser Ala Gln Pro Gly Pro Thr Val Ala
                   325                 330                 335
   Ser Gly Glu Asn Val Thr Leu Leu Cys Gln Ser Trp Trp Gln Phe Asp
               340                 345                 350
   Thr Phe Leu Leu Thr Lys Glu Gly Ala Ala His Pro Pro Leu Arg Leu
           355                 360                 365
   Arg Ser Met Tyr Gly Ala His Lys Tyr Gln Ala Glu Phe Pro Met Ser
       370                 375                 380
   Pro Val Thr Ser Ala His Ala Gly Thr Tyr Arg Cys Tyr Gly Ser Tyr
   385                 390                 395                 400
   Ser Ser Asn Pro His Leu Leu Ser His Pro Ser Glu Pro Leu Glu Leu
                   405                 410                 415
   Val Val Ser Gly His Ser Gly Gly Ser Ser Leu Pro Pro Thr Gly Pro
               420                 425                 430
   Pro Ser Thr Pro Gly Leu Gly Arg Tyr Leu Glu Val Leu Ile Gly Val
           435                 440                 445
   Ser Val Ala Phe Val Leu Leu Leu Phe Leu Leu Leu Phe Leu Leu Leu
       450                 455                 460
   Arg Arg Gln Arg His Ser Lys His Arg Thr Ser Asp Gln Arg Lys Thr
   465                 470                 475                 480
   Asp Phe Gln Arg Pro Ala Gly Ala Ala Glu Thr Glu Pro Lys Asp Arg
                   485                 490                 495
   Gly Leu Leu Arg Arg Ser Ser Pro Ala Ala Asp Val Gln Glu Glu Asn
               500                 505                 510
   Leu Tyr Ala Ala Val Lys Asp Thr Gln Ser Glu Asp Arg Val Glu Leu
           515                 520                 525
   Asp Ser Gln Ser Pro His Asp Glu Asp Pro Gln Ala Val Thr Tyr Ala
       530                 535                 540
   Pro Val Lys His Ser Ser Pro Arg Arg Glu Met Ala Ser Pro Pro Ser
   545                 550                 555                 560
   Ser Leu Ser Gly Glu Phe Leu Asp Thr Lys Asp Arg Gln Val Glu Glu
                   565                 570                 575
   Asp Arg Gln Met Asp Thr Glu Ala Ala Ala Ser Glu Ala Ser Gln Asp
               580                 585                 590
   Val Thr Tyr Ala Gln Leu His Ser Leu Thr Leu Arg Arg Lys Ala Thr
           595                 600                 605
   Glu Pro Pro Pro Ser Gln Glu Gly Glu Pro Pro Ala Glu Pro Ser Ile
       610                 615                 620
   Tyr Ala Thr Leu Ala Ile His
   625                 630
<210> 167
   <211> 253
   <212> PRT
   <213> Homo sapiens
<400> 167
   Met Ala Pro Trp Ser His Pro Ser Ala Gln Leu Gln Pro Val Gly Gly
    1               5                  10                  15
   Asp Ala Val Ser Pro Ala Leu Met Val Leu Leu Cys Leu Gly Asn Leu
               20                  25                  30
   Ser Lys Ala Thr Leu Trp Ala Glu Pro Gly Ser Val Ile Ser Arg Gly
           35                  40                  45
   Asn Ser Val Thr Ile Arg Cys Gln Gly Thr Leu Glu Ala Gln Glu Tyr
       50                  55                  60
   Arg Leu Val Lys Glu Gly Ser Pro Glu Pro Trp Asp Thr Gln Asn Pro
   65                  70                  75                  80
   Leu Glu Pro Lys Asn Lys Ala Arg Phe Ser Ile Pro Ser Met Thr Glu
                   85                  90                  95
   His His Ala Gly Arg Tyr Arg Cys Tyr Tyr Tyr Ser Pro Ala Gly Trp
               100                 105                 110
   Ser Glu Pro Ser Asp Pro Leu Glu Leu Val Val Thr Gly Phe Tyr Asn
           115                 120                 125
   Lys Pro Thr Leu Ser Ala Leu Pro Ser Pro Val Val Thr Ser Gly Glu
       130                 135                 140
   Asn Val Thr Leu Gln Cys Gly Ser Arg Leu Arg Phe Asp Arg Phe Ile
   145                 150                 155                 160
   Leu Thr Glu Glu Gly Asp His Lys Leu Ser Trp Thr Leu Asp Ser Gln
                   165                 170                 175
   Leu Thr Pro Ser Gly Gln Phe Gln Ala Leu Phe Pro Val Gly Pro Val
               180                 185                 190
   Thr Pro Ser His Arg Trp Met Leu Arg Cys Tyr Gly Ser Arg Arg His
           195                 200                 205
   Ile Leu Gln Val Trp Ser Glu Pro Ser Asp Leu Leu Glu Ile Pro Val
       210                 215                 220
   Ser Gly Glu Glu Ala Thr Val Phe Ser Ser Thr Ile Gln Gly Ser Gln
   225                 230                 235                 240
   Thr Gly Cys Gly Glu Leu Tyr Arg Gln Gly Ser Pro Cys
                   245                 250
<210> 168
   <211> 176
   <212> PRT
   <213> Homo sapiens
<400> 168
   Met Gly Ala Pro Ala Thr Arg Arg Cys Val Glu Trp Leu Leu Gly Leu
    1               5                  10                  15
   Tyr Phe Leu Ser His Ile Pro Ile Thr Leu Phe Met Asp Leu Gln Ala
               20                  25                  30
   Val Leu Pro Arg Glu Leu Tyr Pro Val Glu Phe Arg Asn Leu Leu Lys
           35                  40                  45
   Trp Tyr Ala Lys Glu Phe Lys Asp Pro Leu Leu Gln Glu Pro Pro Ala
       50                  55                  60
   Trp Phe Lys Ser Phe Leu Phe Cys Glu Leu Val Phe Gln Leu Pro Phe
   65                  70                  75                  80
   Phe Pro Ile Ala Thr Tyr Ala Phe Leu Lys Gly Ser Cys Lys Trp Ile
                   85                  90                  95
   Arg Thr Pro Ala Ile Ile Tyr Ser Val His Thr Met Thr Thr Leu Ile
               100                 105                 110
   Pro Ile Leu Ser Thr Phe Leu Phe Glu Asp Phe Ser Lys Ala Ser Gly
           115                 120                 125
   Phe Lys Gly Gln Arg Pro Glu Thr Leu His Glu Arg Leu Thr Leu Val
       130                 135                 140
   Ser Val Tyr Ala Pro Tyr Leu Leu Ile Pro Phe Ile Leu Leu Ile Phe
   145                 150                 155                 160
   Met Leu Arg Ser Pro Tyr Tyr Lys Tyr Glu Glu Lys Arg Lys Lys Lys
                   165                 170                 175
<210> 169
   <211> 380
   <212> PRT
   <213> Homo sapiens
<400> 169
   Met Arg Trp Leu Leu Leu Tyr Tyr Ala Leu Cys Phe Ser Leu Ser Lys
   1               5                   10                  15
   Ala Ser Ala His Thr Val Glu Leu Asn Asn Met Phe Gly Gln Ile Gln
               20                  25                  30
   Ser Pro Gly Tyr Pro Asp Ser Tyr Pro Ser Asp Ser Glu Val Thr Trp
           35                  40                  45
   Asn Ile Thr Val Pro Asp Gly Phe Arg Ile Lys Leu Tyr Phe Met His
       50                  55                  60
   Phe Asn Leu Glu Ser Ser Tyr Leu Cys Glu Tyr Asp Tyr Val Lys Val
   65                  70                  75                  80
   Glu Thr Glu Asp Gln Val Leu Ala Thr Phe Cys Gly Arg Glu Thr Thr
                   85                  90                  95
   Asp Thr Glu Gln Thr Pro Gly Gln Glu Val Val Leu Ser Pro Gly Ser
               100                 105                 110
   Phe Met Ser Ile Thr Phe Arg Ser Asp Phe Ser Asn Glu Glu Arg Phe
           115                 120                 125
   Thr Gly Phe Asp Ala His Tyr Met Ala Val Asp Val Asp Glu Cys Lys
       130                 135                 140
   Glu Arg Glu Asp Glu Glu Leu Ser Cys Asp His Tyr Cys His Asn Tyr
   145                 150                 155                 160
   Ile Gly Gly Tyr Tyr Cys Ser Cys Arg Phe Gly Tyr Ile Leu His Thr
                   165                 170                 175
   Asp Asn Arg Thr Cys Arg Val Glu Cys Ser Asp Asn Leu Phe Thr Gln
               180                 185                 190
   Arg Thr Gly Val Ile Thr Ser Pro Asp Phe Pro Asn Pro Tyr Pro Lys
           195                 200                 205
   Ser Ser Glu Cys Leu Tyr Thr Ile Glu Leu Glu Glu Gly Phe Met Val
       210                 215                 220
   Asn Leu Gln Phe Glu Asp Ile Phe Asp Ile Glu Asp His Pro Glu Val
   225                 230                 235                 240
   Pro Cys Pro Tyr Asp Tyr Ile Lys Ile Lys Val Gly Pro Lys Val Leu
                   245                 250                 255
   Gly Pro Phe Cys Gly Glu Lys Ala Pro Glu Pro Ile Ser Thr Gln Ser
               260                 265                 270
   His Ser Val Leu Ile Leu Phe His Ser Asp Asn Ser Gly Glu Asn Arg
           275                 280                 285
   Gly Trp Arg Leu Ser Tyr Arg Ala Ala Gly Asn Glu Cys Pro Glu Leu
       290                 295                 300
   Gln Pro Pro Val His Gly Lys Ile Glu Pro Ser Gln Ala Lys Tyr Phe
   305                 310                 315                 320
   Phe Lys Asp Gln Val Leu Val Ser Cys Asp Thr Gly Tyr Lys Val Leu
                   325                 330                 335
   Lys Asp Asn Val Glu Met Asp Thr Phe Gln Ile Glu Cys Leu Lys Asp
               340                 345                 350
   Gly Thr Trp Ser Asn Lys Ile Pro Thr Cys Lys Lys Asn Glu Ile Asp
           355                 360                 365
   Leu Glu Ser Glu Leu Lys Ser Glu Gln Val Thr Glu
       370                 375                 380
<210> 170
   <211> 1857
   <212> PRT
   <213> Homo sapiens
<400> 170
   Met Ala Arg Lys Lys Leu Lys Lys Phe Thr Thr Leu Glu Ile Val Leu
    1              5                   10                  15
   Ser Val Leu Leu Leu Val Leu Phe Ile Ile Ser Ile Val Leu Ile Val
               20                  25                  30
   Leu Leu Ala Lys Glu Ser Leu Lys Ser Thr Ala Pro Asp Pro Gly Thr
           35                  40                  45
   Thr Gly Thr Pro Asp Pro Gly Thr Thr Gly Thr Pro Asp Pro Gly Thr
       50                  55                  60
   Thr Gly Thr Thr His Ala Arg Thr Thr Gly Pro Pro Asp Pro Gly Thr
   65                  70                  75                  80
   Thr Gly Thr Thr Pro Val Ser Ala Glu Cys Pro Val Val Asn Glu Leu
                   85                  90                  95
   Glu Arg Ile Asn Cys Ile Pro Asp Gln Pro Pro Thr Lys Ala Thr Cys
               100                 105                 110
   Asp Gln Arg Gly Cys Cys Trp Asn Pro Gln Gly Ala Val Ser Val Pro
           115                 120                 125
   Trp Cys Tyr Tyr Ser Lys Asn His Ser Tyr His Val Glu Gly Asn Leu
       130                 135                 140
   Val Asn Thr Asn Ala Gly Phe Thr Ala Arg Leu Lys Asn Leu Pro Ser
   145                 150                 155                 160
   Ser Pro Val Phe Gly Ser Asn Val Asp Asn Val Leu Leu Thr Ala Glu
                   165                 170                 175
   Tyr Gln Thr Ser Asn Arg Phe His Phe Lys Leu Thr Asp Gln Thr Asn
               180                 185                 190
   Asn Arg Phe Glu Val Pro His Glu His Val Gln Ser Phe Ser Gly Asn
           195                 200                 205
   Ala Ala Ala Ser Leu Thr Tyr Gln Val Glu Ile Ser Arg Gln Pro Phe
       210                 215                 220
   Ser Ile Lys Val Thr Arg Arg Ser Asn Asn Arg Val Leu Phe Asp Ser
   225                 230                 235                 240
   Ser Ile Gly Pro Leu Leu Phe Ala Asp Gln Phe Leu Gln Leu Ser Thr
                   245                 250                 255
   Arg Leu Pro Ser Thr Asn Val Tyr Gly Leu Gly Glu His Val His Gln
               260                 265                 270
   Gln Tyr Arg His Asp Met Asn Trp Lys Thr Trp Pro Ile Phe Asn Arg
           275                 280                 285
   Asp Thr Thr Pro Asn Gly Asn Gly Thr Asn Leu Tyr Gly Ala Gln Thr
       290                 295                 300
   Phe Phe Leu Cys Leu Glu Asp Ala Ser Gly Leu Ser Phe Gly Val Phe
   305                 310                 315                 320
   Leu Met Asn Ser Asn Ala Met Glu Val Val Leu Gln Pro Ala Pro Ala
                   325                 330                 335
   Ile Thr Tyr Arg Thr Ile Gly Gly Ile Leu Asp Phe Tyr Val Phe Leu
               340                 345                 350
   Gly Asn Thr Pro Glu Gln Val Val Gln Glu Tyr Leu Glu Leu Ile Gly
           355                 360                 365
   Arg Pro Ala Leu Pro Ser Tyr Trp Ala Leu Gly Phe His Leu Ser Arg
       370                 375                 380
   Tyr Glu Tyr Gly Thr Leu Asp Asn Met Arg Glu Val Val Glu Arg Asn
   385                 390                 395                 400
   Arg Ala Ala Gln Leu Pro Tyr Asp Val Gln His Ala Asp Ile Asp Tyr
                   405                 410                 415
   Met Asp Glu Arg Arg Asp Phe Thr Tyr Asp Ser Val Asp Phe Lys Gly
               420                 425                 430
   Phe Pro Glu Phe Val Asn Glu Leu His Asn Asn Gly Gln Lys Leu Val
           435                 440                 445
   Ile Ile Val Asp Pro Ala Ile Ser Asn Asn Ser Ser Ser Ser Lys Pro
       450                 455                 460
   Tyr Gly Pro Tyr Asp Arg Gly Ser Asp Met Lys Ile Trp Val Asn Ser
   465                 470                 475                 480
   Ser Asp Gly Val Thr Pro Leu Ile Gly Glu Val Trp Pro Gly Gln Thr
                   485                 490                 495
   Val Phe Pro Asp Tyr Thr Asn Pro Asn Cys Ala Val Trp Trp Thr Lys
               500                 505                 510
   Glu Phe Glu Leu Phe His Asn Gln Val Glu Phe Asp Gly Ile Trp Ile
           515                 520                 525
   Asp Met Asn Glu Val Ser Asn Phe Val Asp Gly Ser Val Ser Gly Cys
       530                 535                 540
   Ser Thr Asn Asn Leu Asn Asn Pro Pro Phe Thr Pro Arg Ile Leu Asp
   545                 550                 555                 560
   Gly Tyr Leu Phe Cys Lys Thr Leu Cys Met Asp Ala Val Gln His Trp
                   565                 570                 575
   Gly Lys Gln Tyr Asp Ile His Asn Leu Tyr Gly Tyr Ser Met Ala Val
               580                 585                 590
   Ala Thr Ala Glu Ala Ala Lys Thr Val Phe Pro Asn Lys Arg Ser Phe
           595                 600                 605
   Ile Leu Thr Arg Ser Thr Phe Ala Gly Ser Gly Lys Phe Ala Ala His
       610                 615                 620
   Trp Leu Gly Asp Asn Thr Ala Thr Trp Asp Asp Leu Arg Trp Ser Ile
   625                 630                 635                 640
   Pro Gly Val Leu Glu Phe Asn Leu Phe Gly Ile Pro Met Val Gly Pro
                   645                 650                 655
   Asp Ile Cys Gly Phe Ala Leu Asp Thr Pro Glu Glu Leu Cys Arg Arg
               660                 665                 670
   Trp Met Gln Leu Gly Ala Phe Tyr Pro Phe Ser Arg Asn His Asn Gly
           675                 680                 685
   Gln Gly Tyr Lys Asp Gln Asp Pro Ala Ser Phe Gly Ala Asp Ser Leu
       690                 695                 700
   Leu Leu Asn Ser Ser Arg His Tyr Leu Asn Ile Arg Tyr Thr Leu Leu
   705                 710                 715                 720
   Pro Tyr Leu Tyr Thr Leu Phe Phe Arg Ala His Ser Arg Gly Asp Thr
                   725                 730                 735
   Val Ala Arg Pro Leu Leu His Glu Phe Tyr Glu Asp Asn Ser Thr Trp
               740                 745                 750
   Asp Val His Gln Gln Phe Leu Trp Gly Pro Gly Leu Leu Ile Thr Pro
           755                 760                 765
   Val Leu Asp Glu Gly Ala Glu Lys Val Met Ala Tyr Val Pro Asp Ala
       770                 775                 780
   Val Trp Tyr Asp Tyr Glu Thr Gly Ser Gln Val Arg Trp Arg Lys Gln
   785                 790                 795                 800
   Lys Val Glu Met Glu Leu Pro Gly Asp Lys Ile Gly Leu His Leu Arg
                   805                 810                 815
   Gly Gly Tyr Ile Phe Pro Thr Gln Gln Pro Asn Thr Thr Thr Leu Ala
               820                 825                 830
   Ser Arg Lys Asn Pro Leu Gly Leu Ile Ile Ala Leu Asp Glu Asn Lys
           835                 840                 845
   Glu Ala Lys Gly Glu Leu Phe Trp Asp Asn Gly Glu Thr Lys Asp Thr
       850                 855                 860
   Val Ala Asn Lys Val Tyr Leu Leu Cys Glu Phe Ser Val Thr Gln Asn
   865                 870                 875                 880
   Arg Leu Glu Val Asn Ile Ser Gln Ser Thr Tyr Lys Asp Pro Asn Asn
                   885                 890                 895
   Leu Ala Phe Asn Glu Ile Lys Ile Leu Gly Thr Glu Glu Pro Ser Asn
               900                 905                 910
   Val Thr Val Lys His Asn Gly Val Pro Ser Gln Thr Ser Pro Thr Val
           915                 920                 925
   Thr Tyr Asp Ser Asn Leu Lys Val Ala Ile Ile Thr Asp Ile Asp Leu
       930                 935                 940
   Leu Leu Gly Glu Ala Tyr Thr Val Glu Trp Ser Ile Lys Ile Arg Asp
   945                 950                 955                 960
   Glu Glu Lys Ile Asp Cys Tyr Pro Asp Glu Asn Gly Ala Ser Ala Glu
                   965                 970                 975
   Asn Cys Thr Ala Arg Gly Cys Ile Trp Glu Ala Ser Asn Ser Ser Gly
               980                 985                 990
   Val Pro Phe Cys Tyr Phe Val Asn Asp Leu Tyr Ser Val Ser Asp Val
   995 1000 1005
   Gln Tyr Asn Ser His Gly Ala Thr Ala Asp Ile Ser Leu Lys Ser Ser
   1010 1015 1020
   Val Tyr Ala Asn Ala Phe Pro Ser Thr Pro Val Asn Pro Leu Arg Leu
   1025 1030 1035 1040
   Asp Val Thr Tyr His Lys Asn Glu Met Leu Gln Phe Lys Ile Tyr Asp
   1045 1050 1055
   Pro Asn Lys Asn Arg Tyr Glu Val Pro Val Pro Leu Asn Ile Pro Ser
   1060 1065 1070
   Met Pro Ser Ser Thr Pro Glu Gly Gln Leu Tyr Asp Val Leu Ile Lys
   1075 1080 1085
   Lys Asn Pro Phe Gly Ile Glu Ile Arg Arg Lys Ser Thr Gly Thr Ile
   1090 1095 1100
   Ile Trp Asp Ser Gln Leu Leu Gly Phe Thr Phe Ser Asp Met Phe Ile
   1105 1110 1115 1120
   Arg Ile Ser Thr Arg Leu Pro Ser Lys Tyr Leu Tyr Gly Phe Gly Glu
   1125 1130 1135
   Thr Glu His Arg Ser Tyr Arg Arg Asp Leu Glu Trp His Thr Trp Gly
   1140 1145 1150
   Met Phe Ser Arg Asp Gln Pro Pro Gly Tyr Lys Lys Asn Ser Tyr Gly
   1155 1160 1165
   Val His Pro Tyr Tyr Met Gly Leu Glu Glu Asp Gly Ser Ala His Gly
   1170 1175 1180
   Val Leu Leu Leu Asn Ser Asn Ala Met Asp Val Thr Phe Gln Pro Leu
   1185 1190 1195 1200
   Pro Ala Leu Thr Tyr Arg Thr Thr Gly Gly Val Leu Asp Phe Tyr Val
   1205 1210 1215
   Phe Leu Gly Pro Thr Pro Glu Leu Val Thr Gln Gln Tyr Thr Glu Leu
   1220 1225 1230
   Ile Gly Arg Pro Val Met Val Pro Tyr Trp Ser Leu Gly Phe Gln Leu
   1235 1240 1245
   Cys Arg Tyr Gly Tyr Gln Asn Asp Ser Glu Ile Ala Ser Leu Tyr Asp
   1250 1255 1260
   Glu Met Val Ala Ala Gln Ile Pro Tyr Asp Val Gln Tyr Ser Asp Ile
   1265 1270 1275 1280
   Asp Tyr Met Glu Arg Gln Leu Asp Phe Thr Leu Ser Pro Lys Phe Ala
   1285 1290 1295
   Gly Phe Pro Ala Leu Ile Asn Arg Met Lys Ala Asp Gly Met Arg Val
   1300 1305 1310
   Ile Leu Ile Leu Asp Pro Ala Ile Ser Gly Asn Glu Thr Gln Pro Tyr
   1315 1320 1325
   Pro Ala Phe Thr Arg Gly Val Glu Asp Asp Val Phe Ile Lys Tyr Pro
   1330 1335 1340
   Asn Asp Gly Asp Ile Val Trp Gly Lys Val Trp Pro Asp Phe Pro Asp
   1345 1350 1355 1360
   Val Val Val Asn Gly Ser Leu Asp Trp Asp Ser Gln Val Glu Leu Tyr
   1365 1370 1375
   Arg Ala Tyr Val Ala Phe Pro Asp Phe Phe Arg Asn Ser Thr Ala Lys
   1380 1385 1390
   Trp Trp Lys Arg Glu Ile Glu Glu Leu Tyr Asn Asn Pro Gln Asn Pro
   1395 1400 1405
   Glu Arg Ser Leu Lys Phe Asp Gly Met Trp Ile Asp Met Asn Glu Pro
   1410 1415 1420
   Ser Ser Phe Val Asn Gly Ala Val Ser Pro Gly Cys Arg Asp Ala Ser
   1425 1430 1435 1440
   Leu Asn His Pro Pro Tyr Met Pro His Leu Glu Ser Arg Asp Arg Gly
   1445 1450 1455
   Leu Ser Ser Lys Thr Leu Cys Met Glu Ser Gln Gln Ile Leu Pro Asp
   1460 1465 1470
   Gly Ser Leu Val Gln His Tyr Asn Val His Asn Leu Tyr Gly Trp Ser
           1475                1480                1485
   Gln Thr Arg Pro Thr Tyr Glu Ala Val Gln Glu Val Thr Gly Gln Arg
       1490                1495                1500
   Gly Val Val Ile Thr Arg Ser Thr Phe Pro Ser Ser Gly Arg Trp Ala
   1505                1510                1515                1520
   Gly His Trp Leu Gly Asp Asn Thr Ala Ala Trp Asp Gln Leu Lys Lys
                   1525                1530                1535
   Ser Ile Ile Gly Met Met Glu Phe Ser Leu Phe Gly Ile Ser Tyr Thr
               1540                1545                1550
   Gly Ala Asp Ile Cys Gly Phe Phe Gln Asp Ala Glu Tyr Glu Met Cys
           1555                1560                1565
   Val Arg Trp Met Gln Leu Gly Ala Phe Tyr Pro Phe Ser Arg Asn His
       1570                1575                1580
   Asn Thr Ile Gly Thr Arg Arg Gln Asp Pro Val Ser Trp Asp Val Ala
   1585                1590                1595                1600
   Phe Val Asn Ile Ser Arg Thr Val Leu Gln Thr Arg Tyr Thr Leu Leu
                   1605                1610                1615
   Pro Tyr Leu Tyr Thr Leu Met His Lys Ala His Thr Glu Gly Val Thr
               1620                1625                1630
   Val Val Arg Pro Leu Leu His Glu Phe Val Ser Asp Gln Val Thr Trp
           1635                1640                1645
   Asp Ile Asp Ser Gln Phe Leu Leu Gly Pro Ala Phe Leu Val Ser Pro
       1650                1655                1660
   Val Leu Glu Arg Asn Ala Arg Asn Val Thr Ala Tyr Phe Pro Arg Ala
   1665                1670                1675                1680
   Arg Trp Tyr Asp Tyr Tyr Thr Gly Val Asp Ile Asn Ala Arg Gly Glu
                   1685                1690                1695
   Trp Lys Thr Leu Pro Ala Pro Leu Asp His Ile Asn Leu His Val Arg
               1700                1705                1710
   Gly Gly Tyr Ile Leu Pro Trp Gln Glu Pro Ala Leu Asn Thr His Leu
           1715                1720                1725
   Ser Arg Gln Lys Phe Met Gly Phe Lys Ile Ala Leu Asp Asp Glu Gly
       1730                1735                1740
   Thr Ala Gly Gly Trp Leu Phe Trp Asp Asp Gly Gln Ser Ile Asp Thr
   1745                1750                1755                1760
   Tyr Gly Lys Gly Leu Tyr Tyr Leu Ala Ser Phe Ser Ala Ser Gln Asn
                   1765                1770                1775
   Thr Met Gln Ser His Ile Ile Phe Asn Asn Tyr Ile Thr Gly Thr Asn
               1780                1785                1790
   Pro Leu Lys Leu Gly Tyr Ile Glu Ile Trp Gly Val Gly Ser Val Pro
           1795                1800                1805
   Val Thr Ser Val Ser Ile Ser Val Ser Gly Met Val Ile Thr Pro Ser
       1810                1815                1820
   Phe Asn Asn Asp Pro Thr Thr Gln Val Leu Ser Ile Asp Val Thr Asp
   1825                1830                1835                1840
   Arg Asn Ile Ser Leu His Asn Phe Thr Ser Leu Thr Trp Ile Ser Thr
                   1845                1850                1855
   Leu
<210> 171
   <211> 407
   <212> PRT
   <213> Homo sapiens
<400> 171
   Met Val Asn Glu Tyr Lys Lys Ile Leu Leu Leu Lys Gly Phe Glu Leu
    1               5                  10                  15
   Met Asp Asp Tyr His Phe Thr Ser Ile Lys Ser Leu Leu Ala Tyr Asp
               20                  25                  30
   Leu Gly Leu Thr Thr Lys Met Gln Glu Glu Tyr Asn Arg Ile Lys Ile
           35                  40                  45
   Thr Asp Leu Met Glu Lys Lys Phe Gln Gly Val Ala Cys Leu Asp Lys
       50                  55                  60
   Leu Ile Glu Leu Ala Lys Asp Met Pro Ser Leu Lys Asn Leu Val Asn
   65                  70                  75                  80
   Asn Leu Arg Lys Glu Lys Ser Lys Val Ala Lys Lys Ile Lys Thr Gln
                   85                  90                  95
   Glu Lys Ala Pro Val Lys Lys Ile Asn Gln Glu Glu Val Gly Leu Ala
               100                 105                 110
   Ala Pro Ala Pro Thr Ala Arg Asn Lys Leu Thr Ser Glu Ala Arg Gly
           115                 120                 125
   Arg Ile Pro Val Ala Gln Lys Arg Lys Thr Pro Asn Lys Glu Lys Thr
       130                 135                 140
   Glu Ala Lys Arg Asn Lys Val Ser Gln Glu Gln Ser Lys Pro Pro Gly
   145                 150                 155                 160
   Pro Ser Gly Ala Ser Thr Ser Ala Ala Val Asp His Pro Pro Leu Pro
                   165                 170                 175
   Gln Thr Ser Ser Ser Thr Pro Ser Asn Thr Ser Phe Thr Pro Asn Gln
               180                 185                 190
   Glu Thr Gln Ala Gln Arg Gln Val Asp Ala Arg Arg Asn Val Pro Gln
           195                 200                 205
   Asn Asp Pro Val Thr Val Val Val Leu Lys Ala Thr Ala Pro Phe Lys
       210                 215                 220
   Tyr Glu Ser Pro Glu Asn Gly Lys Ser Thr Met Phe His Ala Thr Val
   225                 230                 235                 240
   Ala Ser Lys Thr Gln Tyr Phe His Val Lys Val Phe Asp Ile Asn Leu
                   245                 250                 255
   Lys Glu Lys Phe Val Arg Lys Lys Val Ile Thr Ile Ser Asp Tyr Ser
               260                 265                 270
   Glu Cys Lys Gly Val Met Glu Ile Lys Glu Ala Ser Ser Val Ser Asp
           275                 280                 285
   Phe Asn Gln Asn Phe Glu Val Pro Asn Arg Ile Ile Glu Ile Ala Asn
       290                 295                 300
   Lys Thr Pro Lys Ile Ser Gln Leu Tyr Lys Gln Ala Ser Gly Thr Met
   305                 310                 315                 320
   Val Tyr Gly Leu Phe Met Leu Gln Lys Lys Ser Val His Lys Lys Asn
                   325                 330                 335
   Thr Ile Tyr Glu Ile Gln Asp Asn Thr Gly Ser Met Asp Val Val Gly
               340                 345                 350
   Ser Gly Lys Trp His Asn Ile Lys Cys Glu Lys Gly Asp Lys Leu Arg
           355                 360                 365
   Leu Phe Cys Leu Gln Leu Arg Thr Val Asp Arg Lys Leu Lys Leu Val
       370                 375                 380
   Cys Gly Ser His Ser Phe Ile Lys Val Ile Lys Ala Lys Lys Asn Lys
   385                 390                 395                 400
   Glu Gly Pro Met Asn Val Asn
                   405
<210> 172
   <211> 273
   <212> PRT
   <213> Homo sapiens
<400> 172
   Met Thr Phe Asp Asp Leu Lys Ile Gln Thr Val Lys Asp Gln Pro Asp
    1               5                  10                  15
   Glu Lys Ser Asn Gly Lys Lys Ala Lys Gly Leu Gln Phe Leu Tyr Ser
               20                  25                  30
   Pro Trp Trp Cys Leu Ala Ala Ala Thr Leu Gly Val Leu Cys Leu Gly
           35                  40                  45
   Leu Val Val Thr Ile Met Val Leu Gly Met Gln Leu Ser Gln Val Ser
       50                  55                  60
   Asp Leu Leu Thr Gln Glu Gln Ala Asn Leu Thr His Gln Lys Lys Lys
   65                  70                  75                  80
   Leu Glu Gly Gln Ile Ser Ala Arg Gln Gln Ala Glu Glu Ala Ser Gln
                   85                  90                  95
   Glu Ser Glu Asn Glu Leu Lys Glu Met Ile Glu Thr Leu Ala Arg Lys
               100                 105                 110
   Leu Asn Glu Lys Ser Lys Glu Gln Met Glu Leu His His Gln Asn Leu
           115                 120                 125
   Asn Leu Gln Glu Thr Leu Lys Arg Val Ala Asn Cys Ser Ala Pro Cys
       130                 135                 140
   Pro Gln Asp Trp Ile Trp His Gly Glu Asn Cys Tyr Leu Phe Ser Ser
   145                 150                 155                 160
   Gly Ser Phe Asn Trp Glu Lys Ser Gln Glu Lys Cys Leu Ser Leu Asp
                   165                 170                 175
   Ala Lys Leu Leu Lys Ile Asn Ser Thr Ala Asp Leu Asp Phe Ile Gln
               180                 185                 190
   Gln Ala Ile Ser Tyr Ser Ser Phe Pro Phe Trp Met Gly Leu Ser Arg
           195                 200                 205
   Arg Asn Pro Ser Tyr Pro Trp Leu Trp Glu Asp Gly Ser Pro Leu Met
       210                 215                 220
   Pro His Leu Phe Arg Val Arg Gly Ala Val Ser Gln Thr Tyr Pro Ser
   225                 230                 235                 240
   Gly Thr Cys Ala Tyr Ile Gln Arg Gly Ala Val Tyr Ala Glu Asn Cys
                   245                 250                 255
   Ile Leu Ala Ala Phe Ser Ile Cys Gln Lys Lys Ala Asn Leu Arg Ala
               260                 265                 270
   Gln
<210> 173
   <211> 252
   <212> PRT
   <213> Homo sapiens
<400> 173
   Met Gly Val Leu Leu Thr Gln Arg Thr Leu Leu Ser Leu Val Leu Ala
    1               5                  10                  15
   Leu Leu Phe Pro Ser Met Ala Ser Met Ala Ala Ile Gly Ser Cys Ser
               20                  25                  30
   Lys Glu Tyr Arg Val Leu Leu Gly Gln Leu Gln Lys Gln Thr Asp Leu
           35                  40                  45
   Met Gln Asp Thr Ser Arg Leu Leu Asp Pro Tyr Ile Arg Ile Gln Gly
       50                  55                  60
   Leu Asp Val Pro Lys Leu Arg Glu His Cys Arg Glu Arg Pro Gly Ala
   65                  70                  75                  80
   Phe Pro Ser Glu Glu Thr Leu Arg Gly Leu Gly Arg Arg Gly Phe Leu
                   85                  90                  95
   Gln Thr Leu Asn Ala Thr Leu Gly Cys Val Leu His Arg Leu Ala Asp
               100                 105                 110
   Leu Glu Gln Arg Leu Pro Lys Ala Gln Asp Leu Glu Arg Ser Gly Leu
           115                 120                 125
   Asn Ile Glu Asp Leu Glu Lys Leu Gln Met Ala Arg Pro Asn Ile Leu
       130                 135                 140
   Gly Leu Arg Asn Asn Ile Tyr Cys Met Ala Gln Leu Leu Asp Asn Ser
   145                 150                 155                 160
   Asp Thr Ala Glu Pro Thr Lys Ala Gly Arg Gly Ala Ser Gln Pro Pro
                   165                 170                 175
   Thr Pro Thr Pro Ala Ser Asp Ala Phe Gln Arg Lys Leu Glu Gly Cys
               180                 185                 190
   Arg Phe Leu His Gly Tyr His Arg Phe Met His Ser Val Gly Arg Val
           195                 200                 205
   Phe Ser Lys Trp Gly Glu Ser Pro Asn Arg Ser Arg Arg His Ser Pro
       210                 215                 220
   His Gln Ala Leu Arg Lys Gly Val Arg Arg Thr Arg Pro Ser Arg Lys
   225                 230                 235                 240
   Gly Lys Arg Leu Met Thr Arg Gly Gln Leu Pro Arg
                   245                 250
<210> 174
   <211> 1627
   <212> PRT
   <213> Homo sapiens
<400> 174
   Met Arg Leu Trp Ser Trp Val Leu His Leu Gly Leu Leu Ser Ala Ala
    1               5                  10                  15
   Leu Gly Cys Gly Leu Ala Glu Arg Pro Arg Arg Ala Arg Arg Asp Pro
               20                  25                  30
   Arg Ala Gly Arg Pro Pro Arg Pro Ala Ala Gly Pro Ala Thr Cys Ala
           35                  40                  45
   Thr Arg Ala Ala Arg Gly Arg Arg Ala Ser Pro Pro Pro Pro Pro Pro
       50                  55                  60
   Pro Gly Gly Ala Trp Glu Ala Val Arg Val Pro Arg Arg Arg Gln Gln
   65                  70                  75                  80
   Arg Glu Ala Arg Gly Ala Thr Glu Glu Pro Ser Pro Pro Ser Arg Ala
                   85                  90                  95
   Leu Tyr Phe Ser Gly Arg Gly Glu Gln Leu Arg Leu Arg Ala Asp Leu
               100                 105                 110
   Glu Leu Pro Arg Asp Ala Phe Thr Leu Gln Val Trp Leu Arg Ala Glu
           115                 120                 125
   Gly Gly Gln Arg Ser Pro Ala Val Ile Thr Gly Leu Tyr Asp Lys Cys
       130                 135                 140
   Ser Tyr Ile Ser Arg Asp Arg Gly Trp Val Val Gly Ile His Thr Ile
   145                 150                 155                 160
   Ser Asp Gln Asp Asn Lys Asp Pro Arg Tyr Phe Phe Ser Leu Lys Thr
                   165                 170                 175
   Asp Arg Ala Arg Gln Val Thr Thr Ile Asn Ala His Arg Ser Tyr Leu
               180                 185                 190
   Pro Gly Gln Trp Val Tyr Leu Ala Ala Thr Tyr Asp Gly Gln Phe Met
           195                 200                 205
   Lys Leu Tyr Val Asn Gly Ala Gln Val Ala Thr Ser Gly Glu Gln Val
       210                 215                 220
   Gly Gly Ile Phe Ser Pro Leu Thr Gln Lys Cys Lys Val Leu Met Leu
   225                 230                 235                 240
   Gly Gly Ser Ala Leu Asn His Asn Tyr Arg Gly Tyr Ile Glu His Phe
                   245                 250                 255
   Ser Leu Trp Lys Val Ala Arg Thr Gln Arg Glu Ile Leu Ser Asp Met
               260                 265                 270
   Glu Thr His Gly Ala His Thr Ala Leu Pro Gln Leu Leu Leu Gln Glu
           275                 280                 285
   Asn Trp Asp Asn Val Lys His Ala Trp Ser Pro Met Lys Asp Gly Ser
       290                 295                 300
   Ser Pro Lys Val Glu Phe Ser Asn Ala His Gly Phe Leu Leu Asp Thr
   305                 310                 315                 320
   Ser Leu Glu Pro Pro Leu Cys Gly Gln Thr Leu Cys Asp Asn Thr Glu
                   325                 330                 335
   Val Ile Ala Ser Tyr Asn Gln Leu Ser Ser Phe Arg Gln Pro Lys Val
               340                 345                 350
   Val Arg Tyr Arg Val Val Asn Leu Tyr Glu Asp Asp His Lys Asn Pro
           355                 360                 365
   Thr Val Thr Arg Glu Gln Val Asp Phe Gln His His Gln Leu Ala Glu
       370                 375                 380
   Ala Phe Lys Gln Tyr Asn Ile Ser Trp Glu Leu Asp Val Leu Glu Val
   385                 390                 395                 400
   Ser Asn Ser Ser Leu Arg Arg Arg Leu Ile Leu Ala Asn Cys Asp Ile
                   405                 410                 415
   Ser Lys Ile Gly Asp Glu Asn Cys Asp Pro Glu Cys Asn His Thr Leu
               420                 425                 430
   Thr Gly His Asp Gly Gly Asp Cys Arg His Leu Arg His Pro Ala Phe
           435                 440                 445
   Val Lys Lys Gln His Asn Gly Val Cys Asp Met Asp Cys Asn Tyr Glu
       450                 455                 460
   Arg Phe Asn Phe Asp Gly Gly Glu Cys Cys Asp Pro Glu Ile Thr Asn
   465                 470                 475                 480
   Val Thr Gln Thr Cys Phe Asp Pro Asp Ser Pro His Arg Ala Tyr Leu
                   485                 490                 495
   Asp Val Asn Glu Leu Lys Asn Ile Leu Lys Leu Asp Gly Ser Thr His
               500                 505                 510
   Leu Asn Ile Phe Phe Ala Lys Ser Ser Glu Glu Glu Leu Ala Gly Val
           515                 520                 525
   Ala Thr Trp Pro Trp Asp Lys Glu Ala Leu Met His Leu Gly Gly Ile
       530                 535                 540
   Val Leu Asn Pro Ser Phe Tyr Gly Met Pro Gly His Thr His Thr Met
   545                 550                 555                 560
   Ile His Glu Ile Gly His Ser Leu Gly Leu Tyr His Val Phe Arg Gly
                   565                 570                 575
   Ile Ser Glu Ile Gln Ser Cys Ser Asp Pro Cys Met Glu Thr Glu Pro
               580                 585                 590
   Ser Phe Glu Thr Gly Asp Leu Cys Asn Asp Thr Asn Pro Ala Pro Lys
           595                 600                 605
   His Lys Ser Cys Gly Asp Pro Gly Pro Gly Asn Asp Thr Cys Gly Phe
       610                 615                 620
   His Ser Phe Phe Asn Thr Pro Tyr Asn Asn Phe Met Ser Tyr Ala Asp
   625                 630                 635                 640
   Asp Asp Cys Thr Asp Ser Phe Thr Pro Asn Gln Val Ala Arg Met His
                   645                 650                 655
   Cys Tyr Leu Asp Leu Val Tyr Gln Gly Trp Gln Pro Ser Arg Lys Pro
               660                 665                 670
   Ala Pro Val Ala Leu Ala Pro Gln Val Leu Gly His Thr Thr Asp Ser
           675                 680                 685
   Val Thr Leu Glu Trp Phe Pro Pro Ile Asp Gly His Phe Phe Glu Arg
       690                 695                 700
   Glu Leu Gly Ser Ala Cys His Leu Cys Leu Glu Gly Arg Ile Leu Val
   705                 710                 715                 720
   Gln Tyr Ala Ser Asn Ala Ser Ser Pro Met Pro Cys Ser Pro Ser Gly
                   725                 730                 735
   His Trp Ser Pro Arg Glu Ala Glu Gly His Pro Asp Val Glu Gln Pro
               740                 745                 750
   Cys Lys Ser Ser Val Arg Thr Trp Ser Pro Asn Ser Ala Val Asn Pro
           755                 760                 765
   His Thr Val Pro Pro Ala Cys Pro Glu Pro Gln Gly Cys Tyr Leu Glu
       770                 775                 780
   Leu Glu Phe Leu Tyr Pro Leu Val Pro Glu Ser Leu Thr Ile Trp Val
   785                 790                 795                 800
   Thr Phe Val Ser Thr Asp Trp Asp Ser Ser Gly Ala Val Asn Asp Ile
                   805                 810                 815
   Lys Leu Leu Ala Val Ser Gly Lys Asn Ile Ser Leu Gly Pro Gln Asn
               820                 825                 830
   Val Phe Cys Asp Val Pro Leu Thr Ile Arg Leu Trp Asp Val Gly Glu
           835                 840                 845
   Glu Val Tyr Gly Ile Gln Ile Tyr Thr Leu Asp Glu His Leu Glu Ile
       850                 855                 860
   Asp Ala Ala Met Leu Thr Ser Thr Ala Asp Thr Pro Leu Cys Leu Gln
   865                 870                 875                 880
   Cys Lys Pro Leu Lys Tyr Lys Val Val Arg Asp Pro Pro Leu Gln Met
                   885                 890                 895
   Asp Val Ala Ser Ile Leu His Leu Asn Arg Lys Phe Val Asp Met Asp
               900                 905                 910
   Leu Asn Leu Gly Ser Val Tyr Gln Tyr Trp Val Ile Thr Ile Ser Gly
           915                 920                 925
   Thr Glu Glu Ser Glu Pro Ser Pro Ala Val Thr Tyr Ile His Gly Ser
       930                 935                 940
   Gly Tyr Cys Gly Asp Gly Ile Ile Gln Lys Asp Gln Gly Glu Gln Cys
   945                 950                 955                 960
   Asp Asp Met Asn Lys Ile Asn Gly Asp Gly Cys Ser Leu Phe Cys Arg
                   965                 970                 975
   Gln Glu Val Ser Phe Asn Cys Ile Asp Glu Pro Ser Arg Cys Tyr Phe
               980                 985                 990
   His Asp Gly Asp Gly Val Cys Glu Glu Phe Glu Gln Lys Thr Ser Ile
           995                 1000                1005
   Lys Asp Cys Gly Val Tyr Thr Pro Gln Gly Phe Leu Asp Gln Trp Ala
       1010                1015                1020
   Ser Asn Ala Ser Val Ser His Gln Asp Gln Gln Cys Pro Gly Trp Val
   1025                1030                1035                1040
   Ile Ile Gly Gln Pro Ala Ala Ser Gln Val Cys Arg Thr Lys Val Ile
                   1045                1050                1055
   Asp Leu Ser Glu Gly Ile Ser Gln His Ala Trp Tyr Pro Cys Thr Ile
               1060                1065                1070
   Ser Tyr Pro Tyr Ser Gln Leu Ala Gln Thr Thr Phe Trp Leu Arg Ala
           1075                1080                1085
   Tyr Phe Ser Gln Pro Met Val Ala Ala Ala Val Ile Val His Leu Val
       1090                1095                1100
   Thr Asp Gly Thr Tyr Tyr Gly Asp Gln Lys Gln Glu Thr Ile Ser Val
   1105                1110                1115                1120
   Gln Leu Leu Asp Thr Lys Asp Gln Ser His Asp Leu Gly Leu His Val
                   1125                1130                1135
   Leu Ser Cys Arg Asn Asn Pro Leu Ile Ile Pro Val Val His Asp Leu
               1140                1145                1150
   Ser Gln Pro Phe Tyr His Ser Gln Ala Val Arg Val Ser Phe Ser Ser
           1155                1160                1165
   Pro Leu Val Ala Ile Ser Gly Val Ala Leu Arg Ser Phe Asp Asn Phe
       1170                1175                1180
   Asp Pro Val Thr Leu Ser Ser Cys Gln Arg Gly Glu Thr Tyr Ser Pro
   1185                1190                1195                1200
   Ala Glu Gln Ser Cys Val His Phe Ala Cys Glu Lys Thr Asp Cys Pro
                   1205                1210                1215
   Glu Leu Ala Val Glu Asn Ala Ser Leu Asn Cys Ser Ser Ser Asp Arg
               1220                1225                1230
   Tyr His Gly Ala Gln Cys Thr Val Ser Cys Arg Thr Gly Tyr Val Leu
           1235                1240                1245
   Gln Ile Arg Arg Asp Asp Glu Leu Ile Lys Ser Gln Thr Gly Pro Ser
       1250                1255                1260
   Val Thr Val Thr Cys Thr Glu Gly Lys Trp Asn Lys Gln Val Ala Cys
   1265 1270 1275 1280
   Glu Pro Val Asp Cys Ser Ile Pro Asp His His Gln Val Tyr Ala Ala
   1285 1290 1295
   Ser Phe Ser Cys Pro Glu Gly Thr Thr Phe Gly Ser Gln Cys Ser Phe
   1300 1305 1310
   Gln Cys Arg His Pro Ala Gln Leu Lys Gly Asn Asn Ser Leu Leu Thr
   1315 1320 1325
   Cys Met Glu Asp Gly Leu Trp Ser Phe Pro Glu Ala Leu Cys Glu Leu
   1330 1335 1340
   Met Cys Leu Ala Pro Pro Pro Val Pro Asn Ala Asp Leu Gln Thr Ala
   1345 1350 1355 1360
   Arg Cys Arg Glu Asn Lys His Lys Val Gly Ser Phe Cys Lys Tyr Lys
   1365 1370 1375
   Cys Lys Pro Gly Tyr His Val Pro Gly Ser Ser Arg Lys Ser Lys Lys
   1380 1385 1390
   Arg Ala Phe Lys Thr Gln Cys Thr Gln Asp Gly Ser Trp Gln Glu Gly
   1395 1400 1405
   Ala Cys Val Pro Val Thr Cys Asp Pro Pro Pro Pro Lys Phe His Gly
   1410 1415 1420
   Leu Tyr Gln Cys Thr Asn Gly Phe Gln Phe Asn Ser Glu Cys Arg Ile
   1425 1430 1435 1440
   Lys Cys Glu Asp Ser Asp Ala Ser Gln Gly Leu Gly Ser Asn Val Ile
   1445 1450 1455
   His Cys Arg Lys Asp Gly Thr Trp Asn Gly Ser Phe His Val Cys Gln
   1460 1465 1470
   Glu Met Gln Gly Gln Cys Ser Val Pro Asn Glu Leu Asn Ser Asn Leu
   1475 1480 1485
   Lys Leu Gln Cys Pro Asp Gly Tyr Ala Ile Gly Ser Glu Cys Ala Thr
   1490 1495 1500
   Ser Cys Leu Asp His Asn Ser Glu Ser Ile Ile Leu Pro Met Asn Val
   1505 1510 1515 1520
   Thr Val Arg Asp Ile Pro His Trp Leu Asn Pro Thr Arg Val Glu Arg
   1525 1530 1535
   Val Val Cys Thr Ala Gly Leu Lys Trp Tyr Pro His Pro Ala Leu Ile
   1540 1545 1550
   His Cys Val Lys Gly Cys Glu Pro Phe Met Gly Asp Asn Tyr Cys Asp
   1555 1560 1565
   Ala Ile Asn Asn Arg Ala Phe Cys Asn Tyr Asp Gly Gly Asp Cys Cys
   1570 1575 1580
   Thr Ser Thr Val Lys Thr Lys Lys Val Thr Pro Phe Pro Met Ser Cys
   1585 1590 1595 1600
   Asp Leu Gln Gly Asp Cys Ala Cys Arg Asp Pro Gln Ala Gln Glu His
   1605 1610 1615
   Ser Arg Lys Asp Leu Arg Gly Tyr Ser His Gly
   1620 1625
<210> 175
   <211> 491
   <212> PRT
   <213> Homo sapiens
<400> 175
<210> 176
   <211> 951
   <212> PRT
   <213> Homo sapiens
<400> 176
   Met Arg Gly Arg Gly Asn Ala Arg Ser Ser Gln Ala Leu Gly Val Ser
    1               5                  10                  15
   Trp Cys Pro Ala Thr Trp His Pro Arg Leu Asp Met Gly Arg Leu His
               20                  25                  30
   Arg Pro Arg Ser Ser Thr Ser Tyr Arg Asn Leu Pro His Leu Phe Leu
           35                  40                  45
   Phe Phe Leu Phe Val Gly Pro Phe Ser Cys Leu Gly Ser Tyr Ser Arg
       50                  55                  60
   Ala Thr Glu Leu Leu Tyr Ser Leu Asn Glu Gly Leu Pro Ala Gly Val
   65                  70                  75                  80
   Leu Ile Gly Ser Leu Ala Glu Asp Leu Arg Leu Leu Pro Arg Ser Ala
                   85                  90                  95
   Gly Arg Pro Asp Pro Gln Ser Gln Leu Pro Glu Arg Thr Gly Ala Glu
               100                 105                 110
   Trp Asn Pro Pro Leu Ser Phe Ser Leu Ala Ser Arg Gly Leu Ser Gly
           115                 120                 125
   Gln Tyr Val Thr Leu Asp Asn Arg Ser Gly Glu Leu His Thr Ser Ala
       130                 135                 140
   Gln Glu Ile Asp Arg Glu Ala Leu Cys Val Glu Gly Gly Gly Gly Thr
   145                 150                 155                 160
   Ala Trp Ser Gly Ser Val Ser Ile Ser Ser Ser Pro Ser Asp Ser Cys
                   165                 170                 175
   Leu Leu Leu Leu Asp Val Leu Val Leu Pro Gln Glu Tyr Phe Arg Phe
               180                 185                 190
   Val Lys Val Lys Ile Ala Ile Arg Asp Ile Asn Asp Asn Ala Pro Gln
           195                 200                 205
   Phe Pro Val Ser Gln Ile Ser Val Trp Val Pro Glu Asn Ala Pro Val
       210                 215                 220
   Asn Thr Arg Leu Ala Ile Glu His Pro Ala Val Asp Pro Asp Val Gly
   225                 230                 235                 240
   Ile Asn Gly Val Gln Thr Tyr Arg Leu Leu Asp Tyr His Gly Met Phe
                   245                 250                 255
   Thr Leu Asp Val Glu Glu Asn Glu Asn Gly Glu Arg Thr Pro Tyr Leu
               260                 265                 270
   Ile Val Met Gly Ala Leu Asp Arg Glu Thr Gln Asp Gln Tyr Val Ser
           275                 280                 285
   Ile Ile Ile Ala Glu Asp Gly Gly Ser Pro Pro Leu Leu Gly Ser Ala
       290                 295                 300
   Thr Leu Thr Ile Gly Ile Ser Asp Ile Asn Asp Asn Cys Pro Leu Phe
   305                 310                 315                 320
   Thr Asp Ser Gln Ile Asn Val Thr Val Tyr Gly Asn Ala Thr Val Gly
                   325                 330                 335
   Thr Pro Ile Ala Ala Val Gln Ala Val Asp Lys Asp Leu Gly Thr Asn
               340                 345                 350
   Ala Gln Ile Thr Tyr Ser Tyr Ser Gln Lys Val Pro Gln Ala Ser Lys
           355                 360                 365
   Asp Leu Phe His Leu Asp Glu Asn Thr Gly Val Ile Lys Leu Phe Ser
       370                 375                 380
   Lys Ile Gly Gly Ser Val Leu Glu Ser His Lys Leu Thr Ile Leu Ala
   385                 390                 395                 400
   Asn Gly Pro Gly Cys Ile Pro Ala Val Ile Thr Ala Leu Val Ser Ile
                   405                 410                 415
   Ile Lys Val Ile Phe Arg Pro Pro Glu Ile Val Pro Arg Tyr Ile Ala
               420                 425                 430
   Asn Glu Ile Asp Gly Val Val Tyr Leu Lys Glu Leu Glu Pro Val Asn
           435                 440                 445
   Thr Pro Ile Ala Phe Phe Thr Ile Arg Asp Pro Glu Gly Lys Tyr Lys
       450                 455                 460
   Val Asn Cys Tyr Leu Asp Gly Glu Gly Pro Phe Arg Leu Ser Pro Tyr
   465                 470                 475                 480
   Lys Pro Tyr Asn Asn Glu Tyr Leu Leu Glu Thr Thr Lys Pro Met Asp
                   485                 490                 495
   Tyr Glu Leu Gln Gln Phe Tyr Glu Val Ala Val Val Ala Trp Asn Ser
               500                 505                 510
   Glu Gly Phe His Val Lys Arg Val Ile Lys Val Gln Leu Leu Asp Asp
           515                 520                 525
   Asn Asp Asn Ala Pro Ile Phe Leu Gln Pro Leu Ile Glu Leu Thr Ile
       530                 535                 540
   Glu Glu Asn Asn Ser Pro Asn Ala Phe Leu Thr Lys Leu Tyr Ala Thr
   545                 550                 555                 560
   Asp Ala Asp Ser Glu Glu Arg Gly Gln Val Ser Tyr Phe Leu Gly Pro
                   565                 570                 575
   Asp Ala Pro Ser Tyr Phe Ser Leu Asp Ser Val Thr Gly Ile Leu Thr
               580                 585                 590
   Val Ser Thr Gln Leu Asp Arg Glu Glu Lys Glu Lys Tyr Arg Tyr Thr
           595                 600                 605
   Val Arg Ala Val Asp Cys Gly Lys Pro Pro Arg Glu Ser Val Ala Thr
       610                 615                 620
   Val Ala Leu Thr Val Leu Asp Lys Asn Asp Asn Ser Pro Arg Phe Ile
   625                 630                 635                 640
   Asn Lys Asp Phe Ser Phe Phe Val Pro Glu Asn Phe Pro Gly Tyr Gly
                   645                 650                 655
   Glu Ile Gly Val Ile Ser Val Thr Asp Ala Asp Ala Gly Arg Asn Gly
               660                 665                 670
   Trp Val Ala Leu Ser Val Val Asn Gln Ser Asp Ile Phe Val Ile Asp
           675                 680                 685
   Thr Gly Lys Gly Met Leu Arg Ala Lys Val Ser Leu Asp Arg Glu Gln
       690                 695                 700
   Gln Ser Ser Tyr Thr Leu Trp Val Glu Ala Val Asp Gly Gly Glu Pro
   705                 710                 715                 720
   Ala Leu Ser Ser Thr Ala Lys Ile Thr Ile Leu Leu Leu Asp Ile Asn
                   725                 730                 735
   Asp Asn Pro Pro Leu Val Leu Phe Pro Gln Ser Asn Met Ser Tyr Leu
               740                 745                 750
   Leu Val Leu Pro Ser Thr Leu Pro Gly Ser Pro Val Thr Glu Val Tyr
           755                 760                 765
   Ala Val Asp Lys Asp Thr Gly Met Asn Ala Val Ile Ala Tyr Ser Ile
       770                 775                 780
   Ile Gly Arg Arg Gly Pro Arg Pro Glu Ser Phe Arg Ile Asp Pro Lys
   785                 790                 795                 800
   Thr Gly Asn Ile Thr Leu Glu Glu Ala Leu Leu Gln Thr Asp Tyr Gly
                   805                 810                 815
   Leu His Arg Leu Leu Val Lys Val Ser Asp His Gly Tyr Pro Glu Pro
               820                 825                 830
   Leu His Ser Thr Val Met Val Asn Leu Phe Val Asn Asp Thr Val Ser
           835                 840                 845
   Asn Glu Ser Tyr Ile Glu Ser Leu Leu Arg Lys Glu Pro Glu Ile Asn
       850                 855                 860
   Ile Glu Glu Lys Glu Pro Gln Ile Ser Ile Glu Pro Thr His Arg Lys
   865                 870                 875                 880
   Val Glu Ser Val Ser Cys Met Pro Thr Leu Val Ala Leu Ser Val Ile
                   885                 890                 895
   Ser Leu Gly Ser Ile Thr Leu Val Thr Gly Met Gly Ile Tyr Ile Cys
               900                 905                 910
   Leu Arg Lys Gly Glu Lys His Pro Arg Glu Asp Glu Asn Leu Glu Val
           915                 920                 925
   Gln Ile Pro Leu Lys Gly Lys Ile Asp Leu His Met Arg Glu Arg Lys
             930                 935                 940
   Pro Met Asp Ile Ser Asn Ile
   945                 950
<210> 177
   <211> 564
   <212> PRT
   <213> Homo sapiens
<400> 177
   Met Lys Glu His Gly Gly Thr Phe Ser Ser Thr Gly Ile Ser Gly Gly
    1               5                  10                  15
   Ser Gly Asp Ser Ala Met Asp Ser Leu Gln Pro Leu Gln Pro Asn Tyr
               20                  25                  30
   Met Pro Val Cys Leu Phe Ala Glu Glu Ser Tyr Gln Lys Leu Ala Met
           35                  40                  45
   Glu Thr Leu Glu Glu Leu Asp Trp Cys Leu Asp Gln Leu Glu Thr Ile
       50                  55                  60
   Gln Thr Tyr Arg Ser Val Ser Glu Met Ala Ser Asn Lys Phe Lys Arg
   65                  70                  75                  80
   Met Leu Asn Arg Glu Leu Thr His Leu Ser Glu Met Ser Arg Ser Gly
                   85                  90                  95
   Asn Gln Val Ser Glu Tyr Ile Ser Asn Thr Phe Leu Asp Lys Gln Asn
               100                 105                 110
   Asp Val Glu Ile Pro Ser Pro Thr Gln Lys Asp Arg Glu Lys Lys Lys
           115                 120                 125
   Lys Gln Gln Leu Met Thr Gln Ile Ser Gly Val Lys Lys Leu Met His
       130                 135                 140
   Ser Ser Ser Leu Asn Asn Thr Ser Ile Ser Arg Phe Gly Val Asn Thr
   145                 150                 155                 160
   Glu Asn Glu Asp His Leu Ala Lys Glu Leu Glu Asp Leu Asn Lys Trp
                   165                 170                 175
   Gly Leu Asn Ile Phe Asn Val Ala Gly Tyr Ser His Asn Arg Pro Leu
               180                 185                 190
   Thr Cys Ile Met Tyr Ala Ile Phe Gln Glu Arg Asp Leu Leu Lys Thr
           195                 200                 205
   Phe Arg Ile Ser Ser Asp Thr Phe Ile Thr Tyr Met Met Thr Leu Glu
       210                 215                 220
   Asp His Tyr His Ser Asp Val Ala Tyr His Asn Ser Leu His Ala Ala
   225                 230                 235                 240
   Asp Val Ala Gln Ser Thr His Val Leu Leu Ser Thr Pro Ala Leu Asp
                   245                 250                 255
   Ala Val Phe Thr Asp Leu Glu Ile Leu Ala Ala Ile Phe Ala Ala Ala
               260                 265                 270
   Ile His Asp Val Asp His Pro Gly Val Ser Asn Gln Phe Leu Ile Asn
           275                 280                 285
   Thr Asn Ser Glu Leu Ala Leu Met Tyr Asn Asp Glu Ser Val Leu Glu
       290                 295                 300
   Asn His His Leu Ala Val Gly Phe Lys Leu Leu Gln Glu Glu His Cys
   305                 310                 315                 320
   Asp Ile Phe Met Asn Leu Thr Lys Lys Gln Arg Gln Thr Leu Arg Lys
                   325                 330                 335
   Met Val Ile Asp Met Val Leu Ala Thr Asp Met Ser Lys His Met Ser
               340                 345                 350
   Leu Leu Ala Asp Leu Lys Thr Met Val Glu Thr Lys Lys Val Thr Ser
           355                 360                 365
   Ser Gly Val Leu Leu Leu Asp Asn Tyr Thr Asp Arg Ile Gln Val Leu
       370                 375                 380
   Arg Asn Met Val His Cys Ala Asp Leu Ser Asn Pro Thr Lys Ser Leu
   385                 390                 395                 400
   Glu Leu Tyr Arg Gln Trp Thr Asp Arg Ile Met Glu Glu Phe Phe Gln
                   405                 410                 415
   Gln Gly Asp Lys Glu Arg Glu Arg Gly Met Glu Ile Ser Pro Met Cys
               420                 425                 430
   Asp Lys His Thr Ala Ser Val Glu Lys Ser Gln Val Gly Phe Ile Asp
           435                 440                 445
   Tyr Ile Val His Pro Leu Trp Glu Thr Trp Ala Asp Leu Val Gln Pro
       450                 455                 460
   Asp Ala Gln Asp Ile Leu Asp Thr Leu Glu Asp Asn Arg Asn Trp Tyr
   465                 470                 475                 480
   Gln Ser Met Ile Pro Gln Ser Pro Ser Pro Pro Leu Asp Glu Gln Asn
                   485                 490                 495
   Arg Asp Cys Gln Gly Leu Met Glu Lys Phe Gln Phe Glu Leu Thr Leu
               500                 505                 510
   Asp Glu Glu Asp Ser Glu Gly Pro Glu Lys Glu Gly Glu Gly His Ser
           515                 520                 525
   Tyr Phe Ser Ser Thr Lys Thr Leu Cys Val Ile Asp Pro Glu Asn Arg
       530                 535                 540
   Asp Ser Leu Gly Glu Thr Asp Ile Asp Ile Ala Thr Glu Asp Lys Ser
   545                 550                 555                 560
   Pro Val Asp Thr
<210> 178
   <211> 303
   <212> PRT
   <213> Homo sapiens
<400> 178
   Met Gly Arg Pro Leu Leu Leu Pro Leu Leu Pro Leu Leu Leu Pro Pro
    1               5                  10                  15
   Ala Phe Leu Gln Pro Ser Gly Ser Thr Gly Ser Gly Pro Ser Tyr Leu
               20                  25                  30
   Tyr Gly Val Thr Gln Pro Lys His Leu Ser Ala Ser Met Gly Gly Ser
           35                  40                  45
   Val Glu Ile Pro Phe Ser Phe Tyr Tyr Pro Trp Glu Leu Ala Thr Ala
       50                  55                  60
   Pro Asp Val Arg Ile Ser Trp Arg Arg Gly His Phe His Arg Gln Ser
   65                  70                  75                  80
   Phe Tyr Ser Thr Arg Pro Pro Ser Ile His Lys Asp Tyr Val Asn Arg
                   85                  90                  95
   Leu Phe Leu Asn Trp Thr Glu Gly Gln Lys Ser Gly Phe Leu Arg Ile
               100                 105                 110
   Ser Asn Leu Gln Lys Gln Asp Gln Ser Val Tyr Phe Cys Arg Val Glu
           115                 120                 125
   Leu Asp Thr Arg Ser Ser Gly Arg Gln Gln Trp Gln Ser Ile Glu Gly
       130                 135                 140
   Thr Lys Leu Ser Ile Thr Gln Ala Val Thr Thr Thr Thr Gln Arg Pro
   145                 150                 155                 160
   Ser Ser Met Thr Thr Thr Trp Arg Leu Ser Ser Thr Thr Thr Thr Thr
                   165                 170                 175
   Gly Leu Arg Val Thr Gln Gly Lys Arg Arg Ser Asp Ser Trp His Ile
               180                 185                 190
   Ser Leu Glu Thr Ala Val Gly Val Ala Val Ala Val Thr Val Leu Gly
           195                 200                 205
   Ile Met Ile Leu Gly Leu Ile Cys Leu Leu Arg Trp Arg Arg Arg Lys
       210                 215                 220
   Gly Gln Gln Arg Thr Lys Ala Thr Thr Pro Ala Arg Glu Pro Phe Gln
   225                 230                 235                 240
   Asn Thr Glu Glu Pro Tyr Glu Asn Ile Arg Asn Glu Gly Gln Asn Thr
                   245                 250                 255
   Asp Pro Lys Leu Asn Pro Lys Asp Asp Gly Ile Val Tyr Ala Ser Leu
               260                 265                 270
   Ala Leu Ser Ser Ser Thr Ser Pro Arg Ala Pro Pro Ser His Arg Pro
           275                 280                 285
   Leu Lys Ser Pro Gln Asn Glu Thr Leu Tyr Ser Val Leu Lys Ala
       290                 295                 300
<210> 179
   <211> 350
   <212> PRT
   <213> Homo sapiens
<400> 179
   Met Glu Pro Lys Arg Ile Arg Glu Gly Tyr Leu Val Lys Lys Gly Ser
    1               5                  10                  15
   Val Phe Asn Thr Trp Lys Pro Met Trp Val Val Leu Leu Glu Asp Gly
               20                  25                  30
   Ile Glu Phe Tyr Lys Lys Lys Ser Asp Asn Ser Pro Lys Gly Met Ile
           35                  40                  45
   Pro Leu Lys Gly Ser Thr Leu Thr Ser Pro Cys Gln Asp Phe Gly Lys
       50                  55                  60
   Arg Met Phe Val Phe Lys Ile Thr Thr Thr Lys Gln Gln Asp His Phe
   65                  70                  75                  80
   Phe Gln Ala Ala Phe Leu Glu Glu Arg Asp Ala Trp Val Arg Asp Ile
                   85                  90                  95
   Lys Lys Ala Ile Lys Cys Ile Glu Gly Gly Gln Lys Phe Ala Arg Lys
               100                 105                 110
   Ser Thr Arg Arg Ser Ile Arg Leu Pro Glu Thr Ile Asp Leu Gly Ala
           115                 120                 125
   Leu Tyr Leu Ser Met Lys Asp Thr Glu Lys Gly Ile Lys Glu Leu Asn
       130                 135                 140
   Leu Glu Lys Asp Lys Lys Ile Phe Asn His Cys Phe Thr Gly Asn Cys
   145                 150                 155                 160
   Val Ile Asp Trp Leu Val Ser Asn Gln Ser Val Arg Asn Arg Gln Glu
                   165                 170                 175
   Gly Leu Met Ile Ala Ser Ser Leu Leu Asn Glu Gly Tyr Leu Gln Pro
               180                 185                 190
   Ala Gly Asp Met Ser Lys Ser Ala Val Asp Gly Thr Ala Glu Asn Pro
           195                 200                 205
   Phe Leu Asp Asn Pro Asp Ala Phe Tyr Tyr Phe Pro Asp Ser Gly Phe
       210                 215                 220
   Phe Cys Glu Glu Asn Ser Ser Asp Asp Asp Val Ile Leu Lys Glu Glu
   225                 230                 235                 240
   Phe Arg Gly Val Ile Ile Lys Gln Gly Cys Leu Leu Lys Gln Gly His
                   245                 250                 255
   Arg Arg Lys Asn Trp Lys Val Arg Lys Phe Ile Leu Arg Glu Asp Pro
               260                 265                 270
   Ala Tyr Leu His Tyr Tyr Asp Pro Ala Gly Ala Glu Asp Pro Leu Gly
           275                 280                 285
   Ala Ile His Leu Arg Gly Cys Val Val Thr Ser Val Glu Ser Asn Ser
       290                 295                 300
   Asn Gly Arg Lys Ser Glu Glu Glu Asn Leu Phe Glu Ile Ile Thr Ala
   305                 310                 315                 320
   Asp Glu Val His Tyr Phe Leu Gln Ala Ala Thr Pro Lys Glu Arg Thr
                   325                 330                 335
   Glu Trp Ile Arg Ala Ile Gln Met Ala Ser Arg Thr Gly Lys
               340                 345                 350
<210> 180
   <211> 158
   <212> PRT
   <213> Homo sapiens
<400> 180
   Met Met Gln Lys Leu Leu Lys Cys Ser Arg Leu Val Leu Ala Leu Ala
    1              5                  10                  15
   Leu Ile Leu Val Leu Glu Ser Ser Val Gln Gly Tyr Pro Thr Arg Arg
               20                  25                  30
   Ala Arg Tyr Gln Trp Val Arg Cys Asn Pro Asp Ser Asn Ser Ala Asn
           35                  40                  45
   Cys Leu Glu Glu Lys Gly Pro Met Phe Glu Leu Leu Pro Gly Glu Ser
       50                  55                  60
   Asn Lys Ile Pro Arg Leu Arg Thr Asp Leu Phe Pro Lys Thr Arg Ile
   65                  70                  75                  80
   Gln Asp Leu Asn Arg Ile Phe Pro Leu Ser Glu Asp Tyr Ser Gly Ser
                   85                  90                  95
   Gly Phe Gly Ser Gly Ser Gly Ser Gly Ser Gly Ser Gly Ser Gly Phe
               100                 105                 110
   Leu Thr Glu Met Glu Gln Asp Tyr Gln Leu Val Asp Glu Ser Asp Ala
           115                 120                 125
   Phe His Asp Asn Leu Arg Ser Leu Asp Arg Asn Leu Pro Ser Asp Ser
       130                 135                 140
   Gln Asp Leu Gly Gln His Gly Leu Glu Glu Asp Phe Met Leu
   145                 150                 155
<210> 181
   <211> 129
   <212> PRT
   <213> Homo sapiens
<400> 181
   Met Arg Ser Leu Cys Cys Ala Pro Leu Leu Leu Leu Leu Leu Leu Pro
    1              5                  10                  15
   Pro Leu Leu Leu Thr Pro Arg Ala Gly Asp Ala Ala Val Ile Thr Gly
               20                  25                  30
   Ala Cys Asp Lys Asp Ser Gln Cys Gly Gly Gly Met Cys Cys Ala Val
           35                  40                  45
   Ser Ile Trp Val Lys Ser Ile Arg Ile Cys Thr Pro Met Gly Lys Leu
       50                  55                  60
   Gly Asp Ser Cys His Pro Leu Thr Arg Lys Asn Asn Phe Gly Asn Gly
   65                  70                  75                  80
   Arg Gln Glu Arg Arg Lys Arg Lys Arg Ser Lys Arg Lys Lys Glu Val
                   85                  90                  95
   Pro Phe Phe Gly Arg Arg Met His His Thr Cys Pro Cys Leu Pro Gly
               100                 105                 110
   Leu Ala Cys Leu Arg Thr Ser Phe Asn Arg Phe Ile Cys Leu Ala Gln
           115                 120                 125
   Lys
<210> 182
   <211> 604
   <212> PRT
   <213> Homo sapiens
<400> 182
   Met Leu Ala Arg Ala Leu Leu Leu Cys Ala Val Leu Ala Leu Ser His
    1               5                  10                  15
   Thr Ala Asn Pro Cys Cys Ser His Pro Cys Gln Asn Arg Gly Val Cys
               20                  25                  30
   Met Ser Val Gly Phe Asp Gln Tyr Lys Cys Asp Cys Thr Arg Thr Gly
           35                  40                  45
   Phe Tyr Gly Glu Asn Cys Ser Thr Pro Glu Phe Leu Thr Arg Ile Lys
       50                  55                  60
   Leu Phe Leu Lys Pro Thr Pro Asn Thr Val His Tyr Ile Leu Thr His
   65                  70                  75                  80
   Phe Lys Gly Phe Trp Asn Val Val Asn Asn Ile Pro Phe Leu Arg Asn
                   85                  90                  95
   Ala Ile Met Ser Tyr Val Leu Thr Ser Arg Ser His Leu Ile Asp Ser
               100                 105                 110
   Pro Pro Thr Tyr Asn Ala Asp Tyr Gly Tyr Lys Ser Trp Glu Ala Phe
           115                 120                 125
   Ser Asn Leu Ser Tyr Tyr Thr Arg Ala Leu Pro Pro Val Pro Asp Asp
       130                 135                 140
   Cys Pro Thr Pro Leu Gly Val Lys Gly Lys Lys Gln Leu Pro Asp Ser
   145                 150                 155                 160
   Asn Glu Ile Val Glu Lys Leu Leu Leu Arg Arg Lys Phe Ile Pro Asp
                   165                 170                 175
   Pro Gln Gly Ser Asn Met Met Phe Ala Phe Phe Ala Gln His Phe Thr
               180                 185                 190
   His Gln Phe Phe Lys Thr Asp His Lys Arg Gly Pro Ala Phe Thr Asn
           195                 200                 205
   Gly Leu Gly His Gly Val Asp Leu Asn His Ile Tyr Gly Glu Thr Leu
       210                 215                 220
   Ala Arg Gln Arg Lys Leu Arg Leu Phe Lys Asp Gly Lys Met Lys Tyr
   225                 230                 235                 240
   Gln Ile Ile Asp Gly Glu Met Tyr Pro Pro Thr Val Lys Asp Thr Gln
                   245                 250                 255
   Ala Glu Met Ile Tyr Pro Pro Gln Val Pro Glu His Leu Arg Phe Ala
               260                 265                 270
   Val Gly Gln Glu Val Phe Gly Leu Val Pro Gly Leu Met Met Tyr Ala
           275                 280                 285
   Thr Ile Trp Leu Arg Glu His Asn Arg Val Cys Asp Val Leu Lys Gln
       290                 295                 300
   Glu His Pro Glu Trp Gly Asp Glu Gln Leu Phe Gln Thr Ser Arg Leu
   305                 310                 315                 320
   Ile Leu Ile Gly Glu Thr Ile Lys Ile Val Ile Glu Asp Tyr Val Gln
                   325                 330                 335
   His Leu Ser Gly Tyr His Phe Lys Leu Lys Phe Asp Pro Glu Leu Leu
               340                 345                 350
   Phe Asn Lys Gln Phe Gln Tyr Gln Asn Arg Ile Ala Ala Glu Phe Asn
           355                 360                 365
   Thr Leu Tyr His Trp His Pro Leu Leu Pro Asp Thr Phe Gln Ile His
       370                 375                 380
   Asp Gln Lys Tyr Asn Tyr Gln Gln Phe Ile Tyr Asn Asn Ser Ile Leu
   385                 390                 395                 400
   Leu Glu His Gly Ile Thr Gln Phe Val Glu Ser Phe Thr Arg Gln Ile
                   405                 410                 415
   Ala Gly Arg Val Ala Gly Gly Arg Asn Val Pro Pro Ala Val Gln Lys
               420                 425                 430
   Val Ser Gln Ala Ser Ile Asp Gln Ser Arg Gln Met Lys Tyr Gln Ser
           435                 440                 445
   Phe Asn Glu Tyr Arg Lys Arg Phe Met Leu Lys Pro Tyr Glu Ser Phe
       450                 455                 460
   Glu Glu Leu Thr Gly Glu Lys Glu Met Ser Ala Glu Leu Glu Ala Leu
   465                 470                 475                 480
   Tyr Gly Asp Ile Asp Ala Val Glu Leu Tyr Pro Ala Leu Leu Val Glu
                   485                 490                 495
   Lys Pro Arg Pro Asp Ala Ile Phe Gly Glu Thr Met Val Glu Val Gly
               500                 505                 510
   Ala Pro Phe Ser Leu Lys Gly Leu Met Gly Asn Val Ile Cys Ser Pro
           515                 520                 525
   Ala Tyr Trp Lys Pro Ser Thr Phe Gly Gly Glu Val Gly Phe Gln Ile
       530                 535                 540
   Ile Asn Thr Ala Ser Ile Gln Ser Leu Ile Cys Asn Asn Val Lys Gly
   545                 550                 555                 560
   Cys Pro Phe Thr Ser Phe Ser Val Pro Asp Pro Glu Leu Ile Lys Thr
                   565                 570                 575
   Val Thr Ile Asn Ala Ser Ser Ser Arg Ser Gly Leu Asp Asp Ile Asn
               580                 585                 590
   Pro Thr Val Leu Leu Lys Glu Arg Ser Thr Glu Leu
           595                 600
<210> 183
   <211> 604
   <212> PRT
   <213> Homo sapiens
<400> 183
   Met Leu Ala Arg Ala Leu Leu Leu Cys Ala Val Leu Ala Leu Ser His
    1               5                  10                  15
   Thr Ala Asn Pro Cys Cys Ser His Pro Cys Gln Asn Arg Gly Val Cys
               20                  25                  30
   Met Ser Val Gly Phe Asp Gln Tyr Lys Cys Asp Cys Thr Arg Thr Gly
           35                  40                  45
   Phe Tyr Gly Glu Asn Cys Ser Thr Pro Glu Phe Leu Thr Arg Ile Lys
       50                  55                  60
   Leu Phe Leu Lys Pro Thr Pro Asn Thr Val His Tyr Ile Leu Thr His
   65                  70                  75                  80
   Phe Lys Gly Phe Trp Asn Val Val Asn Asn Ile Pro Phe Leu Arg Asn
                   85                  90                  95
   Ala Ile Met Ser Tyr Val Leu Thr Ser Arg Ser His Leu Ile Asp Ser
               100                 105                 110
   Pro Pro Thr Tyr Asn Ala Asp Tyr Gly Tyr Lys Ser Trp Glu Ala Phe
           115                 120                 125
   Ser Asn Leu Ser Tyr Tyr Thr Arg Ala Leu Pro Pro Val Pro Asp Asp
       130                 135                 140
   Cys Pro Thr Pro Leu Gly Val Lys Gly Lys Lys Gln Leu Pro Asp Ser
   145                 150                 155                 160
   Asn Glu Ile Val Glu Lys Leu Leu Leu Arg Arg Lys Phe Ile Pro Asp
                   165                 170                 175
   Pro Gln Gly Ser Asn Met Met Phe Ala Phe Phe Ala Gln His Phe Thr
               180                 185                 190
   His Gln Phe Phe Lys Thr Asp His Lys Arg Gly Pro Ala Phe Thr Asn
           195                 200                 205
   Gly Leu Gly His Gly Val Asp Leu Asn His Ile Tyr Gly Glu Thr Leu
       210                 215                 220
   Ala Arg Gln Arg Lys Leu Arg Leu Phe Lys Asp Gly Lys Met Lys Tyr
   225                 230                 235                 240
   Gln Ile Ile Asp Gly Glu Met Tyr Pro Pro Thr Val Lys Asp Thr Gln
                   245                 250                 255
   Ala Glu Met Ile Tyr Pro Pro Gln Val Pro Glu His Leu Arg Phe Ala
                   260                 265                 270
   Val Gly Gln Glu Val Phe Gly Leu Val Pro Gly Leu Met Met Tyr Ala
               275                 280                 285
   Thr Ile Trp Leu Arg Glu His Asn Arg Val Cys Asp Val Leu Lys Gln
       290                 295                 300
   Glu His Pro Glu Trp Gly Asp Glu Gln Leu Phe Gln Thr Ser Arg Leu
   305                 310                 315                 320
   Ile Leu Ile Gly Glu Thr Ile Lys Ile Val Ile Glu Asp Tyr Val Gln
                   325                 330                 335
   His Leu Ser Gly Tyr His Phe Lys Leu Lys Phe Asp Pro Glu Leu Leu
               340                 345                 350
   Phe Asn Lys Gln Phe Gln Tyr Gln Asn Arg Ile Ala Ala Glu Phe Asn
           355                 360                 365
   Thr Leu Tyr His Trp His Pro Leu Leu Pro Asp Thr Phe Gln Ile His
       370                 375                 380
   Asp Gln Lys Tyr Asn Tyr Gln Gln Phe Ile Tyr Asn Asn Ser Ile Leu
   385                 390                 395                 400
   Leu Glu His Gly Ile Thr Gln Phe Val Glu Ser Phe Thr Arg Gln Ile
                   405                 410                 415
   Ala Gly Arg Val Ala Gly Gly Arg Asn Val Pro Pro Ala Val Gln Lys
               420                 425                 430
   Val Ser Gln Ala Ser Ile Asp Gln Ser Arg Gln Met Lys Tyr Gln Ser
           435                 440                 445
   Phe Asn Glu Tyr Arg Lys Arg Phe Met Leu Lys Pro Tyr Glu Ser Phe
       450                 455                 460
   Glu Glu Leu Thr Gly Glu Lys Glu Met Ser Ala Glu Leu Glu Ala Leu
   465                 470                 475                 480
   Tyr Gly Asp Ile Asp Ala Val Glu Leu Tyr Pro Ala Leu Leu Val Glu
                   485                 490                 495
   Lys Pro Arg Pro Asp Ala Ile Phe Gly Glu Thr Met Val Glu Val Gly
               500                 505                 510
   Ala Pro Phe Ser Leu Lys Gly Leu Met Gly Asn Val Ile Cys Ser Pro
           515                 520                 525
   Ala Tyr Trp Lys Pro Ser Thr Phe Gly Gly Glu Val Gly Phe Gln Ile
       530                 535                 540
   Ile Asn Thr Ala Ser Ile Gln Ser Leu Ile Cys Asn Asn Val Lys Gly
   545                 550                 555                 560
   Cys Pro Phe Thr Ser Phe Ser Val Pro Asp Pro Glu Leu Ile Lys Thr
                   565                 570                 575
   Val Thr Ile Asn Ala Ser Ser Ser Arg Ser Gly Leu Asp Asp Ile Asn
               580                 585                 590
   Pro Thr Val Leu Leu Lys Glu Arg Ser Thr Glu Leu
           595                 600
<210> 184
   <211> 673
   <212> PRT
   <213> Homo sapiens
<400> 184
   Met Asn Arg Lys Asp Ser Lys Arg Lys Ser His Gln Glu Cys Thr Gly
    1               5                  10                  15
   Lys Ile Gly Gly Arg Gly Arg Pro Arg Gln Val Arg Arg His Lys Thr
               20                  25                  30
   Cys Pro Ser Pro Arg Glu Ile Ser Lys Val Met Ala Ser Met Asn Leu
           35                  40                  45
   Gly Leu Leu Ser Glu Gly Gly Cys Ser Glu Asp Glu Leu Leu Glu Lys
       50                  55                  60
   Cys Ile Gln Ser Phe Asp Ser Ala Gly Ser Leu Cys His Glu Asp His
   65                  70                  75                  80
   Met Leu Asn Met Val Leu Ala Met His Ser Trp Val Leu Pro Ser Ala
                   85                  90                  95
   Asp Leu Ala Ala Arg Leu Leu Thr Ser Tyr Gln Lys Ala Thr Gly Asp
               100                 105                 110
   Thr Gln Glu Leu Arg Arg Leu Gln Ile Cys His Leu Val Arg Tyr Trp
           115                 120                 125
   Leu Met Arg His Pro Glu Val Met His Gln Asp Pro Gln Leu Glu Glu
       130                 135                 140
   Val Ile Gly Arg Phe Trp Ala Thr Val Ala Arg Glu Gly Asn Ser Ala
   145                 150                 155                 160
   Gln Arg Arg Leu Gly Asp Ser Ser Asp Leu Leu Ser Pro Gly Gly Pro
                   165                 170                 175
   Gly Pro Pro Leu Pro Met Ser Ser Pro Gly Leu Gly Lys Lys Arg Lys
               180                 185                 190
   Val Ser Leu Leu Phe Asp His Leu Glu Thr Gly Glu Leu Ala Gln His
           195                 200                 205
   Leu Thr Tyr Leu Glu Phe Arg Ser Phe Gln Ala Ile Thr Pro Gln Asp
       210                 215                 220
   Leu Arg Ser Tyr Val Leu Gln Gly Ser Val Arg Gly Cys Pro Ala Leu
   225                 230                 235                 240
   Glu Gly Ser Val Gly Leu Ser Asn Ser Val Ser Arg Trp Val Gln Val
                   245                 250                 255
   Met Val Leu Ser Arg Pro Gly Pro Leu Gln Arg Ala Gln Val Leu Asp
               260                 265                 270
   Lys Phe Ile His Val Ala Gln Arg Leu His Gln Leu Gln Asn Phe Asn
           275                 280                 285
   Thr Leu Met Ala Val Thr Gly Gly Leu Cys His Ser Ala Ile Ser Arg
       290                 295                 300
   Leu Lys Asp Ser His Ala His Leu Ser Pro Asp Ser Thr Lys Ala Leu
   305                 310                 315                 320
   Leu Glu Leu Thr Glu Leu Leu Ala Ser His Asn Asn Tyr Ala Arg Tyr
                   325                 330                 335
   Arg Arg Thr Trp Ala Gly Cys Ala Gly Phe Arg Leu Pro Val Leu Gly
               340                 345                 350
   Val His Leu Lys Asp Leu Val Ser Leu His Glu Ala Gln Pro Asp Arg
           355                 360                 365
   Leu Pro Asp Gly Arg Leu His Leu Pro Lys Leu Asn Asn Leu Tyr Leu
       370                 375                 380
   Arg Leu Gln Glu Leu Val Ala Leu Gln Gly Gln His Pro Pro Cys Ser
   385                 390                 395                 400
   Ala Asn Glu Asp Leu Leu His Leu Leu Thr Leu Ser Leu Asp Leu Phe
                   405                 410                 415
   Tyr Thr Glu Asp Glu Ile Tyr Glu Leu Ser Tyr Ala Arg Glu Pro Arg
               420                 425                 430
   Cys Pro Lys Ser Leu Pro Pro Ser Pro Phe Asn Ala Pro Leu Val Val
           435                 440                 445
   Glu Trp Ala Pro Gly Val Thr Pro Lys Pro Asp Arg Val Thr Leu Gly
       450                 455                 460
   Arg His Val Glu Gln Leu Val Glu Ser Val Phe Lys Asn Tyr Asp Pro
   465                 470                 475                 480
   Glu Gly Arg Gly Thr Ile Ser Gln Glu Asp Phe Glu Arg Leu Ser Gly
                   485                 490                 495
   Asn Phe Pro Phe Ala Cys His Gly Leu His Pro Pro Pro Arg Gln Gly
               500                 505                 510
   Arg Gly Ser Phe Ser Arg Glu Glu Leu Thr Gly Tyr Leu Leu Arg Ala
           515                 520                 525
   Ser Ala Ile Cys Ser Lys Leu Gly Leu Ala Phe Leu His Thr Phe His
       530                 535                 540
   Glu Val Thr Phe Arg Lys Pro Thr Phe Cys Asp Ser Cys Ser Gly Phe
   545 550 555 560
   Leu Trp Gly Val Thr Lys Gln Gly Tyr Arg Cys Arg Glu Cys Gly Leu
   565 570 575
   Cys Cys His Lys His Cys Arg Asp Gln Val Lys Val Glu Cys Lys Lys
   580 585 590
   Arg Pro Gly Ala Lys Gly Asp Ala Gly Pro Pro Gly Ala Pro Val Pro
   595 600 605
   Ser Thr Pro Ala Pro His Ala Ser Cys Gly Ser Glu Glu Asn His Ser
   610 615 620
   Tyr Thr Leu Ser Leu Glu Pro Glu Thr Gly Cys Gln Leu Arg His Ala
   625 630 635 640
   Trp Thr Gln Thr Glu Ser Pro His Pro Ser Trp Glu Thr Asp Thr Val
   645 650 655
   Pro Cys Pro Val Met Asp Pro Pro Ser Thr Ala Ser Ser Lys Leu Asp
   660 665 670
   Ser
<210> 185
   <211> 211
   <212> PRT
   <213> Homo sapiens
<400> 185
   Met Gln Ser Ala Met Phe Leu Ala Val Gln His Asp Cys Arg Pro Met
   1 5 10 15
   Asp Lys Ser Ala Gly Ser Gly His Lys Ser Glu Glu Lys Arg Glu Lys
   20 25 30
   Met Lys Arg Thr Leu Leu Lys Asp Trp Lys Thr Arg Leu Ser Tyr Phe
   35 40 45
   Leu Gln Asn Ser Ser Thr Pro Gly Lys Pro Lys Thr Gly Lys Lys Ser
   50 55 60
   Lys Gln Gln Ala Phe Ile Lys Pro Ser Pro Glu Glu Ala Gln Leu Trp
   65 70 75 80
   Ser Glu Ala Phe Asp Glu Leu Leu Ala Ser Lys Tyr Gly Leu Ala Ala
   85 90 95
   Phe Arg Ala Phe Leu Lys Ser Glu Phe Cys Glu Glu Asn Ile Glu Phe
   100 105 110
   Trp Leu Ala Cys Glu Asp Phe Lys Lys Thr Lys Ser Pro Gln Lys Leu
   115 120 125
   Ser Ser Lys Ala Arg Lys Ile Tyr Thr Asp Phe Ile Glu Lys Glu Ala
   130 135 140
   Pro Lys Glu Ile Asn Ile Asp Phe Gln Thr Lys Thr Leu Ile Ala Gln
   145 150 155 160
   Asn Ile Gln Glu Ala Thr Ser Gly Cys Phe Thr Thr Ala Gln Lys Arg
   165 170 175
   Val Tyr Ser Leu Met Glu Asn Asn Ser Tyr Pro Arg Phe Leu Glu Ser
   180 185 190
   Glu Phe Tyr Gln Asp Leu Cys Lys Lys Pro Gln Ile Thr Thr Glu Pro
   195 200 205
   His Ala Thr
   210
<210> 186
   <211> 551
   <212> PRT
   <213> Homo sapiens
<400> 186
   Met Arg Asp Tyr Asp Glu Val Ile Ala Phe Leu Gly Glu Trp Gly Pro
    1               5                  10                  15
   Phe Gln Arg Leu Ile Phe Phe Leu Leu Ser Ala Ser Ile Ile Pro Asn
               20                  25                  30
   Gly Phe Asn Gly Met Ser Val Val Phe Leu Ala Gly Thr Pro Glu His
           35                  40                  45
   Arg Cys Arg Val Pro Asp Ala Ala Asn Leu Ser Ser Ala Trp Arg Asn
       50                  55                  60
   Asn Ser Val Pro Leu Arg Leu Arg Asp Gly Arg Glu Val Pro His Ser
   65                  70                  75                  80
   Cys Ser Arg Tyr Arg Leu Ala Thr Ile Ala Asn Phe Ser Ala Leu Gly
                   85                  90                  95
   Leu Glu Pro Gly Arg Asp Val Asp Leu Gly Gln Leu Glu Gln Glu Ser
               100                 105                 110
   Cys Leu Asp Gly Trp Glu Phe Ser Gln Asp Val Tyr Leu Ser Thr Val
           115                 120                 125
   Val Thr Glu Trp Asn Leu Val Cys Glu Asp Asn Trp Lys Val Pro Leu
       130                 135                 140
   Thr Thr Ser Leu Phe Phe Val Gly Val Leu Leu Gly Ser Phe Val Ser
   145                 150                 155                 160
   Gly Gln Leu Ser Asp Arg Phe Gly Arg Lys Asn Val Leu Phe Ala Thr
                   165                 170                 175
   Met Ala Val Gln Thr Gly Phe Ser Phe Leu Gln Ile Phe Ser Ile Ser
               180                 185                 190
   Trp Glu Met Phe Thr Val Leu Phe Val Ile Val Gly Met Gly Gln Ile
           195                 200                 205
   Ser Asn Tyr Val Val Ala Phe Ile Leu Gly Thr Glu Ile Leu Gly Lys
       210                 215                 220
   Ser Val Arg Ile Ile Phe Ser Thr Leu Gly Val Cys Thr Phe Phe Ala
   225                 230                 235                 240
   Val Gly Tyr Met Leu Leu Pro Leu Phe Ala Tyr Phe Ile Arg Asp Trp
                   245                 250                 255
   Arg Met Leu Leu Leu Ala Leu Thr Val Pro Gly Val Leu Cys Val Pro
               260                 265                 270
   Leu Trp Trp Phe Ile Pro Glu Ser Pro Arg Trp Leu Ile Ser Gln Arg
           275                 280                 285
   Arg Phe Arg Glu Ala Glu Asp Ile Ile Gln Lys Ala Ala Lys Met Asn
       290                 295                 300
   Asn Ile Ala Val Pro Ala Val Ile Phe Asp Ser Val Glu Glu Leu Asn
   305                 310                 315                 320
   Pro Leu Lys Gln Gln Lys Ala Phe Ile Leu Asp Leu Phe Arg Thr Arg
                   325                 330                 335
   Asn Ile Ala Ile Met Thr Ile Met Ser Leu Leu Leu Trp Met Leu Thr
               340                 345                 350
   Ser Val Gly Tyr Phe Ala Leu Ser Leu Asp Ala Pro Asn Leu His Gly
           355                 360                 365
   Asp Ala Tyr Leu Asn Cys Phe Leu Ser Ala Leu Ile Glu Ile Pro Ala
       370                 375                 380
   Tyr Ile Thr Ala Trp Leu Leu Leu Arg Thr Leu Pro Arg Arg Tyr Ile
   385                 390                 395                 400
   Ile Ala Ala Val Leu Phe Trp Gly Gly Gly Val Leu Leu Phe Ile Gln
                   405                 410                 415
   Leu Val Pro Val Asp Tyr Tyr Phe Leu Ser Ile Gly Leu Val Met Leu
               420                 425                 430
   Gly Lys Phe Gly Ile Thr Ser Ala Phe Ser Met Leu Tyr Val Phe Thr
           435                 440                 445
   Ala Glu Leu Tyr Pro Thr Leu Val Arg Asn Met Ala Val Gly Val Thr
       450                 455                 460
   Ser Thr Ala Ser Arg Val Gly Ser Ile Ile Ala Pro Tyr Phe Val Tyr
   465                 470                 475                 480
   Leu Gly Ala Tyr Asn Arg Met Leu Pro Tyr Ile Val Met Gly Ser Leu
                   485                 490                 495
   Thr Val Leu Ile Gly Ile Leu Thr Leu Phe Phe Pro Glu Ser Leu Gly
               500                 505                 510
   Met Thr Leu Pro Glu Thr Leu Glu Gln Met Gln Lys Val Lys Trp Phe
           515                 520                 525
   Arg Ser Gly Lys Lys Thr Arg Asp Ser Met Glu Thr Glu Glu Asn Pro
       530                 535                 540
   Lys Val Leu Ile Thr Ala Phe
   545                 550
<210> 187
   <211> 112
   <212> PRT
   <213> Homo sapiens
<400> 187
   Met Asp Val Phe Met Lys Gly Leu Ser Lys Ala Lys Glu Gly Val Val
    1               5                  10                  15
   Ala Ala Ala Glu Lys Thr Lys Gln Gly Val Ala Glu Ala Ala Gly Lys
               20                  25                  30
   Thr Lys Glu Gly Val Leu Tyr Val Gly Ser Lys Thr Lys Glu Gly Val
           35                  40                  45
   Val His Gly Val Ala Thr Val Ala Glu Lys Thr Lys Glu Gln Val Thr
       50                  55                  60
   Asn Val Gly Gly Ala Val Val Thr Gly Val Thr Ala Val Ala Gln Lys
   65                  70                  75                  80
   Thr Val Glu Gly Ala Gly Ser Ile Ala Ala Ala Thr Gly Phe Val Lys
                   85                  90                  95
   Lys Asp Gln Leu Gly Lys Glu Gly Tyr Gln Asp Tyr Glu Pro Glu Ala
               100                 105                 110
<210> 188
   <211> 222
   <212> PRT
   <213> Homo sapiens
<400> 188
   Met Leu Ser Arg Ala Val Cys Gly Thr Ser Arg Gln Leu Ala Pro Val
    1              5                   10                  15
   Leu Gly Tyr Leu Gly Ser Arg Gln Lys His Ser Leu Pro Asp Leu Pro
               20                  25                  30
   Tyr Asp Tyr Gly Ala Leu Glu Pro His Ile Asn Ala Gln Ile Met Gln
           35                  40                  45
   Leu His His Ser Lys His His Ala Ala Tyr Val Asn Asn Leu Asn Val
       50                  55                  60
   Thr Glu Glu Lys Tyr Gln Glu Ala Leu Ala Lys Gly Asp Val Thr Ala
   65                  70                  75                  80
   Gln Ile Ala Leu Gln Pro Ala Leu Lys Phe Asn Gly Gly Gly His Ile
                   85                  90                  95
   Asn His Ser Ile Phe Trp Thr Asn Leu Ser Pro Asn Gly Gly Gly Glu
               100                 105                 110
   Pro Lys Gly Glu Leu Leu Glu Ala Ile Lys Arg Asp Phe Gly Ser Phe
           115                 120                 125
   Asp Lys Phe Lys Glu Lys Leu Thr Ala Ala Ser Val Gly Val Gln Gly
       130                 135                 140
   Ser Gly Trp Gly Trp Leu Gly Phe Asn Lys Glu Arg Gly His Leu Gln
   145                 150                 155                 160
   Ile Ala Ala Cys Pro Asn Gln Asp Pro Leu Gln Gly Thr Thr Gly Leu
                   165                 170                 175
   Ile Pro Leu Leu Gly Ile Asp Val Trp Glu His Ala Tyr Tyr Leu Gln
               180                 185                 190
   Tyr Lys Asn Val Arg Pro Asp Tyr Leu Lys Ala Ile Trp Asn Val Ile
           195                 200                 205
   Asn Trp Glu Asn Val Thr Glu Arg Tyr Met Ala Cys Lys Lys
       210                 215                 220
<210> 189
   <211> 247
   <212> PRT
   <213> Homo sapiens
<400> 189
   Met Leu Gln Asn Ser Ala Val Leu Leu Val Leu Val Ile Ser Ala Ser
    1               5                  10                  15
   Ala Thr His Glu Ala Glu Gln Asn Asp Ser Val Ser Pro Arg Lys Ser
               20                  25                  30
   Arg Val Ala Ala Gln Asn Ser Ala Glu Val Val Arg Cys Leu Asn Ser
           35                  40                  45
   Ala Leu Gln Val Gly Cys Gly Ala Phe Ala Cys Leu Glu Asn Ser Thr
       50                  55                  60
   Cys Asp Thr Asp Gly Met Tyr Asp Ile Cys Lys Ser Phe Leu Tyr Ser
   65                  70                  75                  80
   Ala Ala Lys Phe Asp Thr Gln Gly Lys Ala Phe Val Lys Glu Ser Leu
                   85                  90                  95
   Lys Cys Ile Ala Asn Gly Val Thr Ser Lys Val Phe Leu Ala Ile Arg
               100                 105                 110
   Arg Cys Ser Thr Phe Gln Arg Met Ile Ala Glu Val Gln Glu Glu Cys
           115                 120                 125
   Tyr Ser Lys Leu Asn Val Cys Ser Ile Ala Lys Arg Asn Pro Glu Ala
       130                 135                 140
   Ile Thr Glu Val Val Gln Leu Pro Asn His Phe Ser Asn Arg Tyr Tyr
   145                 150                 155                 160
   Asn Arg Leu Val Arg Ser Leu Leu Glu Cys Asp Glu Asp Thr Val Ser
                   165                 170                 175
   Thr Ile Arg Asp Ser Leu Met Glu Lys Ile Gly Pro Asn Met Ala Ser
               180                 185                 190
   Leu Phe His Ile Leu Gln Thr Asp His Cys Ala Gln Thr His Pro Arg
           195                 200                 205
   Ala Asp Phe Asn Arg Arg Arg Thr Asn Glu Pro Gln Lys Leu Lys Val
       210                 215                 220
   Leu Leu Arg Asn Leu Arg Gly Glu Glu Asp Ser Pro Ser His Ile Lys
   225                 230                 235                 240
   Arg Thr Ser His Glu Ser Ala
                   245
<210> 190
   <211> 247
   <212> PRT
   <213> Homo sapiens
<400> 190
   Met Leu Gln Asn Ser Ala Val Leu Leu Val Leu Val Ile Ser Ala Ser
    1               5                  10                  15
   Ala Thr His Glu Ala Glu Gln Asn Asp Ser Val Ser Pro Arg Lys Ser
                   20                  25                  30
   Arg Val Ala Ala Gln Asn Ser Ala Glu Val Val Arg Cys Leu Asn Ser
               35                  40                  45
   Ala Leu Gln Val Gly Cys Gly Ala Phe Ala Cys Leu Glu Asn Ser Thr
       50                  55                  60
   Cys Asp Thr Asp Gly Met Tyr Asp Ile Cys Lys Ser Phe Leu Tyr Ser
   65                  70                  75                  80
   Ala Ala Lys Phe Asp Thr Gln Gly Lys Ala Phe Val Lys Glu Ser Leu
                   85                  90                  95
   Lys Cys Ile Ala Asn Gly Val Thr Ser Lys Val Phe Leu Ala Ile Arg
               100                 105                 110
   Arg Cys Ser Thr Phe Gln Arg Met Ile Ala Glu Val Gln Glu Glu Cys
           115                 120                 125
   Tyr Ser Lys Leu Asn Val Cys Ser Ile Ala Lys Arg Asn Pro Glu Ala
       130                 135                 140
   Ile Thr Glu Val Val Gln Leu Pro Asn His Phe Ser Asn Arg Tyr Tyr
   145                 150                 155                 160
   Asn Arg Leu Val Arg Ser Leu Leu Glu Cys Asp Glu Asp Thr Val Ser
                   165                 170                 175
   Thr Ile Arg Asp Ser Leu Met Glu Lys Ile Gly Pro Asn Met Ala Ser
               180                 185                 190
   Leu Phe His Ile Leu Gln Thr Asp His Cys Ala Gln Thr His Pro Arg
           195                 200                 205
   Ala Asp Phe Asn Arg Arg Arg Thr Asn Glu Pro Gln Lys Leu Lys Val
       210                 215                 220
   Leu Leu Arg Asn Leu Arg Gly Glu Glu Asp Ser Pro Ser His Ile Lys
   225                 230                 235                 240
   Arg Thr Ser His Glu Ser Ala
                   245
<210> 191
   <211> 266
   <212> PRT
   <213> Homo sapiens
<400> 191
   Met Lys Leu Arg Ser Ser His Asn Ala Ser Lys Thr Leu Asn Ala Asn
   1               5                   10                  15
   Asn Met Glu Thr Leu Ile Glu Cys Gln Ser Glu Gly Asp Ile Lys Glu
               20                  25                  30
   His Pro Leu Leu Ala Ser Cys Glu Ser Glu Asp Ser Ile Cys Gln Leu
           35                  40                  45
   Ile Glu Val Lys Lys Arg Lys Lys Val Leu Ser Trp Pro Phe Leu Met
       50                  55                  60
   Arg Arg Leu Ser Pro Ala Ser Asp Phe Ser Gly Ala Leu Glu Thr Asp
   65                  70                  75                  80
   Leu Lys Ala Ser Leu Phe Asp Gln Pro Leu Ser Ile Ile Cys Gly Asp
                   85                  90                  95
   Ser Asp Thr Leu Pro Arg Pro Ile Gln Asp Ile Leu Thr Ile Leu Cys
               100                 105                 110
   Leu Lys Gly Pro Ser Thr Glu Gly Ile Phe Arg Arg Ala Ala Asn Glu
           115                 120                 125
   Lys Ala Arg Lys Glu Leu Lys Glu Glu Leu Asn Ser Gly Asp Ala Val
       130                 135                 140
   Asp Leu Glu Arg Leu Pro Val His Leu Leu Ala Val Val Phe Lys Asp
   145                 150                 155                 160
   Phe Leu Arg Ser Ile Pro Arg Lys Leu Leu Ser Ser Asp Leu Phe Glu
                   165                 170                 175
   Glu Trp Met Gly Ala Leu Glu Met Gln Asp Glu Glu Asp Arg Ile Glu
               180                 185                 190
   Ala Leu Lys Gln Val Ala Asp Lys Leu Pro Arg Pro Asn Leu Leu Leu
           195                 200                 205
   Leu Lys His Leu Val Tyr Val Leu His Leu Ile Ser Lys Asn Ser Glu
       210                 215                 220
   Val Asn Arg Met Asp Ser Ser Asn Leu Ala Ile Cys Ile Gly Pro Asn
   225                 230                 235                 240
   Met Leu Thr Leu Glu Asn Asp Gln Ser Leu Ser Phe Glu Ala Gln Lys
                   245                 250                 255
   Asp Leu Asn Asn Lys Val Cys Ser Ala Tyr
               260                 265
<210> 192
   <211> 304
   <212> PRT
   <213> Homo sapiens
<400> 192
   Met Ile Tyr Thr Met Lys Lys Val His Ala Leu Trp Ala Ser Val Cys
    1               5                  10                  15
   Leu Leu Leu Asn Leu Ala Pro Ala Pro Leu Asn Ala Asp Ser Glu Glu
               20                  25                  30
   Asp Glu Glu His Thr Ile Ile Thr Asp Thr Glu Leu Pro Pro Leu Lys
           35                  40                  45
   Leu Met His Ser Phe Cys Ala Phe Lys Ala Asp Asp Gly Pro Cys Lys
       50                  55                  60
   Ala Ile Met Lys Arg Phe Phe Phe Asn Ile Phe Thr Arg Gln Cys Glu
   65                  70                  75                  80
   Glu Phe Ile Tyr Gly Gly Cys Glu Gly Asn Gln Asn Arg Phe Glu Ser
                   85                  90                  95
   Leu Glu Glu Cys Lys Lys Met Cys Thr Arg Asp Asn Ala Asn Arg Ile
               100                 105                 110
   Ile Lys Thr Thr Leu Gln Gln Glu Lys Pro Asp Phe Cys Phe Leu Glu
           115                 120                 125
   Glu Asp Pro Gly Ile Cys Arg Gly Tyr Ile Thr Arg Tyr Phe Tyr Asn
       130                 135                 140
   Asn Gln Thr Lys Gln Cys Glu Arg Phe Lys Tyr Gly Gly Cys Leu Gly
   145                 150                 155                 160
   Asn Met Asn Asn Phe Glu Thr Leu Glu Glu Cys Lys Asn Ile Cys Glu
                   165                 170                 175
   Asp Gly Pro Asn Gly Phe Gln Val Asp Asn Tyr Gly Thr Gln Leu Asn
               180                 185                 190
   Ala Val Asn Asn Ser Leu Thr Pro Gln Ser Thr Lys Val Pro Ser Leu
           195                 200                 205
   Phe Glu Phe His Gly Pro Ser Trp Cys Leu Thr Pro Ala Asp Arg Gly
       210                 215                 220
   Leu Cys Arg Ala Asn Glu Asn Arg Phe Tyr Tyr Asn Ser Val Ile Gly
   225                 230                 235                 240
   Lys Cys Arg Pro Phe Lys Tyr Ser Gly Cys Gly Gly Asn Glu Asn Asn
                   245                 250                 255
   Phe Thr Ser Lys Gln Glu Cys Leu Arg Ala Cys Lys Lys Gly Phe Ile
               260                 265                 270
   Gln Arg Ile Ser Lys Gly Gly Leu Ile Lys Thr Lys Arg Lys Arg Lys
           275                 280                 285
   Lys Gln Arg Val Lys Ile Ala Tyr Glu Glu Ile Phe Val Lys Asn Met
       290                 295                 300
<210> 193
   <211> 784
   <212> PRT
   <213> Homo sapiens
<400> 193
   Met Pro His Thr Leu Trp Met Val Trp Val Leu Gly Val Ile Ile Ser
    1               5                  10                  15
   Leu Ser Lys Glu Glu Ser Ser Asn Gln Ala Ser Leu Ser Cys Asp Arg
               20                  25                  30
   Asn Gly Ile Cys Lys Gly Ser Ser Gly Ser Leu Asn Ser Ile Pro Ser
           35                  40                  45
   Gly Leu Thr Glu Ala Val Lys Ser Leu Asp Leu Ser Asn Asn Arg Ile
       50                  55                  60
   Thr Tyr Ile Ser Asn Ser Asp Leu Gln Arg Cys Val Asn Leu Gln Ala
   65                  70                  75                  80
   Leu Val Leu Thr Ser Asn Gly Ile Asn Thr Ile Glu Glu Asp Ser Phe
                   85                  90                  95
   Ser Ser Leu Gly Ser Leu Glu His Leu Asp Leu Ser Tyr Asn Tyr Leu
               100                 105                 110
   Ser Asn Leu Ser Ser Ser Trp Phe Lys Pro Leu Ser Ser Leu Thr Phe
           115                 120                 125
   Leu Asn Leu Leu Gly Asn Pro Tyr Lys Thr Leu Gly Glu Thr Ser Leu
       130                 135                 140
   Phe Ser His Leu Thr Lys Leu Gln Ile Leu Arg Val Gly Asn Met Asp
   145                 150                 155                 160
   Thr Phe Thr Lys Ile Gln Arg Lys Asp Phe Ala Gly Leu Thr Phe Leu
                   165                 170                 175
   Glu Glu Leu Glu Ile Asp Ala Ser Asp Leu Gln Ser Tyr Glu Pro Lys
               180                 185                 190
   Ser Leu Lys Ser Ile Gln Asn Val Ser His Leu Ile Leu His Met Lys
           195                 200                 205
   Gln His Ile Leu Leu Leu Glu Ile Phe Val Asp Val Thr Ser Ser Val
       210                 215                 220
   Glu Cys Leu Glu Leu Arg Asp Thr Asp Leu Asp Thr Phe His Phe Ser
   225                 230                 235                 240
   Glu Leu Ser Thr Gly Glu Thr Asn Ser Leu Ile Lys Lys Phe Thr Phe
                   245                 250                 255
   Arg Asn Val Lys Ile Thr Asp Glu Ser Leu Phe Gln Val Met Lys Leu
               260                 265                 270
   Leu Asn Gln Ile Ser Gly Leu Leu Glu Leu Glu Phe Asp Asp Cys Thr
           275                 280                 285
   Leu Asn Gly Val Gly Asn Phe Arg Ala Ser Asp Asn Asp Arg Val Ile
       290                 295                 300
   Asp Pro Gly Lys Val Glu Thr Leu Thr Ile Arg Arg Leu His Ile Pro
   305                 310                 315                 320
   Arg Phe Tyr Leu Phe Tyr Asp Leu Ser Thr Leu Tyr Ser Leu Thr Glu
                   325                 330                 335
   Arg Val Lys Arg Ile Thr Val Glu Asn Ser Lys Val Phe Leu Val Pro
               340                 345                 350
   Cys Leu Leu Ser Gln His Leu Lys Ser Leu Glu Tyr Leu Asp Leu Ser
           355                 360                 365
   Glu Asn Leu Met Val Glu Glu Tyr Leu Lys Asn Ser Ala Cys Glu Asp
       370                 375                 380
   Ala Trp Pro Ser Leu Gln Thr Leu Ile Leu Arg Gln Asn His Leu Ala
   385                 390                 395                 400
   Ser Leu Glu Lys Thr Gly Glu Thr Leu Leu Thr Leu Lys Asn Leu Thr
                   405                 410                 415
   Asn Ile Asp Ile Ser Lys Asn Ser Phe His Ser Met Pro Glu Thr Cys
               420                 425                 430
   Gln Trp Pro Glu Lys Met Lys Tyr Leu Asn Leu Ser Ser Thr Arg Ile
           435                 440                 445
   His Ser Val Thr Gly Cys Ile Pro Lys Thr Leu Glu Ile Leu Asp Val
       450                 455                 460
   Ser Asn Asn Asn Leu Asn Leu Phe Ser Leu Asn Leu Pro Gln Leu Lys
   465                 470                 475                 480
   Glu Leu Tyr Ile Ser Arg Asn Lys Leu Met Thr Leu Pro Asp Ala Ser
                   485                 490                 495
   Leu Leu Pro Met Leu Leu Val Leu Lys Ile Ser Arg Asn Ala Ile Thr
               500                 505                 510
   Thr Phe Ser Lys Glu Gln Leu Asp Ser Phe His Thr Leu Lys Thr Leu
           515                 520                 525
   Glu Ala Gly Gly Asn Asn Phe Ile Cys Ser Cys Glu Phe Leu Ser Phe
       530                 535                 540
   Thr Gln Glu Gln Gln Ala Leu Ala Lys Val Leu Ile Asp Trp Pro Ala
   545                 550                 555                 560
   Asn Tyr Leu Cys Asp Ser Pro Ser His Val Arg Gly Gln Gln Val Gln
                   565                 570                 575
   Asp Val Arg Leu Ser Val Ser Glu Cys His Arg Thr Ala Leu Val Ser
               580                 585                 590
   Gly Met Cys Cys Ala Leu Phe Leu Leu Ile Leu Leu Thr Gly Val Leu
           595                 600                 605
   Cys His Arg Phe His Gly Leu Trp Tyr Met Lys Met Met Trp Ala Trp
       610                 615                 620
   Leu Gln Ala Lys Arg Lys Pro Arg Lys Ala Pro Ser Arg Asn Ile Cys
   625                 630                 635                 640
   Tyr Asp Ala Phe Val Ser Tyr Ser Glu Arg Asp Ala Tyr Trp Val Glu
                   645                 650                 655
   Asn Leu Met Val Gln Glu Leu Glu Asn Phe Asn Pro Pro Phe Lys Leu
               660                 665                 670
   Cys Leu His Lys Arg Asp Phe Ile Pro Gly Lys Trp Ile Ile Asp Asn
           675                 680                 685
   Ile Ile Asp Ser Ile Glu Lys Ser His Lys Thr Val Phe Val Leu Ser
       690                 695                 700
   Glu Asn Phe Val Lys Ser Glu Trp Cys Lys Tyr Glu Leu Asp Phe Ser
   705                 710                 715                 720
   His Phe Arg Leu Phe Asp Glu Asn Asn Asp Ala Ala Ile Leu Ile Leu
                   725                 730                 735
   Leu Glu Pro Ile Glu Lys Lys Ala Ile Pro Gln Arg Phe Cys Lys Leu
               740                 745                 750
   Arg Lys Ile Met Asn Thr Lys Thr Tyr Leu Glu Trp Pro Met Asp Glu
           755                 760                 765
   Ala Gln Arg Glu Gly Phe Trp Val Asn Leu Arg Ala Ala Ile Lys Ser
       770                 775                 780
<210> 194
   <211> 839
   <212> PRT
   <213> Homo sapiens
<400> 194
   Met Met Ser Ala Ser Arg Leu Ala Gly Thr Leu Ile Pro Ala Met Ala
    1               5                  10                  15
   Phe Leu Ser Cys Val Arg Pro Glu Ser Trp Glu Pro Cys Val Glu Val
               20                  25                  30
   Val Pro Asn Ile Thr Tyr Gln Cys Met Glu Leu Asn Phe Tyr Lys Ile
           35                  40                  45
   Pro Asp Asn Leu Pro Phe Ser Thr Lys Asn Leu Asp Leu Ser Phe Asn
       50                  55                  60
   Pro Leu Arg His Leu Gly Ser Tyr Ser Phe Phe Ser Phe Pro Glu Leu
   65                  70                  75                  80
   Gln Val Leu Asp Leu Ser Arg Cys Glu Ile Gln Thr Ile Glu Asp Gly
                   85                  90                  95
   Ala Tyr Gln Ser Leu Ser His Leu Ser Thr Leu Ile Leu Thr Gly Asn
               100                 105                 110
   Pro Ile Gln Ser Leu Ala Leu Gly Ala Phe Ser Gly Leu Ser Ser Leu
           115                 120                 125
   Gln Lys Leu Val Ala Val Glu Thr Asn Leu Ala Ser Leu Glu Asn Phe
       130                 135                 140
   Pro Ile Gly His Leu Lys Thr Leu Lys Glu Leu Asn Val Ala His Asn
   145                 150                 155                 160
   Leu Ile Gln Ser Phe Lys Leu Pro Glu Tyr Phe Ser Asn Leu Thr Asn
                   165                 170                 175
   Leu Glu His Leu Asp Leu Ser Ser Asn Lys Ile Gln Ser Ile Tyr Cys
               180                 185                 190
   Thr Asp Leu Arg Val Leu His Gln Met Pro Leu Leu Asn Leu Ser Leu
           195                 200                 205
   Asp Leu Ser Leu Asn Pro Met Asn Phe Ile Gln Pro Gly Ala Phe Lys
       210                 215                 220
   Glu Ile Arg Leu His Lys Leu Thr Leu Arg Asn Asn Phe Asp Ser Leu
   225                 230                 235                 240
   Asn Val Met Lys Thr Cys Ile Gln Gly Leu Ala Gly Leu Glu Val His
                   245                 250                 255
   Arg Leu Val Leu Gly Glu Phe Arg Asn Glu Gly Asn Leu Glu Lys Phe
               260                 265                 270
   Asp Lys Ser Ala Leu Glu Gly Leu Cys Asn Leu Thr Ile Glu Glu Phe
           275                 280                 285
   Arg Leu Ala Tyr Leu Asp Tyr Tyr Leu Asp Asp Ile Ile Asp Leu Phe
       290                 295                 300
   Asn Cys Leu Thr Asn Val Ser Ser Phe Ser Leu Val Ser Val Thr Ile
   305                 310                 315                 320
   Glu Arg Val Lys Asp Phe Ser Tyr Asn Phe Gly Trp Gln His Leu Glu
                   325                 330                 335
   Leu Val Asn Cys Lys Phe Gly Gln Phe Pro Thr Leu Lys Leu Lys Ser
               340                 345                 350
   Leu Lys Arg Leu Thr Phe Thr Ser Asn Lys Gly Gly Asn Ala Phe Ser
           355                 360                 365
   Glu Val Asp Leu Pro Ser Leu Glu Phe Leu Asp Leu Ser Arg Asn Gly
       370                 375                 380
   Leu Ser Phe Lys Gly Cys Cys Ser Gln Ser Asp Phe Gly Thr Thr Ser
   385                 390                 395                 400
   Leu Lys Tyr Leu Asp Leu Ser Phe Asn Gly Val Ile Thr Met Ser Ser
                   405                 410                 415
   Asn Phe Leu Gly Leu Glu Gln Leu Glu His Leu Asp Phe Gln His Ser
               420                 425                 430
   Asn Leu Lys Gln Met Ser Glu Phe Ser Val Phe Leu Ser Leu Arg Asn
           435                 440                 445
   Leu Ile Tyr Leu Asp Ile Ser His Thr His Thr Arg Val Ala Phe Asn
       450                 455                 460
   Gly Ile Phe Asn Gly Leu Ser Ser Leu Glu Val Leu Lys Met Ala Gly
   465                 470                 475                 480
   Asn Ser Phe Gln Glu Asn Phe Leu Pro Asp Ile Phe Thr Glu Leu Arg
                   485                 490                 495
   Asn Leu Thr Phe Leu Asp Leu Ser Gln Cys Gln Leu Glu Gln Leu Ser
               500                 505                 510
   Pro Thr Ala Phe Asn Ser Leu Ser Ser Leu Gln Val Leu Asn Met Ser
           515                 520                 525
   His Asn Asn Phe Phe Ser Leu Asp Thr Phe Pro Tyr Lys Cys Leu Asn
       530                 535                 540
   Ser Leu Gln Val Leu Asp Tyr Ser Leu Asn His Ile Met Thr Ser Lys
   545                 550                 555                 560
   Lys Gln Glu Leu Gln His Phe Pro Ser Ser Leu Ala Phe Leu Asn Leu
                   565                 570                 575
   Thr Gln Asn Asp Phe Ala Cys Thr Cys Glu His Gln Ser Phe Leu Gln
               580                 585                 590
   Trp Ile Lys Asp Gln Arg Gln Leu Leu Val Glu Val Glu Arg Met Glu
           595                 600                 605
   Cys Ala Thr Pro Ser Asp Lys Gln Gly Met Pro Val Leu Ser Leu Asn
       610                 615                 620
   Ile Thr Cys Gln Met Asn Lys Thr Ile Ile Gly Val Ser Val Leu Ser
   625                 630                 635                 640
   Val Leu Val Val Ser Val Val Ala Val Leu Val Tyr Lys Phe Tyr Phe
                   645                 650                 655
   His Leu Met Leu Leu Ala Gly Cys Ile Lys Tyr Gly Arg Gly Glu Asn
               660                 665                 670
   Ile Tyr Asp Ala Phe Val Ile Tyr Ser Ser Gln Asp Glu Asp Trp Val
           675                 680                 685
   Arg Asn Glu Leu Val Lys Asn Leu Glu Glu Gly Val Pro Pro Phe Gln
       690                 695                 700
   Leu Cys Leu His Tyr Arg Asp Phe Ile Pro Gly Val Ala Ile Ala Ala
   705                 710                 715                 720
   Asn Ile Ile His Glu Gly Phe His Lys Ser Arg Lys Val Ile Val Val
                   725                 730                 735
   Val Ser Gln His Phe Ile Gln Ser Arg Trp Cys Ile Phe Glu Tyr Glu
               740                 745                 750
   Ile Ala Gln Thr Trp Gln Phe Leu Ser Ser Arg Ala Gly Ile Ile Phe
           755                 760                 765
   Ile Val Leu Gln Lys Val Glu Lys Thr Leu Leu Arg Gln Gln Val Glu
       770                 775                 780
   Leu Tyr Arg Leu Leu Ser Arg Asn Thr Tyr Leu Glu Trp Glu Asp Ser
   785                 790                 795                 800
   Val Leu Gly Arg His Ile Phe Trp Arg Arg Leu Arg Lys Ala Leu Leu
                   805                 810                 815
   Asp Gly Lys Ser Trp Asn Pro Glu Gly Thr Val Gly Thr Gly Cys Asn
               820                 825                 830
   Trp Gln Glu Ala Thr Ser Ile
           835
<210> 195
   <211> 277
   <212> PRT
   <213> Homo sapiens
<400> 195
   Met Ile Ile Leu Ile Tyr Leu Phe Leu Leu Leu Trp Glu Asp Thr Gln
    1               5                  10                  15
   Gly Trp Gly Phe Lys Asp Gly Ile Phe His Asn Ser Ile Trp Leu Glu
               20                  25                  30
   Arg Ala Ala Gly Val Tyr His Arg Glu Ala Arg Ser Gly Lys Tyr Lys
           35                  40                  45
   Leu Thr Tyr Ala Glu Ala Lys Ala Val Cys Glu Phe Glu Gly Gly His
       50                  55                  60
   Leu Ala Thr Tyr Lys Gln Leu Glu Ala Ala Arg Lys Ile Gly Phe His
   65                  70                  75                  80
   Val Cys Ala Ala Gly Trp Met Ala Lys Gly Arg Val Gly Tyr Pro Ile
                   85                  90                  95
   Val Lys Pro Gly Pro Asn Cys Gly Phe Gly Lys Thr Gly Ile Ile Asp
               100                 105                 110
   Tyr Gly Ile Arg Leu Asn Arg Ser Glu Arg Trp Asp Ala Tyr Cys Tyr
           115                 120                 125
   Asn Pro His Ala Lys Glu Cys Gly Gly Val Phe Thr Asp Pro Lys Gln
       130                 135                 140
   Ile Phe Lys Ser Pro Gly Phe Pro Asn Glu Tyr Glu Asp Asn Gln Ile
   145                 150                 155                 160
   Cys Tyr Trp His Ile Arg Leu Lys Tyr Gly Gln Arg Ile His Leu Ser
                   165                 170                 175
   Phe Leu Asp Phe Asp Leu Glu Asp Asp Pro Gly Cys Leu Ala Asp Tyr
               180                 185                 190
   Val Glu Ile Tyr Asp Ser Tyr Asp Asp Val His Gly Phe Val Gly Arg
           195                 200                 205
   Tyr Cys Gly Asp Glu Leu Pro Asp Asp Ile Ile Ser Thr Gly Asn Val
       210                 215                 220
   Met Thr Leu Lys Phe Leu Ser Asp Ala Ser Val Thr Ala Gly Gly Phe
   225                 230                 235                 240
   Gln Ile Lys Tyr Val Ala Met Asp Pro Val Ser Lys Ser Ser Gln Gly
                   245                 250                 255
   Lys Asn Thr Ser Thr Thr Ser Thr Gly Asn Lys Asn Phe Leu Ala Gly
               260                 265                 270
   Arg Phe Ser His Leu
           275
<210> 196
   <211> 218
   <212> PRT
   <213> Homo sapiens
<400> 196
   Met Ala Arg Ile Pro Lys Thr Leu Lys Phe Val Val Val Ile Val Ala
   1               5                   10                  15
   Val Leu Leu Pro Val Leu Ala Tyr Ser Ala Thr Thr Ala Arg Gln Glu
               20                  25                  30
   Glu Val Pro Gln Gln Thr Val Ala Pro Gln Gln Gln Arg His Ser Phe
           35                  40                  45
   Lys Gly Glu Glu Cys Pro Ala Gly Ser His Arg Ser Glu His Thr Gly
       50                  55                  60
   Ala Cys Asn Pro Cys Thr Glu Gly Val Asp Tyr Thr Asn Ala Ser Asn
   65                  70                  75                  80
   Asn Glu Pro Ser Cys Phe Pro Cys Thr Val Cys Lys Ser Asp Gln Lys
                   85                  90                  95
   His Lys Ser Ser Cys Thr Met Thr Arg Asp Thr Val Cys Gln Cys Lys
               100                 105                 110
   Glu Gly Thr Phe Arg Asn Glu Asn Ser Pro Glu Met Cys Arg Lys Cys
           115                 120                 125
   Ser Arg Cys Pro Ser Gly Glu Val Gln Val Ser Asn Cys Thr Ser Trp
       130                 135                 140
   Asp Asp Ile Gln Cys Val Glu Glu Phe Gly Ala Asn Ala Thr Val Glu
   145                 150                 155                 160
   Thr Pro Ala Ala Glu Glu Thr Met Asn Thr Ser Pro Gly Thr Pro Ala
                   165                 170                 175
   Pro Ala Ala Glu Glu Thr Met Asn Thr Ser Pro Gly Thr Pro Ala Pro
               180                 185                 190
   Ala Ala Glu Glu Thr Met Thr Thr Ser Pro Gly Thr Pro Ala Pro Ala
           195                 200                 205
   Ala Glu Glu Thr Met Thr Thr Ser Pro Gly
       210                 215
<210> 197
   <211> 259
   <212> PRT
   <213> Homo sapiens
<400> 197
   Met Ala Arg Ile Pro Lys Thr Leu Lys Phe Val Val Val Ile Val Ala
   1 5 10 15
   Val Leu Leu Pro Val Leu Ala Tyr Ser Ala Thr Thr Ala Arg Gln Glu
   20 25 30
   Glu Val Pro Gln Gln Thr Val Ala Pro Gln Gln Gln Arg His Ser Phe
   35 40 45
   Lys Gly Glu Glu Cys Pro Ala Gly Ser His Arg Ser Glu His Thr Gly
   50 55 60
   Ala Cys Asn Pro Cys Thr Glu Gly Val Asp Tyr Thr Asn Ala Ser Asn
   65 70 75 80
   Asn Glu Pro Ser Cys Phe Pro Cys Thr Val Cys Lys Ser Asp Gln Lys
   85 90 95
   His Lys Ser Ser Cys Thr Met Thr Arg Asp Thr Val Cys Gln Cys Lys
   100 105 110
   Glu Gly Thr Phe Arg Asn Glu Asn Ser Pro Glu Met Cys Arg Lys Cys
   115 120 125
   Ser Arg Cys Pro Ser Gly Glu Val Gln Val Ser Asn Cys Thr Ser Trp
   130 135 140
   Asp Asp Ile Gln Cys Val Glu Glu Phe Gly Ala Asn Ala Thr Val Glu
   145 150 155 160
   Thr Pro Ala Ala Glu Glu Thr Met Asn Thr Ser Pro Gly Thr Pro Ala
   165 170 175
   Pro Ala Ala Glu Glu Thr Met Asn Thr Ser Pro Gly Thr Pro Ala Pro
   180 185 190
   Ala Ala Glu Glu Thr Met Thr Thr Ser Pro Gly Thr Pro Ala Pro Ala
   195 200 205
   Ala Glu Glu Thr Met Thr Thr Ser Pro Gly Thr Pro Ala Pro Ala Ala
   210 215 220
   Glu Glu Thr Met Thr Thr Ser Pro Gly Thr Pro Ala Ser Ser His Tyr
   225 230 235 240
   Leu Ser Cys Thr Ile Val Gly Ile Ile Val Leu Ile Val Leu Leu Ile
   245 250 255
   Val Phe Val
<210> 198
   <211> 155
   <212> PRT
   <213> Homo sapiens
<400> 198
<210> 199
   <211> 401
   <212> PRT
   <213> Homo sapiens
<400> 199
   Met Asn Asn Leu Leu Cys Cys Ala Leu Val Phe Leu Asp Ile Ser Ile
    1               5                  10                  15
   Lys Trp Thr Thr Gln Glu Thr Phe Pro Pro Lys Tyr Leu His Tyr Asp
               20                  25                  30
   Glu Glu Thr Ser His Gln Leu Leu Cys Asp Lys Cys Pro Pro Gly Thr
           35                  40                  45
   Tyr Leu Lys Gln His Cys Thr Ala Lys Trp Lys Thr Val Cys Ala Pro
       50                  55                  60
   Cys Pro Asp His Tyr Tyr Thr Asp Ser Trp His Thr Ser Asp Glu Cys
   65                  70                  75                  80
   Leu Tyr Cys Ser Pro Val Cys Lys Glu Leu Gln Tyr Val Lys Gln Glu
                   85                  90                  95
   Cys Asn Arg Thr His Asn Arg Val Cys Glu Cys Lys Glu Gly Arg Tyr
               100                 105                 110
   Leu Glu Ile Glu Phe Cys Leu Lys His Arg Ser Cys Pro Pro Gly Phe
           115                 120                 125
   Gly Val Val Gln Ala Gly Thr Pro Glu Arg Asn Thr Val Cys Lys Arg
       130                 135                 140
   Cys Pro Asp Gly Phe Phe Ser Asn Glu Thr Ser Ser Lys Ala Pro Cys
   145                 150                 155                 160
   Arg Lys His Thr Asn Cys Ser Val Phe Gly Leu Leu Leu Thr Gln Lys
                   165                 170                 175
   Gly Asn Ala Thr His Asp Asn Ile Cys Ser Gly Asn Ser Glu Ser Thr
               180                 185                 190
   Gln Lys Cys Gly Ile Asp Val Thr Leu Cys Glu Glu Ala Phe Phe Arg
           195                 200                 205
   Phe Ala Val Pro Thr Lys Phe Thr Pro Asn Trp Leu Ser Val Leu Val
       210                 215                 220
   Asp Asn Leu Pro Gly Thr Lys Val Asn Ala Glu Ser Val Glu Arg Ile
   225                 230                 235                 240
   Lys Arg Gln His Ser Ser Gln Glu Gln Thr Phe Gln Leu Leu Lys Leu
                   245                 250                 255
   Trp Lys His Gln Asn Lys Asp Gln Asp Ile Val Lys Lys Ile Ile Gln
               260                 265                 270
   Asp Ile Asp Leu Cys Glu Asn Ser Val Gln Arg His Ile Gly His Ala
           275                 280                 285
   Asn Leu Thr Phe Glu Gln Leu Arg Ser Leu Met Glu Ser Leu Pro Gly
       290                 295                 300
   Lys Lys Val Gly Ala Glu Asp Ile Glu Lys Thr Ile Lys Ala Cys Lys
   305                 310                 315                 320
   Pro Ser Asp Gln Ile Leu Lys Leu Leu Ser Leu Trp Arg Ile Lys Asn
                   325                 330                 335
   Gly Asp Gln Asp Thr Leu Lys Gly Leu Met His Ala Leu Lys His Ser
               340                 345                 350
   Lys Thr Tyr His Phe Pro Lys Thr Val Thr Gln Ser Leu Lys Lys Thr
           355                 360                 365
   Ile Arg Phe Leu His Ser Phe Thr Met Tyr Lys Leu Tyr Gln Lys Leu
       370                 375                 380
   Phe Leu Glu Met Ile Gly Asn Gln Val Gln Ser Val Lys Ile Ser Cys
   385                 390                 395                 400
   Leu
<210> 200
   <211> 234
   <212> PRT
   <213> Homo sapiens
<400> 200
   Met Arg Lys Thr Arg Leu Trp Gly Leu Leu Trp Met Leu Phe Val Ser
    1               5                  10                  15
   Glu Leu Arg Ala Ala Thr Lys Leu Thr Glu Glu Lys Tyr Glu Leu Lys
               20                  25                  30
   Glu Gly Gln Thr Leu Asp Val Lys Cys Asp Tyr Thr Leu Glu Lys Phe
           35                  40                  45
   Ala Ser Ser Gln Lys Ala Trp Gln Ile Ile Arg Asp Gly Glu Met Pro
       50                  55                  60
   Lys Thr Leu Ala Cys Thr Glu Arg Pro Ser Lys Asn Ser His Pro Val
   65                  70                  75                  80
   Gln Val Gly Arg Ile Ile Leu Glu Asp Tyr His Asp His Gly Leu Leu
                   85                  90                  95
   Arg Val Arg Met Val Asn Leu Gln Val Glu Asp Ser Gly Leu Tyr Gln
               100                 105                 110
   Cys Val Ile Tyr Gln Pro Pro Lys Glu Pro His Met Leu Phe Asp Arg
           115                 120                 125
   Ile Arg Leu Val Val Thr Lys Gly Phe Ser Gly Thr Pro Gly Ser Asn
       130                 135                 140
   Glu Asn Ser Thr Gln Asn Val Tyr Lys Ile Pro Pro Thr Thr Thr Lys
   145                 150                 155                 160
   Ala Leu Cys Pro Leu Tyr Thr Ser Pro Arg Thr Val Thr Gln Ala Pro
                   165                 170                 175
   Pro Lys Ser Thr Ala Asp Val Ser Thr Pro Asp Ser Glu Ile Asn Leu
               180                 185                 190
   Thr Asn Val Thr Asp Ile Ile Arg Val Pro Val Phe Asn Ile Val Ile
           195                 200                 205
   Leu Leu Ala Gly Gly Phe Leu Ser Lys Ser Leu Val Phe Ser Val Leu
       210                 215                 220
   Phe Ala Val Thr Leu Arg Ser Phe Val Pro
   225                 230
<210> 201
   <211> 380
   <212> PRT
   <213> Homo sapiens
<400> 201
<210> 202
   <211> 380
   <212> PRT
   <213> Homo sapiens
<400> 202
<210> 203
   <211> 234
   <212> PRT
   <213> Homo sapiens
<400> 203
   Met Arg Ser Gly Glu Pro Ala Cys Thr Met Asp Gln Ala Arg Gly Leu
    1               5                  10                  15
   Asp Asp Ala Ala Ala Arg Gly Gly Gln Cys Pro Gly Leu Gly Pro Ala
               20                  25                  30
   Pro Thr Pro Thr Pro Pro Gly Arg Leu Gly Ala Pro Tyr Ser Glu Ala
           35                  40                  45
   Trp Gly Tyr Phe His Leu Ala Pro Gly Arg Pro Gly His Pro Ser Gly
       50                  55                  60
   His Trp Ala Thr Cys Arg Leu Cys Gly Glu Gln Val Gly Arg Gly Pro
   65                  70                  75                  80
   Gly Phe His Ala Gly Thr Ser Ala Leu Trp Arg His Leu Arg Ser Ala
                   85                  90                  95
   His Arg Arg Glu Leu Glu Ser Ser Gly Ala Gly Ser Ser Pro Pro Ala
               100                 105                 110
   Ala Pro Cys Pro Pro Pro Pro Gly Pro Ala Ala Ala Pro Glu Gly Asp
           115                 120                 125
   Trp Ala Arg Leu Leu Glu Gln Met Gly Ala Leu Ala Val Arg Gly Ser
       130                 135                 140
   Arg Arg Glu Arg Glu Leu Glu Arg Arg Glu Leu Ala Val Glu Gln Gly
   145                 150                 155                 160
   Glu Arg Ala Leu Glu Arg Arg Arg Arg Ala Leu Gln Glu Glu Glu Arg
                   165                 170                 175
   Ala Ala Ala Gln Ala Arg Arg Glu Leu Gln Ala Glu Arg Glu Ala Leu
               180                 185                 190
   Gln Ala Arg Leu Arg Asp Val Ser Arg Arg Glu Gly Ala Leu Gly Trp
           195                 200                 205
   Ala Pro Ala Ala Pro Pro Pro Leu Lys Asp Asp Pro Glu Gly Asp Arg
       210                 215                 220
   Asp Gly Cys Val Ile Thr Lys Val Leu Leu
   225                 230

## Description

### FIELD OF THE INVENTION

The invention relates to the identification of expression profiles and the nucleic acids indicative of TNF mediated disorders such as ulcerative colitis, and to the use of such expression profiles and nucleic acids in diagnosis of ulcerative colitis and related diseases. The invention further relates to methods for identifying, using, and testing candidate agents and/or targets which modulate ulcerative colitis.

### BACKGROUND OF THE INVENTION

The treatment of ulcerative colitis with biologics presents a number of challenges. Determining which patient population to study, predicting which subjects will respond to treatment, and which subjects will lose responsiveness following treatment are issues that have significant impact upon treatment and clinical study design. Biomarkers can be useful in answering these questions.

Biomarkers are defined as a characteristic that is objectively measured and evaluated as an indicator of normal biologic processes, pathogenic processes, or pharmacologic responses to a therapeutic intervention (Biomarkers Working Group, 2001, *infra*). The definition of a biomarker has recently been further defined as proteins in which a change in the expression of may correlate with an increased risk of disease or progression, or predictive of a response of a disease to a given treatment.

Tumor necrosis factor alpha (TNFα) is an important cytokine in the innate immune response, which provides immediate host defense against invading organisms before activation of the adaptive immune system. TNFα is expressed as a transmembrane precursor that undergoes proteolytic processing to form a soluble trimer. The binding of both the membrane-bound and soluble forms of TNF to its receptors, TNFRSF1A and TNFRSF1B (also known as TNFR1 and TNFR2 respectively), initiates the expression of several other pro-inflammatory cytokines and general inflammatory markers. TNFα is a known mediator of many chronic immune-mediated inflammatory diseases.

There are many biologic TNFα antagonists, such as infliximab (Remicade®), golimumab (Simponi®), adalimumab (Humira®), and etanercept (Enbrel®), that have been approved for patient use. The primary mechanism is to reduce the levels of TNF in the circulation thereby reducing the overall inflammation and ameliorate the clinical signs of disease, without causing systemic immunosuppression in the patient. They have been shown so far to be efficacious in treating rheumatoid arthritis (RA), psoriatic arthritis (PsA), Crohn's disease (CD), ulcerative colitis (UC), psoriasis, and ankylosing spondylitis.

Ulcerative colitis (UC) is an episodic inflammatory bowel disease of the colon. UC pathogenesis depends upon an interaction among genetic factors, immune response, microbial infection, and environmental factors, resulting in an abnormal response to bacteria commonly found in the gastrointestinal tract. Gene expression not only controls the overall course of the disease, but also reflects the processes that underlie the clinical expression of active disease and disease in remission.

Current UC treatments include anti-inflammatory agents, immunomodulators, and anti-tumor necrosis factor α (TNFα) agents, including infliximab. Infliximab induces and maintains clinical response and clinical remission in patients with UC as demonstrated in the ACT I and ACT 2 trials.

Although none of these anti-TNFα agents has been approved to treat ulcerative colitis, TNFα has been implicated in many aspects of inflammatory gastrointeslinal disorders. However, there are individual differences in response to anti-TNF therapy and some patients may not receive therapeutic benefit. It has been hypothesized that multiple genetic variations may play a key role in the varied response.

Accordingly, there is a need to identify and characterize new gene markers from serum or plasma useful in developing methods for diagnosing and treating immune-mediated inflammatory disorders, such as ulcerative colitis, as well as other diseases and conditions, and a method for predicting how a patient would respond to a therapeutic intervention. Marker panels in general are already known (e.g. WO 2008/028044).

### SUMMARY OF THE INVENTION

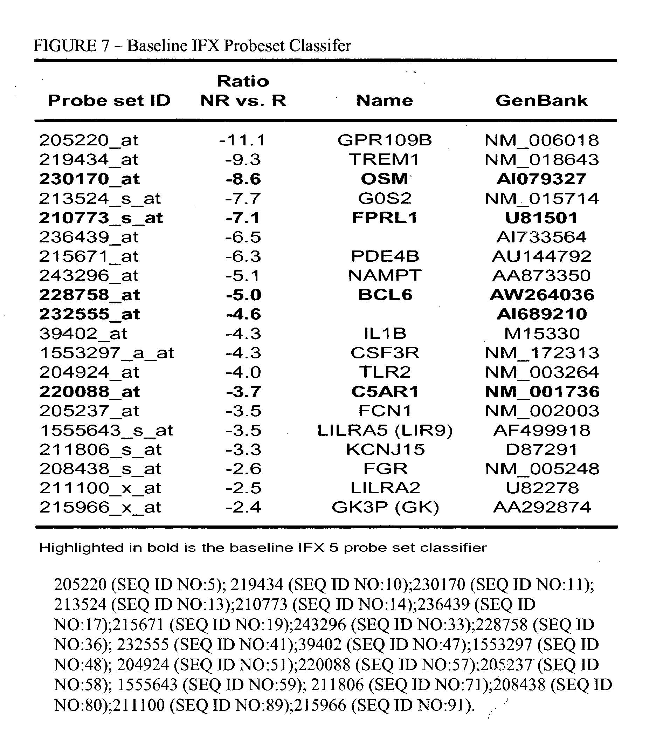

The present invention relates to a method or kit for diagnosing and/or treating ulcerative colitis and/or related diseases or disorders and/or predicting the suitability of candidate agents for treatment. The present invention includes the discovery of particular genes of interest that have modified expression levels in patients responsive to treatment for ulcerative colitis (effective in reducing the symptoms of ulcerative colitis) versus patients nonresponsive to treatment or placebo treated patients. The modified expression levels constitute a profile that can serve as a biomarker profile predictive of a patient's responsiveness to treatment and/or provide preferred dosage routes.
1. The subject matter disclosed herein relates to the genetic association of a set of genes (and expression of those genes) that are up- or down- regulated with anti-TNF therapy in subjects having an inflammatory gastrointestinal disorder, such as ulcerative colitis. The genes up- or down regulated include BCL6 (Genbank Acc. No. AW264036; SEQ ID NO: 118) and a gene (Genbank Acc. No. AI689210; SEQ ID NO: 123), optionally further comprising C5AR1 ((Genbank Acc. No.NM001736; SEQ ID NO: 119), FOLR1 ((Genbank Acc. No. U81501; SEQ ID NO: 142) and/or OSM (Genbank Acc. No. A1079327; SEQ ID NO: 173), and optionally further comprising a gene presented in Figure 7 (*e.g.*, SEQ ID NO:s 110 through 203). The expression profiles of these genes may be measured using one or more nucleic acid probes selected from the group consisting of SEQ ID NOs: 1 through 109.

The subject exhibits a therapeutic response to at least one anti-TNFα agents (*e*.*g*., infliximab, golimumab, Enbrel™, Humira™, or other anti-TNF biologic or small molecule drug), wherein the subject has been diagnosed with ulcerative colitis or another gastrointestinal disorder, such as but not limited to Crohn's disease, inflammatory bowel disorder and the like, including mild, moderate, severe, pediatric or adult forms, as well as other forms such as but not limited to steroid, methotrexate or NSAID resistant forms of gastrointestinal disorders.

In one embodiment, the present invention uses a gene panel in a method of assessing the effectiveness of candidate agents for treatment of ulcerative colitis or related disorders, for example, at early time points of treatment where the effectiveness of treatment may not be measurable by symptoms or traditional disease characteristics.

In a particular embodiment, the present invention comprises a method of predicting the suitability of a treatment for ulcerative colitis based on the pattern of gene expression of one or more genes which constitute the profile prior to treatment.

In addition, the present invention comprises a method of identifying subjects with ulcerative colitis and/or related diseases or disorders that are candidates for treatment with a particular therapeutic agent by evaluating their expression profile of one or more these TNF receptor SNPs of the panel.

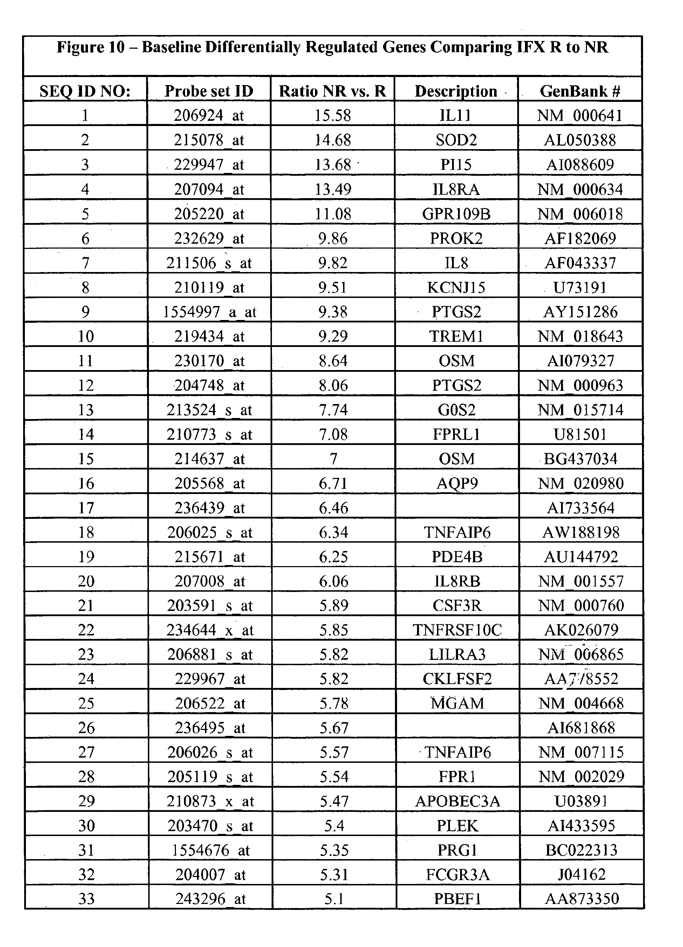

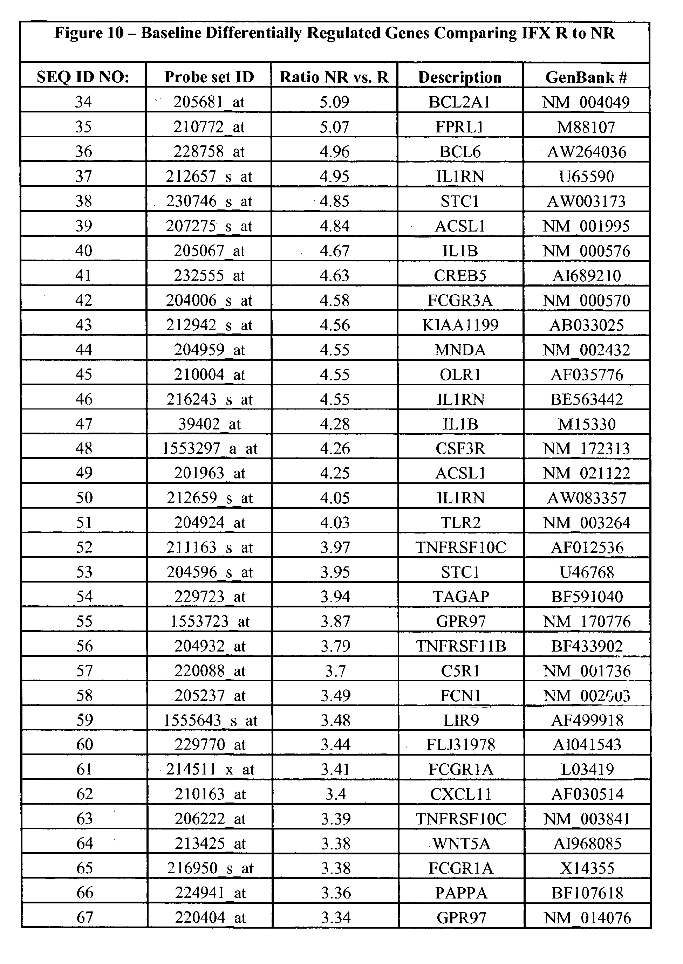

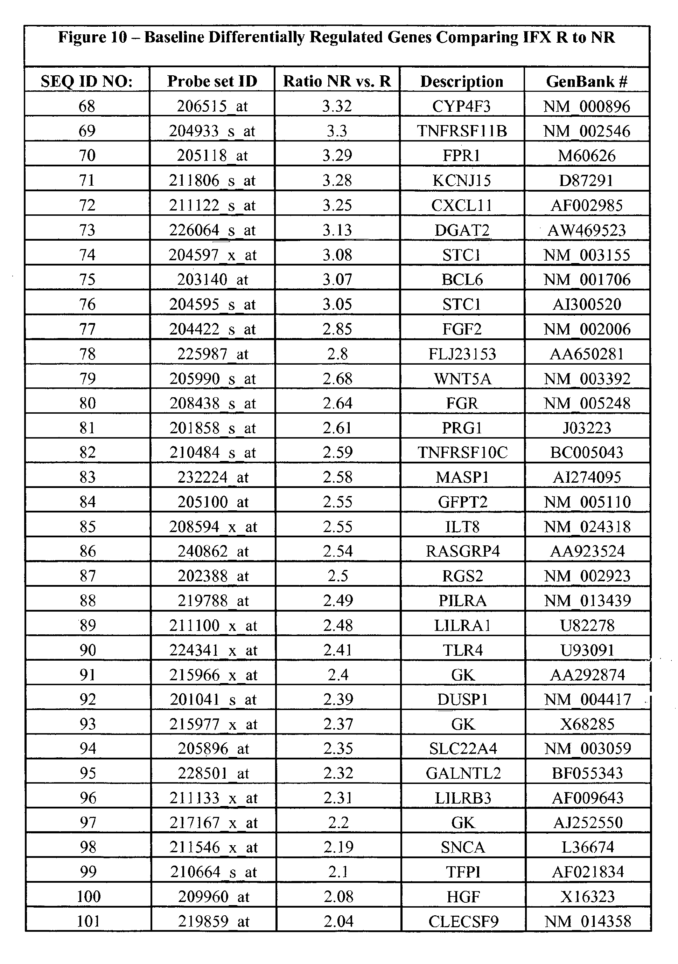

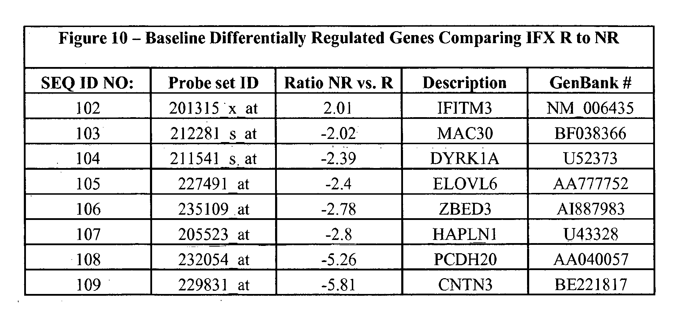

In a further embodiment, the ulcerative colitis-related gene profile is used to create an array-based method for prognostic or diagnostic purposes, the method comprising:
a) preparing a sample of nucleic acids from a specimen obtained from the subject; and
b) contacting the sample with a panel of nucleic acid segments (*e*.*g*., SEQ ID NOs: 1-109) comprising a portion of a BCL6 (Genbank Acc. No. AW264036; SEQ ID NO: 118) gene and tx82a04.x1 (Genbank Acc. No. AI689210; SEQ ID NO: 123), optionally further comprising C5AR1 ((Genbank Acc. No.NM001736; SEQ ID NO: 119), FOLR1 ((Genbank Acc. No. U81501; SEQ ID NO: 142) and OSM (Genbank Acc. No. A1079327; SEQ ID NO: 173), optionally further comprising a gene listed in Figure 7 or 10 (*e*.*g*., SEQ ID NOs: 110-203) with a therapeutic response to an TNFα agent (*e*.*g*., infliximab, golimumab, Enbrel™, Humira ™, or other anti-TNF biologic or small molecule drug), in subjects with ulcerative colitis or other gastrointestinal disorder, such as but not limited to Crohn's disease, inflammatory bowel disorder and the like, including mild, moderate, severe, pediatric or adult forms, as well as other forms such as but not limited to steroid, methotrexate or NSAID resistant forms of gastrointestinal disorders.

Optionally, statistical analysis is performed on the changes in expression levels of members of the gene SNP corresponding panel to evaluate the significance of these changes and to identify which members are meaningful members of the panel.

In an alternative embodiment, the present invention comprises a kit for predicting the suitability of candidate agents for treating ulcerative colitis and/or related diseases or disorders based on the pattern of gene expression.

### DESCRIPTION OF THE FIGURES

Figure 1: Baseline pathway distribution analysis between infliximab responders and non-responders. Pathway distribution analysis of the genes differentially expressed at baseline between responders and non-responders shown as percentage of genes (X axis) in a given pathway among the total number of genes within the input list having GeneOntology annotations. All enrichments are statistically significant (Fisher's exact test score p-value < 0.05).
Figure 2: Hierarchical clustering using the 20 probe set baseline classifier. Hierarchical clustering of the 20 probe set classifier across 12 responder vs. 11 non-responder samples before infliximab treatment. The similarity matrix was calculated using the Pearson correlation around 0 (standard correlation in GeneSpring).
Figure 3: Hierarchical clustering using the 5 probe set baseline classifier. Hierarchical clustering of the 5 probe set classifier across either 12 responder vs. 11 non-responder samples from the training set (A) or 8 responders and 16 non-responders from the test set before infliximab treatment. The similarity matrix was calculated using the Pearson correlation around 0 (standard correlation in GeneSpring).
Figure 4: Infliximab responder/Non-responder expression profile of the 5 probe set classifier. Dot plot representation comparing infliximab responders to non-responders of the 5 probe set classifier. Shown are the normalized intensities of each sample (black circle). Also shown is the median intensity, the 75 and 25 percentile and the minimum and maximum values for each responder and non-responder population for each of the 5 genes.
Figure 5: Leuven Cohort hierarchical clustering of the 20 probe set classifier among the 8 responders and 16 non-responders showed that the 4 misclassified non-responders and the 1 misclassified responder have expression profiles very similar to responders and non-responders respectively.
Figure 6: Common and unique probe sets comparing the ACT1 with the validation cohort
Figure 7: Baseline IFX Probeset Classifer
Figure 8: Characteristics of Baseline IFX Predictive Signatures

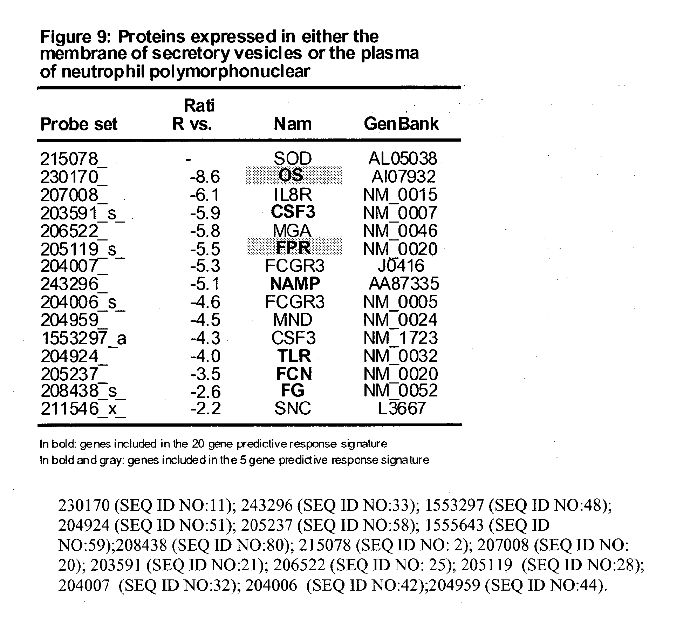

Figure 9: Proteins expressed in either the membrane of secretory vesicles or the plasma of neutrophil polymorphonuclear leukocytes.
Figure 10. Baseline differentially regulated genes comparing IFXR to NR.

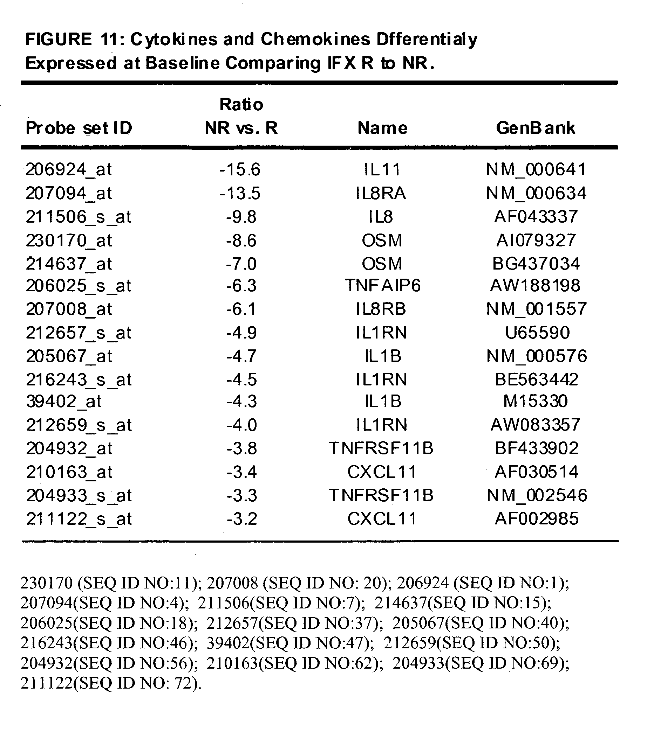

Figure 11. Cytokines and chemokines differentially expressed at baseline comparing IFXR to NR.

### DETAILED DESCRIPTION OF THE INVENTION

### Definitions

An "activity," a biological activity, and a functional activity of a polypeptide refers to an activity exerted by a gene, or protein encoded by a gene, of the ulcerative colitis-related gene panel in response to its specific interaction with another protein or molecule as determined *in vivo*, *in situ*, *or in vitro*, according to standard techniques. Such activities can be a direct activity, such as an association with or an enzymatic activity on a second protein, or an indirect activity, such as a cellular process mediated by interaction of the protein with a second protein or a series of interactions as in intracellular signaling or the coagulation cascade.

An "antibody" includes any polypeptide or peptide containing molecule that comprises at least a portion of an immunoglobulin molecule, such as but not limited to, at least one complementarity determining region (CDR) of a heavy or light chain or a ligand binding portion thereof, a heavy chain or light chain variable region, a heavy chain or light chain constant region, a framework region, or any portion, fragment or variant thereof. The term "antibody" is further intended to encompass antibodies, digestion fragments, specified portions and variants thereof, including antibody mimetics or comprising portions of antibodies that mimic the structure and/or function of an antibody or specified fragment or portion thereof, including single chain antibodies and fragments thereof. For example, antibody fragments include, but are not limited to, Fab (*e*.*g*., by papain digestion), Fab' (*e*.*g*., by pepsin digestion and partial reduction) and F(ab')2 (*e*.*g*., by pepsin digestion), facb (*e*.*g*., by plasmin digestion), pFc' (*e.g.*, by pepsin or plasmin digestion), Fd (*e.g.*, by pepsin digestion, partial reduction and reaggregation), Fv or scFv (*e.g.*, by molecular biology techniques) fragments, and single domain antibodies (*e*.*g*., V_{H} or V_{L}), are encompassed by the invention (see, *e*.*g*., Colligan, et al., eds., Current Protocols in Immunology, John Wiley & Sons, Inc., NY (1994-2001); Colligan et al., Current Protocols in Polypeptide Science, John Wiley & Sons, NY (1997-2001)).

The terms "array" or "microarray" or "biochip" or "chip" as used herein refer to articles of manufacture or devices comprising a plurality of immobilized target elements, each target element comprising a "clone," "feature," "spot" or defined area comprising a particular composition, such as a biological molecule, *e*.*g*., a nucleic acid molecule or polypeptide, immobilized to a solid surface, as discussed in further detail, below.

"Complement of" or "complementary to" a nucleic acid sequence of the invention refers to a polynucleotide molecule having a complementary base sequence and reverse orientation as compared to a first polynucleotide.

"Identity," as known in the art, is a relationship between two or more polypeptide sequences or two or more polynucleotide sequences, as determined by comparing the sequences. In the art, "identity" also means the degree of sequence relatedness between polypeptide or polynucleotide sequences, as determined by the match between strings of such sequences. "Identity" and "similarity" can be readily calculated by known methods, including, but not limited to, those described in Computational Molecular Biology, Lesk, A. M., ed., Oxford University Press, New York, 1988; Biocomputing:Informatics and Genome Projects, Smith, D. W., ed., Academic Press, New York, 1993; Computer Analysis of Sequence Data, Part I, Griffin, A. M., and Griffin, H. G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991; and Carillo, H., and Lipman, D., Siam J. Applied Math., 48:1073 (1988). In addition, values for percentage identity can be obtained from amino acid and nucleotide sequence alignments generated using the default settings for the AlignX component of Vector NTI Suite 8.0 (Informax, Frederick, MD).

The terms "specifically hybridize to," "hybridizing specifically to," "specific hybridization" and "selectively hybridize to," as used herein refer to the binding, duplexing, or hybridizing of a nucleic acid molecule preferentially to a particular nucleotide sequence under stringent conditions. The term "stringent conditions" refers to conditions under which a probe will hybridize preferentially to its target subsequence; and to a lesser extent to, or not at all to, other sequences. A "stringent hybridization" and "stringent hybridization wash conditions" in the context of nucleic acid hybridization (*e*.*g*., as in array; Southern or Northern hybridizations) are sequence dependent, and are different under different environmental parameters. Alternative hybridization conditions that can be used to practice the invention are described in detail, below. In alternative aspects, the hybridization and/or wash conditions are carried out under moderate conditions, stringent conditions and very stringent conditions, as described in further detail, below. Alternative wash conditions are also used in different aspects, as described in further detail, herein.

The phrases "labeled biological molecule" or "labeled with a detectable composition" or "labeled with a detectable moiety" as used herein refer to a biological molecule, *e*.*g*., a nucleic acid, comprising a detectable composition, *i*.*e*., a label, as described in detail, below. The label can also be another biological molecule, as a nucleic acid, *e*.*g*., a nucleic acid in the form of a stem-loop structure as a "molecular beacon," as described below. This includes incorporation of labeled bases (or, bases which can bind to a detectable label) into the nucleic acid by, *e*.*g*., nick translation, random primer extension, amplification with degenerate primers, and the like. Any label can be used, *e.g.*, chemiluminescent labels, radiolabels, enzymatic labels and the like. The label can be detectable by any means, *e*.*g*., visual, spectroscopic, photochemical, biochemical, immunochemical, physical, chemical and/or chemiluminescent detection. The invention can use arrays comprising immobilized nucleic acids comprising detectable labels.

The term "nucleic acid" as used herein refers to a deoxyribonucleotide (DNA) or ribonucleotide (RNA) in either single- or double-stranded form. The term encompasses nucleic acids containing known analogues of natural nucleotides. The term nucleic acid is used interchangeably with gene, DNA, RNA, cDNA, mRNA, oligonucleotide primer, probe and amplification product. The term also encompasses DNA backbone analogues, such as phosphodiester, phosphorothioate, phosphorodithioate, methylphosphonate, phosphoramidate, alkyl phosphotriester, sulfamate, 3'-thioacetal, methylene (methylimino), 3'-N-carbamate, morpholino carbamate, and peptide nucleic acids (PNAs).

The terms "sample" or "sample of nucleic acids" as used herein refer to a sample comprising a DNA or RNA, or nucleic acid representative of DNA or RNA isolated from a natural source. A "sample of nucleic acids" is in a form suitable for hybridization (*e*.*g*., as a soluble aqueous solution) to another nucleic acid *(e.g.,* immobilized probes). The sample nucleic acid can be isolated, cloned, or extracted from particular cells or tissues. The cell or tissue sample from which the nucleic acid sample is prepared is typically taken from a patient having or suspected of having UC or a related disease or condition. Methods of isolating cell and tissue samples are well known to those of skill in the art and include, but are not limited to, aspirations, tissue sections, needle biopsies, and the like. Frequently the sample will be a "clinical sample" which is a sample derived from a patient, including sections of tissues such as frozen sections or paraffin sections taken for histological purposes. The sample can also be derived from supernatants (of cells) or the cells themselves taken from patients or from cell cultures, cells from tissue culture and other media in which it can be desirable to detect the response to drug candidates. In some cases, the nucleic acids can be amplified using standard techniques such as PCR, prior to the hybridization. The probe can be produced from and collectively can be representative of a source of nucleic acids from one or more particular (pre-selected) portions of, *e*.*g*., a collection of polymerase chain reaction (PCR) amplification products, substantially an entire chromosome or a chromosome fragment, or substantially an entire genome, *e*.*g*., as a collection of clones, *e*.*g*., BACs, PACs, YACs, and the like (see below).

"Nucleic acids" are polymers of nucleotides, wherein a nucleotide comprises a base linked to a sugar which sugars are in turn linked one to another by an interceding at least bivalent molecule, such as phosphoric acid. In naturally occurring nucleic acids, the sugar is either 2'-deoxyribose (DNA) or ribose (RNA). Unnatural poly- or oligonucleotides contain modified bases, sugars, or linking molecules, but are generally understood to mimic the complementary nature of the naturally occurring nucleic acids after which they are designed. An example of an unnatural oligonucleotide is an antisense molecule composition that has a phosphorothioate backbone. An "oligonucleotide" generally refers to a nucleic acid molecule having less than 30 nucleotides.

The term "profile" means a pattern and relates to the magnitude and direction of change of a number of features. The profile can be interpreted stringently, *i.e.*, where the variation in the magnitude and/or number of features within the profile displaying the characteristic is substantially similar to a reference profile or it can be interpreted less stringently, for example, by requiring a trend rather than an absolute match of all or a subset of feature characteristics.

The terms "protein," "polypeptide," and "peptide" include "analogs," or "conservative variants" and "mimetics" or "peptidomimetics" with structures and activity that substantially correspond to the polypeptide from which the variant was derived, as discussed in detail above.

A "polypeptide" is a polymer of amino acid residues joined by peptide bonds, and a peptide generally refers to amino acid polymers of 12 or less residues. Peptide bonds can be produced naturally as directed by the nucleic acid template or synthetically by methods well known in the art.

A "protein" is a macromolecule comprising one or more polypeptide chains. A protein may further comprise substituent groups attached to the side groups of the amino acids not involved in formation of the peptide bonds. Typically, proteins formed by eukaryotic cell expression also contain carbohydrates. Proteins are defined herein in terms of their amino acid sequence or backbone and substituents are not specified, whether known or not.

The term "receptor" denotes a molecule having the ability to affect biological activity, in *e*.*g*., a cell, as a result of interaction with a specific ligand or binding partner. Cell membrane bound receptors are characterized by an extracellular ligand-binding domain, one or more membrane spanning or transmembrane domains, and an intracellular effector domain that is typically involved in signal transduction. Ligand binding to cell membrane receptors causes changes in the extracellular domain that are communicated across the cell membrane, direct or indirect interaction with one or more intracellular proteins, and alters cellular properties, such as enzyme activity, cell shape, or gene expression profile. Receptors may also be untethered to the cell surface and can be cytosolic, nuclear, or released from the cell altogether. Non-cell associated receptors are termed soluble receptors or ligands.

All publications or patents cited herein show the state of the art at the time of the present invention and/or provide description and enablement of the present invention. Publications refer to any scientific or patent publications, or any other information available in any media format, including all recorded, electronic or printed formats, and include: Ausubel, et al., ed., Current Protocols in Molecular Biology, John Wiley & Sons, Inc., NY (1987-2008); Sambrook, et al., Molecular Cloning: A Laboratory Manual, 2nd Edition, Cold Spring Harbor, NY (1989); Harlow and Lane, antibodies, a Laboratory Manual, Cold Spring Harbor, NY (1989); Colligan, et al., eds., Current Protocols in Immunology, John Wiley & Sons, Inc., NY (1994-2008); Colligan et al., Current Protocols in Protein Science, John Wiley & Sons, NY (1997-2008).

### Gene Panel Identification and Validation

The present invention provides novel methods for screening for molecules that modulate the symptoms of ulcerative colitis. This invention discloses the genetic association of a set of gene up- or down- regulation with anti-TNF responders in inflammatory gastrointestinal disorder, such as ulcerative colitis, with BCL6 (Genbank Acc. No. AW264036; SEQ ID NO: 118) and tx82a04.x1 (Genbank Acc. No. AI689210; SEQ ID NO: 123), optionally further comprising C5AR1 ((Genbank Acc. No.NM001736; SEQ ID NO: 119), FOLR1 ((Genbank Acc. No. U81501; SEQ ID NO: 142) and OSM (Genbank Acc. No.A1079327; SEQ ID NO: 173)), optionally further comprising a gene presented in Figure 7 (*e*.*g*., SEQ ID NOs: 110-203) with a therapeutic response anti-TNFα agents (*e*.*g*., infliximab, golimumab, Enbrel™, Humira ™, or other anti-TNF biologic or small molecule drug), in subjects with ulcerative colitis or other gastrointestinal disorder, such as but not limited to Crohn's disease, inflammatory bowel disorder and the like, including mild, moderate, severe, pediatric or adult forms, as well as other forms such as but not limited to steroid, methotrexate or NSAID resistant forms of gastrointestinal disorders.

The identification of these sequences (genes or hereinafter "ulcerative colitis-related gene sequences") that are differentially expressed in disease tissue alone or in response to anti-TNF therapy allows the use of this information in a number of ways. For example, the evaluation of a particular treatment regime can be evaluated with the information provided by the expression profile of the UC-associated biomarker genes.

This can be done by making biochips comprising sets of the complementary these sequences, which can then be used in these screens (*e*.*g*., SEQ ID NOs: 1-109). These methods can also be performed on the protein basis; that is, protein expression levels of the ulcerative colitis-related as these gene product proteins can be evaluated for diagnostic purposes or to select anti-TNF treatment responders or to screen additional candidate therapeutics. In addition, the nucleic acid sequences comprising the ulcerative colitis-related gene profile can be used to measure whether a patient is likely to respond to a therapeutic prior to treatment.

Ulcerative colitis-related gene sequences can include both nucleic acid and amino acid sequences. In a preferred embodiment, the ulcerative colitis-related gene sequences are recombinant nucleic acids. By the term "recombinant nucleic acid" herein is meant nucleic acid, originally formed *in vitro*, in general, by the manipulation of nucleic acid by polymerases and endonucleases, in a form not normally found in nature. Thus, an isolated nucleic acid, in a linear form, or an expression vector formed *in vitro* by ligating DNA molecules that are not normally joined, are both considered recombinant for the purposes of this invention. It is understood that once a recombinant nucleic acid is made and reintroduced into a host cell or organism, it will replicate non-recombinantly, *i.e.*, using the *in vivo* cellular machinery of the host cell rather than *in vitro* manipulations; however, such nucleic acids, once produced recombinantly, although subsequently replicated non-recombinantly, are still considered recombinant for the purposes of the invention.

### Method of Practicing the Invention

The invention provides *in vitro*, *in situ*, or *in silico*, nucleic acid, protein and/or array-based methods relying on the relative amount of a binding molecule (*e*.*g*., nucleic acid sequence) in two or more samples. Also provided are computer-implemented methods for determining the relative amount of a binding molecule (*e.g.*, nucleic acid sequence) in two or more samples and using the determined relative binding amount to predict responsiveness to a particular therapy, and monitor and enhance therapeutic treatment.

In practicing the methods of the invention, one or more samples of labeled biological molecules (*e*.*g*., nucleic acid) are applied to two or more assays or arrays, where the assays or arrays have substantially the same complement of immobilized binding molecule (*e*.*g*., immobilized nucleic acid capable of hybridizing to labeled sample nucleic acid). The two or more arrays are typically multiple copies of the same array. However, because each "spot," "clone" or "feature" on the array has similar biological molecules (*e*.*g*., nucleic acids of the same sequence) and the biological molecules (*e*.*g*., nucleic acid) in each spot is known, as is typical of nucleic acid and other arrays, it is not necessary that the multiple arrays used in the invention be identical in configuration it is only necessary that the position of each feature on the substrate be known, that is, have an address. Thus, in one aspect, multiple biological molecules (*e.g.*, nucleic acid) in samples are comparatively bound to the array (*e*.*g*., hybridized simultaneously) and the information gathered is coded so that the results are based on the inherent properties of the feature (*e*.*g*., the nucleic acid sequence) and not the position on the substrate.

### Amplification of Nucleic Acids

Well known methods of nucleic acid amplification using oligonucleotide primers can be used to generate nucleic acids used in the compositions and methods of the invention, to detect or measure levels of test or control samples hybridized to an array, and the like, *e*.*g*., to detect the presence of TNFR SNP polymorphisms of the present invention. The skilled artisan can select and design suitable oligonucleotide amplification primers. Amplification methods are also well known in the art, and include, *e*.*g*., polymerase chain reaction, PCR (PCR PROTOCOLS, A GUIDE TO METHODS AND APPLICATIONS, ed. Innis, Academic Press, N.Y. (1990) and PCR STRATEGIES (1995), ed. Innis, Academic Press, Inc., N.Y., ligase chain reaction (LCR) (see, *e*.*g*., Wu (1989) Genomics 4:560; Landegren (1988) Science 241:1077; Barringer (1990) Gene 89:117); transcription amplification (see, *e*.*g*., Kwoh (1989) Proc. Natl. Acad. Sci. USA 86:1173); and, self-sustained sequence replication (see, *e*.*g*., Guatelli (1990) Proc. Natl. Acad. Sci. USA 87:1874); Q Beta replicase amplification (see, *e.g.*, Smith (1997) J. Clin. Microbiol. 35:1477-1491), automated Q-beta replicase amplification assay (see, *e*.*g*., Burg (1996) Mol. Cell. Probes 10:257-271) and other RNA polymerase mediated techniques (*e*.*g*., NASBA, Cangene, Mississauga, Ontario); see also Berger (1987) Methods Enzymol. 152:307-316; Sambrook; Ausubel; U.S. Pat. Nos. 4,683,195 and 4,683,202; Sooknanan (1995) Biotechnology 13:563-564.

### Hybridizing Nucleic Acids

In practicing the methods of the invention, test and control samples of nucleic acid are hybridized to immobilized nucleic acid probes, *e*.*g*., on arrays. In alternative aspects, the hybridization and/or wash conditions are carried out under moderate conditions, stringent conditions and very stringent conditions. An extensive guide to the hybridization of nucleic acids is found in, *e*.*g*., Sambrook Ausubel, Tijssen. Generally, highly stringent hybridization and wash conditions are selected to be about 5°C lower than the thermal melting point (Tm) for the specific sequence at a defined ionic strength and pH. The Tm is the temperature (under defined ionic strength and pH) at which 50% of the target sequence hybridizes to a perfectly matched probe. Very stringent conditions are selected to be equal to the Tm for a particular probe. An example of stringent hybridization conditions for hybridization of complementary nucleic acids which have more than 100 complementary residues on an array or a filter in a Southern or northern blot is 42°C using standard hybridization solutions (see, *e*.*g*., Sambrook), with the hybridization being carried out overnight. An example of highly stringent wash conditions is 0.15 M NaCl at 72°C for about 15 minutes. An example of stringent wash conditions is a 0.2×SSC wash at 65°C for 15 minutes (see, *e*.*g*., Sambrook). Often, a high stringency wash is preceded by a medium or low stringency wash to remove background probe signal. An example medium stringency wash for a duplex of, *e*.*g*., more than 100 nucleotides, is 1×SSC at 45°C for 15 minutes. An example of a low stringency wash for a duplex of, *e*.*g*., more than 100 nucleotides, is 4× to 6×SSC at 40° C for 15 minutes.

In alternative aspects of the compositions and methods of the invention, *e.g.*, in practicing comparative nucleic acid hybridization, such as comparative genomic hybridization (CGH) with arrays, the fluorescent dyes Cy3® and Cy5® are used to differentially label nucleic acid fragments from two samples, *e*.*g*., the array-immobilized nucleic acid versus the sample nucleic acid, or, nucleic acid generated from a control versus a test cell or tissue. Many commercial instruments are designed to accommodate the detection of these two dyes. To increase the stability of Cy5®, or fluors or other oxidation-sensitive compounds, antioxidants and free radical scavengers can be used in hybridization mixes, the hybridization and/or the wash solutions. Thus, Cy5® signals are dramatically increased and longer hybridization times are possible. See WO 0194630 A2 and U.S. Patent Application No. 20020006622.

To increase the hybridization sensitivity, hybridization can be carried out in a controlled, unsaturated humidity environment; thus, hybridization efficiency is significantly improved if the humidity is not saturated. See WO 0194630 A2 and U.S. Patent Application No. 20020006622. The hybridization efficiency can be improved if the humidity is dynamically controlled, *i.e.*, if the humidity changes during hybridization. Mass transfer will be facilitated in a dynamically balanced humidity environment. The humidity in the hybridization environment can be adjusted stepwise or continuously. Array devices comprising housings and controls that allow the operator to control the humidity during pre-hybridization, hybridization, wash and/or detection stages can be used. The device can have detection, control and memory components to allow pre-programming of the humidity and temperature controls (which are constant and precise or which fluctuate), and other parameters during the entire procedural cycle, including pre-hybridization, hybridization, wash and detection steps. See WO 0194630 A2 and U.S. Patent Application No. 20020006622.

The methods of the invention can comprise hybridization conditions comprising osmotic fluctuation. Hybridization efficiency (*i.e*., time to equilibrium) can also be enhanced by a hybridization environment that comprises changing hyper-/hypo-tonicity, *e*.*g*., a solute gradient. A solute gradient is created in the device. For example, a low salt hybridization solution is placed on one side of the array hybridization chamber and a higher salt buffer is placed on the other side to generate a solute gradient in the chamber. See WO 0194630 A2 and U.S. Patent Application No. 20020006622.

### Blocking the Ability of Repetitive Nucleic Acid Sequences to Hybridize

The methods of the invention can comprise a step of blocking the ability of repetitive nucleic acid sequences to hybridize (*i.e.*, blocking "hybridization capacity") in the immobilized nucleic acid segments. The hybridization capacity of repetitive nucleic acid sequences in the sample nucleic acid sequences can be blocked by mixing sample nucleic acid sequences with unlabeled or alternatively labeled repetitive nucleic acid sequences. Sample nucleic acid sequences can be mixed with repetitive nucleic acid sequences before the step of contacting with the array-immobilized nucleic acid segments. Blocking sequences are for example, Cot-1 DNA, salmon sperm DNA, or specific repetitive genomic sequences. The repetitive nucleic acid sequences can be unlabeled. A number of methods for removing and/or disabling the hybridization capacity of repetitive sequences using, *e.g.*, Cot-1 are known; see, *e*.*g*., Craig (1997) Hum. Genet. 100:472-476; WO 93/18186. Repetitive DNA sequences can be removed from library probes by means of magnetic purification and affinity PCR, see, *e*.*g*., Rauch (2000) J. Biochem. Biophys. Methods 44:59-72.

Arrays are generically a plurality of target elements immobilized onto the surface of the plate as defined "spots" or "clusters," or "features," with each target element comprising one or more biological molecules (*e*.*g*., nucleic acids or polypeptides) immobilized to a solid surface for specific binding (*e*.*g*., hybridization) to a molecule in a sample. The immobilized nucleic acids can contain sequences from specific messages (*e*.*g*., as cDNA libraries) or genes (*e*.*g*., genomic libraries), including a human genome. Other target elements can contain reference sequences and the like. The biological molecules of the arrays can be arranged on the solid surface at different sizes and different densities. The densities of the biological molecules in a cluster and the number of clusters on the array will depend upon a number of factors, such as the nature of the label, the solid support, the degree of hydrophobicity of the substrate surface, and the like. Each feature may comprise substantially the same biological molecule (*e*.*g*., nucleic acid), or, a mixture of biological molecules (*e*.*g*., nucleic acids of different lengths and/or sequences). Thus, for example, a feature may contain more than one copy of a cloned piece of DNA, and each copy can be broken into fragments of different lengths.

Array substrate surfaces onto which biological molecules (*e*.*g*., nucleic acids) are immobilized can include nitrocellulose, glass, quartz, fused silica, plastics and the like, as discussed further, below. The compositions and methods of the invention can incorporate in whole or in part designs of arrays, and associated components and methods, as described, *e.g.*, in U.S. Pat. Nos. 6,344,316; 6,197,503; 6,174,684; 6,159,685; 6,156,501; 6,093,370; 6,087,112; 6,087, 103; 6,087,102; 6,083,697; 6, 080,585; 6,054,270; 6,048,695; 6,045,996; 6,022,963; 6,013,440; 5,959,098; 5,856,174; 5,843,655; 5,837,832; 5,770,456; 5,723,320; 5,700,637; 5,695, 940; 5,556,752; 5,143,854; see also, *e*.*g*., WO 99/51773; WO 99/09217; WO 97/46313; WO 96/17958; WO 89/10977; see also, *e*.*g*., Johnston (1998) Curr. Biol. 8:R171-174; Schummer (1997) Biotechniques 23:1087-1092; Kern (1997) Biotechniques 23:120-124; Solinas- Toldo (1997) Genes, Chromosomes & Cancer 20:399-407; Bowtell (1999) Nature Genetics Supp. 21:25-32; Epstein (2000) Current Opinion in Biotech. 11:36-41; Mendoza (1999 Biotechniques 27: 778-788; Lueking (1999) Anal. Biochem. 270:103-111; Davies (1999) Biotechniques 27:1258-1261.

### Substrate Surfaces

Substrate surfaces that can be used in the compositions and methods of the invention include, for example, glass (see, *e*.*g*., U.S. Pat No. 5,843,767), ceramics, and quartz. The arrays can have substrate surfaces of a rigid, semi-rigid or flexible material. The substrate surface can be flat or planar, be shaped as wells, raised regions, etched trenches, pores, beads, filaments, or the like. Substrate surfaces can also comprise various materials such as nitrocellulose, paper, crystalline substrates (*e*.*g*., gallium arsenide), metals, metalloids, polacryloylmorpholide, various plastics and plastic copolymers, Nylon®, Teflon®, polyethylene, polypropylene, latex, polymethacrylate, poly (ethylene terephthalate), rayon, nylon, poly(vinyl butyrate), and cellulose acetate. The substrates can be coated and the substrate and the coating can be functionalized to, *e*.*g*., enable conjugation to an amine.

### Arrays Comprising Calibration Sequences

The invention contemplates the use of arrays comprising immobilized calibration sequences for normalizing the results of array-based hybridization reactions, and methods for using these calibration sequences, *e*.*g*., to determine the copy number of a calibration sequence to "normalize" or "calibrate" ratio profiles. The calibration sequences can be substantially the same as a unique sequence in an immobilized nucleic acid sequence on an array. For example, a "marker" sequence from each "spot" or "biosite" on an array (which is present only on that spot, making it a "marker" for that spot) is represented by a corresponding sequence on one or more "control" or "calibration" spot(s).

The "control spots" or "calibration spots" are used for "normalization" to provide information that is reliable and repeatable. Control spots can provide a consistent result independent of the labeled sample hybridized to the array (or a labeled binding molecule from a sample). The control spots can be used to generate a "normalization" or "calibration" curve to offset possible intensity errors between the two arrays (or more) used in the in silico, array-based methods of the invention.

One method of generating a control on the array would be to use an equimolar mixture of all the biological molecules (*e.g.*, nucleic acid sequences) spotted on the array and generating a single spot. This single spot would have equal amounts of the biological molecules (*e.g.*, nucleic acid sequences) from all the other spots on the array. Multiple control spots can be generated by varying the concentration of the equimolar mixture.

### Samples and Specimens

The sample nucleic acid can be isolated, cloned, or extracted from particular cells, tissues, or other specimens. The cell or tissue sample from which the nucleic acid sample is prepared is typically taken from a patient having or suspected of having ulcerative colitis or a related condition. Methods of isolating cell and tissue samples are well known to those of skill in the art and include, but are not limited to, aspirations, tissue sections, needle biopsies, and the like. Frequently, the sample will be a "clinical sample" which is a sample derived from a patient, including whole blood, serum, plasma, or sections of tissues, such as frozen sections or paraffin sections taken for histological purposes. The sample can also be derived from supernatants (of cells) or the cells themselves taken from patients or from cell cultures, cells from tissue culture and other media in which it can be desirable to detect the response to drug candidates. In some cases, the nucleic acids can be amplified using standard techniques such as PCR, prior to the hybridization.

In one embodiment, the present invention is a pre-treatment method of predicting disease regression or resolution. The method includes (1) taking a suitable tissue biopsy or other specimen from an individual diagnosed with ulcerative colitis or a related disease or disorder, (2) measuring the expression levels of the profile genes of the panel, (3) comparing the pre-treatment expression level of the genes with a pre-treatment reference profile from treatment responders, and (4) predicting treatment response by monitoring the expression levels of the gene panel.

### Methods of Assessing Biomarker Utility

The prognostic utility of the present biomarker gene panel for assessing a patient's response to treatment or prognosis of disease can be validated by using other means for assessing a patient's state of disease. For example, gross measurement of disease can be assessed and recorded by certain imaging methods, such as but not limited to: imaging by photographic, radiometric, or magnetic resonance technology. General indices of health or disease further include serum or blood composition (protein, liver enzymes, pH, electrolytes, red cell volume, hematocrit, hemoglobin, or specific protein). However, in some diseases, the etiology is still poorly understood. ulcerative colitis is an example of one such disease.

### Patient Assessment and Monitoring

The expression patterns of the genes over the course of treatment have not been studied in the treatment of ulcerative colitis or other gastrointestinal disorders, and none has been identified as having predictive value. The panel of gene expression biomarkers disclosed herein permits the generation of methods for rapid and reliable prediction, diagnostic tools that predict the clinical outcome of a ulcerative colitis trial, or prognostic tools for tracking the efficacy of ulcerative colitis therapy. Prognostic methods based on detecting these genes in a sample are provided. These compositions can be used, for example, in connection with the diagnosis, prevention and treatment of a range of immune-mediated inflammatory diseases.

### Therapeutic agents

### Antagonists

As used herein, the term "antagonists" refer to substances which inhibit or neutralize the biologic activity of the gene product of the ulcerative colitis-related gene panel of the invention. Such antagonists accomplish this effect in a variety of ways. One class of antagonists will bind to the gene product protein with sufficient affinity and specificity to neutralize the biologic effects of the protein. Included in this class of molecules are antibodies and antibody fragments (such as, for example, F(ab) or F(ab')₂ molecules). Another class of antagonists comprises fragments of the gene product protein, muteins or small organic molecules, *i.e.*, peptidomimetics, that will bind to the cognate binding partners or ligands of the gene product, thereby inhibiting the biologic activity of the specific interaction of the gene product with its cognate ligand or receptor. The ulcerative colitis-related gene antagonist can be of any of these classes as long as it is a substance that inhibits a biological activity of the gene product.

Antagonists include antibodies directed to one or more regions of the gene product protein or fragments thereof, antibodies directed to the cognate ligand or receptor, and partial peptides of the gene product or its cognate ligand which inhibit a biological activity of the gene product. Another class of antagonists includes siRNAs, shRNAs, antisense molecules and DNAzymes targeting the gene sequence as known in the art are disclosed herein.

Suitable antibodies include those that compete for binding to ulcerative colitis-related gene product with monoclonal antibodies that block ulcerative colitis-related gene product activation or prevent ulcerative colitis-related gene product binding to its cognate ligand, or prevent ulcerative colitis-related gene product signaling.

A therapeutic targeting the inducer of the ulcerative colitis-related gene product may provide better chances of success. Gene expression can be modulated in several different ways including by the use of siRNAs, shRNAs, antisense molecules and DNAzymes. Synthetic siRNAs, shRNAs, and DNAzymes can be designed to specifically target one or more genes and they can easily be delivered to cells *in vitro* or *in vivo*.

Disclosed herein are antisense nucleic acid molecules, *i.e.*, molecules that are complementary to a sense nucleic acid encoding a ulcerative colitis-related gene product polypeptide, *e*.*g*., complementary to the coding strand of a double-stranded cDNA molecule or complementary to an mRNA sequence. Accordingly, an antisense nucleic acid can hydrogen bond to a sense nucleic acid. The antisense nucleic acid can be complementary to an entire coding strand, or to only a portion thereof, *e.g.*, all or part of the protein coding region (or open reading frame). An antisense nucleic acid molecule can be antisense to all or part of a non-coding region of the coding strand of a nucleotide sequence encoding a ulcerative colitis-related gene product polypeptide. The non-coding regions ("5' and 3' untranslated regions") are the 5' and 3' sequences that flank the coding region and are not translated into amino acids.

Disclosed herein are chimeric or fusion proteins. As used herein, a "chimeric protein" or "fusion protein" comprises all or part (preferably biologically active) of a ulcerative colitis-related gene product polypeptide operably linked to a heterologous polypeptide (*i.e.*, a polypeptide other than the same UC-related gene product polypeptide). Within the fusion protein, the term "operably linked" is intended to indicate that the ulcerative colitis-related gene product polypeptide and the heterologous polypeptide are fused in-frame to each other. The heterologous polypeptide can be fused to the amino-terminus or the carboxyl-terminus of the ulcerative colitis-related gene product polypeptide. In another embodiment, a ulcerative colitis-related gene product polypeptide or a domain or active fragment thereof can be fused with a heterologous protein sequence or fragment thereof to form a chimeric protein, where the polypeptides, domains or fragments are not fused end to end but are interposed within the heterologous protein framework.

In yet another embodiment, the fusion protein is an immunoglobulin fusion protein in which all or part of a ulcerative colitis-related gene product polypeptide is fused to sequences derived from a member of the immunoglobulin protein family. The immunoglobulin fusion proteins of the invention can be incorporated into pharmaceutical compositions and administered to a subject to inhibit an interaction between a ligand (soluble or membrane-bound) and a protein on the surface of a cell (receptor), to thereby suppress signal transduction *in vivo*. The immunoglobulin fusion protein can be used to affect the bioavailability of a cognate ligand of a ulcerative colitis-related gene product polypeptide. Inhibition of ligand/receptor interaction can be useful therapeutically, both for treating proliferative and differentiative disorders and for modulating (*e.g.*, promoting or inhibiting) cell survival. Moreover, the immunoglobulin fusion proteins of the invention can be used as immunogens to produce antibodies directed against a ulcerative colitis-related gene product polypeptide in a subject, to purify ligands and in screening assays to identify molecules that inhibit the interaction of receptors with ligands.

### Compositions and Their Uses

The neutralizing anti-ulcerative colitis-related gene product antagonists, such as monoclonal antibodies, described herein can be used to inhibit ulcerative colitis-related gene product activity. Additionally, such antagonists can be used to inhibit the pathogenesis of ulcerative colitis and related inflammatory diseases amenable to such treatment, which may include, but are not limited to, rheumatic diseases. The individual to be treated can be any mammal and is preferably a primate, a companion animal which is a mammal and most preferably a human patient. The amount of antagonist administered will vary according to the purpose it is being used for and the method of administration.

The ulcerative colitis-related gene antagonists can be administered by any number of methods that result in an effect in tissue in which pathological activity is desired to be prevented or halted. Further, the anti-ulcerative colitis-related gene product antagonists need not be present locally to impart an effect on the ulcerative colitis-related gene product activity, therefore, they can be administered wherever access to body compartments or fluids containing ulcerative colitis-related gene product is achieved. In the case of inflamed, malignant, or otherwise compromised tissues, these methods may include direct application of a formulation containing the antagonists. Such methods include intravenous administration of a liquid composition, transdermal administration of a liquid or solid formulation, oral, topical administration, or interstitial or inter-operative administration. Administration can be affected by the implantation of a device whose primary function may not be as a drug delivery vehicle.

For antibodies, the preferred dosage is about 0.1 mg/kg to 100 mg/kg of body weight (generally about 10 mg/kg to 20 mg/kg). If the antibody is to act in the brain, a dosage of about 50 mg/kg to 100 mg/kg is usually appropriate. Generally, partially human antibodies and fully human antibodies have a longer half-life within the human body than other antibodies. Accordingly, the use of lower dosages and less frequent administration is often possible. Modifications, such as lipidation, can be used to stabilize antibodies and to enhance uptake and tissue penetration (*e*.*g*., into the brain). A method for lipidation of antibodies is described by Cruikshank *et al.* ((1997) *J. Acquired Immune Deficiency Syndromes and Human Retrovirology* 14:193).

The ulcerative colitis-related gene product antagonist nucleic acid molecules can be inserted into vectors and used as gene therapy vectors. Gene therapy vectors can be delivered to a subject by, for example, intravenous injection, local administration (U.S. Pat. No. 5,328,470), or by stereotactic injection (see, *e*.*g*., Chen et al. (1994) Proc. Natl. Acad. Sci. USA 91:3054- 3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, *e*.*g*., retroviral vectors, the pharmaceutical preparation can include one or more cells which produce the gene delivery system.

The pharmaceutical compositions can be included in a container, pack, or dispenser together with instructions for administration.

### Pharmacogenomics

Agents, or modulators that have a stimulatory or inhibitory effect on activity or expression of a ulcerative colitis-related gene product polypeptide as identified by a screening assay described herein, can be administered to individuals to treat (prophylactically or therapeutically) disorders associated with aberrant activity of the polypeptide. In conjunction with such treatment, the pharmacogenomics *(i.e.,* the study of the relationship between an individual's genotype and that individual's response to a foreign compound or drug) of the individual can be considered. Differences in metabolism of therapeutics can lead to severe toxicity or therapeutic failure by altering the relation between dose and blood concentration of the pharmacologically active drug. Thus, the pharmacogenomics of the individual permits the selection of effective agents (*e*.*g*., drugs) for prophylactic or therapeutic treatments based on a consideration of the individual's genotype. Such pharmacogenomics can further be used to determine appropriate dosages and therapeutic regimens. Accordingly, the activity of a ulcerative colitis-related gene product polypeptide, expression of a ulcerative colitis-related gene product nucleic acid, or mutation content of a ulcerative colitis-related gene product gene in an individual can be determined to thereby select an appropriate agent(s) for therapeutic or prophylactic treatment of the individual.

Pharmacogenomics deals with clinically significant hereditary variations in the response to drugs due to altered drug disposition and abnormal action in affected persons. See, *e*.*g*., Linder (1997) Clin. Chem. 43(2): 254-266. In general, two types of pharmacogenetic conditions can be differentiated. Genetic conditions transmitted as a single factor altering the way drugs act on the body are referred to as "altered drug action." Genetic conditions transmitted as single factors altering the way the body acts on drugs are referred to as "altered drug metabolism." These pharmacogenetic conditions can occur either as rare defects or as polymorphisms. For example, glucose-6-phosphate dehydrogenase (G6PD) deficiency is a common inherited enzymopathy in which the main clinical complication is hemolysis after ingestion of oxidant drugs (anti-malarials, sulfonamides, analgesics, nitrofurans) and consumption of fava beans.

As an illustrative embodiment, the activity of drug metabolizing enzymes is a major determinant of both the intensity and duration of drug action. The discovery of genetic polymorphisms of drug metabolizing enzymes (*e*.*g*., N-acetyltransferase 2 (NAT 2) and cytochrome P450 enzymes CYP2D6 and CYP2C19) has provided an explanation as to why some patients do not obtain the expected drug effects or show exaggerated drug response and serious toxicity after taking the standard and safe dose of a drug. These polymorphisms are expressed in two phenotypes in the population, the extensive metabolizer (EM) and poor metabolizer (PM). The prevalence of PM is different among different populations. For example, the gene coding for CYP2D6 is highly polymorphic and several mutations have been identified in PM, which all lead to the absence of functional CYP2D6. Poor metabolizers of CYP2D6 and CYP2C19 quite frequently experience exaggerated drug response and side effects when they receive standard doses. If a metabolite is the active therapeutic moiety, a PM will show no therapeutic response, as demonstrated for the analgesic effect of codeine mediated by its CYP2D6-formed metabolite morphine. The other extreme are the so called ultra-rapid metabolizers who do not respond to standard doses. Recently, the molecular basis of ultra-rapid metabolism has been identified to be due to CYP2D6 gene amplification.

Thus, the activity of a ulcerative colitis or other gastrointestinal disorder-related gene product polypeptide, expression of a nucleic acid encoding the polypeptide, or mutation content of a gene encoding the polypeptide in an individual can be determined to thereby select appropriate agent(s) for therapeutic or prophylactic treatment of the individual. In addition, pharmacogenetic studies can be used to apply genotyping of polymorphic alleles encoding drug-metabolizing enzymes to the identification of an individual's drug responsiveness phenotype. This knowledge, when applied to dosing or drug selection, can avoid adverse reactions or therapeutic failure and thus enhance therapeutic or prophylactic efficiency when treating a subject with a modulator of activity or expression of the polypeptide, such as a modulator identified by one of the exemplary screening assays described herein.

### Methods of Treatment

Disclosed herein are prophylactic and therapeutic methods of treating a subject at risk of (or susceptible to) a disorder or having a disorder associated with aberrant expression or activity of a ulcerative colitis-related gene product polypeptide and/or in which the ulcerative colitis-related gene product polypeptide is involved.

Disclosed herein is a method for modulating or treating ulcerative colitis-related gene product related disease or condition, in a cell, tissue, organ, animal, or patient, as known in the art or as described herein, using an ulcerative colitis-related gene product antagonist. Compositions of ulcerative colitis-related gene product antagonist may find therapeutic use in the treatment of ulcerative colitis or related conditions, such as ulcerative colitis or other TNF mediated disorders.

Disclosed herein is a method for modulating or treating a TNF mediated, immune related disease, in a cell, tissue, organ, animal, or patient including, but not limited to, gastric ulcer, inflammatory bowel disease, ulcerative colitis, Crohn's pathology, and the like. See, *e*.*g*., the Merck Manual, 12th-17th Editions, Merck & Company, Rahway, NJ (1972, 1977, 1982, 1987, 1992, 1999), Pharmacotherapy Handbook, Wells et al., eds., Second Edition, Appleton and Lange, Stamford, Conn. (1998, 2000).

Disorders characterized by aberrant expression or activity of the ulcerative colitis-related gene product polypeptides are further described elsewhere in this disclosure.

### Prophylactic Methods

Disclosed herein is a method for substantially preventing in a subject, a disease or condition associated with an aberrant expression or activity of a ulcerative colitis-related gene product polypeptide, by administering to the subject an agent that modulates expression or an activity of the polypeptide. Subjects at risk for a disease that is caused or contributed to by aberrant expression or activity of a ulcerative colitis-related gene product can be identified by, for example, any or a combination of diagnostic or prognostic assays as described herein. Administration of a prophylactic agent can occur prior to the manifestation of symptoms characteristic of the aberrancy, such that a disease or disorder is prevented or, alternatively, delayed in its progression. Depending on the type of aberrancy, for example, an agonist or antagonist agent can be used for treating the subject. The appropriate agent can be determined based on screening assays described herein.

### Therapeutic Methods.

Disclosed herein is methods of modulating expression or activity of ulcerative colitis-related gene or gene product for therapeutic purposes. The modulatory method involves contacting a cell with an agent that modulates one or more of the activities of the polypeptide. An agent that modulates activity can be an agent as described herein, such as a nucleic acid or a protein, a naturally-occurring cognate ligand of the polypeptide, a peptide, a peptidomimetic, or other small molecule. In one embodiment, the agent stimulates one or more of the biological activities of the polypeptide. In another embodiment, the agent inhibits one or more of the biological activities of the ulcerative colitis-related gene or gene product polypeptide. Examples of such inhibitory agents include antisense nucleic acid molecules and antibodies and other methods described herein. These modulatory methods can be performed *in vitro* (*e.g.*, by culturing the cell with the agent) or, alternatively, *in vivo* (*e.g.*, by administering the agent to a subject). As such, disclosed herein are methods of treating an individual afflicted with a disease or disorder characterized by aberrant expression or activity of a ulcerative colitis-related gene product polypeptide. In one embodiment, the method involves administering an agent (*e*.*g*., an agent identified by a screening assay described herein), or combination of agents that modulate (*e*.*g*., up-regulates or down-regulates) expression or activity. Inhibition of activity is desirable *in situ*ations in which activity or expression is abnormally high or up-regulated and/or in which decreased activity is likely to have a beneficial effect.

While having described the invention in general terms, the embodiments of the invention will be further disclosed in the following examples which should not be construed as limiting the scope of the claims.

### Abbreviations:

- ACT 1: Active Ulcerative Colitis Trial 1
- ANOVA: analysis of variance
- C5a: complement 5a
- DC: dendritic cell
- FDR: false discovery rate
- IEC: intestinal epithelial cell
- IL: interleukin
- mRNA: messenger RNA
- PMN: polymorphonuclear leucocytes
- TLR: toll-like receptor
- TNFα: tumor necrosis factor alpha
- Th: helper T-cells
- TREM-1: triggering receptor expressed on myeloid cells
- UC: ulcerative colitis

### EXAMPLE 1

Using colonic mucosal biopsies obtained from a subgroup of patients in a clinical study, a retrospective analysis of messenger RNA (mRNA) expression was performed. mRNA expression patterns were identified that provide a molecular profile prior to infliximab treatment that distinguishes responders from non-responders to treatment. The identified molecules were used to define 20- and 5-gene molecular response signatures, which accurately classified patients as responders or non-responders.

The response signature probe sets were applied to a second, independent cohort of patients. The results confirmed the ability of the probe sets to accurately classify subjects prior to infliximab treatment.

Infliximab, an anti-TNFα monoclonal antibody, is an effective treatment for ulcerative colitis (UC) inducing over 60% of patients to respond to treatment. Consequently, about 40% of patients do not respond. This experiment analyzed mucosal gene expression from patients enrolled in a clinical trial ("ACT1") to provide a predictive response signature for infliximab treatment.

Twenty-two patients underwent colonoscopies with biopsy before infliximab treatment. Response to infliximab was defined as endoscopic and histologic healing at week 8. Messenger RNA was isolated from pre-infliximab biopsies, labeled and hybridized to an Affymetrix HGU133Plus_2.0 Array. The predictive response signature was verified by an independent data set.

When the mucosal expression profile of 12 infliximab responders and 10 infliximab non-responders were compared, 109 differentially expressed probe sets were identified. Genes linked to neutrophil cell functions, mucosal epithelial barrier function, and innate immune responses were enriched. From these 109 genes, a predictive response signature of 20 probe sets was defined showing an overall accuracy of 95.4% (21/22), sensitivity of 91.7% (11/12 responders) and specificity of 100% (10/10 non-responders). A reduction to five probe sets maintained the overall accuracy at 90.9% (20/22), sensitivity at 91.7% (11/12 responders) and specificity at 90.0% (9/10 non-responders). Both probe set signatures were validated on an independent cohort yielding overall accuracies of 75% (18/24), sensitivities of 87.5% (7/8 responders), and specificities of 68.8% (11/16 non-responders). The microarray data were deposited at Gene Expression Omnibus under the series accession number GSE12251 (http :// www. ncbi. nlm. nih. gov /geo /query/ acc. cgi? token=rdatvkmqcaiqizk&acc=GSE12251).

### Materials and Methods

Patient biopsies were collected at protocol-specified time points from a subset of ACT1 randomized patients. Twenty-three biopsies obtained at Week 0 were analyzed from a subgroup of 22 patients who received either infliximab 5 or 10 mg/kg (11 biopsies from 10 non-responders and 12 biopsies from 12 responders; two of the non-responder biopsies were obtained within two weeks from the same subject). Response was determined at week 8. Response to infliximab was defined as complete mucosal healing (*i.e.*, Mayo endoscopic subscore of 0 or 1) and a grade of 0 or I on the histological score for UC. Patients who did not achieve mucosal healing were considered non-responders, although some patients showed histologic improvement. The baseline characteristics of this cohort are presented in Table 1.

**Table 1: Baseline Characteristics of the Study Cohorts**

| | **ACT1 Cohort** | |
|---|---|---|
| **Characteristics** | **Responders (n=12)** | **Non-responders (n=10)** |
| Male/Female | 6/6 | 4/6 |
| Median age at baseline (years) | 39.0 (29-70) | 51.5 (24-68) |
| Median weight at baseline (kg) | 75 (63.0-159.0) | 69.0 (46.0-102.0) |
| Median duration of disease at baseline (years) | 5.9 (1.6-42.1) | 5.7 (2.9-26.8) |
| Median C-reactive protein at baseline (mg/dL) | 0.7 (0.2-2.9) | 1.35 (0.2-6.8) |

| **Concomitant medication at baseline** | | |
|---|---|---|
| 5-Aminosalicylates | 1 | 1 |
| Corticosteroids | 8 | 6 |
| Azathioprine/6-Mercaptopurine | 2 | 3 |
| Corticosteroids + Immunosuppressants | 0 | 0 |
| Active smoking at baseline | 1 | 0 |

Intestinal biopsies were collected 15 to 20 centimeters distal from the anal verge during endoscopies conducted at Week 0. Biopsies were quick frozen in liquid nitrogen, and stored at minus 80C until processing. Total RNA was isolated with RNeasy mini-kit according to the manufacturer's instructions (Qiagen Inc., Valencia, CA). RNA quality and quantity were analyzed with a 2100 Bioanalyzer (Agilent Technologies Inc., Palo Alto, CA).

An independent validation cohort of biopsy mRNA expression data from 24 UC patients treated with IFX was obtained from University Hospital Gasthuisberg, Leuven Belgium. Biopsies were obtained within one week prior to the intravenous infusion of 5 mg/kg IFX. The biopsies were frozen at -80°C prior to processing for mRNA expression. The response to IFX was determined at 4-6 weeks post-infusion, with response defined as above. The Leuven cohort specimens were processed for mRNA isolation and hybridization using the same methods as were used for the ACT1 specimens.

Microarray hybridization was performed on GeneChip Human Genome U133 Plus 2.0 arrays according to the manufacturer's protocol (Affymetrix, Santa Clara, CA). The chip allows expression analysis of more than 47,000 transcripts and variants, including 38,500 well-characterized human genes. The chips were scanned with a GeneChip Scanner 3000, and fluorescence intensity for each feature of the array was obtained with GeneChip Operating Software version 1.4 (Affymetrix, Santa Clara, CA). The data were deposited at Gene Expression Omnibus under the series accession number GSE12251 (http:// www. ncbi. nlm. nih. Gov /geo/query/acc.cgi?token=rdatvkmqcaiqizk&acc=GSE12251. The data from the Validation cohort were also deposited at Gene Expression Omnibus under the series accession number GSE10892 (http:// www. ncbi. nlm. nih. gov/geo/query/acc.Ggi?loken=dlyphyokcomkmto&acc=GSE 10892).

Data quality was assessed by hybridization intensity distribution and Pearson's correlation with Partek Genomic Suite 6.3 (Partek Inc., St. Charles, MO). Pearson's correlation coefficients ranged from 0.80 to 1.0. The intensity of probe sets were normalized across all samples using GeneSpringGX 7.3 (Agilent Technologies, Palo Alto, CA).

Significant differences between infliximab non-responder and responder samples were identified using analysis of variance (ANOVA) on log-2 transformed normalized intensities. A 5% false discovery rate (FDR) was applied for multiple testing corrections. Transcripts with more than 2-fold differential expression were selected for the comparison to be analyzed. To exclude probe sets that passed ANOVA and fold change filtering but were undetected in both conditions of a paired comparison, only those samples designated as "present" (detected) or "marginal" (limited detection) among samples representing the condition were selected.

Classification - of infliximab responsiveness for each patient sample was generated with the 'K-Nearest Neighbours" algorithm using GeneSpring GX 7.3. A classifier containing transcripts showing significant differential expression between non-responder (n=11 samples) and responder (n=12 samples) before infliximab treatment was evaluated by leave-one-out cross-validation. A p-value was calculated to measure the probability of a test sample being classified by chance. Fisher's Exact Test was used to select the top predictive transcripts.

Hierarchical clustering analysis was applied to data obtained from the microarray data analysis. Clustering was run using Pearson correlation between the expression profiles of two genes or patients to calculate the similarity matrix in GeneSpringGX 7.3. Results were visualized as a 2-dimensional heat map with two dendrograms, one indicating the similarity between patients and the other indicating the similarity between genes.

Gene-annotation enrichment analysis was conducted using National Institutes of Health DAVID online (http :// david. abcc.ncifcrf.gov/). Statistical significance was determined using Fisher's exact test. Functional categories with a p-value ≤ 0.05 were considered significant.

### Summary of Results

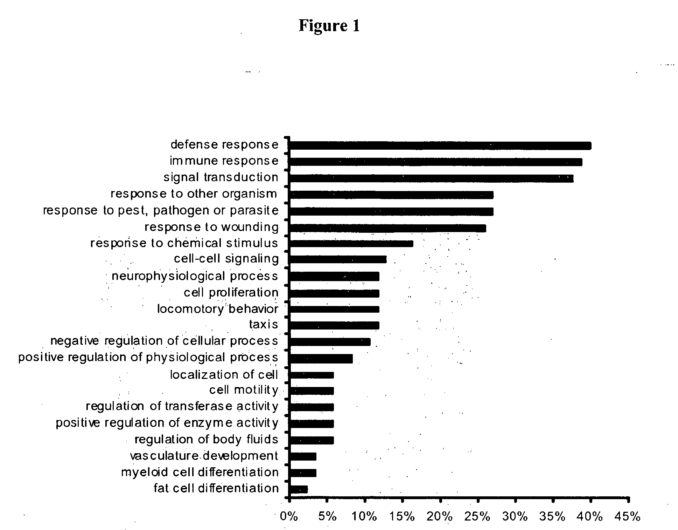

The expression profile of mucosal biopsies at Week 0 before infliximab treatment was established from 22 patients (11 biopsies from 10 non-responders and 12 biopsies from 12 responders; two of the non-responder biopsies were obtained within two weeks from the same subject) based upon the response to infliximab at Week 8. A total of 109 probe sets, 102 downregulated and 7 upregulated, passed an FDR of 5% and a two-fold differential cut-off representing 86 genes (Figure 10). When classified into biological processes, there was a predominance of innate immune processes. The five most predominant innate immune processes were defense response, immune response, signal transduction, response to other organisms, and response to pests, pathogens or parasites (Figure 1). Ten probe sets were cytokines/chemokines or cytokine/chemokine receptors. Included in the probe set was CXCL8/interleukin (IL)-8, a chemotactic chemokine for neutrophil polymorphonuclear leucocytes (PMN). The receptors for CXCL8/IL-8, CXCR1/IL-8RA and CXCR2/IL-8RB were also present. Also present was CXCL11/I-TAC, a chemotactic factor that activates T cells and natural killer cells. Finally, IL-1β, IL-1RN, and IL-11, were all downregulated more than four-fold when comparing responders to non-responders (Figure 10).

### Class prediction analysis of Responders and Non-Responders

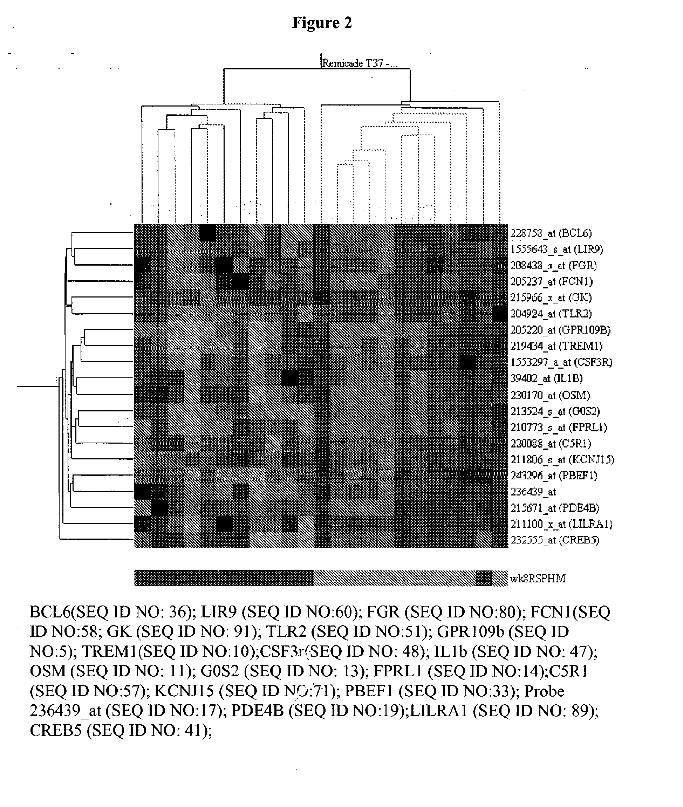

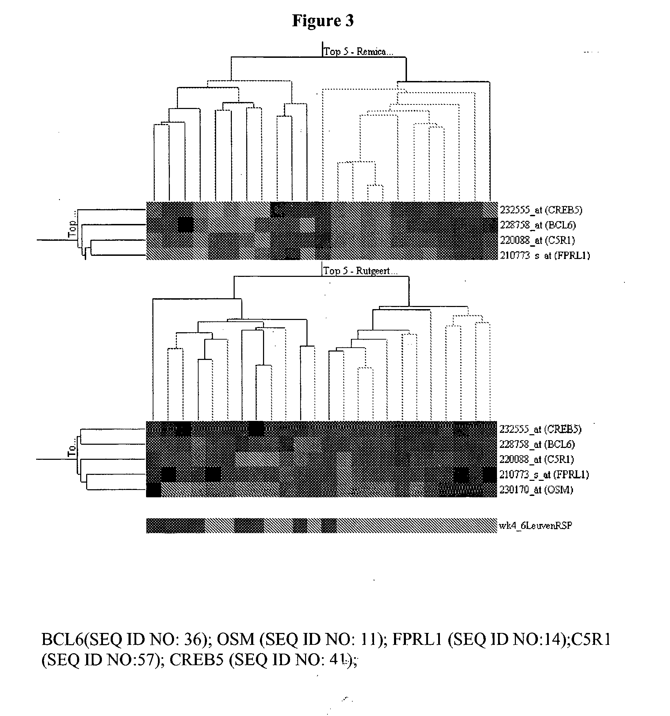

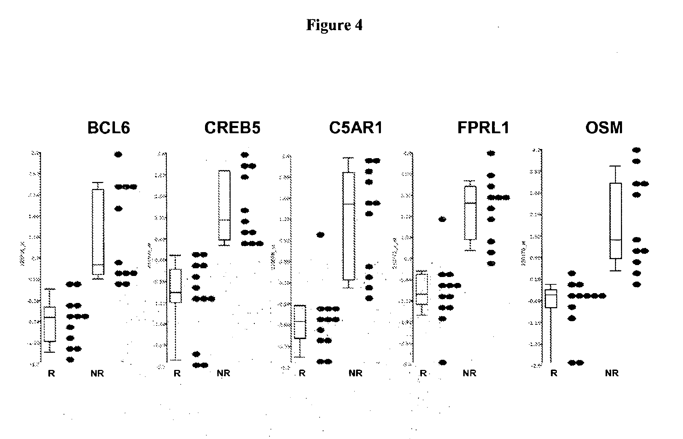

Fisher's Exact Test was used to select the top predictive probe sets distinguishing responders from non-responders within the 109 probe sets shown to be differentially expressed at Week 0 (Figure 10). A subset of 20 probe sets classified responders and non-responders at Week 0 (Figure 7) with an overall accuracy of 95.4% (21/22), sensitivity of 91.7% (11/12 responders) and specificity of 100% (10/10 non-responders) (Figure 7). Genes involved in immune responses (*e.g.* IL-1β, TLR2, TREM1 or LILRA2), signal transduction (*e.g.* PDE4B, NAMPT or FCN1) or G-protein-coupled receptor protein signaling pathways (*e.g.* GPR109B, C5AR1 or FPRL1) were represented. Eight of these 20 genes were expressed by PMNs, which are present in large numbers in the colonic mucosa from patients with active UC. Hierarchical clustering of the 20 probe set classifier showed a clear separation among responders and non-responders (Figure 3). The minimal number of transcripts allowing for an equivalent classification was subsequently determined. A classifier containing as few as 5 probe sets selected from the above 20 was able to reach an overall accuracy of 90.9% (20/22), sensitivity of 91.7% (11/12 responders) and specificity of 90.0% (9/10 non-responders) (Figures 3 and 7). The 5 genes obtained were BCL6, CSAR1, FPRL1, OSM and tx82a04.x1 a gene similar to CREB5. Both BCL6 and tx82a04.x1 had slightly higher predictive strength while the remaining 3 genes had equal predictive strength. Of note, any of the remaining 15 genes could replace C5AR1, FPRL1, and OSM without any loss to the predictive quality of the 5 probe set classifier (data not shown). Hierarchical clustering of the 5 probe set classifier across the 10 non-responders and 12 responders showed remarkable separation (Figure 3) with a very contrasted expression profile across all 5 probe sets when comparing responders to non-responders. The single misclassified responder had an expression profile very similar to non-responders with the exception of tx82a04.x1, which is similar to CREB5 (Figures 2 and 3). Finally, Figure 4 shows a dot plot representation of the 5-gene classifier using the normalized raw intensities where a marked difference is seen for each of the five genes at baseline comparing responders to non-responders.

### Class prediction validation

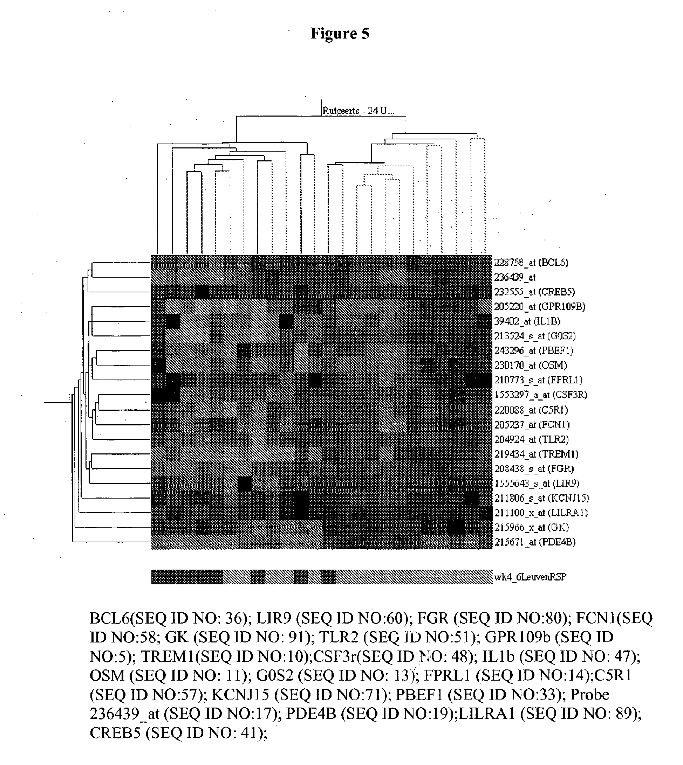

We next validated both the 20 and the 5 probe set classifiers using the Leuven cohort as an independent validation test set composed of 8 responders and 16 non-responders Overall accuracies of 75% (18/24), sensitivities of 87.5% (7/8 responders) and specificities of 68.8% (11/16 non-responders) were obtained for both classifiers. Hierarchical clustering of the 20 probe set classifier among the 8 responders and 16 non-responders showed that the 4 misclassified non-responders and the 1 misclassified responder have expression profiles very similar to responders and non-responders respectively (Figure 5).

Two stringent criteria defining infliximab response (endoscopic and histologic healing) yielded 109 differentially expressed probe sets at Week 0 representing 86 genes. These probe sets all passed a 5% FDR and a two-fold differential expression cutoff. Two predictive response signatures, one a 20 probe set and one of 5 sub-set of the 20, were established and verified using an independent cohort. Four non-responders were misclassified but had expression patterns resembling that of responders for all 109 genes differentially expressed at Week 0. This suggests that these patients are either slow to manifest a clinical benefit from infliximab treatment or have factors influencing their clinical response not evident through mucosal expression profiling at Week 0. One responder from the ACT1 cohort was misclassified by the 20 and 5 probe set classifiers, while one responder in the independent validation cohort was misclassified (Figures 2, 3, and 5).

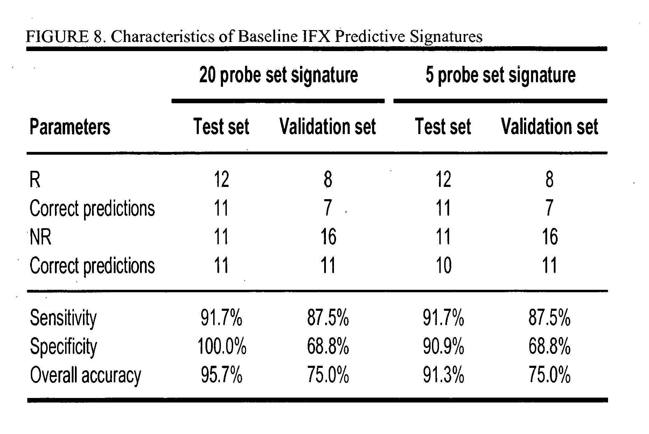

A number of the genes expressed prior to infliximab treatment are involved in regulating the function of PMNs. PMNs represent a central component of the innate immune response acting as primary responders to microbial challenge. PMNs rapidly migrate into inflamed tissues employing phagocytosis, production of reactive oxygen species, and the release of inflammatory mediators and antimicrobial substances as potent effector mechanisms. PMN influx is common in UC as shown by histological examination of colonic mucosal biopsies obtained from patients with active UC, and is paralleled by extensive mucosal and/or transmural injury including edema, loss of goblet cells, decreased mucous production, crypt cell hyperplasia, erosions, and ulcerations. Therefore, PMNs represent an important cell-type driving and influencing UC pathogenesis. Normalization of PMN influx and activity is an integral part of the response to infliximab treatment. Thirteen genes differentially expressed at Week 0 code for proteins either in the membrane of secretory vesicles or the plasma membrane of PMNs (Figure 8). Seven (CSF3R, FCN1, FGR, FPRL1, OSM, NAMPT, TLR2) are included in the predictive 20 probe set response gene panel and two (FPRL and OSM) are included in the predictive 5 probe set response gene panel (Figure 7). In addition, all are downregulated among infliximab responders when comparing to non-responders at Week 0 suggesting a differential PMN activation state.

Complement 5a (C5a) binding to complement component 5a receptor (C5aR1) plays an essential role in PMN innate immunity. C5a-C5aR1 interactions preserve PMN innate immune functions (chemotaxis, phagocytosis, respiratory burst), attenuate inflammatory reaction, ameliorate coagulopathy, alter adhesion molecule expression, and modulate apoptosis. However, excessive interaction of C5a-C5aR1 has been described to result in the paralysis of PMN functions, which in turn may lead to compromised host defenses. Because C5aRl was downregulated when comparing infliximab responders to non-responders at Week 0 (Figure 8), it could be hypothesized that PMN functions may be partially or totally paralyzed in infliximab non-responders resulting in compromised host defense.

Toll-like receptor (TLR) 2 and TLR4 are two receptors involved in host defense, which recognize specific pathogen-associated molecular patterns. Both were downregulated at Week 0 when comparing infliximab responders to non-responders (Figure 7 and Figure 10). TLR2 and TLR4 are expressed on leukocytes including PMNs and are over-expressed in UC when compared to normal controls. Dendritic cells (DCs) recognize and respond to microbial structures through TLRs. In the intestine, DCs are pivotal in tolerance induction and direct the differentiation of T cells. In healthy individuals, few intestinal DCs express TLR2 or TLR4; however, both are significantly enhanced in UC. Due to the importance of TLR signals in the maintenance of intestinal epithelial cell (IEC) homoeostasis and as commensal bacteria have a significant role in the maintenance of IEC homoeostasis, deregulated TLRs signaling could be a contributing factor in UC.

Triggering receptor expressed on myeloid cells (TREM-1) is a receptor expressed on the surface of PMNs and monocytes in the presence of microbial components. TREM-1 was downregulated when comparing responders to non-responders at Week 0 (Figure 7). CXCL8/IL-8, CXCRI/IL-8RA and CXCR2/IL-8RB (Figure 7 and Figure 10) were also downregulated, a finding highlighted by the increased expression of these molecules in the intestinal mucosal specimens of UC patients. Also, increased levels of CXCL8/IL-8 in the rectal mucosa is significantly associated with UC relapse. Finally, CXCL8/IL-8 can initiate PMN transepithelial migration. These genes also point towards a difference in PMN activity in infliximab non-responders.

BCL6 and the gene similar to CREB5 were the two genes expressed at Week 0 in the 20 or 5 predictive panel with the highest predictive value. BCL6 is a proto-oncogene, which encodes a nuclear transcriptional repressor, with pivotal roles in germinal center formation and regulation of lymphocyte function, differentiation, and survival. BCL6 mice display a phenotype characterized by a defect in germinal center formation and a massive multiorgan inflammatory response, especially the heart and lung, corresponding to a typical Th2 hyper-immune response. Also, BCL6 regulates the expression of multiple Th2 cytokines by controlling expression of the GATA-3 transcription factor, suggesting that a more active Th2 response is contributing to the status of infliximab non-responders. IL-13Ralpha2 was the most significant gene probe distinguishing between responders and non-responders in UC and IBD overall in the validation cohort. IL-13 is a regulator of the Th2 response and IL-13Ralpha2 receptor has recently been hypothesized to be involved in fibrogenesis.

The altered expression of IL-11 may be involved in UC pathogenesis. Interleukin-11 has anti-inflammatory properties, and the downregulation of this gene in UC patients who respond to infliximab may have positive effects on the disease course. While interleukin-11 and interleukin-1RN gene polymorphisms have been associated with UC, IL-11 has been associated with increased intestinal permeability mediated by increased MLCK protein expression and activity.

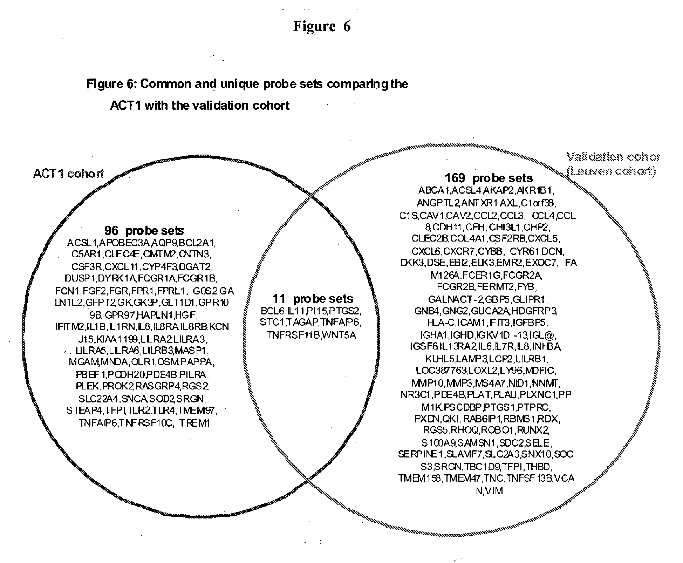

When the results from both cohorts are compared a very significant overlap between the two gene signatures exists highlighting innate immunity and Th2 skewing as essential for the resolution of UC (Figure 6). These results also demonstrate that multiple gene expression signatures in common pathways can be used to predict response to IFX.

We have used mRNA expression analysis to develop a predictive response signature to infliximab treatment in UC. This signature provides insight into the action of infliximab at a molecular level. The expression of genes that affect PMN and Th2 cell functions were the most influential in predicting the response to infliximab as was the case in the results described for the validation cohort. We propose that in UC patients, the mucosal gene of these genes largely accounts for the lack of response to infliximab treatment observed in some patients following 8 weeks of treatment.

## Claims

1. A method for predicting the suitability of treatment with anti-TNF α therapy for a TNF α mediated-related disorder in a subject, comprising:
a) preparing a sample of nucleic acids from a specimen obtained from the subject; and
b) assaying said sample with a panel of labeled nucleic acid segments that hybridize to BCL6 (Genbank Acc. No. AW264036; SEQ ID NO:118) and tx82a04.x1 (Genbank Acc. No. AI689210; SEQ ID NO:123), wherein down regulation of said genes correlates with therapeutic efficacy for the treatment of an inflammatory gastrointestinal disorder.

2. A method according to claim 1, wherein said assaying further comprises assaying a nucleic acid hybridization to a member selected from the group consisting of C5AR1 ((Genbank Acc. No.NM001736; SEQ ID NO: 119), FOLR1 ((Genbank Acc. No. U81501; SEQ ID NO: 142) and OSM (Genbank Acc. No. A1079327; SEQ ID NO: 173).

3. A method according to claim 1, wherein said assaying further comprises assaying nucleic acid hybridization to a gene selected from SEQ ID NO:s 110 through 203.

4. A method according to claim 1, wherein said anti-TNF α therapy is selected from the group consisting of infliximab, golimumab, Enbrel™, and Humira™.

5. A method according to claim 1, wherein said inflammatory gastrointestinal disorder is selected from the group consisting of Crohn's disease, inflammatory bowel disease and ulcerative colitis.

6. A method according to claim 1, wherein said inflammatory gastrointestinal disorder is selected from the group consisting of mild, moderate, severe, pediatric or adult forms.

7. The method of claim 4, wherein the anti-TNF α antibody is golimumab or infliximab and the TNF α mediated-related disorder is ulcerative colitis.

8. The method of claim 1, wherein the expression profile of the genes is measured using one or more nucleic acid probes selected from the group consisting of SEQ ID NOs: 1 through 109.

9. A method according to claim 1 which is an array-based testing method for predicting the suitability of treatment with an anti-TNF α therapy for a TNF α mediated-related disorder in a patient, comprising:
a) preparing a mixture of nucleic acids from a specimen obtained from the patient;
b) assaying said sample with a panel of labeled nucleic acid probes that hybridize to BCL6 (Genbank Acc. No. AW264036; SEQ ID NO: 118) and tx82a04.x1 (Genbank Acc. No. AI689210; SEQ ID NO:123), wherein said probes comprise SEQ ID NO: 37, and SEQ ID NO: 41, wherein down regulation of said genes correlates with therapeutic efficacy for the treatment of an inflammatory gastrointestinal disorder.

10. A method according to claim 9, wherein said assaying further comprises assaying C5AR1 ((Genbank Acc. No.NM001736; SEQ ID NO: 119), FOLR1 ((Genbank Acc. No. U81501; SEQ ID NO: 142) or OSM (Genbank Acc. No. A1079327; SEQ ID NO: 173).

11. A method according to claim 9, wherein said assaying further comprises assaying one of the genes of SEQ ID NOs: 110- 203

12. A method according to claim 9, wherein said anti-TNF α therapy is selected from the group consisting of infliximab, golimumab, Enbrel™, and Humira ™, or another anti-TNF biologic or small molecule drug.

13. Use of a kit comprising oligonucleotides or oligonucleotides complementary to a polynucleotide encoding a marker gene or the complementary strand thereof and cells expressing the marker genes, wherein the marker genes are selected from the group consisting of the nucleotide sequences encoding a portion of BCL6 (Genbank Acc. No. AW264036; ; SEQ ID NO: 118) and tx82a04.x1 (Genbank Acc. No. AI689210; SEQ ID NO: 123), in the method of any one of claims 1-12.

14. The use according to claim 13, wherein said marker genes further comprise C5AR1 ((Genbank Acc. No.NM001736; SEQ ID NO: 119), FOLR1 ((Genbank Acc. No. U81501; SEQ ID NO: 142) or OSM (Genbank Acc. No. A1079327; SEQ ID NO: 173).

15. The use according to claim 14 wherein said marker genes further comprise at a gene listed in SEQ ID NOs: 110-203 and said oligonucleotides comprise a probe of any one of SEQ ID NOs 1-109.

## Patentansprüche

1. Verfahren zum Prognostizieren der Eignung einer Behandlung mit anti-TNFα-Therapie für ein TNFα-vermitteltes/assoziiertes Leiden in einem Individuum, wobei das Verfahren Folgendes umfasst:
a) Herstellen einer Nukleinsäureprobe aus einem von dem Individuum stammenden Untersuchungsmaterial; und
b) Testen der Probe mit einem Panel von markierten Nukleinsäuresegmenten, die mit BCL6 (Genbank Zugangs-Nr. AW264036; SEQ ID NO: 118) und tx82a04.x1 (Genbank Zugangs-Nr. AI689210; SEQ ID NO: 123) hybridisieren, wobei ein Hinunterregulieren der Gene mit einer therapeutischen Wirksamkeit für die Behandlung eines entzündlichen Magen-Darm-Leidens korreliert.

2. Verfahren nach Anspruch 1, wobei das Testen weiterhin das Testen einer Nukleinsäurehybridisierung mit einem Mitglied aus der Gruppe bestehend aus C5AR1 ((Genbank Zugangs-Nr. NM001736; SEQ ID NO: 119), FOLR1 ((Genbank Zugangs-Nr. U81501; SEQ ID NO: 142) und OSM (Genbank Zugangs-Nr. A1079327; SEQ ID NO: 173) beinhaltet.

3. Verfahren nach Anspruch 1, wobei das Testen weiterhin das Testen einer Nukleinsäurehybridisierung mit einem Gen aus der Reihe SEQ ID NO: 110 bis 203 umfasst.

4. Verfahren nach Anspruch 1, wobei die anti-TNFα-Therapie aus der Reihe Infliximab, Golimumab, Enbrel™ und Humira™ ausgewählt ist.

5. Verfahren nach Anspruch 1, wobei das entzündliche Magen-Darm-Leiden aus der Gruppe Morbus Crohn, entzündliche Darmerkrankung und Colitis ulcerosa ausgewählt ist.

6. Verfahren nach Anspruch 1, wobei das entzündliche Magen-Darm-Leiden aus der Gruppe bestehend aus milden, mäßigen, schweren, pädiatrischen oder adulten Formen ausgewählt ist.

7. Verfahren nach Anspruch 4, wobei es sich bei dem anti-TNFα-Antikörper um Golimumab oder Infliximab und bei dem TNFα-vermittelten/assoziierten Leiden um Colitis ulcerosa handelt.

8. Verfahren nach Anspruch 1, wobei das Expressionsprofil der Gene mit einer oder mehreren Nukleinsäuresonden ausgewählt aus der Gruppe bestehend aus SEQ ID NO: 1 bis 109 gemessen wird.

9. Verfahren nach Anspruch 1, bei dem es sich um ein Array-basiertes Testverfahren zum Prognostizieren der Eignung einer Behandlung mit einer anti-TNFα-Therapie für ein TNFα-vermitteltes/assoziiertes Leiden in einem Patienten handelt, wobei das Verfahren Folgendes umfasst:
a) Herstellen einer Mischung aus Nukleinsäuren aus einem von dem Individuum stammenden Untersuchungsmaterial; und
b) Testen der Probe mit einem Panel von markierten Nukleinsäuresonden, die mit BCL6 (Genbank Zugangs-Nr. AW264036; SEQ ID NO: 118) und tx82a04.x1 (Genbank Zugangs-Nr. AI689210; SEQ ID NO: 123) hybridisieren, wobei die Sonden SEQ ID NO: 37 und SEQ ID NO: 41 umfassen, wobei ein Hinunterregulieren der Gene mit einer therapeutischen Wirksamkeit für die Behandlung eines entzündlichen Magen-Darm-Leidens korreliert.

10. Verfahren nach Anspruch 9, wobei das Testen weiterhin das Testen von C5AR1 ((Genbank Zugangs-Nr. NM001736; SEQ ID NO: 119), FOLR1 ((Genbank Zugangs-Nr. U81501; SEQ ID NO: 142) oder OSM (Genbank Zugangs-Nr. A1079327; SEQ ID NO: 173) umfasst.

11. Verfahren nach Anspruch 9, wobei das Testen weiterhin das Testen von einem der Gene von SEQ ID NO: 110-203 umfasst.

12. Verfahren nach Anspruch 9, wobei die anti-TNFα-Therapie aus der Reihe Infliximab, Golimumab, Enbrel^{™} und Humira^{™} oder einem sonstigen anti-TNF-Biologikum oder kleinmolekularem anti-TNF-Arzneistoff ausgewählt ist.

13. Verwendung eines Kits, umfassend Oligonukleotide oder Oligonukleotide mit Komplementarität zu einem Polynukleotid, das für ein Markergen oder den Komplementärstrang davon codiert, und Zellen, die die Markergene exprimieren, wobei die Markergene aus der Gruppe bestehend aus den Nukleotidsequenzen, die für einen Abschnitt von BCL6 (Genbank Zugangs-Nr. AW264036; SEQ ID NO: 118) und tx82a04.x1 (Genbank Zugangs-Nr. AI689210; SEQ ID NO: 123) codieren, ausgewählt sind, in dem Verfahren nach einem der Ansprüche 1-12.

14. Verwendung nach Anspruch 13, wobei die Markergene weiterhin C5AR1 ((Genbank Zugangs-Nr. NM001736; SEQ ID NO: 119), FOLR1 ((Genbank Zugangs-Nr. U81501; SEQ ID NO: 142) oder OSM (Genbank Zugangs-Nr. A1079327; SEQ ID NO: 173) umfassen.

15. Verwendung nach Anspruch 14, wobei die Markergene weiterhin bei einem Gen, das in SEQ ID NO: 110-203 angeführt ist, umfassen und die Oligonukleotide eine Sonde gemäß einer der SEQ ID NO: 1-109 umfassen.

## Revendications

1. Procédé pour prédire la conformité d'un traitement avec une thérapie anti-TNFα pour un trouble médié par/associé à TNFα chez un sujet, comprenant :
a) la préparation d'un échantillon d'acides nucléiques à partir d'un spécimen obtenu à partir du sujet ; et
b) le dosage dudit échantillon avec un panel de segments d'acide nucléique marqués qui s'hybrident avec BCL6 (Acc. Genbank n° AW264036 ; SEQ ID NO: 118) et tx82a04.x1 (Acc. Genbank n° AI689210 ; SEQ ID NO: 123), la régulation à la baisse desdits gènes étant corrélée à l'efficacité thérapeutique pour le traitement d'un trouble gastro-intestinal inflammatoire.

2. Procédé selon la revendication 1, ledit dosage comprenant en outre le dosage d'une hybridation d'acide nucléique avec un composant choisi dans le groupe constitué de C5AR1 ((Acc. Genbank n° NMOO1736 ; SEQ ID NO: 119), FOLR1 (Acc. Genbank n° U81501 ; SEQ ID NO: 142) et OSM (Acc. Genbank n° A1079327 ; SEQ ID NO: 173).

3. Procédé selon la revendication 1, ledit dosage comprenant en outre le dosage d'hybridation d'acide nucléique avec un gène choisi parmi SEQ ID NO: 110 à 203.

4. Procédé selon la revendication 1, ladite thérapie anti-TNFα étant choisie dans le groupe constitué d'infliximab, golimumab, Enbrel™, et Humira^{™}.

5. Procédé selon la revendication 1, ledit trouble gastro-intestinal inflammatoire étant choisi dans le groupe constitué de la maladie de Crohn, une affection abdominale inflammatoire et la rectocolite hémorragique.

6. Procédé selon la revendication 1, ledit trouble gastro-intestinal inflammatoire étant choisi dans le groupe constitué de formes légères, modérées, graves, pédiatriques ou adultes.

7. Procédé de la revendication 4, l'anticorps anti-TNFα étant le golimumab ou l'infliximab et le trouble médié par/associé à TNFα étant la rectocolite hémorragique.

8. Procédé de la revendication 1, le profil d'expression des gènes étant mesuré en utilisant une ou plusieurs sondes d'acide nucléique choisies dans le groupe constitué de SEQ ID NO: 1 à 109.

9. Procédé selon la revendication 1 qui est un procédé de test à base de puce pour prédire la conformité d'un traitement avec une thérapie anti-TNFα pour un trouble médié par/associé à TNFα chez un patient, comprenant :
a) la préparation d'un mélange d'acides nucléiques à partir d'un spécimen obtenu à partir du patient ;
b) le dosage dudit échantillon avec un panel de sondes d'acide nucléique marquées qui s'hybrident avec BCL6 (Acc. Genbank n° AW264036 ; SEQ ID NO: 118) et tx82a04.x1 (Acc. Genbank n° AI689210 ; SEQ ID NO: 123), lesdites sondes comprenant SEQ ID NO: 37, et SEQ ID NO: 41, la régulation à la baisse desdits gènes étant corrélée à l'efficacité thérapeutique pour le traitement d'un trouble gastro-intestinal inflammatoire.

10. Procédé selon la revendication 9, ledit dosage comprenant en outre le dosage de C5AR1 (Acc. Genbank n° NM001736 ; SEQ ID NO: 119), FOLR1 (Acc. Genbank n° U81501 ; SEQ ID NO: 142) ou OSM (Acc. Genbank n° A1079327 ; SEQ ID NO: 173).

11. Procédé selon la revendication 9, ledit dosage comprenant en outre le dosage d'un des gènes de SEQ ID NO: 110 à 203.

12. Procédé selon la revendication 9, ladite thérapie anti-TNFα étant choisie dans le groupe constitué d'infliximab, golimumab, Enbrel™, et Humira^{™}, ou un autre agent biologique ou médicament à petite molécule anti-TNFα.

13. Utilisation d'un kit comprenant des oligonucléotides ou des oligonucléotides complémentaires d'un polynucléotide codant pour un gène marqueur du brin complémentaire de celui-ci et de cellules exprimant les gènes marqueurs, les gènes marqueurs étant choisis dans le groupe constitué des séquences nucléotidiques codant pour une partie de BCL6 (Acc. Genbank n° AW264036 ; SEQ ID NO: 118) et tx82a04.x1 (Acc. Genbank n° AI689210 ; SEQ ID NO: 123), dans le procédé de l'une quelconque des revendications 1 à 12.

14. Utilisation selon la revendication 13 ; lesdits gènes marqueurs comprenant en outre C5AR1 ((Acc. Genbank n° NM001736 ; SEQ ID NO: 119), FOLR1 (Acc. Genbank n° U81501 ; SEQ ID NO: 142) ou OSM (Acc. Genbank n° A1079327 ; SEQ ID NO: 173).

15. Utilisation selon la revendication 14, lesdits gènes marqueurs comprenant en outre à un gène répertorié dans SEQ ID NO: 110 à 203 et lesdits oligonucléotides comprennent une sonde de l'un quelconque de SEQ ID NO: 1 à 109.
